(19) Europäisches Patentamt
European Patent Office
Office européen des brevets

(11) EP 2 361 985 A1

(12) EUROPEAN PATENT APPLICATION

(43) Date of publication:
**31.08.2011 Bulletin 2011/35**

(51) Int Cl.:
***C12N 15/82*** *(2006.01)* ***A01H 5/00*** *(2006.01)*

(21) Application number: **10154801.4**

(22) Date of filing: **26.02.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
- **BASF Plant Science Company GmbH**
  **67056 Ludwigshafen (DE)**
- **VIB VZW**
  **9052 Gent (BE)**
- **Universiteit Gent**
  **9000 Gent (BE)**

(72) Inventor: **Russinova, Jenny**
**9800 Astene (BE)**

(74) Representative: **Krieger, Stephan Gerhard et al**
**BASF SE**
**GVX/B - C 6**
**Carl-Bosch-Strasse 38**
**67056 Ludwigshafen (DE)**

# (54) Plants having enhanced yield-related traits and a method for making the same

(57) The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding an POI (Protein Of Interest) polypeptide. The present invention also concerns plants having modulated expression of a nucleic acid encoding an POI polypeptide, which plants have enhanced yield-related traits relative to control plants. The invention also provides hitherto unknown POI-encoding nucleic acids, and constructs comprising the same, useful in performing the methods of the invention.



Printed by Jouve, 75001 PARIS (FR)

## Description

The present invention relates generally to the field of molecular biology and concerns a method for enhancing yield-related traits in plants by modulating expression in a plant of a nucleic acid encoding a Hydroxyproline-rich glycoprotein family protein (HRGP) The present invention also concerns plants having modulated expression of a nucleic acid encoding a Hydroxyproline-rich glycoprotein family protein (HRGP), which plants have enhanced yield-related traits relative to corresponding wild type plants or other control plants. The invention also provides constructs useful in the methods of the invention.

A trait of particular economic interest relates to an increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, and leaf senescence. Root development, nutrient uptake, stress tolerance and early vigour may also be important factors in determining yield. Optimizing the abovementioned factors may therefore contribute to increasing crop yield.

Under field conditions, plant performance, for example in terms of growth, development, biomass accumulation and seed generation, depends on a plant's tolerance and acclimation ability to numerous environmental conditions, changes and stresses.

Agricultural biotechnologists use measurements of several parameters that indicate the potential impact of a transgene on crop yield. For forage crops like alfalfa, silage corn, and hay, the plant biomass correlates with the total yield. For grain crops, however, other parameters have been used to estimate yield, such as plant size, as measured by total plant dry and fresh weight, above ground and below ground dry and fresh weight, leaf area, stem volume, plant height, leaf length, root length, tiller number, and leaf number. Plant size at an early developmental stage will typically correlate with plant size later in development. A larger plant with a greater leaf area can typically absorb more light and carbon dioxide than a smaller plant and therefore will likely gain a greater weight during the same period. There is a strong genetic component to plant size and growth rate, and so for a range of diverse genotypes plant size under one environmental condition is likely to correlate with size under another. In this way a standard environment can be used to approximate the diverse and dynamic environments encountered by crops in the field. Plants that exhibit tolerance of one abiotic stress often exhibit tolerance of another environmental stress. This phenomenon of cross-tolerance is not understood at a mechanistic level. Nonetheless, it is reasonable to expect that plants exhibiting enhanced tolerance to low temperature, e.g. chilling temperatures and/or freezing temperatures, due to the expression of a transgene may also exhibit tolerance to drought and/or salt and/or other abiotic stresses. Some genes that are involved in stress responses, water use, and/or biomass in plants have been characterized, but to date, success at developing transgenic crop plants with improved yield has been limited.

Consequently, there is a need to identify genes which confer, when overexpressed or downregulated, increased tolerance to various stresses and/or improved yield under optimal and/or suboptimal growth conditions.

It has now been found that the yield can be increased and various yield-related traits may be improved in plants by modulating the expression in the plant of a nucleic acid encoding a POI (Protein Of Interest) polypeptide.

### *Summary*

Surprisingly, it has now been found that modulating expression of a nucleic acid encoding the Hydroxyproline-rich glycoprotein family protein (HRGP) gives plants having enhanced yield and yield-related traits, in particular seed and/or root yield as measured by the total weight and number of seeds, and improved yield-related traits, in particular seed filling rate, number of seeds filled, shoot and/or root biomass relative to control plants.

According to one embodiment, there is provided a method for improving yield-related traits in plants relative to control plants, comprising modulating expression in a plant of a nucleic acid encoding the Hydroxyproline-rich glycoprotein family protein (HRGP).

In accordance with the invention, therefore, the genes identified here may be employed to enhance yield-related traits. Increased yield may be determined in field trials of transgenic plants and their suitable control plants. Alternatively, a transgene's ability to increase yield may be determined in a model plant under optimal, controlled, growth conditions. An increased yield trait may be determined by measuring any one or any combination of the following phenotypes, in comparison to control plants: yield of dry harvestable parts of the plant, yield of dry above ground harvestable parts of the plant, yield of below ground dry harvestable parts of the plant, yield of fresh weight harvestable parts of the plant, yield of above ground fresh weight harvestable parts of the plant yield of below ground fresh weight harvestable parts of the plant, yield of the plant's fruit (both fresh and dried), yield of seeds (both fresh and dry), grain dry weight, and the like. Increased intrinsic yield capacity of a plant can be demonstrated by an improvement of its seed yield (e.g. increased seed/ grain size, increased ear number, increased seed number per ear, improvement of seed filling, improvement of seed composition, and the like); a modification of its inherent growth and development (e.g. plant height, plant growth rate, pod number, number of internodes, flowering time, pod shattering, efficiency of nodulation and nitrogen fixation, efficiency of carbon assimilation, improvement of seedling vigour/early vigour, enhanced efficiency of germination, improvement in plant architecture, cell cycle modifications and/or the like). Yield-related traits may also be improved to increase tolerance of the plants to abiotic environmental stress. Abiotic stresses include drought, low temperature, salinity, osmotic stress, shade, high plant density, mechanical stresses, and oxidative stress. Additional phenotypes that can be monitored to determine enhanced tolerance to abiotic environmental stress include, but is not limited to, wilting; leaf browning; turgor pressure,; drooping and/or shedding of leaves or needles; premature senescence of leaves or needles; loss of chlorophyll in leaves or needles and/or yellowing of the leaves. Any of the yield-related phenotypes described above may be monitored in crop plants in field trials or in model plants under controlled growth conditions to demonstrate that a transgenic plant has increased tolerance to abiotic environmental stress(es).

***Definitions***

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |

(continued)

| Residue | Conservative Substitutions | Residue | Conservative Substitutions |
|---|---|---|---|
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Table A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA$^d$ hybrids:

For <20 nucleotides: $T_m = 2 (l_n)$
For 20—35 nucleotides: $T_m = 22 + 1.46 (l_n)$
$^a$ or for other monovalent cation, but only accurate in the 0.01—0.4 M range.
$^b$ only accurate for %GC in the 30% to 75% range.
$^c$ L = length of duplex in base pairs.
$^d$ oligo, oligonucleotide; $l_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |

(continued)

| Gene Source | Reference |
|---|---|
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

Table 2b: Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |

(continued)

| Gene Source | Reference |
|---|---|
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1 Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |

(continued)

| Gene source | Reference |
|---|---|
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophos-phorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136 rice alanine aminotransferase | unpublished |
| PRO0147 trypsin inhibitor ITR1 (barley) | unpublished |
| PRO0151, rice WS118 | WO 2004/070039 |
| PRO0175, rice RAB21 | WO 2004/070039 |
| PRO005 | WO 2004/070039 |
| PRO009 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |

(continued)

| Gene source | Reference |
|---|---|
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO017 | WO 2004/070039 |
| PROO05 | WO 2004/070039 |
| PROO095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |

(continued)

| Gene | Expression | Reference |
|---|---|---|
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| **Gene source** | **Expression pattern** | **Reference** |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette — for example the naturally occurring combination of the natural promoter of the nucleic acid sequences with the corresponding nucleic acid sequence encoding a polypeptide useful in the methods of the present invention, as defined above — becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5’ and 3’ sequences that flank the coding region that are transcribed but not translated into amino acids (also referred to as 5’ and 3’ untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5’ and 3’ UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517—527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

## Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al.

(Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Traits

Yield related traits comprise one or more of yield, biomass, seed yield, early vigour, greenness index, increased growth rate, improved agronomic traits (such as improved Water Use Efficiency (WUE), Nitrogen Use Efficiency (NUE), etc.).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Nutrient deficiency may result from a lack of nutrients such as nitrogen, phosphates and other phosphorous-containing compounds, potassium, calcium, magnesium, manganese, iron and boron, amongst others.

The term salt stress is not restricted to common salt (NaCl), but may be any one or more of: NaCl, KCl, LiCl, $MgCl_2$, $CaCl_2$, amongst others.

<u>Increase/Improve/Enhance</u>

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

<u>Roots</u>

The term root as used herein encompasses all 'below ground' or 'under ground' parts of the plant that serves as support, draws minerals and water from the surrounding soil, and/or store nutrient reserves. These include bulbs, corms, tubers, tuberous roots, rhizomes and fleshy roots. Increased roots yield may manifest itself as one or more of the following: an increase in root biomass (total weight) which may be on an individual basis and/or per plant and/or per square meter; increased harvest index, which is expressed as a ratio of the yield of harvestable parts, such as roots, divided by the total biomass.

An increase in root yield may also be manifested as an increase in root size and/or root volume. Furthermore, an increase in root yield may also manifest itself as an increase in root area and/or root length and/or root width and/or root perimeter. Increased yield may also result in modified architecture, or may occur because of modified architecture.

<u>Seed yield</u>

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts. The phenotype or traits of the control plants are assessed under conditions which allow a comparison with the plant produced according to the invention, e.g. the control plants and the plants produced according to the method of the present invention are grown under similar, preferably identical conditions.

*Detailed description of the invention*

It has now been found that modulating expression in a plant of a nucleic acid encoding a Hydroxyproline-rich glycoprotein family protein (HRGP) gives plants having increased yield and/or enhanced yield-related traits relative to control plants. According to a first embodiment, the present invention provides a method for enhancing yield and/or yield-related traits in plants relative to control plants, wherein said method comprises transforming a plant with a recombinant construct to increase the activity or expression in a plant of a Hydroxyproline-rich glycoprotein family protein (HRGP) and optionally selecting for plants having increased yield and/or enhanced yield-related traits.

A preferred method for modulating the expression and activity of a Hydroxyproline-rich glycoprotein family protein (HRGP) in a plant is by introducing and expressing nucleic acid molecule encoding this Hydroxyproline-rich glycoprotein family protein(HRGP).

Any reference hereinafter to a "protein useful in the methods of the invention" is taken to mean a Hydroxyproline-rich glycoprotein family protein (HRGP) as defined herein. Any reference hereinafter to a "nucleic acid useful in the methods of the invention" is taken to mean a nucleic acid capable of encoding such a Hydroxyproline-rich glycoprotein family protein (HRGP). The nucleic acid to be introduced into a plant (and therefore useful in performing the methods of the invention) is any nucleic acid encoding the type of protein which will now be described, hereafter also named *"POI* nucleic acid" or *"POI* gene".

Preferably, a "Hydroxyproline-rich glycoprotein family protein (HRGP)" of the invention (i.e. the POI polypeptide) as defined herein refers to any polypeptide comprising an amino acid sequence containing a BAR (Bin/amphiphysin/Rvs) domain.

BAR domains (PF03114) are dimerisation, lipid binding and curvature sensing modules found in many different protein families. A BAR domain with an additional N-terminal amphipathic helix (an N-BAR) can drive membrane curvature. These N-BAR domains are found in amphiphysin, endophilin, BRAP and Nadrin. BAR domains are also frequently found alongside domains that determine lipid specificity, like a PH domain (PF00169) and PX domain (PF00787) domains in beta centaurins and sorting nexins respectively (Peter et al, 2004). The crystal structure of these proteins suggest the domain forms a crescent-shaped dimer of a three-helix coiled coil with a characteristic set of conserved hydrophobic, aromatic and hydrophilic amino acids. Proteins containing this domain have been shown to homodimerise, heterodimerise or, in a few cases, interact with small GTPases (Peter BJ, Kent HM, Mills IG, Vallis Y, Butler PJ, Evans PR, McMahon HT; , Science. 2004;303:495-499).

Further, a "Hydroxyproline-rich glycoprotein family protein (HRGP)" of the invention (i.e. the POI polypeptide) as defined herein refers to any polypeptide comprising an amino acid sequence containing a BAR (Bin/amphiphysin/Rvs) domain or an amino acid sequence comprising any one of the polypeptide sequences shown in SEQ ID NO.: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 and a homolog thereof (as described herein) or to a polypeptide encoded by a polynucleotide comprising the nucleic acid molecule as shown in SEQ ID NO.: 1, 3, 5, 7, 9, 11, 13, 15, 17, 19, 21, 23, 25, 27, 29, 31, 33, 35, 37, 39, 41, 43, 45, 47, 49, 51, 53, 55, or 57. and a homolog thereof (as described herein) and/or comprises at least one of any one of motifs 1 to 3. Preferably, the Hydroxyproline-rich glycoprotein family protein (HRGP) comprises an amino acid sequence containing a BAR (Bin/amphiphysin/Rvs) domain and an amino acid sequence having 35% or more identity to any one of the polypeptide sequences shown in SEQ ID NO.: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 or to a polypeptide encode by a polynucleotide comprising the nucleic acid molecule as shown in SEQ ID NO.: 1, 3, 5, 7, 9, 11, 13, 15, 17, 19, 21, 23, 25, 27, 29, 31, 33, 35, 37, 39, 41, 43, 45, 47, 49, 51, 53, 55, or 57 and, even more preferred, also comprises at least one of any one of motifs 1 to 3.

In one embodiment, the Hydroxyproline-rich glycoprotein family protein (HRGP) is characterized as comprising one or more of the following MEME motifs:

Motif 1
[ST]SP[KR]ISELHELPRPP

Motif 2
[ED]MK[QR]QCDEKREVYE

Motif 3
SLK[QE]GQSRSLLTQAARHHAAQ

Motifs 1 to 3 were derived using the MEME algorithm (Bailey and Elkan, Proceedings of the Second International Conference on Intelligent Systems for Molecular Biology, pp. 28-36, AAAI Press, Menlo Park, California, 1994). At each position within a MEME motif, the residues are shown that are present in the query set of sequences with a frequency higher than 0.2. Residues within square brackets represent alternatives.

More preferably, the POI polypeptide comprises at least one of these three motifs.

Additionally, the present invention relates to a homologue of the POI polypeptide and its use in the method of the present invention. The homologue of a POI polypeptide has, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 2, and/or represented by its orthologues and paralogues shown in SEQ ID NO.: 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 provided that the homologous protein comprises any one or more of the motifs or domains as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a POI polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the r Motifs 1 to 3.

The terms "domain", "signature" and "motif' are defined in the "definitions" section herein.

Preferably, the polypeptide sequence which when used in the construction of a phylogenetic tree, such as the one depicted in Figure 1, clusters with the group of Hydroxyproline-rich glycoproteins (HRGP) comprising the amino acid sequence represented by SEQ ID NO: 2 rather than with any other group.

Furthermore, POI polypeptides (at least in their native form) typically are described as Hydroxyproline-rich glycoprotein family protein (HRGP). SEQ ID NO.: 1 encodes for a HRGP family protein of *Populus trichocarpa*. Proteins of this family are associated with the plant cell walls and have been shown to be implicated in a board range of growth and development processes including cell proliferation and extension, fertilization, and response to stresses such as pathogen attack (Shpak et al., 1999; Estevez et al., 2006).

For example, the protein sequence of the POI polypeptide also contains a BAR domain in N-terminus (Peter et al., 2004).

The increase in expression or in the activity of POI polypeptides, when expressed in a plant, e.g. according to the methods of the present invention as outlined in Examples 7 and 8, give plants having increased yield, in particular seed yield as measured by the total weight and number of seeds, and improved yield-related traits (in particular seed filling rate, number of seeds filled, shoot and root biomass) relative to control plants. Furthermore, the positive effect of increase of activity or amount of the POI polypeptide in a plant or plant cell on root biomass and seed filling rate suggest that this increase of activity or amount may also confer positive effect on yield under abiotic stresses, and in particular under drought stresses.

The present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any POI-encoding nucleic acid or POI polypeptide as defined herein, e.g. as listed in Table A and the sequence listing, as the polypeptides shown in SEQ ID No.: 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 and homologues, orthologues or paralogues thereof.

Examples of nucleic acids encoding Hydroxyproline-rich glycoproteins (HRGP) are given in Table A of the Examples section herein. Such nucleic acids are useful in performing the methods of the invention. The amino acid sequences given in Table A of the Examples section are example sequences of orthologues and paralogues of the POI polypeptide represented by SEQ ID NO: 2, the terms "orthologues" and "paralogues" being as defined herein. Further orthologues and paralogues may readily be identified by performing a so-called reciprocal blast search as described in the definitions section; where the query sequence is e.g. SEQ ID NO: 1 or SEQ ID NO: 2, the second BLAST (back-BLAST) would be against the original sequence database, e.g. poplar database.

The invention also provides hitherto unknown POI-encoding nucleic acid molecules and POI polypeptides useful for conferring enhanced yield-related traits in plants relative to control plants.

According to a further embodiment of the present invention, there is therefore provided an isolated nucleic acid molecule selected from:

(i) a nucleic acid represented by (any one of) SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57;

(ii) the complement of a nucleic acid represented by (any one of) SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57;

(iii) a nucleic acid encoding the polypeptide as represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89, preferably as a result of the degeneracy of the genetic code, said isolated nucleic acid can be derived from a polypeptide sequence as represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89and further preferably confers enhanced yield-related traits relative to control plants;

(iv) a nucleic acid having, in increasing order of preference at least 30 %, 31 %, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57 and further preferably conferring enhanced yield-related traits relative to control plants;

(v) a nucleic acid molecule which hybridizes with a nucleic acid molecule of (i) to (iv) under stringent hybridization conditions and preferably confers enhanced yield-related traits relative to control plants;

(vi) a nucleic acid encoding a Hydroxyproline-rich glycoprotein family protein (HRGP)having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71 %, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 and any of the other amino acid sequences in Table A and preferably conferring enhanced yield-related traits relative to control plants.

According to a further embodiment of the present invention, there is also provided an isolated polypeptide selected from:

(i) an amino acid sequence represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89;

(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 and any of the other amino acid sequences in Table A and preferably conferring enhanced yield-related traits relative to control plants;

(iii) derivatives of any of the amino acid sequences given in (i) or (ii) above; or

(iv) an amino acid sequence encoded by the nucleic acid of the invention.

Preferably the nucleic acid molecule of the invention or the polypeptide of the invention does not comprise the sequences SEQ ID NO.: 1 or 2, respectively.
Preferably, the nucleic acid molecule of the invention does not comprise the sequence of POPTRDRAFT 814116 and the polypeptide of the invention does not comprise the sequence of XP_002313513.1.

Accordingly, in one embodiment, the present invention relates to an expression construct comprising the nucleic acid molecule of the invention or conferring the expression of a POI polypeptide of the invention.

Nucleic acid variants may also be useful in practising the methods of the invention. Examples of such variants include nucleic acids encoding homologues and derivatives of any one of the amino acid sequences given in Table A of the Examples section, the terms "homologue" and "derivative" being as defined herein. Also useful in the methods of the invention are nucleic acids encoding homologues and derivatives of orthologues or paralogues of any one of the amino acid sequences given in Table A of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding Hydroxyproline-rich glycoprotein family protein (HRGP), nucleic acids hybridising to nucleic acids encoding Hydroxyproline-rich glycoproteins family protein (HRGP), splice variants of nucleic acids encoding POI, allelic variants of nucleic acids encoding POI polypeptides and variants of nucleic acids encoding POI polypeptides obtained by gene shuffling. The terms hybridising sequence, splice variant, allelic variant and gene shuffling are as described herein.

Nucleic acids encoding POI polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A of the Examples section, or a portion of a nucleic acid encoding an orthologue, paralogue or homologue of any of the amino acid sequences given in Table A of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a POI polypeptide as defined herein, and have substantially the same biological activity as the amino acid sequences given in Table A of the Examples section. Preferably, the portion is a portion of any one of the nucleic acids given in Table A of the Examples section, or is a portion of a nucleic acid encoding an orthologue or paralogue of any one of the amino acid sequences given in Table A of the Examples section. Preferably the portion is at least, 100, 200, 300, 400, 500, 550, or 600 consecutive nucleotides in length, the consecutive nucleotides being of any one of the nucleic acid sequences given in Table A of the Examples section, or of a nucleic acid encoding an orthologue or paralogue of any one of the amino acid sequences given in Table A of the Examples section. Preferably the portion is a portion of the nucleic acid of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57. Most preferably the portion is a portion of the nucleic acid of SEQ ID NO: 1. Preferably, the portion encodes a fragment of an amino acid sequence which, when used in the construction of a phylogenetic tree, such as the one depicted in Figure 1, clusters with the group of POI polypeptides comprising the amino acid sequence represented by SEQ ID NO: 2 rather than with any other group and/or comprises any one or more of the motifs 1 to 3 and/or has biological activity of a HRGP and/or comprises the nucleic acid molecule of the invention, e.g. has at least 50% sequence identity to SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 or is a orthologue or paralogue thereof. For example, the portion encodes a fragment of an amino acid sequence which, when used in the construction of a phylogenetic tree, such as the one depicted in Figure 1, clusters with the group of POI polypeptide comprising the amino acid sequence represented by SEQ ID NO: 2 rather than with any other group and comprises any one or more of the motifs 1 to 3 and has biological activity of a HRGP and has at least 50% sequence identity to SEQ ID NO: 2.

Another nucleic acid variant useful in the methods of the invention is a nucleic acid capable of hybridising, under reduced stringency conditions, preferably under stringent conditions, with a nucleic acid encoding a POI polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A of the Examples section, or comprising introducing and expressing in a plant a nucleic acid capable of hybridising to a nucleic acid encoding an orthologue, paralogue or homologue of any of the nucleic acid sequences given in Table A of the Examples section.

Hybridising sequences useful in the methods of the invention encode a POI polypeptide as defined herein, having substantially the same biological activity as the amino acid sequences given in Table A of the Examples section. Preferably, the hybridising sequence is capable of hybridising to the complement of any one of the nucleic acids given in Table A of the Examples section, or to a portion of any of these sequences, a portion being as defined above, or the hybridising sequence is capable of hybridising to the complement of a nucleic acid encoding an orthologue or paralogue of any one of the amino acid sequences given in Table A of the Examples section. Most preferably, the hybridising sequence is capable of hybridising to the complement of a nucleic acid as represented by SEQ ID NO: 1 or to a portion thereof.

Preferably, the hybridising sequence encodes a polypeptide with an amino acid sequence which, when full-length and used in the construction of a phylogenetic tree, such as the one depicted in Figure 1, clusters with the group of POI polypeptide comprising the amino acid sequence represented by SEQ ID NO: 2 rather than with any other group and/or comprises any one of the motifs 1 to 3 and/or has biological activity of a HRGP and/or has at least 50% sequence identity to SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 or is a orthologue or paralogue thereof. For example, the portion encodes a fragment of an amino acid sequence which, when used in the construction of a phylogenetic tree, such as the one depicted in Figure 1, clusters with the group of POI polypeptide comprising the amino acid sequence represented by SEQ ID NO: 2 rather than with any other group and comprises any one or more of the motifs 1 to 3 and has biological activity of a HRGP and has at least 50% sequence identity to SEQ ID NO: 2.

Another nucleic acid variant useful in the methods of the invention is a splice variant encoding a POI polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A of the Examples section, or a splice variant of a nucleic acid encoding an orthologue, paralogue or homologue of any of the amino acid sequences given in Table A of the Examples section.

Preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant, when used in the construction of a phylogenetic tree, such as the one depicted in Figure 1, clusters with the group of POI polypeptides comprising the amino acid sequence represented by SEQ ID NO: 2 rather than with any other group and/or comprises any one or more of the motifs 1 to 3 and/or has biological activity of a HRGP and/or has at least 50% sequence identity to SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 or an orthologue or paralogue thereof. For example, the portion encodes a fragment of an amino acid sequence which, when used in the construction of a phylogenetic tree, such as the one depicted in Figure 1, clusters with the group of POI polypeptides comprising the amino acid sequence represented by SEQ ID NO: 2 rather than with any other group and comprises any one or more of the motifs 1 to 3 and has biological activity of a HRGP and has at least 50% sequence identity to SEQ ID NO: 2.

Another nucleic acid variant useful in performing the methods of the invention is an allelic variant of a nucleic acid encoding a POI polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A of the Examples section, or comprising introducing and expressing in a plant an allelic variant of a nucleic acid encoding an orthologue, paralogue or homologue of any of the amino acid sequences given in Table A of the Examples section.

The polypeptides encoded by allelic variants useful in the methods of the present invention have substantially the same biological activity as the POI polypeptide of SEQ ID NO: 2 and any of the amino acids depicted in Table A of the Examples section. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant, when used in the construction of a phylogenetic tree, such as the one depicted in Figure 1, clusters with the group of POI polypeptides comprising the amino acid sequence represented by SEQ ID NO: 2 rather than with any other group and/or comprises any one or more of the motifs 1 to 3 and/or has biological activity of a HRGP and/or has at least 50% sequence identity to SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 or a orthologue or paralogue thereof. For example, the portion encodes a fragment of an amino acid sequence which, when used in the construction of a phylogenetic tree, such as the one depicted in Figure 1, clusters with the group of POI polypeptides comprising the amino acid sequence represented by SEQ ID NO: 2 rather than with any other group and comprises any one or more of the motifs 1 to 3 and has biological activity of a HRGP and has at least 50% sequence identity to SEQ ID NO: 2.

Gene shuffling or directed evolution may also be used to generate variants of nucleic acids encoding POI polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A of the Examples section, or comprising introducing and expressing in a plant a variant of a nucleic acid encoding an orthologue, paralogue or homologue of any of the amino acid sequences given in Table A of the Examples section, which variant nucleic acid is obtained by gene shuffling.

Preferably, the amino acid sequence encoded by the variant nucleic acid obtained by gene shuffling, when used in the construction of a phylogenetic tree, such as the one depicted in Figure 1, clusters with the group of POI polypeptides comprising the amino acid sequence represented by SEQ ID NO: 2 rather than with any other group and/or comprises any one or more of the motifs 1 to 3 and/or has biological activity of a HRGP and/or has at least 50% sequence identity to SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 or a orthologue or a paralogue thereof. For example, the portion encodes a fragment of an amino acid sequence which, when used in the construction of a phylogenetic tree, such as the one depicted in Figure 1, clusters with the group of POI polypeptides comprising the amino acid sequence represented by SEQ ID NO: 2 rather than with any other group and comprises any one or more of the motifs 1 to 3 and has biological activity of a HRGP and has at least 50% sequence identity to SEQ ID NO: 2..

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding POI polypeptides may be derived from any natural or artificial source. The nucleic acid may be modified from its native form in composition and/or genomic environment through deliberate human manipulation. Preferably the POI polypeptide-encoding nucleic acid is selected from a organism indicated in Table A, e.g. from a plant

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase early vigour and/or in biomass (weight) of one or more parts of a plant, which may include above ground (harvestable) parts and/or (harvestable) parts below ground. In particular, such harvestable parts are seeds and/or roots, and performance of the methods of the invention results in plants having increased seed filling rate, total seed yield, root and shoot biomass relative to control plants.

The present invention provides a method for increasing yield in comparison to the null control plants, in particular seed and/or root yield as measured by the total weight and number of seeds, and improved yield-related traits (in particular seed filling rate, number of seeds filled, shoot and root biomass) relative to control plants. , which method comprises modulating, preferably increasing expression or activity of a POI polypeptide in a plant, e.g. modulating or increasing expression in a plant of a nucleic acid encoding a POI polypeptide as defined herein. Furthermore, the positive effect of increase of activity or expression of the POI polypeptide in a plant or plant cell on root biomass and seed filling rate suggest that this may also confer positive effect on yield under abiotic stresses, and in particular under drought stresses.

Since the transgenic plants according to the present invention have increased yield, e.g. yield related traits such as increased seed filling rate, root and shoot biomass, it is likely that these plants exhibit an increased growth rate (during at least part of their life cycle), relative to the growth rate of control plants at a corresponding stage in their life cycle.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a POI polypeptide as defined herein.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises modulating expression in a plant of a nucleic acid encoding a POI polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding a POI polypeptide.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding a POI polypeptide.

The invention also provides genetic constructs and vectors to facilitate introduction and/or expression in plants of nucleic acids encoding POI polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a POI polypeptide as defined above;
(b) one or more control sequences capable of driving expression of the nucleic acid sequence of (a); and optionally
(c) a transcription termination sequence.

Preferably, the nucleic acid encoding a POI polypeptide is as defined above. The term "control sequence" and "termination sequence" are as defined herein.

The invention furthermore provides plants transformed with a construct as described above. In particular, the invention provides plants transformed with a construct as described above, which plants have increased yield-related traits as described herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types. Also useful in the methods of the invention is a root-specific promoter.Generally, by "medium strength promoter" is intended a promoter that drives expression of a coding sequence at a lower level than a strong promoter, in particular at a level that is in all instances below that obtained when under the control of a 35S CaMV promoter.

It should be clear that the applicability of the present invention is not restricted to the POI polypeptide-encoding nucleic acid represented by SEQ ID NO: 1, nor is the applicability of the invention restricted to expression of a POI polypeptide-encoding nucleic acid when driven by a constitutive promoter, or when driven by a root-specific promoter.

The constitutive promoter is preferably a medium strength promoter, more preferably selected from a plant derived promoter, such as a PRO0129 promoter, more preferably is the promoter PRO0129 promoter from rice. Further preferably the constitutive promoter is represented by a nucleic acid sequence substantially similar to SEQ ID NO: 89, most preferably the constitutive promoter is as represented by SEQ ID NO: 89. See the "Definitions" section herein for further examples of constitutive promoters.

Optionally, one or more terminator sequences may be used in the construct introduced into a plant. Preferably, the construct comprises an expression cassette comprising a PRO0129 promoter and the nucleic acid encoding the POI polypeptide. Furthermore, one or more sequences encoding selectable markers may be present on the construct introduced into a plant.

According to a preferred feature of the invention, the modulated expression is increased expression or activity, e.g. overexpression of a POI polypeptide encoding nucleic acid molecule, e.g. of a nucleic acid molecule encoding SEQ ID NO.: 1, 3, 5, 7, 9, 11, 13, 15, 17, 19, 21, 23, 25, 27, 29, 31, 33, 35, 37, 39, 41, 43, 45, 47, 49, 51, 53, 55, or 57 or a paralogue or orthologue thereof, e.g. as shown in Table A. Methods for increasing expression of nucleic acids or genes, or gene products, are well documented in the art and examples are provided in the definitions section.

As mentioned above, a preferred method for modulating expression of a nucleic acid encoding a POI polypeptide is by introducing and expressing in a plant a nucleic acid encoding a POI polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The invention also provides a method for the production of transgenic plants having enhanced yield-related traits relative to control plants, comprising introduction and expression in a plant of any nucleic acid encoding a POI polypeptide as defined hereinabove.

More specifically, the present invention provides a method for the production of transgenic plants having enhanced yield-related traits, particularly increased seed yield, seed filling rate, root and shoot biomass in comparison to the null control plants, which method comprises:

(i) introducing and expressing in a plant or plant cell a POI polypeptide-encoding nucleic acid or a genetic construct comprising a POI polypeptide-encoding nucleic acid; and
(ii) cultivating the plant cell under conditions promoting plant growth and development.

Furthermore, the positive effect of this construct on root biomass and seed filling rate suggests that this construct may also confer positive effect on yield under abiotic stresses, and in particular under drought stresses. The nucleic acid of (i) may be any of the nucleic acids capable of encoding a POI polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The present invention clearly extends to any plant cell or plant produced by any of the methods described herein, and to all plant parts and propagules thereof. The present invention encompasses plants or parts thereof (including seeds) obtainable by the methods according to the present invention. The plants or parts thereof comprise a nucleic acid transgene encoding a POI polypeptide as defined above. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a POI polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, beet, sugar beet, sunflower, canola, chicory, carrot, cassava, alfalfa, trefoil, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding a POI polypeptide. The invention furthermore relates to products derived, preferably directly derived, from a harvestable part of such a plant, such as dry pellets or powders, oil, fat and fatty acids, starch or proteins.

The present invention also encompasses use of nucleic acids encoding POI polypeptides as described herein and use of these POI polypeptides in enhancing any of the aforementioned yield-related traits in plants. For example, nucleic acids encoding POI polypeptide described herein, or the POI polypeptides themselves, may find use in breeding programmes in which a DNA marker is identified which may be genetically linked to a POI polypeptide-encoding gene. The nucleic acids/genes, or the POI polypeptides themselves may be used to define a molecular marker. This DNA or protein marker may then be used in breeding programmes to select plants having enhanced yield-related traits as defined hereinabove in the methods of the invention. Furthermore, allelic variants of a POI polypeptide-encoding nucleic acid/ gene may find use in marker-assisted breeding programmes. Nucleic acids encoding POI polypeptides may also be used as probes for genetically and physically mapping the genes that they are a part of, and as markers for traits linked to those genes. Such information may be useful in plant breeding in order to develop lines with desired phenotypes.

*Description of figures*

The present invention will now be described with reference to the following figures in which: Fig. 1 shows phylogenetic relationship of POI polypeptides. The proteins were aligned using MAFT (Katoh and Toh (2008). Briefings in Bioinformatics 9:286-298.). A tree was drawn using Dendroscope2.0.1 (Hudson et al. (2007). Bioinformatics 8(1): 460). At e=1, all genes encoding POI polypeptide homologues were recovered. The tree was generated using the genes of this list that contained at least one of the three MEME motifs.

*Examples*

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

**Example 1: Identification of sequences related to SEQ ID NO: 1 and SEQ ID NO: 2**

Sequences (full length cDNA, ESTs or genomic) related to SEQ ID NO: 1 and SEQ ID NO: 2 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 1 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

The sequence listing provides a list of nucleic acid sequences related to SEQ ID NO: 1 and SEQ ID NO: 2;e.g. selected from Table A:

**Table A:** Examples of POI nucleic acids and polypeptides:

| | | |
|---|---|---|
| P.trichocarpa_scaff_III.1360 | 645 aa | SEQ ID NO.: 3 and 4 |
| A.thaliana_AT2G33490.1 | 623 aa | SEQ ID NO.: 5 and 6 |
| A.thaliana_AT3G26910.1 | 608 aa | SEQ ID NO.: 7 and 8 |
| A.thaliana_AT5G41100.1 | 586 aa | SEQ ID NO.: 9 and 10 |
| G.max_Glyma20g39450 | 2005 aa | SEQ ID NO.: 11 and 12 |
| G.max_Glyma02g46370.1 | 305 aa | SEQ ID NO.: 13 and 14 |
| H.annuus_TC42303 | 223 aa | SEQ ID NO.: 15 and 16 |

(continued)

| | | |
|---|---|---|
| M.truncatula_NP7266312 | 209 aa | SEQ ID NO.: 17 and 18 |
| M.truncatula_AC146972_3.4 | 628 aa | SEQ ID NO.: 19 and 20 |
| M.truncatula_AC192957_6.3 | 544 aa | SEQ ID NO.: 21 and 22 |
| O.sativa_Os09g0509300 | 627 aa | SEQ ID NO.: 23 and 24 |
| O.sativa_Os04g0394700 | 624 aa | SEQ ID NO.: 25 and 26 |
| O.sativa_Os02g0515200 | 623 aa | SEQ ID NO.: 27 and 28 |
| P.trichocarpa_827559 | 625 aa | SEQ ID NO.: 29 and 30 |
| P.trichocarpa_TC123654 | 204 aa | SEQ ID NO.: 31 and 32 |
| P.trichocarpa_TC98441 | 203 aa | SEQ ID NO.: 33 and 34 |
| P.trichocarpa_TC115032 | 179 aa | SEQ ID NO.: 35 and 36 |
| T.aestivum_CA660681 | 132 aa | SEQ ID NO.: 37 and 38 |
| T.aestivum_TC292426 | 279 aa | SEQ ID NO.: 39 and 40 |
| T.aestivum_TC304862 | 150 aa | SEQ ID NO.: 41 and 42 |
| Z.mays_TC469176 | 432 aa | SEQ ID NO.: 43 and 44 |
| Z.mays_c57305964gm030403@806 | 210 aa | SEQ ID NO.: 45 and 46 |
| Z.mays_ZM07MC15404_62013874@15 | 615 aa | SEQ ID NO.: 47 and 48 |
| P.patens_234309 | 993 aa | SEQ ID NO.: 49 and 50 |
| P.trichocarpa_scaff_I.2192 | 608 aa | SEQ ID NO.: 51 and 52 |
| P.trichocarpa_757846 | 574 aa | SEQ ID NO.: 53 and 54 |
| P.thchocarpa_TC90097 | 183 aa | SEQ ID NO.: 55 and 56 |
| B.napus_TC86239 | 176 aa | SEQ ID NO.: 57 and 58 |

as well as SEQ ID NO.: 59 to 81, respectively.

Sequences have been tentatively assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, such as by the Joint Genome Institute. Furthermore, access to proprietary databases, has allowed the identification of novel nucleic acid and polypeptide sequences.

**Example 2: Alignment of POI polypeptide sequences**

Alignment of polypeptide sequences was performed using MAFT (version 6.717, L-INS-I method ; Katoh and Toh (2008). Briefings in Bioinformatics 9:286-298.).

A tree was drawn using Dendroscope2.0.1 (Hudson et al. (2007). Bioinformatics 8(1):460).

Alignment of polypeptide sequences can be performed using the ClustalW (1.83 / 2.0) algorithm of progressive alignment (Thompson et al. (1997) Nucleic Acids Res 25:4876-4882; Chenna et al. (2003). Nucleic Acids Res 31: 3497-3500) with standard setting (slow alignment, similarity matrix: Gonnet (or Blosum 62 (if polypeptides are aligned). , gap opening penalty 10, gap extension penalty: 0.2). Minor manual editing can be done to further optimise the alignment..

A phylogenetic tree of POI polypeptides (Figure 1) can be constructed using a neighbour-joining clustering algorithm as provided in the AlignX programme from the Vector NTI (Invitrogen).

Alignment of polypeptide sequences can be performed using the ClustalW (1.83 / 2.0) algorithm of progressive alignment (Thompson et al. (1997) Nucleic Acids Res 25:4876-4882; Chenna et al. (2003). Nucleic Acids Res 31: 3497-3500) with standard setting (slow alignment, similarity matrix: Gonnet, gap opening penalty 10, gap extension penalty: 0.2). Minor manual editing was done to further optimise the alignment.

**Example 3: Calculation of global percentage identity between polypeptide sequences**

Global percentages of similarity and identity between full length polypeptide sequences were determined using the ClustalW 2.0 algorithm of progressive alignment (Thompson et al. (1997) Nucleic Acids Res 25:4876-4882; Chenna et al. (2003). Nucleic Acids Res 31:3497-3500) with default setting.

Global percentages of similarity and identity between full length polypeptide sequences useful in performing the methods of the invention can be determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix.

**Example 4: Identification of domains comprised in polypeptide sequences useful in performing the methods of the invention**

Motifs were identified by using the MEME algorithm (Bailey and Elkan, Proceedings of the Second International Conference on Intelligent Systems for Molecular Biology, pp. 28-36, AAAI Press, Menlo Park, California, 1994). At each position within a MEME motif, the residues are shown that are present in the query set of sequences with a frequency higher than 0.2. Residues within square brackets represent alternatives.

Domains were identified by using the Pfam database.

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

**Example 5: Topology prediction of the POI polypeptide sequences**

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

**Example 6: Cloning of the POI encoding nucleic acid sequence**

The nucleic acid sequence was amplified by PCR using as template a custom-made *Populus trichocarpa* seedlings cDNA library (in pDONR222.1; Invitrogen, Paisley, UK). PCR was performed using Hifi Taq DNA polymerase in standard conditions, using 200 ng of template in a 50 μl PCR mix. The primers used were prm17524 (SEQ ID NO: 59; sense, start codon in bold):

5' ggggacaagtttgtacaaaaaagcaggcttaaacaatgaaatcatttaggaaattg 3'
and prm17525 (SEQ ID NO: 60; reverse, complementary):
5' ggggaccactttgtacaagaaagctgggtaagaatagaatcaaactgcac 3',

which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", $_P$POI. Plasmid pDONR201 was purchased from Invitrogen, as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice PRO0129 (e.g. SEQ ID NO.: 89) promoter for constitutive expression was located upstream of this Gateway cassette.

After the LR recombination step, the resulting expression vector PRO129::PO1 was transformed into *Agrobacterium* strain LBA4044 according to methods well known in the art.

**Example 7: Plant transformation**

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

Example 8: Transformation of other crops

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat can be performed with the method described by Ishida et al. (1996) Nature Biotech 14(6): 745-50. The cultivar Bobwhite (available from CIMMYT, Mexico) is commonly used in transformation. Immature embryos can be co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots can be transferred from each embryo to rooting medium and incubated at 25 °C for 2-3 weeks, until roots develop. The rooted shoots can be transplanted to soil in the greenhouse. T1 seeds are produced from plants that exhibit tolerance to the selection agent and that contain a single copy of the T-DNA insert.

*Soybean transformation*

Soybean can be transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon can be excised from seven-day old young seedlings. The epicotyl and the remaining cotyledon are further grown to develop axillary nodes. These axillary nodes can be excised and incubated with *Agrobacterium tumefaciens* containing the expression vector. After the cocultivation treatment, the explants are washed and transferred to selection media. Regenerated shoots can be excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling can be used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds can be surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they can be cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds can be produced from plants that exhibit tolerance to the selection agent and that contain a single copy of the T-DNA insert.

*Alfalfa transformation*

A regenerating clone of alfalfa *(Medicago sativa)* can be transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) can be selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants can be washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings can be transplanted into pots and grown in a greenhouse. T1 seeds can be produced from plants that exhibit tolerance to the selection agent and that contain a single copy of the T-DNA insert.

*Cotton transformation*

Cotton can be transformed using *Agrobacterium tumefaciens* according to the method described in US 5,159,135. Cotton seeds can be surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings can be removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues can be transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues can be subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants can be hardened and subsequently moved to the greenhouse for further cultivation.

**Example 10: Phenotypic evaluation procedure**

9.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.
rom the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

9.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant above ground area (or leafy biomass) was determined by counting the total number of pixels on the digital images from above ground plant parts discriminated from the background. This value was averaged for the pictures taken on the same time point from the different angles and was converted to a physical surface value expressed in square mm by calibration. Experiments show that the above ground plant area measured this way correlates with the biomass of plant parts above ground. The above ground area is the area measured at the time point at which the plant had reached its maximal leafy biomass. The early vigour is the plant (seedling) above ground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

A robust indication of the height of the plant is the measurement of the gravity (GravityYMax), i.e.determing the height (in mm) of the gravity centre of the leafy biomass. This avoids influence by a single erect leaf, based on the asymptote of curve fitting or, if the fit is not satisfactory, based on the absolute maximum.

Early vigour was determined by counting the total number of pixels from above ground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

Examples 10: Results of the phenotypic evaluation of the transgenic plants

The results of the evaluation of transgenic rice plants in the T1 generation and expressing a nucleic acid comprising the longest Open Reading Frame in SEQ ID NO: 1 under non-stress conditions are presented below. See previous Examples for details on the generations of the transgenic plants.

The results of the evaluation of transgenic rice plants under non-stress conditions are presented below. An increase of (at least - more than) 5 % was observed for above ground biomass (AreaMax), emergence vigour (early vigour), total seed yield, number of filled seeds, fill rate, number of flowers per panicle, harvest index, and of (2.5-3)% for thousand kernel weight

Transgenic plants over-expressing the POI under the constitutive promoter PRO0129 displayed increased yield in comparison to the null control plants. More particularly, the transgenic plants exhibited increased root and shoot biomass with an overall positive effect on root biomass (RootMax; 8.1%), above ground biomass (AreaMax; 8.7%) and GravityYMax (5.9%) (p-values of 0.0149, 0.0060, and 0.0000, respectively). Transgenic plants were also more fertile with a 8.5% overall increase for flowerperpan (p-value of 0.1252), and displayed increased seed yield: overall increase of 10.5% for total number of seeds (nrtotalseed), 21.9% for total weight of seeds (totalwgseeds), 13.3% for seed filling rate (filling rate), and 24.6% for number of filled seeds (nrfilledseed) (p-values of 0.0241, 0.0004, 0.0023, and 0.0000, respectively).

## Claims

1. A method for enhancing yield in plants relative to control plants, comprising modulating the activity in a plant of a polypeptide, wherein said polypeptide comprises a BAR (Bin/amphiphysin/Rvs) domain.

2. The method of claim 1, comprising modulating expression in a plant of a nucleic acid encoding a polypeptide, wherein said polypeptide comprises a BAR (Bin/amphiphysin/Rvs) domain.

3. Method according to claim 1 or 2, wherein said polypeptide comprises one or more of the following motifs:

   (i) Motif 1:

   [ST]SP[KR]ISELHELPRPP

   (ii) Motif 2:

   [ED]MK[QR]QCDEKREVYE

   (iii) Motif 3:

   SLK[QE]GQSRSLLTQAARHHAAQ

**4.** Method according to claim 2 to 3, wherein said modulated expression is effected by introducing and expressing in a plant a nucleic acid encoding a Hydroxyproline-rich glycoprotein family protein (HRGP).

**5.** Method according to any one of claims 1 to 3, wherein said polypeptide is encoded by a nucleic acid molecule comprising a nucleic acid molecule selected from the group consisting of:

(i) a nucleic acid represented by (any one of) SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57;
(ii) the complement of a nucleic acid represented by (any one of) SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57;
(iii) a nucleic acid encoding the polypeptide as represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89, preferably as a result of the degeneracy of the genetic code, said isolated nucleic acid can be derived from a polypeptide sequence as represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 and further preferably confers enhanced yield-related traits relative to control plants;
(iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, or 57 and further preferably conferring enhanced yield-related traits relative to control plants;
(v) a nucleic acid molecule which hybridizes with a nucleic acid molecule of (i) to (iv) under stringent hybridization conditions and preferably confers enhanced yield-related traits relative to control plants;
(vi) a nucleic acid encoding said polypeptide having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by (any one of) SEQ ID NO: 2, 4, 6, 8, 10, 12, 14, 16, 18, 20, 22, 24, 26, 28, 30, 32, 34, 36, 38, 40, 42, 44, 46, 48, 50, 53, 54, 56, or 58 or 59 to 89 and preferably conferring enhanced yield-related traits relative to control plants.

**6.** Method according to any preceding claim, wherein said enhanced yield-related traits comprise increased yield, preferably seed filling rate, number of seeds filled, shoot and/or root biomass relative to control plants.

**7.** Method according to any one of claims 1 to 6, wherein said enhanced yield-related traits are obtained under non-stress conditions.

**8.** Method according to any one of claims 1 to 6, wherein said enhanced yield-related traits are obtained under conditions of drought stress, salt stress or nitrogen deficiency.

**9.** Method according to any one of claims 2 to 8, wherein said nucleic acid is operably linked to a constitutive promoter, preferably to a PRO0129 promoter, most preferably to a PRO0129 promoter from rice.

**10.** Method according to any one of claims 1 to 9, wherein said polypeptide or said nucleic acid molecule, respectively, is of plant origin, preferably from a dicotyledonous plant, further preferably from the family Salicaceae, more preferably from the genus Populus, most preferably from *Populus trichocarpa.*

**11.** Plant or part thereof, including seeds, obtainable by a method according to any one of claims 1 to 10, wherein said plant or part thereof comprises a recombinant nucleic acid encoding said polypeptide as defined in any one of claims 1 to 10.

**12.** Construct comprising:

(i) nucleic acid encoding said polypeptide as defined in any one of claims 1 to 10;
(ii) one or more control sequences capable of driving expression of the nucleic acid sequence of (a); and optionally
(iii) a transcription termination sequence.

**13.** Construct according to claim 12, wherein one of said control sequences is a constitutive promoter, preferably a PRO0129 promoter, most preferably a PRO0129 promoter from rice.

**14.** Use of a construct according to claim 12 or 13 in a method for making plants having increased yield, particularly seed filling rate, number of seeds filled, shoot and/or root biomass relative to control plants relative to control plants.

**15.** Plant, plant part or plant cell transformed with a construct according to claim 12 or 13.

**16.** Method for the production of a transgenic plant having increased yield, particularly increased biomass and/or increased seed yield relative to control plants, comprising:

(i) introducing and expressing in a plant a nucleic acid encoding said polypeptide as defined in any one of claims 1 to 10; and
(ii) cultivating the plant cell under conditions promoting plant growth and development.

**17.** Plant having increased yield, particularly increased biomass and/or increased seed yield, relative to control plants, resulting from modulated expression of a nucleic acid encoding said polypeptide as defined in any one of claims 1 to 10, or a transgenic plant cell derived from said transgenic plant.

**18.** Plant according to claim 11, 15 or 17, or a transgenic plant cell derived thereof, wherein said plant is a crop plant, such as sugar beet, alfalfa, trefoil, chicory, carrot, cassava, or a monocot, such as sugarcane, or a cereal, such as rice, maize, wheat, barley, millet, rye, triticale, sorghum emmer, spelt, secale, einkorn, teff, milo and oats.

**19.** Harvestable parts of a plant according to claim 18, wherein said harvestable parts are preferably shoot and/or root biomass and/or seeds.

**20.** Products derived from a plant according to claim 18 and/or from harvestable parts of a plant according to claim 19.

**21.** Use of a nucleic acid encoding said polypeptide as defined in any one of claims 1 to 10 in increasing yield, particularly in seed filling rate, number of seeds filled, shoot and/or root biomass relative to control plants.

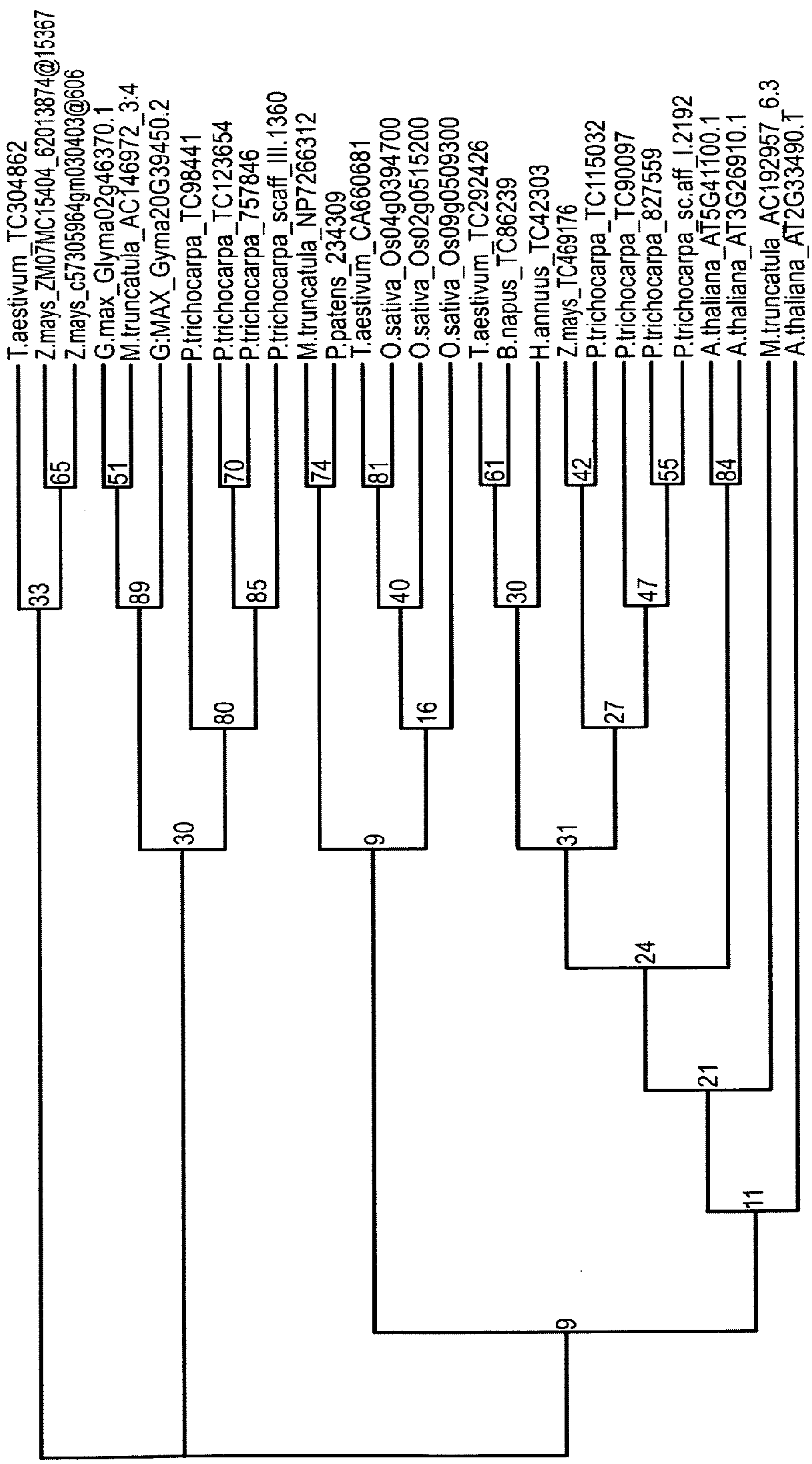

T.aestivum_TC304862
Z.mays_ZM07MC15404_62013874@15367
Z.mays_c57305964gm030403@606
G.max_Glyma02g46370.1
M.truncatula_AC146972_3.4
G.MAX_Gyma20G39450.2
P.trichocarpa_TC98441
P.trichocarpa_TC123654
P.trichocarpa_757846
P.trichocarpa_scaff_III.1360
M.truncatula_NP7266312
P.patens_234309
T.aestivum_CA660681
O.sativa_Os04g0394700
O.sativa_Os02g0515200
O.sativa_Os09g0509300
T.aestivum_TC292426
B.napus_TC86239
H.annuus_TC42303
Z.mays_TC469176
P.trichocarpa_TC115032
P.trichocarpa_TC90097
P.trichocarpa_827559
P.trichocarpa_sc.aff_I.2192
A.thaliana_AT5G41100.1
A.thaliana_AT3G26910.1
M.truncatula_AC192957_6.3
A.thaliana_AT2G33490.1


Figure 1

Europäisches Patentamt
European Patent Office
Office européen des brevets

# EUROPEAN SEARCH REPORT

Application Number
EP 10 15 4801

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online] 24 March 2009 (2009-03-24), "SubName: Full=Predicted protein;" XP002595243 retrieved from EBI accession no. UNIPROT:B9GZC0 Database accession no. B9GZC0 * the whole document * & DATABASE EMBL [Online] 12 October 2006 (2006-10-12), "Populus trichocarpa linkage group III, whole genome shotgun sequence." retrieved from EBI accession no. EMBL:CM000339 Database accession no. CM000339 * compound * ----- | 1-6, 10-12, 14-21 | INV. C12N15/82 A01H5/00 |
| X | DATABASE EMBL [Online] 4 September 2004 (2004-09-04), "B9SP10d10 Populus stem seasonal library Populus deltoides cDNA, mRNA sequence." XP002595244 retrieved from EBI accession no. EMBL:CV130760 Database accession no. CV130760 * the whole document * ----- | 1-6, 10-12, 14-21 | TECHNICAL FIELDS SEARCHED (IPC) C12N A01H |
| X | US 2007/039069 A1 (ROGERS JAMES A [US] ET AL) 15 February 2007 (2007-02-15) * sequences 2103,2113 * ----- | 1-8, 10-12, 14-21 | |
| X | US 2004/031072 A1 (LA ROSA THOMAS J [US] ET AL) 12 February 2004 (2004-02-12) * sequences 170587,27745 * ----- -/-- | 1-8, 10-12, 14-21 | |

~~The present search report has been drawn up for all claims~~

1

| Place of search | Date of completion of the search | Examiner |
|---|---|---|
| The Hague | 4 August 2010 | Maddox, Andrew |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 15 4801

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/216190 A1 (KOVALIC DAVID K [US]) 28 October 2004 (2004-10-28)<br>* sequences 353,5897 *<br>----- | 1-8, 10-12, 14-21 | |
| X | WO 2008/061157 A2 (AGRIGENETICS INC [US]; DAVIES JOHN P [US]; NG HEIN TSOENG MEDARD [US];) 22 May 2008 (2008-05-22)<br>* sequences 33,34 *<br>----- | 1-8, 10-12, 14-21 | |
| A | ROBERTS K ET AL: "Increased extensin levels in Arabidopsis affect inflorescence stem thickening and height"<br>JOURNAL OF EXPERIMENTAL BOTANY, OXFORD UNIVERSITY PRESS, GB LNKD- DOI:10.1093/JXB/ERJ036,<br>vol. 57, no. 3,<br>1 February 2006 (2006-02-01), pages 537-545, XP002576034<br>ISSN: 0022-0957 [retrieved on 2006-01-05]<br>* the whole document *<br>----- | 1-21 | |
| | | | TECHNICAL FIELDS SEARCHED (IPC) |

~~The present search report has been drawn up for all claims~~

1

| Place of search | Date of completion of the search | Examiner |
|---|---|---|
| The Hague | 4 August 2010 | Maddox, Andrew |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 10 15 4801

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-21(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 10 15 4801

The Search Division considers that the present European patent application does not comply with the requirements of unity of invention and relates to several inventions or groups of inventions, namely:

Invention: 1; Claims: 1-21(partially)

Methods, constructs, plants and uses defiend by reference to SEQ ID NO:1 and 2

---

Inventions: 2-60; Claims: 1-21(partially)

Methods, constructs, plants and uses defined by reference to each individual SEQ ID NO pair 3 and 4 through 57 and 58 and each individual sequence of SEQ ID NO:59 through 89. Claims in so far as applicable

---

## ANNEX TO THE EUROPEAN SEARCH REPORT ON EUROPEAN PATENT APPLICATION NO. EP 10 15 4801

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2010

| Patent document cited in search report | Publication date | Patent family member(s) | Publication date |
|---|---|---|---|
| US 2007039069 A1 | 15-02-2007 | NONE | |
| US 2004031072 A1 | 12-02-2004 | US 2004034888 A1<br>US 2006236419 A1 | 19-02-2004<br>19-10-2006 |
| US 2004216190 A1 | 28-10-2004 | NONE | |
| WO 2008061157 A2 | 22-05-2008 | AR 063845 A1<br>CA 2669989 A1<br>US 2008160162 A1 | 25-02-2009<br>22-05-2008<br>03-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling
- WO 9853083 A, Grierson
- WO 9953050 A, Waterhouse
- US 4987071 A, Cech
- US 5116742 A, Cech
- WO 9400012 A
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- US 5164310 A
- US 5159135 A


### Non-patent literature cited in the description


- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696

- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318

- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* 1988, vol. 8, 4395-4405
- **CALLIS et al.** *Genes Dev,* 1987, vol. 1, 1183-1200
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- **F.F. WHITE.** Vectors for Gene Transfer in Higher Plants. *Transgenic Plants, Vol. 1, Engineering and Utilization,* 1993, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **WANG et al.** *Planta,* 2003, vol. 218, 1-14
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346

- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **PETER BJ ; KENT HM ; MILLS IG ; VALLIS Y ; BUTLER PJ ; EVANS PR ; MCMAHON HT.** *Science,* 2004, vol. 303, 495-499
- **BAILEY ; ELKAN.** Proceedings of the Second International Conference on Intelligent Systems for Molecular Biology. AAAI Press, 1994, 28-36
- **KATOH ; TOH.** *Briefings in Bioinformatics,* 2008, vol. 9, 286-298
- **HUDSON et al.** *Bioinformatics,* 2007, vol. 8 (1), 460
- **SAMBROOK.** Molecular Cloning: a laboratory manual. Cold Spring Harbor Laboratory Press, CSH, 2001
- Current Protocols in Molecular Biology. Current Protocols, 1994, vol. 1, 2
- **R.D.D. CROY.** Plant Molecular Biology Labfax. BIOS Scientific Publications, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **PARK ; KANEHISA.** *Bioinformatics,* 2003, vol. 19, 1656-1663
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158

SEQUENCE LISTING

```
<110>  BASF Plant Science Company GmbH + VIB VZW

<120>  Plants having enhanced yield-related traits and a method for
       making the same

<130>  PF70322/CDS8093

<160>  89

<170>  PatentIn version 3.3

<210>  1
<211>  1938
<212>  DNA
<213>  Populus trichocarpa


<220>
<221>  CDS
<222>  (1)..(1938)

<400>  1
atg aaa tca ttt agg aaa ttg aga gga ttt gct tca ctt cac aaa caa       48
Met Lys Ser Phe Arg Lys Leu Arg Gly Phe Ala Ser Leu His Lys Gln
1               5                   10                  15

gta gta cat aaa aac cct agg gat ctt ccc tcc ttc tct caa tca cat       96
Val Val His Lys Asn Pro Arg Asp Leu Pro Ser Phe Ser Gln Ser His
            20                  25                  30

gag ctt gct aaa gcc tct cag gac atg aaa gat atg aaa gat tgc tat      144
Glu Leu Ala Lys Ala Ser Gln Asp Met Lys Asp Met Lys Asp Cys Tyr
        35                  40                  45

gat agc tta ctt tct gct gcc gct gga acc gcc aat tgt gcc ttt gaa      192
Asp Ser Leu Leu Ser Ala Ala Ala Gly Thr Ala Asn Cys Ala Phe Glu
    50                  55                  60

ttc tca gaa tca ttg cgt gaa atg ggt gct tgc ctt ctt gcg aaa act      240
Phe Ser Glu Ser Leu Arg Glu Met Gly Ala Cys Leu Leu Ala Lys Thr
65                  70                  75                  80

gca ttg aat gat gat gaa gaa agt ggc agg gtc ttg ctg atg ctg gga      288
Ala Leu Asn Asp Asp Glu Glu Ser Gly Arg Val Leu Leu Met Leu Gly
                85                  90                  95

aaa gtg caa ttt gaa ctc cag aaa ctt gtt gac tgc tat cgt tct cac      336
Lys Val Gln Phe Glu Leu Gln Lys Leu Val Asp Cys Tyr Arg Ser His
            100                 105                 110

ata aat cag aca atc ata agc ccg tca gag tct ctt ctg aat gaa ctt      384
Ile Asn Gln Thr Ile Ile Ser Pro Ser Glu Ser Leu Leu Asn Glu Leu
        115                 120                 125

caa ata gtt gag gag atg aag cag cag tgt gat gag aaa aga gat gta      432
Gln Ile Val Glu Glu Met Lys Gln Gln Cys Asp Glu Lys Arg Asp Val
    130                 135                 140

tat gct cac atg gcg aga cag aga gaa aga gtg agt gga aga aat gga      480
Tyr Ala His Met Ala Arg Gln Arg Glu Arg Val Ser Gly Arg Asn Gly
145                 150                 155                 160

aag gga gag tgt ttt tct atg cag caa ata caa gca gct cat gat gaa      528
Lys Gly Glu Cys Phe Ser Met Gln Gln Ile Gln Ala Ala His Asp Glu
```

```
                165                 170                 175

tat gat gag gag gcc act ttg ttt gtt ttt cgt ctt aaa tcc ctg aag       576
Tyr Asp Glu Glu Ala Thr Leu Phe Val Phe Arg Leu Lys Ser Leu Lys
            180                 185                 190

gaa gga caa tct cgc agt ctt ctt aca cag gcg gct cgg cac cat gct       624
Glu Gly Gln Ser Arg Ser Leu Leu Thr Gln Ala Ala Arg His His Ala
        195                 200                 205

gct cag ctc tgc ttc ttt aag aag gct ctt caa tct ctt gaa gct gtt       672
Ala Gln Leu Cys Phe Phe Lys Lys Ala Leu Gln Ser Leu Glu Ala Val
    210                 215                 220

gag ccg cat gtc aaa ttg gtc tca gaa cag cag cat ata gat tac cac       720
Glu Pro His Val Lys Leu Val Ser Glu Gln Gln His Ile Asp Tyr His
225                 230                 235                 240

ttt agt ggc ctt gat gat gat ggg agg gat tat gat gat gat gaa gat       768
Phe Ser Gly Leu Asp Asp Asp Gly Arg Asp Tyr Asp Asp Asp Glu Asp
                245                 250                 255

tat gat gat gcc att gat gat gga gaa ttg agc ttt gac tat gga caa       816
Tyr Asp Asp Ala Ile Asp Asp Gly Glu Leu Ser Phe Asp Tyr Gly Gln
            260                 265                 270

aat gac cag gag caa gaa gtt tcc aaa tca ata aag tca atg gag ttg       864
Asn Asp Gln Glu Gln Glu Val Ser Lys Ser Ile Lys Ser Met Glu Leu
        275                 280                 285

gat tta gag gac att aca ttc ccc caa gtt gtg aca ttg gaa atg gca       912
Asp Leu Glu Asp Ile Thr Phe Pro Gln Val Val Thr Leu Glu Met Ala
    290                 295                 300

aag gaa aat cca gat aga agt tac agg aca tcg ttt cct att aag gga       960
Lys Glu Asn Pro Asp Arg Ser Tyr Arg Thr Ser Phe Pro Ile Lys Gly
305                 310                 315                 320

gaa ctt agt gca ggc acc caa tca gct cca ctt ttt gct caa gtg aaa      1008
Glu Leu Ser Ala Gly Thr Gln Ser Ala Pro Leu Phe Ala Gln Val Lys
                325                 330                 335

tct aat cca gct gga aaa aca aaa caa ctg atg cca tca tcc acc cga      1056
Ser Asn Pro Ala Gly Lys Thr Lys Gln Leu Met Pro Ser Ser Thr Arg
            340                 345                 350

aag ttc aac act tat gtg ttg cct act cca gca gac cca aag agt tca      1104
Lys Phe Asn Thr Tyr Val Leu Pro Thr Pro Ala Asp Pro Lys Ser Ser
        355                 360                 365

aat tct aca gga cct ggt agc ccg gtt tct caa aca tta aag aca agt      1152
Asn Ser Thr Gly Pro Gly Ser Pro Val Ser Gln Thr Leu Lys Thr Ser
    370                 375                 380

ttg agt gga cgt cct ctg aat ttg tgg cac tca tcc cct att ggc cat      1200
Leu Ser Gly Arg Pro Leu Asn Leu Trp His Ser Ser Pro Ile Gly His
385                 390                 395                 400

aaa aaa aat gat aag tta cta gga gtt gaa atg tct agc aag ccc cct      1248
Lys Lys Asn Asp Lys Leu Leu Gly Val Glu Met Ser Ser Lys Pro Pro
                405                 410                 415

gct ata aat tcg cgg tca gta ctc aag gag agc aac aac aac acg gca      1296
Ala Ile Asn Ser Arg Ser Val Leu Lys Glu Ser Asn Asn Asn Thr Ala
            420                 425                 430
```

```
tcc acc cga tta cct cct cct cta gct gat gga ctc ttt ttt tca agg      1344
Ser Thr Arg Leu Pro Pro Pro Leu Ala Asp Gly Leu Phe Phe Ser Arg
        435                 440                 445

ctt gag cca cca gct ggc gct gaa tct aag aaa atc aaa aga tat gcc      1392
Leu Glu Pro Pro Ala Gly Ala Glu Ser Lys Lys Ile Lys Arg Tyr Ala
    450                 455                 460

ttt tct ggt cct att aca tcc cag cca tgg tct acc aag gca gtc tcc      1440
Phe Ser Gly Pro Ile Thr Ser Gln Pro Trp Ser Thr Lys Ala Val Ser
465                 470                 475                 480

gca gaa cat cca caa ttg ttc tct gga ccc ctt ttg cga aat cca acg      1488
Ala Glu His Pro Gln Leu Phe Ser Gly Pro Leu Leu Arg Asn Pro Thr
                485                 490                 495

gcc caa tta ttt tca tca cct ccg aaa gtg tct cca aga att tca cca      1536
Ala Gln Leu Phe Ser Ser Pro Pro Lys Val Ser Pro Arg Ile Ser Pro
            500                 505                 510

aaa gta tct cct agt tct tcc cct cca ttt gtg tcc tca ccc aaa atc      1584
Lys Val Ser Pro Ser Ser Ser Pro Pro Phe Val Ser Ser Pro Lys Ile
        515                 520                 525

agc gag cta cat gag ctt ccc aga ccc cca gtc agt tca acc tcc aag      1632
Ser Glu Leu His Glu Leu Pro Arg Pro Pro Val Ser Ser Thr Ser Lys
    530                 535                 540

tct ccg ggg gct gga ggt ttg gtt ggt cat tca gct cca ctg ttg ccc      1680
Ser Pro Gly Ala Gly Gly Leu Val Gly His Ser Ala Pro Leu Leu Pro
545                 550                 555                 560

aaa ggc cac atg ctt cct ggt aca agc aaa acg tca gca tca aat gta      1728
Lys Gly His Met Leu Pro Gly Thr Ser Lys Thr Ser Ala Ser Asn Val
                565                 570                 575

gaa tct caa ctg cct aca cct tct caa gtt gtc cct cgc agt ttt tca      1776
Glu Ser Gln Leu Pro Thr Pro Ser Gln Val Val Pro Arg Ser Phe Ser
            580                 585                 590

ata ccc tct agc aga cat aga gta atg gtc gct cag aat cca ggg att      1824
Ile Pro Ser Ser Arg His Arg Val Met Val Ala Gln Asn Pro Gly Ile
        595                 600                 605

gtt gag aat gtt gcc tca cct ccc cta acc cca ata tct tta tct aac      1872
Val Glu Asn Val Ala Ser Pro Pro Leu Thr Pro Ile Ser Leu Ser Asn
    610                 615                 620

acc cag cca tca tcc act ggt tcc cgg atc gtc aat cag aca gtt caa      1920
Thr Gln Pro Ser Ser Thr Gly Ser Arg Ile Val Asn Gln Thr Val Gln
625                 630                 635                 640

atc aga ggt gca gtt tga                                              1938
Ile Arg Gly Ala Val
                645


<210>  2
<211>  645
<212>  PRT
<213>  Populus trichocarpa

<400>  2

Met Lys Ser Phe Arg Lys Leu Arg Gly Phe Ala Ser Leu His Lys Gln
1               5                   10                  15
```

```
Val Val His Lys Asn Pro Arg Asp Leu Pro Ser Phe Ser Gln Ser His
            20                  25                  30

Glu Leu Ala Lys Ala Ser Gln Asp Met Lys Asp Met Lys Asp Cys Tyr
        35                  40                  45

Asp Ser Leu Leu Ser Ala Ala Ala Gly Thr Ala Asn Cys Ala Phe Glu
    50                  55                  60

Phe Ser Glu Ser Leu Arg Glu Met Gly Ala Cys Leu Leu Ala Lys Thr
65                  70                  75                  80

Ala Leu Asn Asp Asp Glu Glu Ser Gly Arg Val Leu Leu Met Leu Gly
                85                  90                  95

Lys Val Gln Phe Glu Leu Gln Lys Leu Val Asp Cys Tyr Arg Ser His
            100                 105                 110

Ile Asn Gln Thr Ile Ile Ser Pro Ser Glu Ser Leu Leu Asn Glu Leu
        115                 120                 125

Gln Ile Val Glu Glu Met Lys Gln Gln Cys Asp Glu Lys Arg Asp Val
    130                 135                 140

Tyr Ala His Met Ala Arg Gln Arg Glu Arg Val Ser Gly Arg Asn Gly
145                 150                 155                 160

Lys Gly Glu Cys Phe Ser Met Gln Gln Ile Gln Ala Ala His Asp Glu
                165                 170                 175

Tyr Asp Glu Glu Ala Thr Leu Phe Val Phe Arg Leu Lys Ser Leu Lys
            180                 185                 190

Glu Gly Gln Ser Arg Ser Leu Leu Thr Gln Ala Ala Arg His His Ala
        195                 200                 205

Ala Gln Leu Cys Phe Phe Lys Lys Ala Leu Gln Ser Leu Glu Ala Val
    210                 215                 220

Glu Pro His Val Lys Leu Val Ser Glu Gln Gln His Ile Asp Tyr His
225                 230                 235                 240

Phe Ser Gly Leu Asp Asp Asp Gly Arg Asp Tyr Asp Asp Asp Glu Asp
                245                 250                 255

Tyr Asp Asp Ala Ile Asp Asp Gly Glu Leu Ser Phe Asp Tyr Gly Gln
            260                 265                 270
```

```
Asn Asp Gln Glu Gln Glu Val Ser Lys Ser Ile Lys Ser Met Glu Leu
        275                 280                 285

Asp Leu Glu Asp Ile Thr Phe Pro Gln Val Val Thr Leu Glu Met Ala
    290                 295                 300

Lys Glu Asn Pro Asp Arg Ser Tyr Arg Thr Ser Phe Pro Ile Lys Gly
305                 310                 315                 320

Glu Leu Ser Ala Gly Thr Gln Ser Ala Pro Leu Phe Ala Gln Val Lys
                325                 330                 335

Ser Asn Pro Ala Gly Lys Thr Lys Gln Leu Met Pro Ser Ser Thr Arg
            340                 345                 350

Lys Phe Asn Thr Tyr Val Leu Pro Thr Pro Ala Asp Pro Lys Ser Ser
        355                 360                 365

Asn Ser Thr Gly Pro Gly Ser Pro Val Ser Gln Thr Leu Lys Thr Ser
    370                 375                 380

Leu Ser Gly Arg Pro Leu Asn Leu Trp His Ser Ser Pro Ile Gly His
385                 390                 395                 400

Lys Lys Asn Asp Lys Leu Leu Gly Val Glu Met Ser Ser Lys Pro Pro
                405                 410                 415

Ala Ile Asn Ser Arg Ser Val Leu Lys Glu Ser Asn Asn Asn Thr Ala
            420                 425                 430

Ser Thr Arg Leu Pro Pro Pro Leu Ala Asp Gly Leu Phe Phe Ser Arg
        435                 440                 445

Leu Glu Pro Pro Ala Gly Ala Glu Ser Lys Lys Ile Lys Arg Tyr Ala
    450                 455                 460

Phe Ser Gly Pro Ile Thr Ser Gln Pro Trp Ser Thr Lys Ala Val Ser
465                 470                 475                 480

Ala Glu His Pro Gln Leu Phe Ser Gly Pro Leu Leu Arg Asn Pro Thr
                485                 490                 495

Ala Gln Leu Phe Ser Ser Pro Pro Lys Val Ser Pro Arg Ile Ser Pro
            500                 505                 510

Lys Val Ser Pro Ser Ser Ser Pro Pro Phe Val Ser Ser Pro Lys Ile
        515                 520                 525

Ser Glu Leu His Glu Leu Pro Arg Pro Pro Val Ser Ser Thr Ser Lys
    530                 535                 540
```

```
Ser Pro Gly Ala Gly Gly Leu Val Gly His Ser Ala Pro Leu Leu Pro
545                 550                 555                 560

Lys Gly His Met Leu Pro Gly Thr Ser Lys Thr Ser Ala Ser Asn Val
                565                 570                 575

Glu Ser Gln Leu Pro Thr Pro Ser Gln Val Val Pro Arg Ser Phe Ser
            580                 585                 590

Ile Pro Ser Ser Arg His Arg Val Met Val Ala Gln Asn Pro Gly Ile
        595                 600                 605

Val Glu Asn Val Ala Ser Pro Pro Leu Thr Pro Ile Ser Leu Ser Asn
    610                 615                 620

Thr Gln Pro Ser Ser Thr Gly Ser Arg Ile Val Asn Gln Thr Val Gln
625                 630                 635                 640

Ile Arg Gly Ala Val
                645

<210>  3
<211>  1938
<212>  DNA
<213>  Populus.trichocarpa

<220>
<221>  CDS
<222>  (1)..(1938)

<400>  3
atg aaa tca ttt agg aaa ttg aga gga ttt gct tca ctt cac aaa caa       48
Met Lys Ser Phe Arg Lys Leu Arg Gly Phe Ala Ser Leu His Lys Gln
1               5                   10                  15

gta gta cat aaa aac cca agg gat ctt ccc tcc ttc tct caa tca cat       96
Val Val His Lys Asn Pro Arg Asp Leu Pro Ser Phe Ser Gln Ser His
            20                  25                  30

gag ctt gct aaa gcc tct cag gac atg aaa gat atg aaa gat tgc tat      144
Glu Leu Ala Lys Ala Ser Gln Asp Met Lys Asp Met Lys Asp Cys Tyr
        35                  40                  45

gat agc tta ctt tct gct gcc gct gga acc gcc aat tgt gcc ttt gaa      192
Asp Ser Leu Leu Ser Ala Ala Ala Gly Thr Ala Asn Cys Ala Phe Glu
    50                  55                  60

ttc tca gaa tca ctg cgt gaa atg ggt gct tgc ctt ctt gcg aaa act      240
Phe Ser Glu Ser Leu Arg Glu Met Gly Ala Cys Leu Leu Ala Lys Thr
65                  70                  75                  80

gca ttg aat gat gat gaa gaa agt ggc agg gtc ttg ctg atg ctg gga      288
Ala Leu Asn Asp Asp Glu Glu Ser Gly Arg Val Leu Leu Met Leu Gly
                85                  90                  95

aaa gtg caa ttt gaa ctc cag aaa ctt gtt gac tgc tat cgt tct cac      336
```

```
Lys Val Gln Phe Glu Leu Gln Lys Leu Val Asp Cys Tyr Arg Ser His
            100                 105                 110

ata aat cag aca atc ata agc ccg tca gag tct ctt ctg aat gaa ctt      384
Ile Asn Gln Thr Ile Ile Ser Pro Ser Glu Ser Leu Leu Asn Glu Leu
        115                 120                 125

caa ata gtt gag gag atg aag cag cag tgt gat gag aaa aga gat gta      432
Gln Ile Val Glu Glu Met Lys Gln Gln Cys Asp Glu Lys Arg Asp Val
    130                 135                 140

tat gct cac atg gcg aga cag aga gaa aga gtg agt gga aga aat gga      480
Tyr Ala His Met Ala Arg Gln Arg Glu Arg Val Ser Gly Arg Asn Gly
145                 150                 155                 160

aag gga gag tgt ttt tct atg cag caa ata caa gca gct cat gat gaa      528
Lys Gly Glu Cys Phe Ser Met Gln Gln Ile Gln Ala Ala His Asp Glu
                165                 170                 175

tat gat gag gag gcc act ttg ttt gtt ttt cgt ctt aaa tcc ctg aag      576
Tyr Asp Glu Glu Ala Thr Leu Phe Val Phe Arg Leu Lys Ser Leu Lys
            180                 185                 190

gaa gga caa tct cgc agt ctt ctt aca cag gcg gct cgg cac cat gct      624
Glu Gly Gln Ser Arg Ser Leu Leu Thr Gln Ala Ala Arg His His Ala
        195                 200                 205

gct cag ctc tgc ttc ttt aag aag gct ctt caa tct ctt gaa gct gtt      672
Ala Gln Leu Cys Phe Phe Lys Lys Ala Leu Gln Ser Leu Glu Ala Val
    210                 215                 220

gag ccg cat gtc aaa ttg gtc tca gaa cag cag cat ata gat tac cac      720
Glu Pro His Val Lys Leu Val Ser Glu Gln Gln His Ile Asp Tyr His
225                 230                 235                 240

ttt agt ggc ctt gat gat gat ggg agg gat tat gat gat gat gaa gat      768
Phe Ser Gly Leu Asp Asp Asp Gly Arg Asp Tyr Asp Asp Asp Glu Asp
                245                 250                 255

tat gat gat gcc att gat gat gga gaa ttg agc ttt gac tat gga caa      816
Tyr Asp Asp Ala Ile Asp Asp Gly Glu Leu Ser Phe Asp Tyr Gly Gln
            260                 265                 270

aat gac cag gag caa gaa gtt tcc aaa tca ata aag tca atg gag ttg      864
Asn Asp Gln Glu Gln Glu Val Ser Lys Ser Ile Lys Ser Met Glu Leu
        275                 280                 285

gat tta gag gac att aca ttt ccc caa gtt gtg aca ttg gaa atg gca      912
Asp Leu Glu Asp Ile Thr Phe Pro Gln Val Val Thr Leu Glu Met Ala
    290                 295                 300

aag gaa aat cca gat aca agt tac agg aca tcg ttt cct att aag gga      960
Lys Glu Asn Pro Asp Thr Ser Tyr Arg Thr Ser Phe Pro Ile Lys Gly
305                 310                 315                 320

gaa ctt agt gca ggc acc caa tca gct cca ctt ttt gct caa gtg aaa     1008
Glu Leu Ser Ala Gly Thr Gln Ser Ala Pro Leu Phe Ala Gln Val Lys
                325                 330                 335

tct aat cca gct gga aaa aca aaa caa ctg atg cca tca tcc acc cga     1056
Ser Asn Pro Ala Gly Lys Thr Lys Gln Leu Met Pro Ser Ser Thr Arg
            340                 345                 350

aag ttc aac act tat gtg ttg cct act cca gca gac cca aag agt tca     1104
Lys Phe Asn Thr Tyr Val Leu Pro Thr Pro Ala Asp Pro Lys Ser Ser
        355                 360                 365
```

```
aat tct aca gga cct ggt agc ccg gtt tct caa aca tta aag aca agt      1152
Asn Ser Thr Gly Pro Gly Ser Pro Val Ser Gln Thr Leu Lys Thr Ser
    370                 375                 380

ttg agt gga cgt cct ctg aat ttg tgg cac tca tcc cct att ggc cat      1200
Leu Ser Gly Arg Pro Leu Asn Leu Trp His Ser Ser Pro Ile Gly His
385                 390                 395                 400

aaa aaa aat gat aag tta cta gga gtt gaa atg tct agc aag ccc cct      1248
Lys Lys Asn Asp Lys Leu Leu Gly Val Glu Met Ser Ser Lys Pro Pro
                405                 410                 415

gct ata aat tcg cag tca gta ctc aag gag agc aac aac aac acg gca      1296
Ala Ile Asn Ser Gln Ser Val Leu Lys Glu Ser Asn Asn Asn Thr Ala
            420                 425                 430

tcc acc cga tta cct cct cct cta gct gat gga ctc ttt ttt tca agg      1344
Ser Thr Arg Leu Pro Pro Pro Leu Ala Asp Gly Leu Phe Phe Ser Arg
        435                 440                 445

ctt gag cca cca gct ggc gct gaa tct aag aaa atc aaa aga tat gcc      1392
Leu Glu Pro Pro Ala Gly Ala Glu Ser Lys Lys Ile Lys Arg Tyr Ala
    450                 455                 460

ttt tct ggt cct att tca tcc cag cca tgg tct acc aag gca gtc tcc      1440
Phe Ser Gly Pro Ile Ser Ser Gln Pro Trp Ser Thr Lys Ala Val Ser
465                 470                 475                 480

gca gaa cat cca caa ttg ttc tct gga ccc ctt ttg cga aat cca acg      1488
Ala Glu His Pro Gln Leu Phe Ser Gly Pro Leu Leu Arg Asn Pro Thr
                485                 490                 495

gcc caa tta ttt tca tca cct ccg aaa gtg tct cca aga att tca cca      1536
Ala Gln Leu Phe Ser Ser Pro Pro Lys Val Ser Pro Arg Ile Ser Pro
            500                 505                 510

aaa gta tct cct agt tct tcc cct cca ttt gtg tcc tca ccc aaa atc      1584
Lys Val Ser Pro Ser Ser Ser Pro Pro Phe Val Ser Ser Pro Lys Ile
        515                 520                 525

agc gag cta cat gag ctt ccc aga ccc cca gtc agt tca acc tcc aag      1632
Ser Glu Leu His Glu Leu Pro Arg Pro Pro Val Ser Ser Thr Ser Lys
    530                 535                 540

tct ccg ggg gct gga ggt ttg gtt ggt cat tca gct cca ctg ttg ccc      1680
Ser Pro Gly Ala Gly Gly Leu Val Gly His Ser Ala Pro Leu Leu Pro
545                 550                 555                 560

aaa ggc cac atg ctt cct ggt aca agc aaa acg tca gca tca aat gta      1728
Lys Gly His Met Leu Pro Gly Thr Ser Lys Thr Ser Ala Ser Asn Val
                565                 570                 575

gaa tct caa ctg cct aca cct tct caa gtt gtc cct cgc agt ttt tca      1776
Glu Ser Gln Leu Pro Thr Pro Ser Gln Val Val Pro Arg Ser Phe Ser
            580                 585                 590

ata ccc tct agc aga cat aga gta atg gtc gct cag aat tca ggg att      1824
Ile Pro Ser Ser Arg His Arg Val Met Val Ala Gln Asn Ser Gly Ile
        595                 600                 605

gtt gag aat gtt gcc tca cct cct cta acc cca ata tct tta tct aac      1872
Val Glu Asn Val Ala Ser Pro Pro Leu Thr Pro Ile Ser Leu Ser Asn
    610                 615                 620

acc cag cca tca tcc act ggt tcc cgg atc gtc aat cag aca gtt caa      1920
```

Thr Gln Pro Ser Ser Thr Gly Ser Arg Ile Val Asn Gln Thr Val Gln
625 630 635 640

atc aga ggt gca gtt tga 1938
Ile Arg Gly Ala Val
645

<210> 4
<211> 645
<212> PRT
<213> Populus.trichocarpa

<400> 4

Met Lys Ser Phe Arg Lys Leu Arg Gly Phe Ala Ser Leu His Lys Gln
1 5 10 15

Val Val His Lys Asn Pro Arg Asp Leu Pro Ser Phe Ser Gln Ser His
20 25 30

Glu Leu Ala Lys Ala Ser Gln Asp Met Lys Asp Met Lys Asp Cys Tyr
35 40 45

Asp Ser Leu Leu Ser Ala Ala Ala Gly Thr Ala Asn Cys Ala Phe Glu
50 55 60

Phe Ser Glu Ser Leu Arg Glu Met Gly Ala Cys Leu Leu Ala Lys Thr
65 70 75 80

Ala Leu Asn Asp Asp Glu Glu Ser Gly Arg Val Leu Leu Met Leu Gly
85 90 95

Lys Val Gln Phe Glu Leu Gln Lys Leu Val Asp Cys Tyr Arg Ser His
100 105 110

Ile Asn Gln Thr Ile Ile Ser Pro Ser Glu Ser Leu Leu Asn Glu Leu
115 120 125

Gln Ile Val Glu Glu Met Lys Gln Gln Cys Asp Glu Lys Arg Asp Val
130 135 140

Tyr Ala His Met Ala Arg Gln Arg Glu Arg Val Ser Gly Arg Asn Gly
145 150 155 160

Lys Gly Glu Cys Phe Ser Met Gln Gln Ile Gln Ala Ala His Asp Glu
165 170 175

Tyr Asp Glu Glu Ala Thr Leu Phe Val Phe Arg Leu Lys Ser Leu Lys
180 185 190

Glu Gly Gln Ser Arg Ser Leu Leu Thr Gln Ala Ala Arg His His Ala
195 200 205

```
Ala Gln Leu Cys Phe Phe Lys Lys Ala Leu Gln Ser Leu Glu Ala Val
    210                 215                 220

Glu Pro His Val Lys Leu Val Ser Glu Gln Gln His Ile Asp Tyr His
225                 230                 235                 240

Phe Ser Gly Leu Asp Asp Asp Gly Arg Asp Tyr Asp Asp Asp Glu Asp
                245                 250                 255

Tyr Asp Asp Ala Ile Asp Asp Gly Glu Leu Ser Phe Asp Tyr Gly Gln
            260                 265                 270

Asn Asp Gln Glu Gln Glu Val Ser Lys Ser Ile Lys Ser Met Glu Leu
        275                 280                 285

Asp Leu Glu Asp Ile Thr Phe Pro Gln Val Val Thr Leu Glu Met Ala
    290                 295                 300

Lys Glu Asn Pro Asp Thr Ser Tyr Arg Thr Ser Phe Pro Ile Lys Gly
305                 310                 315                 320

Glu Leu Ser Ala Gly Thr Gln Ser Ala Pro Leu Phe Ala Gln Val Lys
                325                 330                 335

Ser Asn Pro Ala Gly Lys Thr Lys Gln Leu Met Pro Ser Ser Thr Arg
            340                 345                 350

Lys Phe Asn Thr Tyr Val Leu Pro Thr Pro Ala Asp Pro Lys Ser Ser
        355                 360                 365

Asn Ser Thr Gly Pro Gly Ser Pro Val Ser Gln Thr Leu Lys Thr Ser
    370                 375                 380

Leu Ser Gly Arg Pro Leu Asn Leu Trp His Ser Ser Pro Ile Gly His
385                 390                 395                 400

Lys Lys Asn Asp Lys Leu Leu Gly Val Glu Met Ser Ser Lys Pro Pro
                405                 410                 415

Ala Ile Asn Ser Gln Ser Val Leu Lys Glu Ser Asn Asn Asn Thr Ala
            420                 425                 430

Ser Thr Arg Leu Pro Pro Pro Leu Ala Asp Gly Leu Phe Phe Ser Arg
        435                 440                 445

Leu Glu Pro Pro Ala Gly Ala Glu Ser Lys Lys Ile Lys Arg Tyr Ala
    450                 455                 460

Phe Ser Gly Pro Ile Ser Ser Gln Pro Trp Ser Thr Lys Ala Val Ser
```

465 470 475 480

Ala Glu His Pro Gln Leu Phe Ser Gly Pro Leu Leu Arg Asn Pro Thr
485 490 495

Ala Gln Leu Phe Ser Ser Pro Pro Lys Val Ser Pro Arg Ile Ser Pro
500 505 510

Lys Val Ser Pro Ser Ser Ser Pro Pro Phe Val Ser Ser Pro Lys Ile
515 520 525

Ser Glu Leu His Glu Leu Pro Arg Pro Pro Val Ser Ser Thr Ser Lys
530 535 540

Ser Pro Gly Ala Gly Gly Leu Val Gly His Ser Ala Pro Leu Leu Pro
545 550 555 560

Lys Gly His Met Leu Pro Gly Thr Ser Lys Thr Ser Ala Ser Asn Val
565 570 575

Glu Ser Gln Leu Pro Thr Pro Ser Gln Val Val Pro Arg Ser Phe Ser
580 585 590

Ile Pro Ser Ser Arg His Arg Val Met Val Ala Gln Asn Ser Gly Ile
595 600 605

Val Glu Asn Val Ala Ser Pro Pro Leu Thr Pro Ile Ser Leu Ser Asn
610 615 620

Thr Gln Pro Ser Ser Thr Gly Ser Arg Ile Val Asn Gln Thr Val Gln
625 630 635 640

Ile Arg Gly Ala Val
645

<210> 5
<211> 1872
<212> DNA
<213> Arabidopsis thaliana

<220>
<221> CDS
<222> (1)..(1872)

<400> 5
atg aaa acc tct ctc cga cgg tta cga ggt gtg ctt cac aag cat gaa 48
Met Lys Thr Ser Leu Arg Arg Leu Arg Gly Val Leu His Lys His Glu
1 5 10 15

tct aaa gac cgg aga gat ctt cga gct ctg gtt cag aaa gat gag ctt 96
Ser Lys Asp Arg Arg Asp Leu Arg Ala Leu Val Gln Lys Asp Glu Leu
20 25 30

```
gcc caa gct tct cag gac gta gaa gac atg aga gat tgc tat gat agc      144
Ala Gln Ala Ser Gln Asp Val Glu Asp Met Arg Asp Cys Tyr Asp Ser
        35                  40                  45

ttg ctc aat gcc gct gct gct act gcc aat agt gct tat gaa ttt tct      192
Leu Leu Asn Ala Ala Ala Ala Thr Ala Asn Ser Ala Tyr Glu Phe Ser
    50                  55                  60

gaa tct ttg cga gaa cta ggt gct tgt ctt ctt gag aaa act gcg cta      240
Glu Ser Leu Arg Glu Leu Gly Ala Cys Leu Leu Glu Lys Thr Ala Leu
65                  70                  75                  80

aat gat gat gaa gaa agt ggt aga gtg ttg att atg ctg gga aag ttg      288
Asn Asp Asp Glu Glu Ser Gly Arg Val Leu Ile Met Leu Gly Lys Leu
                85                  90                  95

cag ttt gaa ttg caa aaa ctt gtt gat aaa tat cgt tct cat att ttt      336
Gln Phe Glu Leu Gln Lys Leu Val Asp Lys Tyr Arg Ser His Ile Phe
            100                 105                 110

caa aca atc aca atc ccc tca gag tca ctt cta aat gaa ctc cgc ata      384
Gln Thr Ile Thr Ile Pro Ser Glu Ser Leu Leu Asn Glu Leu Arg Ile
        115                 120                 125

gtt gag gag atg cag cgg ctt tgt gat gag aaa agg aat gtt tat gaa      432
Val Glu Glu Met Gln Arg Leu Cys Asp Glu Lys Arg Asn Val Tyr Glu
    130                 135                 140

ggc atg ctt aca aga caa aga gag aaa ggg aga tca aag ggt ggg aaa      480
Gly Met Leu Thr Arg Gln Arg Glu Lys Gly Arg Ser Lys Gly Gly Lys
145                 150                 155                 160

gga gaa act ttt tcg cca cag caa cta caa gaa gct cat gat gat tat      528
Gly Glu Thr Phe Ser Pro Gln Gln Leu Gln Glu Ala His Asp Asp Tyr
                165                 170                 175

gaa aac gag acg act tta ttt gtt ttc cgt tta aaa tcc ctg aaa caa      576
Glu Asn Glu Thr Thr Leu Phe Val Phe Arg Leu Lys Ser Leu Lys Gln
            180                 185                 190

ggg caa aca cgt agt ctt ttg act cag gca gca agg cac cat gca gca      624
Gly Gln Thr Arg Ser Leu Leu Thr Gln Ala Ala Arg His His Ala Ala
        195                 200                 205

cag tta tgc ttc ttc aag aag gct ctt agt tcc ctt gaa gaa gtg gac      672
Gln Leu Cys Phe Phe Lys Lys Ala Leu Ser Ser Leu Glu Glu Val Asp
    210                 215                 220

cca cat gta cag atg gta acc gag tca cag cat att gat tac cat ttc      720
Pro His Val Gln Met Val Thr Glu Ser Gln His Ile Asp Tyr His Phe
225                 230                 235                 240

agc gga tta gaa gat gat gac ggg gat gat gaa att gaa aac aat gag      768
Ser Gly Leu Glu Asp Asp Asp Gly Asp Asp Glu Ile Glu Asn Asn Glu
                245                 250                 255

aac gat ggt tct gag gtg cat gat gat gga gag ttg agt ttt gaa tat      816
Asn Asp Gly Ser Glu Val His Asp Asp Gly Glu Leu Ser Phe Glu Tyr
            260                 265                 270

aga gtt aat gac aag gac caa gat gcg gat tct tca gct ggt ggc tcc      864
Arg Val Asn Asp Lys Asp Gln Asp Ala Asp Ser Ser Ala Gly Gly Ser
        275                 280                 285

tca gag ttg ggt aac tca gac atc aca ttt cca caa att gga gga cca      912
Ser Glu Leu Gly Asn Ser Asp Ile Thr Phe Pro Gln Ile Gly Gly Pro
```

```
    290                 295                 300

tat act gca cag gaa aat gag gaa gga aat tac aga aaa tct cat tcg      960
Tyr Thr Ala Gln Glu Asn Glu Glu Gly Asn Tyr Arg Lys Ser His Ser
305                 310                 315                 320

ttt agg aga gat gta agg gca gtg agc caa tca gca cca ctt ttt ccc     1008
Phe Arg Arg Asp Val Arg Ala Val Ser Gln Ser Ala Pro Leu Phe Pro
                325                 330                 335

gag aac cgc aca act cct cct tca gaa aag ctg tta cgg atg cga tca     1056
Glu Asn Arg Thr Thr Pro Pro Ser Glu Lys Leu Leu Arg Met Arg Ser
            340                 345                 350

act ctg aca cgg aaa ttc aac act tat gca ttg cca act cct gtg gaa     1104
Thr Leu Thr Arg Lys Phe Asn Thr Tyr Ala Leu Pro Thr Pro Val Glu
        355                 360                 365

aca acg aga agt ccc tca tct act aca agt cca ggt cac aaa aac gtg     1152
Thr Thr Arg Ser Pro Ser Ser Thr Thr Ser Pro Gly His Lys Asn Val
    370                 375                 380

ggg tca tct aat cct aca aag gca att aca aag cag att tgg tat tca     1200
Gly Ser Ser Asn Pro Thr Lys Ala Ile Thr Lys Gln Ile Trp Tyr Ser
385                 390                 395                 400

tcc cca ctt gaa act cgc gga cct gca aag gtt tcc tca aga tca atg     1248
Ser Pro Leu Glu Thr Arg Gly Pro Ala Lys Val Ser Ser Arg Ser Met
                405                 410                 415

gta gcc ttg aaa gaa caa gtc ctg aga gag agt aac aag aat aca tct     1296
Val Ala Leu Lys Glu Gln Val Leu Arg Glu Ser Asn Lys Asn Thr Ser
            420                 425                 430

cgg ttg cct ccg cct tta gca gat gga ctc ttg ttc tct cgt ctc ggt     1344
Arg Leu Pro Pro Pro Leu Ala Asp Gly Leu Leu Phe Ser Arg Leu Gly
        435                 440                 445

aca ctc aag aga cga tcc ttc tct ggt cca ctg aca agt aaa ccg tta     1392
Thr Leu Lys Arg Arg Ser Phe Ser Gly Pro Leu Thr Ser Lys Pro Leu
    450                 455                 460

ccg aac aag cct ctc tct aca aca tct cac tta tac tct ggt ccg atc     1440
Pro Asn Lys Pro Leu Ser Thr Thr Ser His Leu Tyr Ser Gly Pro Ile
465                 470                 475                 480

cca agg aat cca gtt tct aaa ttg cca aaa gtg tca tca tct cca act     1488
Pro Arg Asn Pro Val Ser Lys Leu Pro Lys Val Ser Ser Ser Pro Thr
                485                 490                 495

gct tcc cct aca ttt gtc tca acc cca aaa ata agt gag ctc cat gag     1536
Ala Ser Pro Thr Phe Val Ser Thr Pro Lys Ile Ser Glu Leu His Glu
            500                 505                 510

ctt cct aga cca cca cca aga agc tct act aag tcc tct agg gaa ttg     1584
Leu Pro Arg Pro Pro Pro Arg Ser Ser Thr Lys Ser Ser Arg Glu Leu
        515                 520                 525

gga tat tca gct ccc ttg gtt tcg agg agc caa ttg ctt agt aaa cct     1632
Gly Tyr Ser Ala Pro Leu Val Ser Arg Ser Gln Leu Leu Ser Lys Pro
    530                 535                 540

ctt att aca aat tca gct tct cct ctt cct ata cca cct gca atc act     1680
Leu Ile Thr Asn Ser Ala Ser Pro Leu Pro Ile Pro Pro Ala Ile Thr
545                 550                 555                 560
```

```
cgt agc ttc tct ata cct act agc aac ctt aga gca tca gat tta gat      1728
Arg Ser Phe Ser Ile Pro Thr Ser Asn Leu Arg Ala Ser Asp Leu Asp
                565                 570                 575

atg tct aag aca agt ctg ggc act aaa aag tta ggc act ccc tcg cct      1776
Met Ser Lys Thr Ser Leu Gly Thr Lys Lys Leu Gly Thr Pro Ser Pro
            580                 585                 590

cct tta acc cca atg tca ttg atc cat cca ccg cca cag gct ctt ccc      1824
Pro Leu Thr Pro Met Ser Leu Ile His Pro Pro Pro Gln Ala Leu Pro
        595                 600                 605

gaa cgt gca gac cac cta atg atg tcc aaa caa gag agg agg att taa      1872
Glu Arg Ala Asp His Leu Met Met Ser Lys Gln Glu Arg Arg Ile
    610                 615                 620


<210>  6
<211>  623
<212>  PRT
<213>  Arabidopsis thaliana

<400>  6

Met Lys Thr Ser Leu Arg Arg Leu Arg Gly Val Leu His Lys His Glu
1               5                   10                  15


Ser Lys Asp Arg Arg Asp Leu Arg Ala Leu Val Gln Lys Asp Glu Leu
            20                  25                  30


Ala Gln Ala Ser Gln Asp Val Glu Asp Met Arg Asp Cys Tyr Asp Ser
        35                  40                  45


Leu Leu Asn Ala Ala Ala Ala Thr Ala Asn Ser Ala Tyr Glu Phe Ser
    50                  55                  60


Glu Ser Leu Arg Glu Leu Gly Ala Cys Leu Leu Glu Lys Thr Ala Leu
65                  70                  75                  80


Asn Asp Asp Glu Glu Ser Gly Arg Val Leu Ile Met Leu Gly Lys Leu
                85                  90                  95


Gln Phe Glu Leu Gln Lys Leu Val Asp Lys Tyr Arg Ser His Ile Phe
            100                 105                 110


Gln Thr Ile Thr Ile Pro Ser Glu Ser Leu Leu Asn Glu Leu Arg Ile
        115                 120                 125


Val Glu Glu Met Gln Arg Leu Cys Asp Glu Lys Arg Asn Val Tyr Glu
    130                 135                 140


Gly Met Leu Thr Arg Gln Arg Glu Lys Gly Arg Ser Lys Gly Gly Lys
145                 150                 155                 160


Gly Glu Thr Phe Ser Pro Gln Gln Leu Gln Glu Ala His Asp Asp Tyr
                165                 170                 175
```

```
Glu Asn Glu Thr Thr Leu Phe Val Phe Arg Leu Lys Ser Leu Lys Gln
            180                 185                 190

Gly Gln Thr Arg Ser Leu Leu Thr Gln Ala Ala Arg His His Ala Ala
        195                 200                 205

Gln Leu Cys Phe Phe Lys Lys Ala Leu Ser Ser Leu Glu Glu Val Asp
    210                 215                 220

Pro His Val Gln Met Val Thr Glu Ser Gln His Ile Asp Tyr His Phe
225                 230                 235                 240

Ser Gly Leu Glu Asp Asp Asp Gly Asp Asp Glu Ile Glu Asn Asn Glu
                245                 250                 255

Asn Asp Gly Ser Glu Val His Asp Asp Gly Glu Leu Ser Phe Glu Tyr
            260                 265                 270

Arg Val Asn Asp Lys Asp Gln Asp Ala Asp Ser Ser Ala Gly Gly Ser
        275                 280                 285

Ser Glu Leu Gly Asn Ser Asp Ile Thr Phe Pro Gln Ile Gly Gly Pro
    290                 295                 300

Tyr Thr Ala Gln Glu Asn Glu Glu Gly Asn Tyr Arg Lys Ser His Ser
305                 310                 315                 320

Phe Arg Arg Asp Val Arg Ala Val Ser Gln Ser Ala Pro Leu Phe Pro
                325                 330                 335

Glu Asn Arg Thr Thr Pro Pro Ser Glu Lys Leu Leu Arg Met Arg Ser
            340                 345                 350

Thr Leu Thr Arg Lys Phe Asn Thr Tyr Ala Leu Pro Thr Pro Val Glu
        355                 360                 365

Thr Thr Arg Ser Pro Ser Ser Thr Thr Ser Pro Gly His Lys Asn Val
    370                 375                 380

Gly Ser Ser Asn Pro Thr Lys Ala Ile Thr Lys Gln Ile Trp Tyr Ser
385                 390                 395                 400

Ser Pro Leu Glu Thr Arg Gly Pro Ala Lys Val Ser Ser Arg Ser Met
                405                 410                 415

Val Ala Leu Lys Glu Gln Val Leu Arg Glu Ser Asn Lys Asn Thr Ser
            420                 425                 430
```

```
Arg Leu Pro Pro Pro Leu Ala Asp Gly Leu Leu Phe Ser Arg Leu Gly
        435                 440                 445

Thr Leu Lys Arg Arg Ser Phe Ser Gly Pro Leu Thr Ser Lys Pro Leu
    450                 455                 460

Pro Asn Lys Pro Leu Ser Thr Thr Ser His Leu Tyr Ser Gly Pro Ile
465                 470                 475                 480

Pro Arg Asn Pro Val Ser Lys Leu Pro Lys Val Ser Ser Ser Pro Thr
                485                 490                 495

Ala Ser Pro Thr Phe Val Ser Thr Pro Lys Ile Ser Glu Leu His Glu
            500                 505                 510

Leu Pro Arg Pro Pro Pro Arg Ser Ser Thr Lys Ser Ser Arg Glu Leu
        515                 520                 525

Gly Tyr Ser Ala Pro Leu Val Ser Arg Ser Gln Leu Leu Ser Lys Pro
    530                 535                 540

Leu Ile Thr Asn Ser Ala Ser Pro Leu Pro Ile Pro Pro Ala Ile Thr
545                 550                 555                 560

Arg Ser Phe Ser Ile Pro Thr Ser Asn Leu Arg Ala Ser Asp Leu Asp
                565                 570                 575

Met Ser Lys Thr Ser Leu Gly Thr Lys Lys Leu Gly Thr Pro Ser Pro
            580                 585                 590

Pro Leu Thr Pro Met Ser Leu Ile His Pro Pro Pro Gln Ala Leu Pro
        595                 600                 605

Glu Arg Ala Asp His Leu Met Met Ser Lys Gln Glu Arg Arg Ile
    610                 615                 620

<210>  7
<211>  1827
<212>  DNA
<213>  Arabidopsis thaliana

<220>
<221>  CDS
<222>  (1)..(1827)

<400>  7
atg aaa gct tcg att gag aag tta agg aga tta aca tcg cat tca cat      48
Met Lys Ala Ser Ile Glu Lys Leu Arg Arg Leu Thr Ser His Ser His
1               5                   10                  15

aag gtt gat gtg aag gag aaa gga gat gtc atg gct aca aca caa atc      96
Lys Val Asp Val Lys Glu Lys Gly Asp Val Met Ala Thr Thr Gln Ile
            20                  25                  30
```

```
gac gaa ctc gat cga gcc ggg aag gat atg caa gat atg aga gaa tgt      144
Asp Glu Leu Asp Arg Ala Gly Lys Asp Met Gln Asp Met Arg Glu Cys
        35                  40                  45

tac gat aga cta ctc gct gca gct gct gcc acg gca aat agc gca tat      192
Tyr Asp Arg Leu Leu Ala Ala Ala Ala Ala Thr Ala Asn Ser Ala Tyr
    50                  55                  60

gag ttc tct gag tcg ttg gga gaa atg ggt tct tgt ttg gag caa atc      240
Glu Phe Ser Glu Ser Leu Gly Glu Met Gly Ser Cys Leu Glu Gln Ile
65                  70                  75                  80

gcg cct cat aac gac gaa gag agc agt aga atc ttg ttt atg ttg ggt      288
Ala Pro His Asn Asp Glu Glu Ser Ser Arg Ile Leu Phe Met Leu Gly
                85                  90                  95

aaa gta cag agt gag ctt caa aga ctt ctt gac aca tat cgt agt cat      336
Lys Val Gln Ser Glu Leu Gln Arg Leu Leu Asp Thr Tyr Arg Ser His
            100                 105                 110

ata ttc gaa act att aca tcc ccg tcc gag gcg ctt ctc aag gac ctc      384
Ile Phe Glu Thr Ile Thr Ser Pro Ser Glu Ala Leu Leu Lys Asp Leu
        115                 120                 125

aga tat gtt gag gat atg aag caa caa tgc gac ggg aag agg aat gtg      432
Arg Tyr Val Glu Asp Met Lys Gln Gln Cys Asp Gly Lys Arg Asn Val
    130                 135                 140

tat gag atg tcg cta gtg aaa gag aaa gga agg cct aaa agc agt aaa      480
Tyr Glu Met Ser Leu Val Lys Glu Lys Gly Arg Pro Lys Ser Ser Lys
145                 150                 155                 160

gga gag aga cat att cct cct gag tct cga cct gct tac agt gag ttt      528
Gly Glu Arg His Ile Pro Pro Glu Ser Arg Pro Ala Tyr Ser Glu Phe
                165                 170                 175

cat gat gaa gcc aca atg tgc att ttt cga ttg aaa tcg ctt aaa gaa      576
His Asp Glu Ala Thr Met Cys Ile Phe Arg Leu Lys Ser Leu Lys Glu
            180                 185                 190

gga caa gct cgt agt ctc cta ata caa gca gtc cgt cac cac act gct      624
Gly Gln Ala Arg Ser Leu Leu Ile Gln Ala Val Arg His His Thr Ala
        195                 200                 205

cag atg cgt ttg ttt cac act gga ctg aaa tcg ctc gag gca gtt gag      672
Gln Met Arg Leu Phe His Thr Gly Leu Lys Ser Leu Glu Ala Val Glu
    210                 215                 220

cgt cat gta aaa gtt gct gta gag aaa caa cac att gac tgt gat cta      720
Arg His Val Lys Val Ala Val Glu Lys Gln His Ile Asp Cys Asp Leu
225                 230                 235                 240

tct gtt cat ggg aac gag atg gaa gct agc gag gat gat gat gat gat      768
Ser Val His Gly Asn Glu Met Glu Ala Ser Glu Asp Asp Asp Asp Asp
                245                 250                 255

ggc cga tac atg aat aga gaa gga gaa ctc agt ttt gat tac aga aca      816
Gly Arg Tyr Met Asn Arg Glu Gly Glu Leu Ser Phe Asp Tyr Arg Thr
            260                 265                 270

aat gag cag aag gta gaa gct tct tct ctc tct aca cca tgg gcc aca      864
Asn Glu Gln Lys Val Glu Ala Ser Ser Leu Ser Thr Pro Trp Ala Thr
        275                 280                 285

aag atg gat gat aca gac ctc tcg ttt cct cgc cct tct aca aca aga      912
```

Lys Met Asp Asp Thr Asp Leu Ser Phe Pro Arg Pro Ser Thr Thr Arg
290 295 300

cca gca gcg gta aat gct gat cat aga gaa gaa tat cca gtt tca acc 960
Pro Ala Ala Val Asn Ala Asp His Arg Glu Glu Tyr Pro Val Ser Thr
305 310 315 320

cgc gat aag tat ttg agc agc cat tca gct ccg ttg ttc cca gaa aag 1008
Arg Asp Lys Tyr Leu Ser Ser His Ser Ala Pro Leu Phe Pro Glu Lys
325 330 335

aaa cct gat gta tca gag agg ttg aga cag gcg aat cca tct ttt aat 1056
Lys Pro Asp Val Ser Glu Arg Leu Arg Gln Ala Asn Pro Ser Phe Asn
340 345 350

gcc tac gta tta cca aca cca aat gat tca agg tac tca aaa ccg gtt 1104
Ala Tyr Val Leu Pro Thr Pro Asn Asp Ser Arg Tyr Ser Lys Pro Val
355 360 365

tcc caa gca tta aat ccg agg cca aca aac cac agt gcc gga aac ata 1152
Ser Gln Ala Leu Asn Pro Arg Pro Thr Asn His Ser Ala Gly Asn Ile
370 375 380

tgg cat tca tct ccg tta gag ccg ata aaa agc ggg aaa gat ggg aaa 1200
Trp His Ser Ser Pro Leu Glu Pro Ile Lys Ser Gly Lys Asp Gly Lys
385 390 395 400

gac gcc gaa agc aac agc ttc tac ggc cgc ctc cct cgg cct tct aca 1248
Asp Ala Glu Ser Asn Ser Phe Tyr Gly Arg Leu Pro Arg Pro Ser Thr
405 410 415

aca gac acg cat cat cat cag cag caa gca gca gga aga cat gca ttt 1296
Thr Asp Thr His His His Gln Gln Gln Ala Ala Gly Arg His Ala Phe
420 425 430

tct gga cct ctc aga ccg tcc tca aca aaa ccc atc acc atg gct gac 1344
Ser Gly Pro Leu Arg Pro Ser Ser Thr Lys Pro Ile Thr Met Ala Asp
435 440 445

agt tat tca ggc gct ttt tgt cct ctg ccg act cct cca gta ctc caa 1392
Ser Tyr Ser Gly Ala Phe Cys Pro Leu Pro Thr Pro Pro Val Leu Gln
450 455 460

tct cac cct cat tca tca tct tct cca aga gtc tcc cct acc gct tca 1440
Ser His Pro His Ser Ser Ser Ser Pro Arg Val Ser Pro Thr Ala Ser
465 470 475 480

cct cct cct gct tct tcc cca agg ctc aac gag ctt cac gag ctt cca 1488
Pro Pro Pro Ala Ser Ser Pro Arg Leu Asn Glu Leu His Glu Leu Pro
485 490 495

aga ccc cca ggc cac ttt gca cca cct cca aga cga gcc aag tcc cct 1536
Arg Pro Pro Gly His Phe Ala Pro Pro Pro Arg Arg Ala Lys Ser Pro
500 505 510

ggt ctg gtt ggt cac tca gcg cct cta acc gca tgg aac caa gaa aga 1584
Gly Leu Val Gly His Ser Ala Pro Leu Thr Ala Trp Asn Gln Glu Arg
515 520 525

agc act gtc act gtt gct gtt ccg tcc gcc acc aac att gtg gcc tcg 1632
Ser Thr Val Thr Val Ala Val Pro Ser Ala Thr Asn Ile Val Ala Ser
530 535 540

ccg ctt ccg gtt cct ccg ttg gtt gtc cct aga agc tac tct ata cct 1680
Pro Leu Pro Val Pro Pro Leu Val Val Pro Arg Ser Tyr Ser Ile Pro
545 550 555 560

```
tca aga aac cag aga gtt gtt tct caa cgg ctg gtc gaa agg aga gat      1728
Ser Arg Asn Gln Arg Val Val Ser Gln Arg Leu Val Glu Arg Arg Asp
                565                 570                 575

gat ata gta gca tcc cca ccg tta aca cca atg agc ctg tct agg cca      1776
Asp Ile Val Ala Ser Pro Pro Leu Thr Pro Met Ser Leu Ser Arg Pro
            580                 585                 590

ctt cct caa gcc aca gga gtt gct cag acc agt caa atc aga ggt tat      1824
Leu Pro Gln Ala Thr Gly Val Ala Gln Thr Ser Gln Ile Arg Gly Tyr
        595                 600                 605

taa                                                                  1827


<210>  8
<211>  608
<212>  PRT
<213>  Arabidopsis thaliana

<400>  8

Met Lys Ala Ser Ile Glu Lys Leu Arg Arg Leu Thr Ser His Ser His
1               5                   10                  15

Lys Val Asp Val Lys Glu Lys Gly Asp Val Met Ala Thr Thr Gln Ile
            20                  25                  30

Asp Glu Leu Asp Arg Ala Gly Lys Asp Met Gln Asp Met Arg Glu Cys
        35                  40                  45

Tyr Asp Arg Leu Leu Ala Ala Ala Ala Ala Thr Ala Asn Ser Ala Tyr
    50                  55                  60

Glu Phe Ser Glu Ser Leu Gly Glu Met Gly Ser Cys Leu Glu Gln Ile
65                  70                  75                  80

Ala Pro His Asn Asp Glu Glu Ser Ser Arg Ile Leu Phe Met Leu Gly
                85                  90                  95

Lys Val Gln Ser Glu Leu Gln Arg Leu Leu Asp Thr Tyr Arg Ser His
            100                 105                 110

Ile Phe Glu Thr Ile Thr Ser Pro Ser Glu Ala Leu Leu Lys Asp Leu
        115                 120                 125

Arg Tyr Val Glu Asp Met Lys Gln Gln Cys Asp Gly Lys Arg Asn Val
    130                 135                 140

Tyr Glu Met Ser Leu Val Lys Glu Lys Gly Arg Pro Lys Ser Ser Lys
145                 150                 155                 160

Gly Glu Arg His Ile Pro Pro Glu Ser Arg Pro Ala Tyr Ser Glu Phe
                165                 170                 175
```

```
His Asp Glu Ala Thr Met Cys Ile Phe Arg Leu Lys Ser Leu Lys Glu
            180                 185                 190

Gly Gln Ala Arg Ser Leu Leu Ile Gln Ala Val Arg His His Thr Ala
        195                 200                 205

Gln Met Arg Leu Phe His Thr Gly Leu Lys Ser Leu Glu Ala Val Glu
    210                 215                 220

Arg His Val Lys Val Ala Val Glu Lys Gln His Ile Asp Cys Asp Leu
225                 230                 235                 240

Ser Val His Gly Asn Glu Met Glu Ala Ser Glu Asp Asp Asp Asp Asp
                245                 250                 255

Gly Arg Tyr Met Asn Arg Glu Gly Glu Leu Ser Phe Asp Tyr Arg Thr
            260                 265                 270

Asn Glu Gln Lys Val Glu Ala Ser Ser Leu Ser Thr Pro Trp Ala Thr
        275                 280                 285

Lys Met Asp Asp Thr Asp Leu Ser Phe Pro Arg Pro Ser Thr Thr Arg
    290                 295                 300

Pro Ala Ala Val Asn Ala Asp His Arg Glu Glu Tyr Pro Val Ser Thr
305                 310                 315                 320

Arg Asp Lys Tyr Leu Ser Ser His Ser Ala Pro Leu Phe Pro Glu Lys
                325                 330                 335

Lys Pro Asp Val Ser Glu Arg Leu Arg Gln Ala Asn Pro Ser Phe Asn
            340                 345                 350

Ala Tyr Val Leu Pro Thr Pro Asn Asp Ser Arg Tyr Ser Lys Pro Val
        355                 360                 365

Ser Gln Ala Leu Asn Pro Arg Pro Thr Asn His Ser Ala Gly Asn Ile
    370                 375                 380

Trp His Ser Ser Pro Leu Glu Pro Ile Lys Ser Gly Lys Asp Gly Lys
385                 390                 395                 400

Asp Ala Glu Ser Asn Ser Phe Tyr Gly Arg Leu Pro Arg Pro Ser Thr
                405                 410                 415

Thr Asp Thr His His His Gln Gln Gln Ala Ala Gly Arg His Ala Phe
            420                 425                 430

Ser Gly Pro Leu Arg Pro Ser Ser Thr Lys Pro Ile Thr Met Ala Asp
```

```
        435                 440                 445

Ser Tyr Ser Gly Ala Phe Cys Pro Leu Pro Thr Pro Pro Val Leu Gln
    450                 455                 460

Ser His Pro His Ser Ser Ser Ser Pro Arg Val Ser Pro Thr Ala Ser
465                 470                 475                 480

Pro Pro Pro Ala Ser Ser Pro Arg Leu Asn Glu Leu His Glu Leu Pro
                485                 490                 495

Arg Pro Pro Gly His Phe Ala Pro Pro Pro Arg Arg Ala Lys Ser Pro
            500                 505                 510

Gly Leu Val Gly His Ser Ala Pro Leu Thr Ala Trp Asn Gln Glu Arg
        515                 520                 525

Ser Thr Val Thr Val Ala Val Pro Ser Ala Thr Asn Ile Val Ala Ser
    530                 535                 540

Pro Leu Pro Val Pro Pro Leu Val Val Pro Arg Ser Tyr Ser Ile Pro
545                 550                 555                 560

Ser Arg Asn Gln Arg Val Val Ser Gln Arg Leu Val Glu Arg Arg Asp
                565                 570                 575

Asp Ile Val Ala Ser Pro Pro Leu Thr Pro Met Ser Leu Ser Arg Pro
            580                 585                 590

Leu Pro Gln Ala Thr Gly Val Ala Gln Thr Ser Gln Ile Arg Gly Tyr
        595                 600                 605


<210>  9
<211>  1761
<212>  DNA
<213>  A.thaliana


<220>
<221>  CDS
<222>  (1)..(1761)

<400>  9
atg atg aaa gct tcg ttt gga agg tta aga aga ttc gca tta ccc aaa         48
Met Met Lys Ala Ser Phe Gly Arg Leu Arg Arg Phe Ala Leu Pro Lys
1               5                   10                  15

gcc gat gcg att gat ata gga gag ctt ttt ccc act gca caa att gaa         96
Ala Asp Ala Ile Asp Ile Gly Glu Leu Phe Pro Thr Ala Gln Ile Glu
            20                  25                  30

ggt ctt gct cga gcc gcc aag gat atg caa gat atg aga gag ggt tat        144
Gly Leu Ala Arg Ala Ala Lys Asp Met Gln Asp Met Arg Glu Gly Tyr
        35                  40                  45
```

```
gat aga tta ctc gaa gta gct gct gct atg gct aat agc gca tat gag      192
Asp Arg Leu Leu Glu Val Ala Ala Ala Met Ala Asn Ser Ala Tyr Glu
    50                  55                  60

ttc tct gaa tca ttg ggg gaa atg ggc tca tgc ttg gag caa atc gcg      240
Phe Ser Glu Ser Leu Gly Glu Met Gly Ser Cys Leu Glu Gln Ile Ala
65                  70                  75                  80

cct cat aat gat caa gaa agt ggt gga att ctt cta atg tta ggt aaa      288
Pro His Asn Asp Gln Glu Ser Gly Gly Ile Leu Leu Met Leu Gly Lys
                85                  90                  95

gtt cag ttt gag ctt aag aaa ctc gtc gac act tat cgt tct caa ata      336
Val Gln Phe Glu Leu Lys Lys Leu Val Asp Thr Tyr Arg Ser Gln Ile
            100                 105                 110

ttc aag acc att aca cga cct tca gag tca ctt ctc agt gac ctt aga      384
Phe Lys Thr Ile Thr Arg Pro Ser Glu Ser Leu Leu Ser Asp Leu Arg
        115                 120                 125

act gtt gag gat atg aag caa caa tgt gaa gaa aag aga gac gtt gtt      432
Thr Val Glu Asp Met Lys Gln Gln Cys Glu Glu Lys Arg Asp Val Val
    130                 135                 140

aag cac atg cta atg gag cat gtg aaa gac aag gta caa gtt aaa ggc      480
Lys His Met Leu Met Glu His Val Lys Asp Lys Val Gln Val Lys Gly
145                 150                 155                 160

act aaa ggg gag aga ctt att cgc cgt cag cta gag act gct cgc gat      528
Thr Lys Gly Glu Arg Leu Ile Arg Arg Gln Leu Glu Thr Ala Arg Asp
                165                 170                 175

gag cta caa gat gag gcg act ctg tgc att ttc cga ttg aaa tct ctc      576
Glu Leu Gln Asp Glu Ala Thr Leu Cys Ile Phe Arg Leu Lys Ser Leu
            180                 185                 190

aag gaa ggg caa gct cga agt ctc ctc aca caa gca gcc cgc cac cac      624
Lys Glu Gly Gln Ala Arg Ser Leu Leu Thr Gln Ala Ala Arg His His
        195                 200                 205

act gct cag atg cat atg ttc ttt gct ggt ctg aaa tcg ctt gag gca      672
Thr Ala Gln Met His Met Phe Phe Ala Gly Leu Lys Ser Leu Glu Ala
    210                 215                 220

gta gag caa cac gtc aga att gct gca gat aga caa cac atc gac tgt      720
Val Glu Gln His Val Arg Ile Ala Ala Asp Arg Gln His Ile Asp Cys
225                 230                 235                 240

gtg ctc tct gat ccc ggg aac gaa atg gat tgt agt gag gat aat gat      768
Val Leu Ser Asp Pro Gly Asn Glu Met Asp Cys Ser Glu Asp Asn Asp
                245                 250                 255

gat gat gac cga ctt gtt aat aga gat gga gaa ctc agt ttt gac tac      816
Asp Asp Asp Arg Leu Val Asn Arg Asp Gly Glu Leu Ser Phe Asp Tyr
            260                 265                 270

ata aca agt gag cag aga gta gaa gtt ata tct aca cct cat gga tcg      864
Ile Thr Ser Glu Gln Arg Val Glu Val Ile Ser Thr Pro His Gly Ser
        275                 280                 285

atg aag atg gat gac aca gat ctc tcg ttt caa cgc cct tca cct gca      912
Met Lys Met Asp Asp Thr Asp Leu Ser Phe Gln Arg Pro Ser Pro Ala
    290                 295                 300

gga tca gca aca gta aat gca gac cct aga gaa gag cac tca gtt tca      960
Gly Ser Ala Thr Val Asn Ala Asp Pro Arg Glu Glu His Ser Val Ser
```

```
305                 310                 315                 320

aac cgt gat cgc aga acg agc agc cat tca gca cct ctg ttt ccg gat      1008
Asn Arg Asp Arg Arg Thr Ser Ser His Ser Ala Pro Leu Phe Pro Asp
                325                 330                 335

aag aaa gct gat tta gca gat aga tcg atg agg cag atg act cca tct      1056
Lys Lys Ala Asp Leu Ala Asp Arg Ser Met Arg Gln Met Thr Pro Ser
            340                 345                 350

gca aat gct tac ata tta cca act ccc gtc gac tca aag tcc tca cca      1104
Ala Asn Ala Tyr Ile Leu Pro Thr Pro Val Asp Ser Lys Ser Ser Pro
        355                 360                 365

atc ttc aca aaa cct gtc acc cag aca aac cac agt gca aac tta tgg      1152
Ile Phe Thr Lys Pro Val Thr Gln Thr Asn His Ser Ala Asn Leu Trp
    370                 375                 380

cat tca tct cca cta gaa cca ata aaa acc gcc cat aaa gat gcg gaa      1200
His Ser Ser Pro Leu Glu Pro Ile Lys Thr Ala His Lys Asp Ala Glu
385                 390                 395                 400

agc aac ctc tat tcc cgt ctt ccc cgt cct tca gaa cac gca ttt tct      1248
Ser Asn Leu Tyr Ser Arg Leu Pro Arg Pro Ser Glu His Ala Phe Ser
                405                 410                 415

gga cca ctc aag cca tcc tca acc cgt ctt cca gta cca gtt gca gtt      1296
Gly Pro Leu Lys Pro Ser Ser Thr Arg Leu Pro Val Pro Val Ala Val
            420                 425                 430

cag gct cag tca tct tct ccc aga ata tct cct acc gct tca ccg cct      1344
Gln Ala Gln Ser Ser Ser Pro Arg Ile Ser Pro Thr Ala Ser Pro Pro
        435                 440                 445

ctt gct tct tcc cca cga atc aac gaa ctc cat gag ctt cca aga cca      1392
Leu Ala Ser Ser Pro Arg Ile Asn Glu Leu His Glu Leu Pro Arg Pro
    450                 455                 460

cca ggt caa ttt gca ccg cca cga cgc tcg aaa tct cct ggt ttg gtt      1440
Pro Gly Gln Phe Ala Pro Pro Arg Arg Ser Lys Ser Pro Gly Leu Val
465                 470                 475                 480

ggt cat tcg gct cca tta acg gct tgg aat caa gaa aga agc aat gta      1488
Gly His Ser Ala Pro Leu Thr Ala Trp Asn Gln Glu Arg Ser Asn Val
                485                 490                 495

gta gtg tca acc aac att gta gca tca cca ctt ccg gtt cca ccg ctt      1536
Val Val Ser Thr Asn Ile Val Ala Ser Pro Leu Pro Val Pro Pro Leu
            500                 505                 510

gtt gta ccg aga agc tac tcg ata cct tct aga aac cag aga gca atg      1584
Val Val Pro Arg Ser Tyr Ser Ile Pro Ser Arg Asn Gln Arg Ala Met
        515                 520                 525

gcc caa caa ccc ttg ccc gag agg aac caa aac aga gta gcc tct cca      1632
Ala Gln Gln Pro Leu Pro Glu Arg Asn Gln Asn Arg Val Ala Ser Pro
    530                 535                 540

ccg cct ctt ccg ctg act cca gcg tct ctg atg aat ctc aga tcg ctg      1680
Pro Pro Leu Pro Leu Thr Pro Ala Ser Leu Met Asn Leu Arg Ser Leu
545                 550                 555                 560

tct cgg tct cat gtc ggg gaa gtt gct cag agc gga cta att aga ggt      1728
Ser Arg Ser His Val Gly Glu Val Ala Gln Ser Gly Leu Ile Arg Gly
                565                 570                 575
```

```
aac gga aac aaa act aac tta ctg tat ttg taa                          1761
Asn Gly Asn Lys Thr Asn Leu Leu Tyr Leu
            580                 585


<210>  10
<211>  586
<212>  PRT
<213>  A.thaliana

<400>  10

Met Met Lys Ala Ser Phe Gly Arg Leu Arg Arg Phe Ala Leu Pro Lys
1               5                   10                  15


Ala Asp Ala Ile Asp Ile Gly Glu Leu Phe Pro Thr Ala Gln Ile Glu
            20                  25                  30


Gly Leu Ala Arg Ala Ala Lys Asp Met Gln Asp Met Arg Glu Gly Tyr
        35                  40                  45


Asp Arg Leu Leu Glu Val Ala Ala Ala Met Ala Asn Ser Ala Tyr Glu
    50                  55                  60


Phe Ser Glu Ser Leu Gly Glu Met Gly Ser Cys Leu Glu Gln Ile Ala
65                  70                  75                  80


Pro His Asn Asp Gln Glu Ser Gly Gly Ile Leu Leu Met Leu Gly Lys
                85                  90                  95


Val Gln Phe Glu Leu Lys Lys Leu Val Asp Thr Tyr Arg Ser Gln Ile
            100                 105                 110


Phe Lys Thr Ile Thr Arg Pro Ser Glu Ser Leu Leu Ser Asp Leu Arg
        115                 120                 125


Thr Val Glu Asp Met Lys Gln Gln Cys Glu Glu Lys Arg Asp Val Val
    130                 135                 140


Lys His Met Leu Met Glu His Val Lys Asp Lys Val Gln Val Lys Gly
145                 150                 155                 160


Thr Lys Gly Glu Arg Leu Ile Arg Arg Gln Leu Glu Thr Ala Arg Asp
                165                 170                 175


Glu Leu Gln Asp Glu Ala Thr Leu Cys Ile Phe Arg Leu Lys Ser Leu
            180                 185                 190


Lys Glu Gly Gln Ala Arg Ser Leu Leu Thr Gln Ala Ala Arg His His
        195                 200                 205


Thr Ala Gln Met His Met Phe Phe Ala Gly Leu Lys Ser Leu Glu Ala
    210                 215                 220
```

```
Val Glu Gln His Val Arg Ile Ala Ala Asp Arg Gln His Ile Asp Cys
225                 230                 235                 240

Val Leu Ser Asp Pro Gly Asn Glu Met Asp Cys Ser Glu Asp Asn Asp
                245                 250                 255

Asp Asp Asp Arg Leu Val Asn Arg Asp Gly Glu Leu Ser Phe Asp Tyr
            260                 265                 270

Ile Thr Ser Glu Gln Arg Val Glu Val Ile Ser Thr Pro His Gly Ser
        275                 280                 285

Met Lys Met Asp Asp Thr Asp Leu Ser Phe Gln Arg Pro Ser Pro Ala
    290                 295                 300

Gly Ser Ala Thr Val Asn Ala Asp Pro Arg Glu Glu His Ser Val Ser
305                 310                 315                 320

Asn Arg Asp Arg Arg Thr Ser Ser His Ser Ala Pro Leu Phe Pro Asp
                325                 330                 335

Lys Lys Ala Asp Leu Ala Asp Arg Ser Met Arg Gln Met Thr Pro Ser
            340                 345                 350

Ala Asn Ala Tyr Ile Leu Pro Thr Pro Val Asp Ser Lys Ser Ser Pro
        355                 360                 365

Ile Phe Thr Lys Pro Val Thr Gln Thr Asn His Ser Ala Asn Leu Trp
    370                 375                 380

His Ser Ser Pro Leu Glu Pro Ile Lys Thr Ala His Lys Asp Ala Glu
385                 390                 395                 400

Ser Asn Leu Tyr Ser Arg Leu Pro Arg Pro Ser Glu His Ala Phe Ser
                405                 410                 415

Gly Pro Leu Lys Pro Ser Ser Thr Arg Leu Pro Val Pro Val Ala Val
            420                 425                 430

Gln Ala Gln Ser Ser Ser Pro Arg Ile Ser Pro Thr Ala Ser Pro Pro
        435                 440                 445

Leu Ala Ser Ser Pro Arg Ile Asn Glu Leu His Glu Leu Pro Arg Pro
    450                 455                 460

Pro Gly Gln Phe Ala Pro Pro Arg Arg Ser Lys Ser Pro Gly Leu Val
465                 470                 475                 480
```

```
Gly His Ser Ala Pro Leu Thr Ala Trp Asn Gln Glu Arg Ser Asn Val
                485                 490                 495

Val Val Ser Thr Asn Ile Val Ala Ser Pro Leu Pro Val Pro Pro Leu
            500                 505                 510

Val Val Pro Arg Ser Tyr Ser Ile Pro Ser Arg Asn Gln Arg Ala Met
        515                 520                 525

Ala Gln Gln Pro Leu Pro Glu Arg Asn Gln Asn Arg Val Ala Ser Pro
    530                 535                 540

Pro Pro Leu Pro Leu Thr Pro Ala Ser Leu Met Asn Leu Arg Ser Leu
545                 550                 555                 560

Ser Arg Ser His Val Gly Glu Val Ala Gln Ser Gly Leu Ile Arg Gly
                565                 570                 575

Asn Gly Asn Lys Thr Asn Leu Leu Tyr Leu
            580                 585


<210>  11
<211>  6018
<212>  DNA
<213>  Glycine max


<220>
<221>  CDS
<222>  (1)..(6018)

<400>  11
atg aag agg tct ctt aga aag ttg gga gtt ctg gcg gta gag aag cat       48
Met Lys Arg Ser Leu Arg Lys Leu Gly Val Leu Ala Val Glu Lys His
1               5                   10                  15

gag cgt gac cgc aga aac att atg cct ttg tcc cag cta gag gag ctc       96
Glu Arg Asp Arg Arg Asn Ile Met Pro Leu Ser Gln Leu Glu Glu Leu
            20                  25                  30

gct cag gct acc cag gag atg caa gac atg aga gac tgc cac gat agc      144
Ala Gln Ala Thr Gln Glu Met Gln Asp Met Arg Asp Cys His Asp Ser
        35                  40                  45

ttg ctt tcc gcg gca gca gtg gcc gcc aac agt gct tat gaa ttc tca      192
Leu Leu Ser Ala Ala Ala Val Ala Ala Asn Ser Ala Tyr Glu Phe Ser
    50                  55                  60

gag tcc ttg gga gaa ttg ggc tct tgc ctt ctt gag aaa acc gcc ttg      240
Glu Ser Leu Gly Glu Leu Gly Ser Cys Leu Leu Glu Lys Thr Ala Leu
65                  70                  75                  80

cat gac gac gaa gaa agc ggc aaa gtt ctc ata atg ctc ggt aaa atg      288
His Asp Asp Glu Glu Ser Gly Lys Val Leu Ile Met Leu Gly Lys Met
                85                  90                  95

cag ttt caa ctc cag aag ctt att gat aac tat cgc tct cat atc acc      336
Gln Phe Gln Leu Gln Lys Leu Ile Asp Asn Tyr Arg Ser His Ile Thr
            100                 105                 110
```

```
cag aca att acc att cct tca gac tct ctt ctc aat gaa ctt cga att      384
Gln Thr Ile Thr Ile Pro Ser Asp Ser Leu Leu Asn Glu Leu Arg Ile
        115                 120                 125

gtt gag gat atg aag caa cac tgt gat gaa aaa aga gaa gta tat gag      432
Val Glu Asp Met Lys Gln His Cys Asp Glu Lys Arg Glu Val Tyr Glu
    130                 135                 140

tcc atg tta aca aga tat aga gaa aga ggt agg tct aga agc gga aaa      480
Ser Met Leu Thr Arg Tyr Arg Glu Arg Gly Arg Ser Arg Ser Gly Lys
145                 150                 155                 160

gca gag aat att tcc ttg cag cac ttg caa att gct cgt gat gaa tat      528
Ala Glu Asn Ile Ser Leu Gln His Leu Gln Ile Ala Arg Asp Glu Tyr
                165                 170                 175

gac gag gag gct aca tta ttt gtt ttc cgc ttg aaa tct ctg aag caa      576
Asp Glu Glu Ala Thr Leu Phe Val Phe Arg Leu Lys Ser Leu Lys Gln
            180                 185                 190

ggg caa tca tgg agc ctt cta aca cag gca gca cgt cac cat gca gct      624
Gly Gln Ser Trp Ser Leu Leu Thr Gln Ala Ala Arg His His Ala Ala
        195                 200                 205

cag ttg tgt ttc ttc aag aaa gca gtt aaa tct ctt gag aca gtg gag      672
Gln Leu Cys Phe Phe Lys Lys Ala Val Lys Ser Leu Glu Thr Val Glu
    210                 215                 220

cca cat gta aaa tca gta aca gag cag cat cac att gat tac caa ttc      720
Pro His Val Lys Ser Val Thr Glu Gln His His Ile Asp Tyr Gln Phe
225                 230                 235                 240

att ggt att gaa gga gag gat gag gat gag gat gaa gat gaa gat gat      768
Ile Gly Ile Glu Gly Glu Asp Glu Asp Glu Asp Glu Asp Glu Asp Asp
                245                 250                 255

gta gat cat gat gat gac agt cat gat gag aat gat gat ggg gag ctg      816
Val Asp His Asp Asp Asp Ser His Asp Glu Asn Asp Asp Gly Glu Leu
            260                 265                 270

agt ttt gac tat gca caa aat gaa tgc gag caa gat gat tct aca tta      864
Ser Phe Asp Tyr Ala Gln Asn Glu Cys Glu Gln Asp Asp Ser Thr Leu
        275                 280                 285

gag aac tca atg aag aaa tcc acc atg aat gaa aca tcc atc aac aac      912
Glu Asn Ser Met Lys Lys Ser Thr Met Asn Glu Thr Ser Ile Asn Asn
    290                 295                 300

atg gag agt tat ctc tac ctc cat ccg agc gag aac ccg gcc act gcc      960
Met Glu Ser Tyr Leu Tyr Leu His Pro Ser Glu Asn Pro Ala Thr Ala
305                 310                 315                 320

ctt gtt tct cca gtc cta gat tcg act aac tac cac tca tgg agc agg     1008
Leu Val Ser Pro Val Leu Asp Ser Thr Asn Tyr His Ser Trp Ser Arg
                325                 330                 335

tcc atg gtc acc gca tta agt gcc aag aac aaa gta gag ttc ata gat     1056
Ser Met Val Thr Ala Leu Ser Ala Lys Asn Lys Val Glu Phe Ile Asp
            340                 345                 350

ggc agc gcg cct gaa cct ctg aaa aca gat aga atg cat gga gcg tgg     1104
Gly Ser Ala Pro Glu Pro Leu Lys Thr Asp Arg Met His Gly Ala Trp
        355                 360                 365

tgt cga tgt aac aat atg gtt gtc tcg tgg ata gtt cac tcg gtg gcc     1152
```

```
Cys Arg Cys Asn Asn Met Val Val Ser Trp Ile Val His Ser Val Ala
    370                 375                 380

acc tcc ata cgt caa agt ata ctt tgg atg gac aaa gcc gaa gaa atc      1200
Thr Ser Ile Arg Gln Ser Ile Leu Trp Met Asp Lys Ala Glu Glu Ile
385                 390                 395                 400

tgg cgc gat ctg aaa tca aga tat tca caa ggt gat ctt ctg cgc atc      1248
Trp Arg Asp Leu Lys Ser Arg Tyr Ser Gln Gly Asp Leu Leu Arg Ile
                405                 410                 415

tcc gac ctt cag caa gaa gct tca aca atg aaa caa ggc aca ctt aca      1296
Ser Asp Leu Gln Gln Glu Ala Ser Thr Met Lys Gln Gly Thr Leu Thr
            420                 425                 430

gtg act gag tat ttc act tgt cta cgc gta att tgg gat gag att gag      1344
Val Thr Glu Tyr Phe Thr Cys Leu Arg Val Ile Trp Asp Glu Ile Glu
        435                 440                 445

aac ttt aga ccc gat ccc atc tgt tcc tgt aat atc agg tgt tcc tgc      1392
Asn Phe Arg Pro Asp Pro Ile Cys Ser Cys Asn Ile Arg Cys Ser Cys
    450                 455                 460

aac gca ttc acc att atc gcg caa cgg aag ctc gag gat aga gcc atg      1440
Asn Ala Phe Thr Ile Ile Ala Gln Arg Lys Leu Glu Asp Arg Ala Met
465                 470                 475                 480

cag ttc cta cga ggc ctg aac gaa cag tat gca aat att cgt tct cat      1488
Gln Phe Leu Arg Gly Leu Asn Glu Gln Tyr Ala Asn Ile Arg Ser His
                485                 490                 495

gtt ctt ctc atg gat ccc ata ccc act ata tcc aaa ata ttc tcc tat      1536
Val Leu Leu Met Asp Pro Ile Pro Thr Ile Ser Lys Ile Phe Ser Tyr
            500                 505                 510

gta gct caa cag gaa agg caa cta ctg ggt aac acc gga cca ggt att      1584
Val Ala Gln Gln Glu Arg Gln Leu Leu Gly Asn Thr Gly Pro Gly Ile
        515                 520                 525

aac ttc gaa ccc aaa gat atc tcc att aac gct gct aag acc gtt tgc      1632
Asn Phe Glu Pro Lys Asp Ile Ser Ile Asn Ala Ala Lys Thr Val Cys
    530                 535                 540

gat ttc tgt gga cgc att ggt cat gtg gaa agt acg tgt tat aag aag      1680
Asp Phe Cys Gly Arg Ile Gly His Val Glu Ser Thr Cys Tyr Lys Lys
545                 550                 555                 560

cat gga gtg ccc tct aat tac gat gca aga aat aag agt aat gga aga      1728
His Gly Val Pro Ser Asn Tyr Asp Ala Arg Asn Lys Ser Asn Gly Arg
                565                 570                 575

aaa gca tgc act cac tgc ggc aag ata gga cat aca gtg gat gtc tgt      1776
Lys Ala Cys Thr His Cys Gly Lys Ile Gly His Thr Val Asp Val Cys
            580                 585                 590

tat cgg aaa cac ggg tac ccg ccg gga tac aag cct tac agt gga agg      1824
Tyr Arg Lys His Gly Tyr Pro Pro Gly Tyr Lys Pro Tyr Ser Gly Arg
        595                 600                 605

acc acc gtg aac aac gta gtg gca gtt gaa agc aaa gcc act gat gat      1872
Thr Thr Val Asn Asn Val Val Ala Val Glu Ser Lys Ala Thr Asp Asp
    610                 615                 620

caa gct cag cat cac gag tct cat gag ttc gtt cgc ttc tcc cct gag      1920
Gln Ala Gln His His Glu Ser His Glu Phe Val Arg Phe Ser Pro Glu
625                 630                 635                 640
```

```
cag tac aag gca ctt ctt gcc tta atc cag gag cca tcc gca ggg aac      1968
Gln Tyr Lys Ala Leu Leu Ala Leu Ile Gln Glu Pro Ser Ala Gly Asn
                645                 650                 655

acg gca ctc act caa ccc aaa cag gtg gct tca atc tca tca tgc acc      2016
Thr Ala Leu Thr Gln Pro Lys Gln Val Ala Ser Ile Ser Ser Cys Thr
            660                 665                 670

gtc aat aat cca aca aac cca ggt atg tca ctt tct ctc agt gcg tct      2064
Val Asn Asn Pro Thr Asn Pro Gly Met Ser Leu Ser Leu Ser Ala Ser
        675                 680                 685

ctc acc tcc tgg ata tta gat tca ggg gct aca gat cac gta acc tgc      2112
Leu Thr Ser Trp Ile Leu Asp Ser Gly Ala Thr Asp His Val Thr Cys
    690                 695                 700

tcc ctg cac aat ctt cat tcc cat aaa cga atc aat cca atc aca gtg      2160
Ser Leu His Asn Leu His Ser His Lys Arg Ile Asn Pro Ile Thr Val
705                 710                 715                 720

aaa ctt ccg aac ggt caa tat gtt cat gca act cac tca gga act gtc      2208
Lys Leu Pro Asn Gly Gln Tyr Val His Ala Thr His Ser Gly Thr Val
                725                 730                 735

cag ctc tcg tcc aac ata acc tta cac gat gtt ctt tac ata ccc tct      2256
Gln Leu Ser Ser Asn Ile Thr Leu His Asp Val Leu Tyr Ile Pro Ser
            740                 745                 750

ttc aca ttc aac ctt att tca ata tca aag ctt gta tct tct att aat      2304
Phe Thr Phe Asn Leu Ile Ser Ile Ser Lys Leu Val Ser Ser Ile Asn
        755                 760                 765

tgt gaa tta atc ttt tct tct aca tca tgt gta ctg cag gaa atg aac      2352
Cys Glu Leu Ile Phe Ser Ser Thr Ser Cys Val Leu Gln Glu Met Asn
    770                 775                 780

aac cat atg aag att ggt ata gtt gaa gca aag cat ggt ctc tat cac      2400
Asn His Met Lys Ile Gly Ile Val Glu Ala Lys His Gly Leu Tyr His
785                 790                 795                 800

ctg ata cca aac cag tta acc acc aaa gct gtg aac tct act atc act      2448
Leu Ile Pro Asn Gln Leu Thr Thr Lys Ala Val Asn Ser Thr Ile Thr
                805                 810                 815

cat cct cga tgc aat gta att ccc ata gat ctt tgg cat ttt agg tta      2496
His Pro Arg Cys Asn Val Ile Pro Ile Asp Leu Trp His Phe Arg Leu
            820                 825                 830

ggt cac cca tca gct gaa agg ata caa tgt atg aaa acc tac tat cct      2544
Gly His Pro Ser Ala Glu Arg Ile Gln Cys Met Lys Thr Tyr Tyr Pro
        835                 840                 845

ctt cta aga aat aat aag aat ttt gtc tgc aac aca tgt cat tat gcg      2592
Leu Leu Arg Asn Asn Lys Asn Phe Val Cys Asn Thr Cys His Tyr Ala
    850                 855                 860

aag cac aag aaa atg cct ttt tct ctt agt aat tca cat gca tct cat      2640
Lys His Lys Lys Met Pro Phe Ser Leu Ser Asn Ser His Ala Ser His
865                 870                 875                 880

gct ttc gat tta ctt cat atg gat ata cgg ggt ccg tgt tca aaa cca      2688
Ala Phe Asp Leu Leu His Met Asp Ile Arg Gly Pro Cys Ser Lys Pro
                885                 890                 895

tca atg cat ggg cat aaa tat ttc ttg acc att gtc gac gat tgt tca      2736
```

```
Ser Met His Gly His Lys Tyr Phe Leu Thr Ile Val Asp Asp Cys Ser
            900                 905                 910

cgc ttc aca tgg gta cat ctc atg aag tct aaa gct gaa acg cga cag      2784
Arg Phe Thr Trp Val His Leu Met Lys Ser Lys Ala Glu Thr Arg Gln
        915                 920                 925

gtc att atg aat ttc att aca ttc att gaa aca cag tac aat ggc aaa      2832
Val Ile Met Asn Phe Ile Thr Phe Ile Glu Thr Gln Tyr Asn Gly Lys
    930                 935                 940

gtt aaa atc atc aga agt gat aat ggg atc gag ttc ttc atg cat cat      2880
Val Lys Ile Ile Arg Ser Asp Asn Gly Ile Glu Phe Phe Met His His
945                 950                 955                 960

tat tat gca tca aag gga atc att cac caa act aca tgt gtt gaa acc      2928
Tyr Tyr Ala Ser Lys Gly Ile Ile His Gln Thr Thr Cys Val Glu Thr
                965                 970                 975

cca gaa caa aac ggt atc gta gaa cga aag cat cag cat cta ctc aat      2976
Pro Glu Gln Asn Gly Ile Val Glu Arg Lys His Gln His Leu Leu Asn
            980                 985                 990

atc aca cgc gca ctc tta ttt cag  gcg agc cta cca cct  agc ttc tgg    3024
Ile Thr Arg Ala Leu Leu Phe Gln  Ala Ser Leu Pro Pro  Ser Phe Trp
        995                 1000                 1005

tgt tac  gcc ttg cca cat gcc  aca tac cta att aat  tgc atc ccc       3069
Cys Tyr  Ala Leu Pro His Ala  Thr Tyr Leu Ile Asn  Cys Ile Pro
    1010                 1015                 1020

aca cca  tac ttg cat aat att  tca cct tac gaa aag  cta cat aag       3114
Thr Pro  Tyr Leu His Asn Ile  Ser Pro Tyr Glu Lys  Leu His Lys
    1025                 1030                 1035

cat cca  tgt gac atc tct aac  ctt cgt gtt ttc ggg  ggt ctg tgc       3159
His Pro  Cys Asp Ile Ser Asn  Leu Arg Val Phe Gly  Gly Leu Cys
    1040                 1045                 1050

tac att  aat acg ctc aaa gca  aat cgg caa aag ctt  gat gct agg       3204
Tyr Ile  Asn Thr Leu Lys Ala  Asn Arg Gln Lys Leu  Asp Ala Arg
    1055                 1060                 1065

gca cac  cca tgc atc ttc att  ggt ttt aaa acg cat  acg aaa gga       3249
Ala His  Pro Cys Ile Phe Ile  Gly Phe Lys Thr His  Thr Lys Gly
    1070                 1075                 1080

tat ctt  gta tat gat ttg cat  tct aat gat gtt acc  gta tct aga       3294
Tyr Leu  Val Tyr Asp Leu His  Ser Asn Asp Val Thr  Val Ser Arg
    1085                 1090                 1095

aat gtc  acg ttt tat gag gat  cat ttt cct tac tat  tct gaa acc       3339
Asn Val  Thr Phe Tyr Glu Asp  His Phe Pro Tyr Tyr  Ser Glu Thr
    1100                 1105                 1110

cag cat  ata aac tca gaa cat  tca gct ccc tct ccg  gga ccc ttt       3384
Gln His  Ile Asn Ser Glu His  Ser Ala Pro Ser Pro  Gly Pro Phe
    1115                 1120                 1125

tcc ggc  aag aac ctt gac cca  caa ata gaa aat tgc  tca tca caa       3429
Ser Gly  Lys Asn Leu Asp Pro  Gln Ile Glu Asn Cys  Ser Ser Gln
    1130                 1135                 1140

ccc aca  ata tca gta cca tca  tcc aat gaa cct tca  aat gag caa       3474
Pro Thr  Ile Ser Val Pro Ser  Ser Asn Glu Pro Ser  Asn Glu Gln
    1145                 1150                 1155
```

```
cct ctt  cca cat ctt agg cga  tcc aca aga gcc aaa  aac acc cca      3519
Pro Leu  Pro His Leu Arg Arg  Ser Thr Arg Ala Lys  Asn Thr Pro
    1160                 1165                 1170

aca tat  ctc caa gac tat cac  aga gac ctt gct tca  tct aca cca      3564
Thr Tyr  Leu Gln Asp Tyr His  Arg Asp Leu Ala Ser  Ser Thr Pro
    1175                 1180                 1185

aat act  tca gcg att gtt cgt  tac cca cta agt tcc  gtg ctt tca      3609
Asn Thr  Ser Ala Ile Val Arg  Tyr Pro Leu Ser Ser  Val Leu Ser
    1190                 1195                 1200

tat tca  cgt ttg tca cct gca  cac agg aat ttc gtc  atg agc att      3654
Tyr Ser  Arg Leu Ser Pro Ala  His Arg Asn Phe Val  Met Ser Ile
    1205                 1210                 1215

tcc tta  aca gca gaa cct acc  tca tac acc gaa gct  tct cgc cat      3699
Ser Leu  Thr Ala Glu Pro Thr  Ser Tyr Thr Glu Ala  Ser Arg His
    1220                 1225                 1230

gac tgc  tgg att aag gca atg  aag gtc gag tta cag  gct ctc caa      3744
Asp Cys  Trp Ile Lys Ala Met  Lys Val Glu Leu Gln  Ala Leu Gln
    1235                 1240                 1245

tcg aac  aac aca tgg aga ctc  aca cct ctt cct cct  cac aag aca      3789
Ser Asn  Asn Thr Trp Arg Leu  Thr Pro Leu Pro Pro  His Lys Thr
    1250                 1255                 1260

gct att  ggg tgt agg tgg ata  tac aaa atc aag tat  cgg aca gat      3834
Ala Ile  Gly Cys Arg Trp Ile  Tyr Lys Ile Lys Tyr  Arg Thr Asp
    1265                 1270                 1275

ggt tcc  ata gag agg cac aag  gcg cga tta gtt gca  aag ggg tac      3879
Gly Ser  Ile Glu Arg His Lys  Ala Arg Leu Val Ala  Lys Gly Tyr
    1280                 1285                 1290

acc caa  atg gag ggg ttg gat  tac ctc gac acg ttc  tct ccg gta      3924
Thr Gln  Met Glu Gly Leu Asp  Tyr Leu Asp Thr Phe  Ser Pro Val
    1295                 1300                 1305

gca aag  ctc acc acg gtg cgt  ctt ctt ctt gcg ata  gct gcg ctc      3969
Ala Lys  Leu Thr Thr Val Arg  Leu Leu Leu Ala Ile  Ala Ala Leu
    1310                 1315                 1320

aac cag  tgg cat tta cgg cag  ctg gac gtc aac aat  gcg ttc ctc      4014
Asn Gln  Trp His Leu Arg Gln  Leu Asp Val Asn Asn  Ala Phe Leu
    1325                 1330                 1335

cat gga  gaa ctt gat gaa gag  gtg tat atg caa ata  cct cca gga      4059
His Gly  Glu Leu Asp Glu Glu  Val Tyr Met Gln Ile  Pro Pro Gly
    1340                 1345                 1350

ctt tcg  gtt gac aat cca cag  ctt gtg tgt cat ctt  caa cgc ttc      4104
Leu Ser  Val Asp Asn Pro Gln  Leu Val Cys His Leu  Gln Arg Phe
    1355                 1360                 1365

tta agc  tct cat ggg ttt caa  cag tcg aat gca gat  cac tcc ctc      4149
Leu Ser  Ser His Gly Phe Gln  Gln Ser Asn Ala Asp  His Ser Leu
    1370                 1375                 1380

ttc ttg  cgc ttc aca gga gtc  atc act aca ata ctc  cta gta tac      4194
Phe Leu  Arg Phe Thr Gly Val  Ile Thr Thr Ile Leu  Leu Val Tyr
    1385                 1390                 1395

gtg gat  gat atc atc ctc acg  gga aac aac ata gct  gaa ata caa      4239
```

```
Val Asp  Asp Ile Ile Leu Thr  Gly Asn Asn Ile Ala  Glu Ile Gln
    1400                 1405                 1410

act atg  atc acc ctc ttg gat  cgc gag ttc aga atc  aag gat ctt      4284
Thr Met  Ile Thr Leu Leu Asp  Arg Glu Phe Arg Ile  Lys Asp Leu
    1415                 1420                 1425

ggg gat  tta aag ttc ttt ctt  ggg ctc gaa att gca  cgc acc tct      4329
Gly Asp  Leu Lys Phe Phe Leu  Gly Leu Glu Ile Ala  Arg Thr Ser
    1430                 1435                 1440

aaa gga  atc cat cta tgt caa  cgt aag tat aca ttg  gat att tta      4374
Lys Gly  Ile His Leu Cys Gln  Arg Lys Tyr Thr Leu  Asp Ile Leu
    1445                 1450                 1455

agt gat  tca gga atg cta ggt  tgc aag ccc aac tca  aca cca atg      4419
Ser Asp  Ser Gly Met Leu Gly  Cys Lys Pro Asn Ser  Thr Pro Met
    1460                 1465                 1470

gat tac  tcc aca aag cta caa  gca gat tca gga agt  ctt ctt tca      4464
Asp Tyr  Ser Thr Lys Leu Gln  Ala Asp Ser Gly Ser  Leu Leu Ser
    1475                 1480                 1485

gct gag  tct tct tcc tcc tat  aga aga ttg ata ggc  aaa ctc ata      4509
Ala Glu  Ser Ser Ser Ser Tyr  Arg Arg Leu Ile Gly  Lys Leu Ile
    1490                 1495                 1500

tac ctt  acc aac aca cga ccc  gat ata aca tac gca  gtc cag caa      4554
Tyr Leu  Thr Asn Thr Arg Pro  Asp Ile Thr Tyr Ala  Val Gln Gln
    1505                 1510                 1515

ctc agc  cag tac atg gcc act  ccc acc aat gtt cat  ctt caa gcc      4599
Leu Ser  Gln Tyr Met Ala Thr  Pro Thr Asn Val His  Leu Gln Ala
    1520                 1525                 1530

gcc ttt  cgc atc ctc cga tac  ctc aag ggt aca cca  ggt tca gga      4644
Ala Phe  Arg Ile Leu Arg Tyr  Leu Lys Gly Thr Pro  Gly Ser Gly
    1535                 1540                 1545

ctc ttc  ttc gcc gcc aca ggc  act cct caa ctt cga  gca ttc agt      4689
Leu Phe  Phe Ala Ala Thr Gly  Thr Pro Gln Leu Arg  Ala Phe Ser
    1550                 1555                 1560

gac tcg  gat tgg gca ggg tgc  aaa gac tca agg aag  tcc act ccg      4734
Asp Ser  Asp Trp Ala Gly Cys  Lys Asp Ser Arg Lys  Ser Thr Pro
    1565                 1570                 1575

ggt tac  tta gtt tac ctt ggt  tct tct ctt gtc tct  tgg caa tca      4779
Gly Tyr  Leu Val Tyr Leu Gly  Ser Ser Leu Val Ser  Trp Gln Ser
    1580                 1585                 1590

aag aag  caa tca act gta tcg  cgc agc tcc tcc gaa  gct gaa tat      4824
Lys Lys  Gln Ser Thr Val Ser  Arg Ser Ser Ser Glu  Ala Glu Tyr
    1595                 1600                 1605

cgc gct  ctc gct tcc act act  tgc gag cta caa tgg  ctg act ttt      4869
Arg Ala  Leu Ala Ser Thr Thr  Cys Glu Leu Gln Trp  Leu Thr Phe
    1610                 1615                 1620

ctg ctt  caa gac ttt cgc gcc  aca ttc att caa cca  gca acc tta      4914
Leu Leu  Gln Asp Phe Arg Ala  Thr Phe Ile Gln Pro  Ala Thr Leu
    1625                 1630                 1635

tat tgc  gac aat cag tca aca  atc caa ata gcc aca  aat ccc gtt      4959
Tyr Cys  Asp Asn Gln Ser Thr  Ile Gln Ile Ala Thr  Asn Pro Val
    1640                 1645                 1650
```

```
ttc cat gaa cga acc aaa cac att gag ata gat tgt cat att gtc      5004
Phe His Glu Arg Thr Lys His Ile Glu Ile Asp Cys His Ile Val
    1655                1660                1665

cga cag aag ctt aac tcg gct ctc ata aaa ctc ctt cca tca aat      5049
Arg Gln Lys Leu Asn Ser Ala Leu Ile Lys Leu Leu Pro Ser Asn
    1670                1675                1680

cgt aag aaa tca cac ttt gtg gtg gtt agg tca gct agc caa tct      5094
Arg Lys Lys Ser His Phe Val Val Val Arg Ser Ala Ser Gln Ser
    1685                1690                1695

gcc cca ctt ttt gtt gat aat aag cgt gat tct agc gaa aag ctg      5139
Ala Pro Leu Phe Val Asp Asn Lys Arg Asp Ser Ser Glu Lys Leu
    1700                1705                1710

aga cag atg cgg cca act tta tct cgg aag ttc aat tca tat gtg      5184
Arg Gln Met Arg Pro Thr Leu Ser Arg Lys Phe Asn Ser Tyr Val
    1715                1720                1725

ctg cct aca cca gtt gat gcg aag agt tca ata tct atg agg tca      5229
Leu Pro Thr Pro Val Asp Ala Lys Ser Ser Ile Ser Met Arg Ser
    1730                1735                1740

agt aat caa gta cct ttg aaa ata aag aca aat tta aat gag cct      5274
Ser Asn Gln Val Pro Leu Lys Ile Lys Thr Asn Leu Asn Glu Pro
    1745                1750                1755

atg aag aat tta tgg cat tca tcc cca cta gaa caa aag aaa tat      5319
Met Lys Asn Leu Trp His Ser Ser Pro Leu Glu Gln Lys Lys Tyr
    1760                1765                1770

gaa aac att ttt gga gat ggg gga tta tct ggt cct aat gca cag      5364
Glu Asn Ile Phe Gly Asp Gly Gly Leu Ser Gly Pro Asn Ala Gln
    1775                1780                1785

tct gta ctg aag gag agt aac agc aat act gcc tac tca aga ttg      5409
Ser Val Leu Lys Glu Ser Asn Ser Asn Thr Ala Tyr Ser Arg Leu
    1790                1795                1800

cca cct cct tta atc gat ggt aat tta tcc tct agt cat gat tat      5454
Pro Pro Pro Leu Ile Asp Gly Asn Leu Ser Ser Ser His Asp Tyr
    1805                1810                1815

att acc gct tac tct aaa aag atc aaa aga cat gcc ttt tct ggt      5499
Ile Thr Ala Tyr Ser Lys Lys Ile Lys Arg His Ala Phe Ser Gly
    1820                1825                1830

cca ttg gta agt aat gca tgg cca acc aag cct gtc tcg tta gaa      5544
Pro Leu Val Ser Asn Ala Trp Pro Thr Lys Pro Val Ser Leu Glu
    1835                1840                1845

agt gtc caa ttg ttt tct gga cct ctt tta cgg acc tca att cct      5589
Ser Val Gln Leu Phe Ser Gly Pro Leu Leu Arg Thr Ser Ile Pro
    1850                1855                1860

cag cct cca tca tca tct cca aaa gta tct ccc agt gct tct ccc      5634
Gln Pro Pro Ser Ser Ser Pro Lys Val Ser Pro Ser Ala Ser Pro
    1865                1870                1875

cct ctt tca tct tct cct aaa ata aat gag ctt cat gaa ctt cca      5679
Pro Leu Ser Ser Ser Pro Lys Ile Asn Glu Leu His Glu Leu Pro
    1880                1885                1890

agg cct cca acc att tcc cca tcc gat tct aag ctt tta ggt ttg      5724
```

```
Arg Pro  Pro Thr Ile Ser Pro  Ser Asp Ser Lys Leu  Leu Gly Leu
    1895                 1900                 1905

gtg ggt  cat tca ggt cca ttg  gtg tcc cga ggt cca  cag ctt tct      5769
Val Gly  His Ser Gly Pro Leu  Val Ser Arg Gly Pro  Gln Leu Ser
    1910                 1915                 1920

gct gca  aat tat ttg gtt aca  tca aat gca gca tct  cca ttg ccg      5814
Ala Ala  Asn Tyr Leu Val Thr  Ser Asn Ala Ala Ser  Pro Leu Pro
    1925                 1930                 1935

atg cct  gct tca gcc atg gct  cga agt ttc tct aca  tct tat cgt      5859
Met Pro  Ala Ser Ala Met Ala  Arg Ser Phe Ser Thr  Ser Tyr Arg
    1940                 1945                 1950

ggt gct  aaa gtt gca gca ata  cat gac tca aga cca  cta gaa gac      5904
Gly Ala  Lys Val Ala Ala Ile  His Asp Ser Arg Pro  Leu Glu Asp
    1955                 1960                 1965

cct aat  aaa tca aca att tct  gag gat att gat tct  cct cct ctg      5949
Pro Asn  Lys Ser Thr Ile Ser  Glu Asp Ile Asp Ser  Pro Pro Leu
    1970                 1975                 1980

atg caa  att gct tta tct act  agt cag cca tca tca  gat ggc tct      5994
Met Gln  Ile Ala Leu Ser Thr  Ser Gln Pro Ser Ser  Asp Gly Ser
    1985                 1990                 1995

gac act  gtt gct cag gct gtc  tag                                   6018
Asp Thr  Val Ala Gln Ala Val
    2000                 2005


<210>  12
<211>  2005
<212>  PRT
<213>  Glycine max

<400>  12

Met Lys Arg Ser Leu Arg Lys Leu Gly Val Leu Ala Val Glu Lys His
1               5                   10                  15


Glu Arg Asp Arg Arg Asn Ile Met Pro Leu Ser Gln Leu Glu Glu Leu
            20                  25                  30


Ala Gln Ala Thr Gln Glu Met Gln Asp Met Arg Asp Cys His Asp Ser
        35                  40                  45


Leu Leu Ser Ala Ala Ala Val Ala Ala Asn Ser Ala Tyr Glu Phe Ser
    50                  55                  60


Glu Ser Leu Gly Glu Leu Gly Ser Cys Leu Leu Glu Lys Thr Ala Leu
65                  70                  75                  80


His Asp Asp Glu Glu Ser Gly Lys Val Leu Ile Met Leu Gly Lys Met
                85                  90                  95


Gln Phe Gln Leu Gln Lys Leu Ile Asp Asn Tyr Arg Ser His Ile Thr
            100                 105                 110
```

Gln Thr Ile Thr Ile Pro Ser Asp Ser Leu Leu Asn Glu Leu Arg Ile
115 120 125

Val Glu Asp Met Lys Gln His Cys Asp Glu Lys Arg Glu Val Tyr Glu
130 135 140

Ser Met Leu Thr Arg Tyr Arg Glu Arg Gly Arg Ser Arg Ser Gly Lys
145 150 155 160

Ala Glu Asn Ile Ser Leu Gln His Leu Gln Ile Ala Arg Asp Glu Tyr
165 170 175

Asp Glu Glu Ala Thr Leu Phe Val Phe Arg Leu Lys Ser Leu Lys Gln
180 185 190

Gly Gln Ser Trp Ser Leu Leu Thr Gln Ala Ala Arg His His Ala Ala
195 200 205

Gln Leu Cys Phe Phe Lys Lys Ala Val Lys Ser Leu Glu Thr Val Glu
210 215 220

Pro His Val Lys Ser Val Thr Glu Gln His His Ile Asp Tyr Gln Phe
225 230 235 240

Ile Gly Ile Glu Gly Glu Asp Glu Asp Glu Asp Glu Asp Glu Asp Asp
245 250 255

Val Asp His Asp Asp Asp Ser His Asp Glu Asn Asp Asp Gly Glu Leu
260 265 270

Ser Phe Asp Tyr Ala Gln Asn Glu Cys Glu Gln Asp Asp Ser Thr Leu
275 280 285

Glu Asn Ser Met Lys Lys Ser Thr Met Asn Glu Thr Ser Ile Asn Asn
290 295 300

Met Glu Ser Tyr Leu Tyr Leu His Pro Ser Glu Asn Pro Ala Thr Ala
305 310 315 320

Leu Val Ser Pro Val Leu Asp Ser Thr Asn Tyr His Ser Trp Ser Arg
325 330 335

Ser Met Val Thr Ala Leu Ser Ala Lys Asn Lys Val Glu Phe Ile Asp
340 345 350

Gly Ser Ala Pro Glu Pro Leu Lys Thr Asp Arg Met His Gly Ala Trp
355 360 365

Cys Arg Cys Asn Asn Met Val Val Ser Trp Ile Val His Ser Val Ala

```
    370                 375                 380

Thr Ser Ile Arg Gln Ser Ile Leu Trp Met Asp Lys Ala Glu Glu Ile
385                 390                 395                 400

Trp Arg Asp Leu Lys Ser Arg Tyr Ser Gln Gly Asp Leu Leu Arg Ile
                405                 410                 415

Ser Asp Leu Gln Gln Glu Ala Ser Thr Met Lys Gln Gly Thr Leu Thr
            420                 425                 430

Val Thr Glu Tyr Phe Thr Cys Leu Arg Val Ile Trp Asp Glu Ile Glu
        435                 440                 445

Asn Phe Arg Pro Asp Pro Ile Cys Ser Cys Asn Ile Arg Cys Ser Cys
    450                 455                 460

Asn Ala Phe Thr Ile Ile Ala Gln Arg Lys Leu Glu Asp Arg Ala Met
465                 470                 475                 480

Gln Phe Leu Arg Gly Leu Asn Glu Gln Tyr Ala Asn Ile Arg Ser His
                485                 490                 495

Val Leu Leu Met Asp Pro Ile Pro Thr Ile Ser Lys Ile Phe Ser Tyr
            500                 505                 510

Val Ala Gln Gln Glu Arg Gln Leu Leu Gly Asn Thr Gly Pro Gly Ile
        515                 520                 525

Asn Phe Glu Pro Lys Asp Ile Ser Ile Asn Ala Ala Lys Thr Val Cys
    530                 535                 540

Asp Phe Cys Gly Arg Ile Gly His Val Glu Ser Thr Cys Tyr Lys Lys
545                 550                 555                 560

His Gly Val Pro Ser Asn Tyr Asp Ala Arg Asn Lys Ser Asn Gly Arg
                565                 570                 575

Lys Ala Cys Thr His Cys Gly Lys Ile Gly His Thr Val Asp Val Cys
            580                 585                 590

Tyr Arg Lys His Gly Tyr Pro Pro Gly Tyr Lys Pro Tyr Ser Gly Arg
        595                 600                 605

Thr Thr Val Asn Asn Val Val Ala Val Glu Ser Lys Ala Thr Asp Asp
    610                 615                 620

Gln Ala Gln His His Glu Ser His Glu Phe Val Arg Phe Ser Pro Glu
625                 630                 635                 640
```

```
Gln Tyr Lys Ala Leu Leu Ala Leu Ile Gln Glu Pro Ser Ala Gly Asn
                645                 650                 655

Thr Ala Leu Thr Gln Pro Lys Gln Val Ala Ser Ile Ser Ser Cys Thr
            660                 665                 670

Val Asn Asn Pro Thr Asn Pro Gly Met Ser Leu Ser Leu Ser Ala Ser
        675                 680                 685

Leu Thr Ser Trp Ile Leu Asp Ser Gly Ala Thr Asp His Val Thr Cys
    690                 695                 700

Ser Leu His Asn Leu His Ser His Lys Arg Ile Asn Pro Ile Thr Val
705                 710                 715                 720

Lys Leu Pro Asn Gly Gln Tyr Val His Ala Thr His Ser Gly Thr Val
                725                 730                 735

Gln Leu Ser Ser Asn Ile Thr Leu His Asp Val Leu Tyr Ile Pro Ser
            740                 745                 750

Phe Thr Phe Asn Leu Ile Ser Ile Ser Lys Leu Val Ser Ser Ile Asn
        755                 760                 765

Cys Glu Leu Ile Phe Ser Ser Thr Ser Cys Val Leu Gln Glu Met Asn
    770                 775                 780

Asn His Met Lys Ile Gly Ile Val Glu Ala Lys His Gly Leu Tyr His
785                 790                 795                 800

Leu Ile Pro Asn Gln Leu Thr Thr Lys Ala Val Asn Ser Thr Ile Thr
                805                 810                 815

His Pro Arg Cys Asn Val Ile Pro Ile Asp Leu Trp His Phe Arg Leu
            820                 825                 830

Gly His Pro Ser Ala Glu Arg Ile Gln Cys Met Lys Thr Tyr Tyr Pro
        835                 840                 845

Leu Leu Arg Asn Asn Lys Asn Phe Val Cys Asn Thr Cys His Tyr Ala
    850                 855                 860

Lys His Lys Lys Met Pro Phe Ser Leu Ser Asn Ser His Ala Ser His
865                 870                 875                 880

Ala Phe Asp Leu Leu His Met Asp Ile Arg Gly Pro Cys Ser Lys Pro
                885                 890                 895

Ser Met His Gly His Lys Tyr Phe Leu Thr Ile Val Asp Asp Cys Ser
```

900 905 910

Arg Phe Thr Trp Val His Leu Met Lys Ser Lys Ala Glu Thr Arg Gln
915 920 925

Val Ile Met Asn Phe Ile Thr Phe Ile Glu Thr Gln Tyr Asn Gly Lys
930 935 940

Val Lys Ile Ile Arg Ser Asp Asn Gly Ile Glu Phe Phe Met His His
945 950 955 960

Tyr Tyr Ala Ser Lys Gly Ile Ile His Gln Thr Thr Cys Val Glu Thr
965 970 975

Pro Glu Gln Asn Gly Ile Val Glu Arg Lys His Gln His Leu Leu Asn
980 985 990

Ile Thr Arg Ala Leu Leu Phe Gln Ala Ser Leu Pro Pro Ser Phe Trp
995 1000 1005

Cys Tyr Ala Leu Pro His Ala Thr Tyr Leu Ile Asn Cys Ile Pro
1010 1015 1020

Thr Pro Tyr Leu His Asn Ile Ser Pro Tyr Glu Lys Leu His Lys
1025 1030 1035

His Pro Cys Asp Ile Ser Asn Leu Arg Val Phe Gly Gly Leu Cys
1040 1045 1050

Tyr Ile Asn Thr Leu Lys Ala Asn Arg Gln Lys Leu Asp Ala Arg
1055 1060 1065

Ala His Pro Cys Ile Phe Ile Gly Phe Lys Thr His Thr Lys Gly
1070 1075 1080

Tyr Leu Val Tyr Asp Leu His Ser Asn Asp Val Thr Val Ser Arg
1085 1090 1095

Asn Val Thr Phe Tyr Glu Asp His Phe Pro Tyr Tyr Ser Glu Thr
1100 1105 1110

Gln His Ile Asn Ser Glu His Ser Ala Pro Ser Pro Gly Pro Phe
1115 1120 1125

Ser Gly Lys Asn Leu Asp Pro Gln Ile Glu Asn Cys Ser Ser Gln
1130 1135 1140

Pro Thr Ile Ser Val Pro Ser Ser Asn Glu Pro Ser Asn Glu Gln
1145 1150 1155

Pro Leu Pro His Leu Arg Arg Ser Thr Arg Ala Lys Asn Thr Pro
1160 1165 1170

Thr Tyr Leu Gln Asp Tyr His Arg Asp Leu Ala Ser Ser Thr Pro
1175 1180 1185

Asn Thr Ser Ala Ile Val Arg Tyr Pro Leu Ser Ser Val Leu Ser
1190 1195 1200

Tyr Ser Arg Leu Ser Pro Ala His Arg Asn Phe Val Met Ser Ile
1205 1210 1215

Ser Leu Thr Ala Glu Pro Thr Ser Tyr Thr Glu Ala Ser Arg His
1220 1225 1230

Asp Cys Trp Ile Lys Ala Met Lys Val Glu Leu Gln Ala Leu Gln
1235 1240 1245

Ser Asn Asn Thr Trp Arg Leu Thr Pro Leu Pro Pro His Lys Thr
1250 1255 1260

Ala Ile Gly Cys Arg Trp Ile Tyr Lys Ile Lys Tyr Arg Thr Asp
1265 1270 1275

Gly Ser Ile Glu Arg His Lys Ala Arg Leu Val Ala Lys Gly Tyr
1280 1285 1290

Thr Gln Met Glu Gly Leu Asp Tyr Leu Asp Thr Phe Ser Pro Val
1295 1300 1305

Ala Lys Leu Thr Thr Val Arg Leu Leu Leu Ala Ile Ala Ala Leu
1310 1315 1320

Asn Gln Trp His Leu Arg Gln Leu Asp Val Asn Asn Ala Phe Leu
1325 1330 1335

His Gly Glu Leu Asp Glu Glu Val Tyr Met Gln Ile Pro Pro Gly
1340 1345 1350

Leu Ser Val Asp Asn Pro Gln Leu Val Cys His Leu Gln Arg Phe
1355 1360 1365

Leu Ser Ser His Gly Phe Gln Gln Ser Asn Ala Asp His Ser Leu
1370 1375 1380

Phe Leu Arg Phe Thr Gly Val Ile Thr Thr Ile Leu Leu Val Tyr
1385 1390 1395

Val Asp Asp Ile Ile Leu Thr Gly Asn Asn Ile Ala Glu Ile Gln

1400 1405 1410

Thr Met Ile Thr Leu Leu Asp Arg Glu Phe Arg Ile Lys Asp Leu
1415 1420 1425

Gly Asp Leu Lys Phe Phe Leu Gly Leu Glu Ile Ala Arg Thr Ser
1430 1435 1440

Lys Gly Ile His Leu Cys Gln Arg Lys Tyr Thr Leu Asp Ile Leu
1445 1450 1455

Ser Asp Ser Gly Met Leu Gly Cys Lys Pro Asn Ser Thr Pro Met
1460 1465 1470

Asp Tyr Ser Thr Lys Leu Gln Ala Asp Ser Gly Ser Leu Leu Ser
1475 1480 1485

Ala Glu Ser Ser Ser Ser Tyr Arg Arg Leu Ile Gly Lys Leu Ile
1490 1495 1500

Tyr Leu Thr Asn Thr Arg Pro Asp Ile Thr Tyr Ala Val Gln Gln
1505 1510 1515

Leu Ser Gln Tyr Met Ala Thr Pro Thr Asn Val His Leu Gln Ala
1520 1525 1530

Ala Phe Arg Ile Leu Arg Tyr Leu Lys Gly Thr Pro Gly Ser Gly
1535 1540 1545

Leu Phe Phe Ala Ala Thr Gly Thr Pro Gln Leu Arg Ala Phe Ser
1550 1555 1560

Asp Ser Asp Trp Ala Gly Cys Lys Asp Ser Arg Lys Ser Thr Pro
1565 1570 1575

Gly Tyr Leu Val Tyr Leu Gly Ser Ser Leu Val Ser Trp Gln Ser
1580 1585 1590

Lys Lys Gln Ser Thr Val Ser Arg Ser Ser Ser Glu Ala Glu Tyr
1595 1600 1605

Arg Ala Leu Ala Ser Thr Thr Cys Glu Leu Gln Trp Leu Thr Phe
1610 1615 1620

Leu Leu Gln Asp Phe Arg Ala Thr Phe Ile Gln Pro Ala Thr Leu
1625 1630 1635

Tyr Cys Asp Asn Gln Ser Thr Ile Gln Ile Ala Thr Asn Pro Val
1640 1645 1650

Phe His Glu Arg Thr Lys His Ile Glu Ile Asp Cys His Ile Val
1655 1660 1665

Arg Gln Lys Leu Asn Ser Ala Leu Ile Lys Leu Leu Pro Ser Asn
1670 1675 1680

Arg Lys Lys Ser His Phe Val Val Val Arg Ser Ala Ser Gln Ser
1685 1690 1695

Ala Pro Leu Phe Val Asp Asn Lys Arg Asp Ser Ser Glu Lys Leu
1700 1705 1710

Arg Gln Met Arg Pro Thr Leu Ser Arg Lys Phe Asn Ser Tyr Val
1715 1720 1725

Leu Pro Thr Pro Val Asp Ala Lys Ser Ser Ile Ser Met Arg Ser
1730 1735 1740

Ser Asn Gln Val Pro Leu Lys Ile Lys Thr Asn Leu Asn Glu Pro
1745 1750 1755

Met Lys Asn Leu Trp His Ser Ser Pro Leu Glu Gln Lys Lys Tyr
1760 1765 1770

Glu Asn Ile Phe Gly Asp Gly Gly Leu Ser Gly Pro Asn Ala Gln
1775 1780 1785

Ser Val Leu Lys Glu Ser Asn Ser Asn Thr Ala Tyr Ser Arg Leu
1790 1795 1800

Pro Pro Pro Leu Ile Asp Gly Asn Leu Ser Ser Ser His Asp Tyr
1805 1810 1815

Ile Thr Ala Tyr Ser Lys Lys Ile Lys Arg His Ala Phe Ser Gly
1820 1825 1830

Pro Leu Val Ser Asn Ala Trp Pro Thr Lys Pro Val Ser Leu Glu
1835 1840 1845

Ser Val Gln Leu Phe Ser Gly Pro Leu Leu Arg Thr Ser Ile Pro
1850 1855 1860

Gln Pro Pro Ser Ser Ser Pro Lys Val Ser Pro Ser Ala Ser Pro
1865 1870 1875

Pro Leu Ser Ser Ser Pro Lys Ile Asn Glu Leu His Glu Leu Pro
1880 1885 1890

Arg Pro Pro Thr Ile Ser Pro Ser Asp Ser Lys Leu Leu Gly Leu

```
    1895                1900                1905

Val Gly His Ser Gly Pro Leu Val Ser Arg Gly Pro Gln Leu Ser
    1910                1915                1920

Ala Ala Asn Tyr Leu Val Thr Ser Asn Ala Ala Ser Pro Leu Pro
    1925                1930                1935

Met Pro Ala Ser Ala Met Ala Arg Ser Phe Ser Thr Ser Tyr Arg
    1940                1945                1950

Gly Ala Lys Val Ala Ala Ile His Asp Ser Arg Pro Leu Glu Asp
    1955                1960                1965

Pro Asn Lys Ser Thr Ile Ser Glu Asp Ile Asp Ser Pro Pro Leu
    1970                1975                1980

Met Gln Ile Ala Leu Ser Thr Ser Gln Pro Ser Ser Asp Gly Ser
    1985                1990                1995

Asp Thr Val Ala Gln Ala Val
    2000                2005


<210>  13
<211>  918
<212>  DNA
<213>  Glycine max


<220>
<221>  CDS
<222>  (1)..(918)

<400>  13
atg ctt cct cca agt caa tca ttc agc ctc tgg cct agc ttg acg agc       48
Met Leu Pro Pro Ser Gln Ser Phe Ser Leu Trp Pro Ser Leu Thr Ser
1               5                   10                  15

gag ctc ggg cta ccc agc cat tta ttt ctt gaa aat gat atc ata cct       96
Glu Leu Gly Leu Pro Ser His Leu Phe Leu Glu Asn Asp Ile Ile Pro
            20                  25                  30

tac cgt aca aat gga tgg att gag caa gaa cct ctt caa gaa gac ttg      144
Tyr Arg Thr Asn Gly Trp Ile Glu Gln Glu Pro Leu Gln Glu Asp Leu
        35                  40                  45

ctt ata tta ctc atc ttg atg act tgc tgc tta acc acg ttt gtt tct      192
Leu Ile Leu Leu Ile Leu Met Thr Cys Cys Leu Thr Thr Phe Val Ser
    50                  55                  60

ttt aca gtt gga tta gga aaa ctt aat gca agt gaa aaa cta aga cag      240
Phe Thr Val Gly Leu Gly Lys Leu Asn Ala Ser Glu Lys Leu Arg Gln
65                  70                  75                  80

atg cgc cca tct tta tca cgg aag ttc agc tca tat gtg cta gcc aca      288
Met Arg Pro Ser Leu Ser Arg Lys Phe Ser Ser Tyr Val Leu Ala Thr
                85                  90                  95
```

```
cca gtt gat gct aag agt tca acc tct tca ggg tca aat aat cca aag      336
Pro Val Asp Ala Lys Ser Ser Thr Ser Ser Gly Ser Asn Asn Pro Lys
            100                 105                 110

cct tcc aat atg cag gca aat tta aga gaa cct aca aag aat ttg tgg      384
Pro Ser Asn Met Gln Ala Asn Leu Arg Glu Pro Thr Lys Asn Leu Trp
        115                 120                 125

cat tca tcc cca ttg gaa caa aag aaa cat gac aaa gat atg gga gat      432
His Ser Ser Pro Leu Glu Gln Lys Lys His Asp Lys Asp Met Gly Asp
    130                 135                 140

gag ttt tct ggt tca att atc aga agt gca caa tca tta ctc aag gaa      480
Glu Phe Ser Gly Ser Ile Ile Arg Ser Ala Gln Ser Leu Leu Lys Glu
145                 150                 155                 160

agt aac tgc ctc ctt caa cat gcc ttt tct ggc cca ttg aca agt aat      528
Ser Asn Cys Leu Leu Gln His Ala Phe Ser Gly Pro Leu Thr Ser Asn
                165                 170                 175

ctt ggg cct acc aac cca gtc aac tgt tct ctg tcg tca tct cca aaa      576
Leu Gly Pro Thr Asn Pro Val Asn Cys Ser Leu Ser Ser Ser Pro Lys
            180                 185                 190

gta tct cct agt gct tct ctt act ctt atg act tca cct caa ata agt      624
Val Ser Pro Ser Ala Ser Leu Thr Leu Met Thr Ser Pro Gln Ile Ser
        195                 200                 205

gag ctt cat gaa ctt cct agg cct cca acc agt ttc ccc tcc aat tca      672
Glu Leu His Glu Leu Pro Arg Pro Pro Thr Ser Phe Pro Ser Asn Ser
    210                 215                 220

agg ctt tta ggt ttg gtg ggt cat tct ggt cca ttg gtg tct aga gtg      720
Arg Leu Leu Gly Leu Val Gly His Ser Gly Pro Leu Val Ser Arg Val
225                 230                 235                 240

gca gca tta cat gtg cca aga aca cta gaa ttc tct cat aga tca tct      768
Ala Ala Leu His Val Pro Arg Thr Leu Glu Phe Ser His Arg Ser Ser
                245                 250                 255

ata ttt gag aat att gct tct cct cct agg atg cca ata gaa tta tct      816
Ile Phe Glu Asn Ile Ala Ser Pro Pro Arg Met Pro Ile Glu Leu Ser
            260                 265                 270

agt atc atc aga tgg ctg ggg cta acc aga act gcc att ggt ata gga      864
Ser Ile Ile Arg Trp Leu Gly Leu Thr Arg Thr Ala Ile Gly Ile Gly
        275                 280                 285

cac cta tca ttg agg aag ttc tat ctt caa agt ttt ggg ttt att aga      912
His Leu Ser Leu Arg Lys Phe Tyr Leu Gln Ser Phe Gly Phe Ile Arg
    290                 295                 300

aaa taa                                                              918
Lys
305


<210>  14
<211>  305
<212>  PRT
<213>  Glycine max

<400>  14

Met Leu Pro Pro Ser Gln Ser Phe Ser Leu Trp Pro Ser Leu Thr Ser
1               5                   10                  15
```

```
Glu Leu Gly Leu Pro Ser His Leu Phe Leu Glu Asn Asp Ile Ile Pro
            20                  25                  30

Tyr Arg Thr Asn Gly Trp Ile Glu Gln Glu Pro Leu Gln Glu Asp Leu
        35                  40                  45

Leu Ile Leu Leu Ile Leu Met Thr Cys Cys Leu Thr Thr Phe Val Ser
    50                  55                  60

Phe Thr Val Gly Leu Gly Lys Leu Asn Ala Ser Glu Lys Leu Arg Gln
65                  70                  75                  80

Met Arg Pro Ser Leu Ser Arg Lys Phe Ser Ser Tyr Val Leu Ala Thr
                85                  90                  95

Pro Val Asp Ala Lys Ser Ser Thr Ser Ser Gly Ser Asn Asn Pro Lys
            100                 105                 110

Pro Ser Asn Met Gln Ala Asn Leu Arg Glu Pro Thr Lys Asn Leu Trp
        115                 120                 125

His Ser Ser Pro Leu Glu Gln Lys Lys His Asp Lys Asp Met Gly Asp
    130                 135                 140

Glu Phe Ser Gly Ser Ile Ile Arg Ser Ala Gln Ser Leu Leu Lys Glu
145                 150                 155                 160

Ser Asn Cys Leu Leu Gln His Ala Phe Ser Gly Pro Leu Thr Ser Asn
                165                 170                 175

Leu Gly Pro Thr Asn Pro Val Asn Cys Ser Leu Ser Ser Ser Pro Lys
            180                 185                 190

Val Ser Pro Ser Ala Ser Leu Thr Leu Met Thr Ser Pro Gln Ile Ser
        195                 200                 205

Glu Leu His Glu Leu Pro Arg Pro Pro Thr Ser Phe Pro Ser Asn Ser
    210                 215                 220

Arg Leu Leu Gly Leu Val Gly His Ser Gly Pro Leu Val Ser Arg Val
225                 230                 235                 240

Ala Ala Leu His Val Pro Arg Thr Leu Glu Phe Ser His Arg Ser Ser
                245                 250                 255

Ile Phe Glu Asn Ile Ala Ser Pro Pro Arg Met Pro Ile Glu Leu Ser
            260                 265                 270
```

```
Ser Ile Ile Arg Trp Leu Gly Leu Thr Arg Thr Ala Ile Gly Ile Gly
        275                 280                 285

His Leu Ser Leu Arg Lys Phe Tyr Leu Gln Ser Phe Gly Phe Ile Arg
    290                 295                 300

Lys
305

<210>  15
<211>  672
<212>  DNA
<213>  H.annuus

<220>
<221>  CDS
<222>  (1)..(672)

<400>  15
atg aag agc ccg ttc aga aaa gtc cgt ggc cta ggg cta cac aaa cga        48
Met Lys Ser Pro Phe Arg Lys Val Arg Gly Leu Gly Leu His Lys Arg
1               5                   10                  15

cac cgt att caa cgc tct tcc gct caa tta gac gag ctc acc caa gct        96
His Arg Ile Gln Arg Ser Ser Ala Gln Leu Asp Glu Leu Thr Gln Ala
            20                  25                  30

act cag gat atg caa gat atg aaa gat tgc tat gac agt tta ctt acg       144
Thr Gln Asp Met Gln Asp Met Lys Asp Cys Tyr Asp Ser Leu Leu Thr
        35                  40                  45

gct gct gcg agc acc acc aac act gct tat gaa ttc tca gat tcg tta       192
Ala Ala Ala Ser Thr Thr Asn Thr Ala Tyr Glu Phe Ser Asp Ser Leu
    50                  55                  60

cag gaa atg ggt agt tgt ctt ctt gag aaa act gca cta agt gac gat       240
Gln Glu Met Gly Ser Cys Leu Leu Glu Lys Thr Ala Leu Ser Asp Asp
65                  70                  75                  80

gaa gac agt ggc aga gtg ttg tta atg ctt gga aag gtg caa ttt gaa       288
Glu Asp Ser Gly Arg Val Leu Leu Met Leu Gly Lys Val Gln Phe Glu
                85                  90                  95

atc cag aag ctg att gat aat tat cga aca cat att tcc caa aca ctc       336
Ile Gln Lys Leu Ile Asp Asn Tyr Arg Thr His Ile Ser Gln Thr Leu
            100                 105                 110

acg gtt cct tca caa tct ctt cta aac gaa ttg ctt aat gtt gag gac       384
Thr Val Pro Ser Gln Ser Leu Leu Asn Glu Leu Leu Asn Val Glu Asp
        115                 120                 125

atg aaa aga caa tgt gat gaa aaa agg aca caa tat gaa gcg ttg aaa       432
Met Lys Arg Gln Cys Asp Glu Lys Arg Thr Gln Tyr Glu Ala Leu Lys
    130                 135                 140

aca caa tac gaa aag gga aag atg aaa ggc agt aaa gta gaa cat gtt       480
Thr Gln Tyr Glu Lys Gly Lys Met Lys Gly Ser Lys Val Glu His Val
145                 150                 155                 160

tct tct cgc cag ctg caa aca gct tgc gat gaa ttt gat gag ggt gct       528
Ser Ser Arg Gln Leu Gln Thr Ala Cys Asp Glu Phe Asp Glu Gly Ala
                165                 170                 175
```

acg gta ttt att ttc cgc atg aaa tct ttg aaa aag gct caa tct cgt 576
Thr Val Phe Ile Phe Arg Met Lys Ser Leu Lys Lys Ala Gln Ser Arg
180 185 190

agt ctt cta aca caa gca gct cgc cac tat gct gct cag atg tcg ttt 624
Ser Leu Leu Thr Gln Ala Ala Arg His Tyr Ala Ala Gln Met Ser Phe
195 200 205

ttc aga aag gcg ctc aaa tca ctg gaa agc aat cga acc aca tgt taa 672
Phe Arg Lys Ala Leu Lys Ser Leu Glu Ser Asn Arg Thr Thr Cys
210 215 220

<210> 16
<211> 223
<212> PRT
<213> H.annuus

<400> 16

Met Lys Ser Pro Phe Arg Lys Val Arg Gly Leu Gly Leu His Lys Arg
1 5 10 15

His Arg Ile Gln Arg Ser Ser Ala Gln Leu Asp Glu Leu Thr Gln Ala
20 25 30

Thr Gln Asp Met Gln Asp Met Lys Asp Cys Tyr Asp Ser Leu Leu Thr
35 40 45

Ala Ala Ala Ser Thr Thr Asn Thr Ala Tyr Glu Phe Ser Asp Ser Leu
50 55 60

Gln Glu Met Gly Ser Cys Leu Leu Glu Lys Thr Ala Leu Ser Asp Asp
65 70 75 80

Glu Asp Ser Gly Arg Val Leu Leu Met Leu Gly Lys Val Gln Phe Glu
85 90 95

Ile Gln Lys Leu Ile Asp Asn Tyr Arg Thr His Ile Ser Gln Thr Leu
100 105 110

Thr Val Pro Ser Gln Ser Leu Leu Asn Glu Leu Leu Asn Val Glu Asp
115 120 125

Met Lys Arg Gln Cys Asp Glu Lys Arg Thr Gln Tyr Glu Ala Leu Lys
130 135 140

Thr Gln Tyr Glu Lys Gly Lys Met Lys Gly Ser Lys Val Glu His Val
145 150 155 160

Ser Ser Arg Gln Leu Gln Thr Ala Cys Asp Glu Phe Asp Glu Gly Ala
165 170 175

Thr Val Phe Ile Phe Arg Met Lys Ser Leu Lys Lys Ala Gln Ser Arg

```
        180                 185                 190

Ser Leu Leu Thr Gln Ala Ala Arg His Tyr Ala Ala Gln Met Ser Phe
            195                 200                 205

Phe Arg Lys Ala Leu Lys Ser Leu Glu Ser Asn Arg Thr Thr Cys
    210                 215                 220
```

<210> 17
<211> 630
<212> DNA
<213> M.truncatula

<220>
<221> CDS
<222> (1)..(630)

<400> 17

```
atg cgg cca tca aag ctc cat cag ata gcg gcg ttg aca tct aag gtt      48
Met Arg Pro Ser Lys Leu His Gln Ile Ala Ala Leu Thr Ser Lys Val
1               5                   10                  15

gac aag aac ata aaa aaa aat gga aaa ttt gga cca att aga gtt cca      96
Asp Lys Asn Ile Lys Lys Asn Gly Lys Phe Gly Pro Ile Arg Val Pro
            20                  25                  30

cgt ggt gta aac aat caa att ttt gag gat tcg aat tct tta aag gtg     144
Arg Gly Val Asn Asn Gln Ile Phe Glu Asp Ser Asn Ser Leu Lys Val
        35                  40                  45

aag gag aag gat aac atg aag cat cac tgt gat gaa aaa aga gaa gtt     192
Lys Glu Lys Asp Asn Met Lys His His Cys Asp Glu Lys Arg Glu Val
    50                  55                  60

tat gaa tac atg att atc caa ccg aaa gaa aag ggg aag tca aac agt     240
Tyr Glu Tyr Met Ile Ile Gln Pro Lys Glu Lys Gly Lys Ser Asn Ser
65                  70                  75                  80

ggt aag ggt gaa cat ata act tca cgg cct ttg caa gct gct cac gat     288
Gly Lys Gly Glu His Ile Thr Ser Arg Pro Leu Gln Ala Ala His Asp
                85                  90                  95

gaa tat gaa gag gaa gct aca ctc gcc aaa caa gga tgt cct cac atc     336
Glu Tyr Glu Glu Glu Ala Thr Leu Ala Lys Gln Gly Cys Pro His Ile
            100                 105                 110

gcc aaa ctc ggc aaa ggt aga aga atc aaa ccg ctc aaa cat tca aac     384
Ala Lys Leu Gly Lys Gly Arg Arg Ile Lys Pro Leu Lys His Ser Asn
        115                 120                 125

ttc aac gaa aga aac tgc aga gac aag ttt aga caa gat cca tgt agt     432
Phe Asn Glu Arg Asn Cys Arg Asp Lys Phe Arg Gln Asp Pro Cys Ser
    130                 135                 140

gaa gca atg tcg cct ctt gat gta tat agt gat agc gtt tat gat gtg     480
Glu Ala Met Ser Pro Leu Asp Val Tyr Ser Asp Ser Val Tyr Asp Val
145                 150                 155                 160

gac act aat agt gat act gaa gat ata gaa gtc aat aac gac atc aat     528
Asp Thr Asn Ser Asp Thr Glu Asp Ile Glu Val Asn Asn Asp Ile Asn
                165                 170                 175
```

```
gtt cgt agt gat aga gat gta ggc agt atg gaa ttc aac cat ggt aat      576
Val Arg Ser Asp Arg Asp Val Gly Ser Met Glu Phe Asn His Gly Asn
            180                 185                 190

ggt gtt tgc agt gag caa ggg aaa aga gaa ttc caa tgg tta caa atg      624
Gly Val Cys Ser Glu Gln Gly Lys Arg Glu Phe Gln Trp Leu Gln Met
        195                 200                 205

cat tag                                                              630
His
```

<210> 18
<211> 209
<212> PRT
<213> M.truncatula

<400> 18

```
Met Arg Pro Ser Lys Leu His Gln Ile Ala Ala Leu Thr Ser Lys Val
1               5                   10                  15

Asp Lys Asn Ile Lys Lys Asn Gly Lys Phe Gly Pro Ile Arg Val Pro
            20                  25                  30

Arg Gly Val Asn Asn Gln Ile Phe Glu Asp Ser Asn Ser Leu Lys Val
        35                  40                  45

Lys Glu Lys Asp Asn Met Lys His His Cys Asp Glu Lys Arg Glu Val
    50                  55                  60

Tyr Glu Tyr Met Ile Ile Gln Pro Lys Glu Lys Gly Lys Ser Asn Ser
65                  70                  75                  80

Gly Lys Gly Glu His Ile Thr Ser Arg Pro Leu Gln Ala Ala His Asp
                85                  90                  95

Glu Tyr Glu Glu Glu Ala Thr Leu Ala Lys Gln Gly Cys Pro His Ile
            100                 105                 110

Ala Lys Leu Gly Lys Gly Arg Arg Ile Lys Pro Leu Lys His Ser Asn
        115                 120                 125

Phe Asn Glu Arg Asn Cys Arg Asp Lys Phe Arg Gln Asp Pro Cys Ser
    130                 135                 140

Glu Ala Met Ser Pro Leu Asp Val Tyr Ser Asp Ser Val Tyr Asp Val
145                 150                 155                 160

Asp Thr Asn Ser Asp Thr Glu Asp Ile Glu Val Asn Asn Asp Ile Asn
                165                 170                 175

Val Arg Ser Asp Arg Asp Val Gly Ser Met Glu Phe Asn His Gly Asn
            180                 185                 190
```

```
Gly Val Cys Ser Glu Gln Gly Lys Arg Glu Phe Gln Trp Leu Gln Met
        195                 200                 205

His


<210>  19
<211>  1887
<212>  DNA
<213>  M.truncatula


<220>
<221>  CDS
<222>  (1)..(1887)

<400>  19
atg aag agt tct ctg aaa aag tta cga ggt ttg gca ctt cac aat cat        48
Met Lys Ser Ser Leu Lys Lys Leu Arg Gly Leu Ala Leu His Asn His
1               5                   10                  15

cat cat cat cat cat cat cat cac aaa cat gat aca att aac aac aaa        96
His His His His His His His His Lys His Asp Thr Ile Asn Asn Lys
            20                  25                  30

aca att ctt cct ctt aaa caa ctt gac gaa ctc gaa aag gct aca agg       144
Thr Ile Leu Pro Leu Lys Gln Leu Asp Glu Leu Glu Lys Ala Thr Arg
        35                  40                  45

gag atg caa gac atg agg gac tgc tat gat acc tta ctt tcc gca gcg       192
Glu Met Gln Asp Met Arg Asp Cys Tyr Asp Thr Leu Leu Ser Ala Ala
    50                  55                  60

gcc gca acc gcc agt agt gct tat gaa ttc gct gag tcg ttg cgg gac       240
Ala Ala Thr Ala Ser Ser Ala Tyr Glu Phe Ala Glu Ser Leu Arg Asp
65                  70                  75                  80

atg ggt tct tgt tta ctc gag aaa act gct ttg aat gat cac gaa gaa       288
Met Gly Ser Cys Leu Leu Glu Lys Thr Ala Leu Asn Asp His Glu Glu
                85                  90                  95

gaa act gga aag gtt ctt ctt atg ctt gga aaa atc cag ttc aaa ctt       336
Glu Thr Gly Lys Val Leu Leu Met Leu Gly Lys Ile Gln Phe Lys Leu
            100                 105                 110

cag aaa ctt att gat aac tat cgt tcc cat ata ata cag aca att aca       384
Gln Lys Leu Ile Asp Asn Tyr Arg Ser His Ile Ile Gln Thr Ile Thr
        115                 120                 125

gtt cca tct gag tca ctc ttg aat gaa ctt cga att gtt gag gag atg       432
Val Pro Ser Glu Ser Leu Leu Asn Glu Leu Arg Ile Val Glu Glu Met
    130                 135                 140

aag cga cag tgt gat gaa aaa aga gat gtg tat gag tat atg ata gca       480
Lys Arg Gln Cys Asp Glu Lys Arg Asp Val Tyr Glu Tyr Met Ile Ala
145                 150                 155                 160

aga tac aga gaa aga ggt agg tct aaa ggt ggc aaa gga gaa act ttt       528
Arg Tyr Arg Glu Arg Gly Arg Ser Lys Gly Gly Lys Gly Glu Thr Phe
                165                 170                 175

aca ttg cag cag ctg caa gct gct cgc gat gaa tat gat gag gag gcc       576
```

```
Thr Leu Gln Gln Leu Gln Ala Ala Arg Asp Glu Tyr Asp Glu Glu Ala
            180                 185                 190

aca ttg ttt gtt ttc cga ttg aaa tct ctg aag caa gga caa tca cgt      624
Thr Leu Phe Val Phe Arg Leu Lys Ser Leu Lys Gln Gly Gln Ser Arg
        195                 200                 205

agt ctt cta acc cag gca gca cgt cat cat gct tct cag tca tgt ttc      672
Ser Leu Leu Thr Gln Ala Ala Arg His His Ala Ser Gln Ser Cys Phe
    210                 215                 220

ttc aag aaa gca gcc aaa tct ctt gag aca gta gag cca cat gtg aag      720
Phe Lys Lys Ala Ala Lys Ser Leu Glu Thr Val Glu Pro His Val Lys
225                 230                 235                 240

tca gta acc gaa caa caa cac att gat tac cac ttc agt ggt ttg gaa      768
Ser Val Thr Glu Gln Gln His Ile Asp Tyr His Phe Ser Gly Leu Glu
                245                 250                 255

gag gag gat ggg gat gaa ggt gat tat gta gac gaa gac gac gag ggt      816
Glu Glu Asp Gly Asp Glu Gly Asp Tyr Val Asp Glu Asp Asp Glu Gly
            260                 265                 270

tat gat gag aat gat gat ggg gaa ctg agt ttt gac tat gga cca aat      864
Tyr Asp Glu Asn Asp Asp Gly Glu Leu Ser Phe Asp Tyr Gly Pro Asn
        275                 280                 285

gaa cag gag cga gat gtt tct aca tct aga aat tca atg gag ctg gac      912
Glu Gln Glu Arg Asp Val Ser Thr Ser Arg Asn Ser Met Glu Leu Asp
    290                 295                 300

cag gtg gaa cat aca ctt ccc aga ggt tca cca gca ggg ggt gct aag      960
Gln Val Glu His Thr Leu Pro Arg Gly Ser Pro Ala Gly Gly Ala Lys
305                 310                 315                 320

gaa aac ttg gat aag ctt caa agg aat ttg ttt tct ttt aag gtt agg     1008
Glu Asn Leu Asp Lys Leu Gln Arg Asn Leu Phe Ser Phe Lys Val Arg
                325                 330                 335

gca ggg agc caa tct gcc cca ctt ttt gcc gat aat aaa cct gat tcc     1056
Ala Gly Ser Gln Ser Ala Pro Leu Phe Ala Asp Asn Lys Pro Asp Ser
            340                 345                 350

agt gaa aag ctg agg cag atg cgc cca tct tta tcg cga aaa ttc agt     1104
Ser Glu Lys Leu Arg Gln Met Arg Pro Ser Leu Ser Arg Lys Phe Ser
        355                 360                 365

tca tat gtg cta cca act cca gtt gat gcg aag agt cca atc tct ttt     1152
Ser Tyr Val Leu Pro Thr Pro Val Asp Ala Lys Ser Pro Ile Ser Phe
    370                 375                 380

ttt cca gat aaa cca aag cct tcc aca atg cag aca aat tta aat gaa     1200
Phe Pro Asp Lys Pro Lys Pro Ser Thr Met Gln Thr Asn Leu Asn Glu
385                 390                 395                 400

cca aca aag aac ttg tgg cat tca tcc cca ttg gat caa aag aaa cat     1248
Pro Thr Lys Asn Leu Trp His Ser Ser Pro Leu Asp Gln Lys Lys His
                405                 410                 415

gaa aaa gat atc aga gat gaa cat tct gat cct act atc aga aac act     1296
Glu Lys Asp Ile Arg Asp Glu His Ser Asp Pro Thr Ile Arg Asn Thr
            420                 425                 430

cag tct gca tta agg gaa agc aac aat aat gct tcc ttc aca aga ctg     1344
Gln Ser Ala Leu Arg Glu Ser Asn Asn Asn Ala Ser Phe Thr Arg Leu
        435                 440                 445
```

```
ccg ctt cct ttg gta gat ggt cct gca tcc tta aat cat gac aat gtt      1392
Pro Leu Pro Leu Val Asp Gly Pro Ala Ser Leu Asn His Asp Asn Val
    450                 455                 460

tct gct tac tct aaa aag att aaa aga cat gcc ttt tcc ggc cca ctg      1440
Ser Ala Tyr Ser Lys Lys Ile Lys Arg His Ala Phe Ser Gly Pro Leu
465                 470                 475                 480

aca agt aat cct tgg cct acc aga cct gtc tca atg gaa aat att cag      1488
Thr Ser Asn Pro Trp Pro Thr Arg Pro Val Ser Met Glu Asn Ile Gln
                485                 490                 495

ttg ttt tct gga cct ctt ttg cct act cga atc cct cag cct cca tca      1536
Leu Phe Ser Gly Pro Leu Leu Pro Thr Arg Ile Pro Gln Pro Pro Ser
            500                 505                 510

tca tcc ccc aaa gtt tct cct agt gct tct cct act atc ttg tct tca      1584
Ser Ser Pro Lys Val Ser Pro Ser Ala Ser Pro Thr Ile Leu Ser Ser
        515                 520                 525

cct aaa ata agc gag ctt cat gaa ctt cca agg cct cca gcc aat tcc      1632
Pro Lys Ile Ser Glu Leu His Glu Leu Pro Arg Pro Pro Ala Asn Ser
    530                 535                 540

ccg ccc aac tct agg ctt tta ggt ttg atg ggg cat tca ggt cca tta      1680
Pro Pro Asn Ser Arg Leu Leu Gly Leu Met Gly His Ser Gly Pro Leu
545                 550                 555                 560

gtg tct aga ggt caa aat gtt tcc gct gca aat aat ttg gtt gtt tca      1728
Val Ser Arg Gly Gln Asn Val Ser Ala Ala Asn Asn Leu Val Val Ser
                565                 570                 575

agt gta gca tct cca ctg cca atg cca ccg cag gct atg tct cgt agt      1776
Ser Val Ala Ser Pro Leu Pro Met Pro Pro Gln Ala Met Ser Arg Ser
            580                 585                 590

ttc tct ata cct tct tct ggt agt gct cga gtt gca gca tta atg ggc      1824
Phe Ser Ile Pro Ser Ser Gly Ser Ala Arg Val Ala Ala Leu Met Gly
        595                 600                 605

caa ggg gac gag aat cct ctc ata cat cat ctc tat ccg agg aaa ttg      1872
Gln Gly Asp Glu Asn Pro Leu Ile His His Leu Tyr Pro Arg Lys Leu
    610                 615                 620

ctt ctc ctc cgc taa                                                  1887
Leu Leu Leu Arg
625


<210>  20
<211>  628
<212>  PRT
<213>  M.truncatula

<400>  20

Met Lys Ser Ser Leu Lys Lys Leu Arg Gly Leu Ala Leu His Asn His
1               5                   10                  15


His His His His His His His His Lys His Asp Thr Ile Asn Asn Lys
            20                  25                  30


Thr Ile Leu Pro Leu Lys Gln Leu Asp Glu Leu Glu Lys Ala Thr Arg
```

```
        35                  40                  45

Glu Met Gln Asp Met Arg Asp Cys Tyr Asp Thr Leu Leu Ser Ala Ala
    50                  55                  60

Ala Ala Thr Ala Ser Ser Ala Tyr Glu Phe Ala Glu Ser Leu Arg Asp
65                  70                  75                  80

Met Gly Ser Cys Leu Leu Glu Lys Thr Ala Leu Asn Asp His Glu Glu
                85                  90                  95

Glu Thr Gly Lys Val Leu Leu Met Leu Gly Lys Ile Gln Phe Lys Leu
            100                 105                 110

Gln Lys Leu Ile Asp Asn Tyr Arg Ser His Ile Ile Gln Thr Ile Thr
        115                 120                 125

Val Pro Ser Glu Ser Leu Leu Asn Glu Leu Arg Ile Val Glu Glu Met
    130                 135                 140

Lys Arg Gln Cys Asp Glu Lys Arg Asp Val Tyr Glu Tyr Met Ile Ala
145                 150                 155                 160

Arg Tyr Arg Glu Arg Gly Arg Ser Lys Gly Gly Lys Gly Glu Thr Phe
                165                 170                 175

Thr Leu Gln Gln Leu Gln Ala Ala Arg Asp Glu Tyr Asp Glu Glu Ala
            180                 185                 190

Thr Leu Phe Val Phe Arg Leu Lys Ser Leu Lys Gln Gly Gln Ser Arg
        195                 200                 205

Ser Leu Leu Thr Gln Ala Ala Arg His His Ala Ser Gln Ser Cys Phe
    210                 215                 220

Phe Lys Lys Ala Ala Lys Ser Leu Glu Thr Val Glu Pro His Val Lys
225                 230                 235                 240

Ser Val Thr Glu Gln Gln His Ile Asp Tyr His Phe Ser Gly Leu Glu
                245                 250                 255

Glu Glu Asp Gly Asp Glu Gly Asp Tyr Val Asp Glu Asp Asp Glu Gly
            260                 265                 270

Tyr Asp Glu Asn Asp Asp Gly Glu Leu Ser Phe Asp Tyr Gly Pro Asn
        275                 280                 285

Glu Gln Glu Arg Asp Val Ser Thr Ser Arg Asn Ser Met Glu Leu Asp
    290                 295                 300
```

Gln Val Glu His Thr Leu Pro Arg Gly Ser Pro Ala Gly Gly Ala Lys
305                 310                 315                 320

Glu Asn Leu Asp Lys Leu Gln Arg Asn Leu Phe Ser Phe Lys Val Arg
                325                 330                 335

Ala Gly Ser Gln Ser Ala Pro Leu Phe Ala Asp Asn Lys Pro Asp Ser
            340                 345                 350

Ser Glu Lys Leu Arg Gln Met Arg Pro Ser Leu Ser Arg Lys Phe Ser
        355                 360                 365

Ser Tyr Val Leu Pro Thr Pro Val Asp Ala Lys Ser Pro Ile Ser Phe
    370                 375                 380

Phe Pro Asp Lys Pro Lys Pro Ser Thr Met Gln Thr Asn Leu Asn Glu
385                 390                 395                 400

Pro Thr Lys Asn Leu Trp His Ser Ser Pro Leu Asp Gln Lys Lys His
                405                 410                 415

Glu Lys Asp Ile Arg Asp Glu His Ser Asp Pro Thr Ile Arg Asn Thr
            420                 425                 430

Gln Ser Ala Leu Arg Glu Ser Asn Asn Asn Ala Ser Phe Thr Arg Leu
        435                 440                 445

Pro Leu Pro Leu Val Asp Gly Pro Ala Ser Leu Asn His Asp Asn Val
    450                 455                 460

Ser Ala Tyr Ser Lys Lys Ile Lys Arg His Ala Phe Ser Gly Pro Leu
465                 470                 475                 480

Thr Ser Asn Pro Trp Pro Thr Arg Pro Val Ser Met Glu Asn Ile Gln
                485                 490                 495

Leu Phe Ser Gly Pro Leu Leu Pro Thr Arg Ile Pro Gln Pro Pro Ser
            500                 505                 510

Ser Ser Pro Lys Val Ser Pro Ser Ala Ser Pro Thr Ile Leu Ser Ser
        515                 520                 525

Pro Lys Ile Ser Glu Leu His Glu Leu Pro Arg Pro Pro Ala Asn Ser
    530                 535                 540

Pro Pro Asn Ser Arg Leu Leu Gly Leu Met Gly His Ser Gly Pro Leu
545                 550                 555                 560

Val Ser Arg Gly Gln Asn Val Ser Ala Ala Asn Asn Leu Val Val Ser

```
                565                 570                 575

Ser Val Ala Ser Pro Leu Pro Met Pro Pro Gln Ala Met Ser Arg Ser
            580                 585                 590

Phe Ser Ile Pro Ser Ser Gly Ser Ala Arg Val Ala Ala Leu Met Gly
        595                 600                 605

Gln Gly Asp Glu Asn Pro Leu Ile His His Leu Tyr Pro Arg Lys Leu
    610                 615                 620

Leu Leu Leu Arg
625

<210>  21
<211>  1635
<212>  DNA
<213>  M.truncatula

<220>
<221>  CDS
<222>  (1)..(1635)

<400>  21
atg aag tcc ttt aac aaa ttg aag aga att gca ctt cac aaa acc gtt       48
Met Lys Ser Phe Asn Lys Leu Lys Arg Ile Ala Leu His Lys Thr Val
1               5                   10                  15

gga aaa gaa aag aag gag ttt ctt cct tcc gtt aag gtc gat gaa ctc       96
Gly Lys Glu Lys Lys Glu Phe Leu Pro Ser Val Lys Val Asp Glu Leu
            20                  25                  30

gct ctc gct gct aag tat atg caa gag atg aga gat tgt tat gat agc      144
Ala Leu Ala Ala Lys Tyr Met Gln Glu Met Arg Asp Cys Tyr Asp Ser
        35                  40                  45

ttg ctt gct gca gct gct gct act gaa aac agc gcg tat gaa ttt tcg      192
Leu Leu Ala Ala Ala Ala Ala Thr Glu Asn Ser Ala Tyr Glu Phe Ser
    50                  55                  60

gag tca ttg caa gaa atg ggt act tgt cta ttg gag aaa act gcc tta      240
Glu Ser Leu Gln Glu Met Gly Thr Cys Leu Leu Glu Lys Thr Ala Leu
65                  70                  75                  80

aat gat gat gaa gag agt ggt aaa gtt ttg ggg atg ctt gga aat gtg      288
Asn Asp Asp Glu Glu Ser Gly Lys Val Leu Gly Met Leu Gly Asn Val
                85                  90                  95

cag ctt gac ctt cag aaa ctt gtt gat ggc tac cgt tct cat gta gca      336
Gln Leu Asp Leu Gln Lys Leu Val Asp Gly Tyr Arg Ser His Val Ala
            100                 105                 110

ctg acc ata acc aga cca tcg gag tct ctt ctt aat gaa cta aga act      384
Leu Thr Ile Thr Arg Pro Ser Glu Ser Leu Leu Asn Glu Leu Arg Thr
        115                 120                 125

gtt gag gac atg aag cgt cag tgt gat gaa aaa aga gaa gtt tat gaa      432
Val Glu Asp Met Lys Arg Gln Cys Asp Glu Lys Arg Glu Val Tyr Glu
    130                 135                 140
```

tac atg att gcc caa cag aaa gaa aaa ggg aag tca aaa agt ggt aag 480
Tyr Met Ile Ala Gln Gln Lys Glu Lys Gly Lys Ser Lys Ser Gly Lys
145 150 155 160

ggt gaa aat ata act tca cag cat ttg aaa gct gct cac gat gaa tat 528
Gly Glu Asn Ile Thr Ser Gln His Leu Lys Ala Ala His Asp Glu Tyr
165 170 175

gaa gag gaa gca aca ctc tgt gct ttt cgg tta aaa tcc ctg aaa caa 576
Glu Glu Glu Ala Thr Leu Cys Ala Phe Arg Leu Lys Ser Leu Lys Gln
180 185 190

ggc cag tca cgt agt ctc cta aca caa gcc gct cgc cac cat gct gct 624
Gly Gln Ser Arg Ser Leu Leu Thr Gln Ala Ala Arg His His Ala Ala
195 200 205

cag ttg aat ttc ttc cgg aag gga ctt aaa tca ctg gag gcc gtt gag 672
Gln Leu Asn Phe Phe Arg Lys Gly Leu Lys Ser Leu Glu Ala Val Glu
210 215 220

cca cat gtt agg atg gtt gcc gag ttg caa cat att gat tac caa ttc 720
Pro His Val Arg Met Val Ala Glu Leu Gln His Ile Asp Tyr Gln Phe
225 230 235 240

agt ggc ctt gaa gat gat gat ggt gaa ggc agt ttt gaa tat gat ggg 768
Ser Gly Leu Glu Asp Asp Asp Gly Glu Gly Ser Phe Glu Tyr Asp Gly
245 250 255

aat gag tat gag tac gag gcc acc gaa ggc ggc gag ttg agt ttt aac 816
Asn Glu Tyr Glu Tyr Glu Ala Thr Glu Gly Gly Glu Leu Ser Phe Asn
260 265 270

tac aga tca aac aag gat gtc ccc aca tcg cca aac tca gca gag gtg 864
Tyr Arg Ser Asn Lys Asp Val Pro Thr Ser Pro Asn Ser Ala Glu Val
275 280 285

gaa gaa tca gac cgt tca aac att cga gct tca acc act gaa act gca 912
Glu Glu Ser Asp Arg Ser Asn Ile Arg Ala Ser Thr Thr Glu Thr Ala
290 295 300

gag aca agt tta gac aag agc cat gta gat ttt aaa gta tca aat aga 960
Glu Thr Ser Leu Asp Lys Ser His Val Asp Phe Lys Val Ser Asn Arg
305 310 315 320

gat cca cca aga gtc agc agc tat tca gct cca ata ttt gca gaa aag 1008
Asp Pro Pro Arg Val Ser Ser Tyr Ser Ala Pro Ile Phe Ala Glu Lys
325 330 335

aaa ttt gac cca gct gaa aaa gta aga caa ctg ttg tca tca tct gca 1056
Lys Phe Asp Pro Ala Glu Lys Val Arg Gln Leu Leu Ser Ser Ser Ala
340 345 350

gca aag cct aat gct tat gta ctt cct tta cct gtc aac atc aaa gaa 1104
Ala Lys Pro Asn Ala Tyr Val Leu Pro Leu Pro Val Asn Ile Lys Glu
355 360 365

aca aaa act gca cca cgc tta agt gca agt gca tct tcg cat gat ttg 1152
Thr Lys Thr Ala Pro Arg Leu Ser Ala Ser Ala Ser Ser His Asp Leu
370 375 380

tgg cat tca tct cct ttg gat gaa aaa aag aac ggg aaa gat ttt gct 1200
Trp His Ser Ser Pro Leu Asp Glu Lys Lys Asn Gly Lys Asp Phe Ala
385 390 395 400

gat ggt aaa ttg tca gaa cca gcc atc cct aga gtc tct att ctc aaa 1248
Asp Gly Lys Leu Ser Glu Pro Ala Ile Pro Arg Val Ser Ile Leu Lys

```
                405                 410                 415

gaa agc aac agt gac act tca tca gcc cag tta cca cgt ccg tcg aca      1296
Glu Ser Asn Ser Asp Thr Ser Ser Ala Gln Leu Pro Arg Pro Ser Thr
            420                 425                 430

gag gga cag tca ctt ccc caa gtt gat att ttt aac gct tct gat cat      1344
Glu Gly Gln Ser Leu Pro Gln Val Asp Ile Phe Asn Ala Ser Asp His
        435                 440                 445

aag aaa ata aaa agg cat gca ttt tca ggt cca ttg act aat aag cca      1392
Lys Lys Ile Lys Arg His Ala Phe Ser Gly Pro Leu Thr Asn Lys Pro
    450                 455                 460

ttg tct gta aaa cct gtc tct ggg ggt ttt tca cgg ttg ccg atg ccc      1440
Leu Ser Val Lys Pro Val Ser Gly Gly Phe Ser Arg Leu Pro Met Pro
465                 470                 475                 480

caa cct tcc tct cca aaa gca tct cct ggt gct tct cct ccc ctt gtt      1488
Gln Pro Ser Ser Pro Lys Ala Ser Pro Gly Ala Ser Pro Pro Leu Val
                485                 490                 495

tct tca cct agg ata agt gaa cta cat gag ctt cct agg ccc cct ggt      1536
Ser Ser Pro Arg Ile Ser Glu Leu His Glu Leu Pro Arg Pro Pro Gly
            500                 505                 510

aat caa acc agc aag gca aca aaa tct tct cga ttc agc atc tcc act      1584
Asn Gln Thr Ser Lys Ala Thr Lys Ser Ser Arg Phe Ser Ile Ser Thr
        515                 520                 525

tcc aac tcc acc cat tac cgt ctc tcg gag ttt ttc cat acc ctc aag      1632
Ser Asn Ser Thr His Tyr Arg Leu Ser Glu Phe Phe His Thr Leu Lys
    530                 535                 540

tag                                                                  1635


<210>  22
<211>  544
<212>  PRT
<213>  M.truncatula

<400>  22

Met Lys Ser Phe Asn Lys Leu Lys Arg Ile Ala Leu His Lys Thr Val
1               5                   10                  15


Gly Lys Glu Lys Lys Glu Phe Leu Pro Ser Val Lys Val Asp Glu Leu
            20                  25                  30


Ala Leu Ala Ala Lys Tyr Met Gln Glu Met Arg Asp Cys Tyr Asp Ser
        35                  40                  45


Leu Leu Ala Ala Ala Ala Ala Thr Glu Asn Ser Ala Tyr Glu Phe Ser
    50                  55                  60


Glu Ser Leu Gln Glu Met Gly Thr Cys Leu Leu Glu Lys Thr Ala Leu
65                  70                  75                  80


Asn Asp Asp Glu Glu Ser Gly Lys Val Leu Gly Met Leu Gly Asn Val
                85                  90                  95
```

```
Gln Leu Asp Leu Gln Lys Leu Val Asp Gly Tyr Arg Ser His Val Ala
            100                 105                 110

Leu Thr Ile Thr Arg Pro Ser Glu Ser Leu Leu Asn Glu Leu Arg Thr
        115                 120                 125

Val Glu Asp Met Lys Arg Gln Cys Asp Glu Lys Arg Glu Val Tyr Glu
    130                 135                 140

Tyr Met Ile Ala Gln Gln Lys Glu Lys Gly Lys Ser Lys Ser Gly Lys
145                 150                 155                 160

Gly Glu Asn Ile Thr Ser Gln His Leu Lys Ala Ala His Asp Glu Tyr
                165                 170                 175

Glu Glu Glu Ala Thr Leu Cys Ala Phe Arg Leu Lys Ser Leu Lys Gln
            180                 185                 190

Gly Gln Ser Arg Ser Leu Leu Thr Gln Ala Ala Arg His His Ala Ala
        195                 200                 205

Gln Leu Asn Phe Phe Arg Lys Gly Leu Lys Ser Leu Glu Ala Val Glu
    210                 215                 220

Pro His Val Arg Met Val Ala Glu Leu Gln His Ile Asp Tyr Gln Phe
225                 230                 235                 240

Ser Gly Leu Glu Asp Asp Asp Gly Glu Gly Ser Phe Glu Tyr Asp Gly
                245                 250                 255

Asn Glu Tyr Glu Tyr Glu Ala Thr Glu Gly Gly Glu Leu Ser Phe Asn
            260                 265                 270

Tyr Arg Ser Asn Lys Asp Val Pro Thr Ser Pro Asn Ser Ala Glu Val
        275                 280                 285

Glu Glu Ser Asp Arg Ser Asn Ile Arg Ala Ser Thr Thr Glu Thr Ala
    290                 295                 300

Glu Thr Ser Leu Asp Lys Ser His Val Asp Phe Lys Val Ser Asn Arg
305                 310                 315                 320

Asp Pro Pro Arg Val Ser Ser Tyr Ser Ala Pro Ile Phe Ala Glu Lys
                325                 330                 335

Lys Phe Asp Pro Ala Glu Lys Val Arg Gln Leu Leu Ser Ser Ser Ala
            340                 345                 350
```

Ala Lys Pro Asn Ala Tyr Val Leu Pro Leu Pro Val Asn Ile Lys Glu
355 360 365

Thr Lys Thr Ala Pro Arg Leu Ser Ala Ser Ala Ser Ser His Asp Leu
370 375 380

Trp His Ser Ser Pro Leu Asp Glu Lys Lys Asn Gly Lys Asp Phe Ala
385 390 395 400

Asp Gly Lys Leu Ser Glu Pro Ala Ile Pro Arg Val Ser Ile Leu Lys
405 410 415

Glu Ser Asn Ser Asp Thr Ser Ser Ala Gln Leu Pro Arg Pro Ser Thr
420 425 430

Glu Gly Gln Ser Leu Pro Gln Val Asp Ile Phe Asn Ala Ser Asp His
435 440 445

Lys Lys Ile Lys Arg His Ala Phe Ser Gly Pro Leu Thr Asn Lys Pro
450 455 460

Leu Ser Val Lys Pro Val Ser Gly Gly Phe Ser Arg Leu Pro Met Pro
465 470 475 480

Gln Pro Ser Ser Pro Lys Ala Ser Pro Gly Ala Ser Pro Pro Leu Val
485 490 495

Ser Ser Pro Arg Ile Ser Glu Leu His Glu Leu Pro Arg Pro Pro Gly
500 505 510

Asn Gln Thr Ser Lys Ala Thr Lys Ser Ser Arg Phe Ser Ile Ser Thr
515 520 525

Ser Asn Ser Thr His Tyr Arg Leu Ser Glu Phe Phe His Thr Leu Lys
530 535 540

<210> 23
<211> 1884
<212> DNA
<213> Oryza sativa

<220>
<221> CDS
<222> (1)..(1884)

<400> 23
atg aag tcg tcg ctg cgg aag ctg cgg ggc ttc gcg ctg cag cgc cac 48
Met Lys Ser Ser Leu Arg Lys Leu Arg Gly Phe Ala Leu Gln Arg His
1 5 10 15

gag cag cgg gtg gac cgc cgc ggg ggc cac tcc ccg gcc gcc gcc gcc 96
Glu Gln Arg Val Asp Arg Arg Gly Gly His Ser Pro Ala Ala Ala Ala
20 25 30

```
gcc gcg aac gag ctc ctc gcc gcc tcg cag gat atg gca gat atg agg      144
Ala Ala Asn Glu Leu Leu Ala Ala Ser Gln Asp Met Ala Asp Met Arg
        35                  40                  45

agc tgt tat gac aat ctg ctt tct gtt gca gca gcg att gca aat agt      192
Ser Cys Tyr Asp Asn Leu Leu Ser Val Ala Ala Ala Ile Ala Asn Ser
    50                  55                  60

gca tat gag ttc tca gaa gca ctg cag gaa atg ggg act tgt tta ctt      240
Ala Tyr Glu Phe Ser Glu Ala Leu Gln Glu Met Gly Thr Cys Leu Leu
65                  70                  75                  80

aaa aga gtc aca cca aac aag gac ggg att aat gat aaa gtt ttg ctg      288
Lys Arg Val Thr Pro Asn Lys Asp Gly Ile Asn Asp Lys Val Leu Leu
                85                  90                  95

ttg ctt gga aag gcc cag ttt gaa ctg cgg aag ctt gta gat agt tat      336
Leu Leu Gly Lys Ala Gln Phe Glu Leu Arg Lys Leu Val Asp Ser Tyr
            100                 105                 110

cgt gtg cat gtt ctt aat acc atc acc act cca tca cag tcc ctt ctc      384
Arg Val His Val Leu Asn Thr Ile Thr Thr Pro Ser Gln Ser Leu Leu
        115                 120                 125

aat gaa ctt caa act gta gag gaa atg aag cac cag tgt gat gaa aaa      432
Asn Glu Leu Gln Thr Val Glu Glu Met Lys His Gln Cys Asp Glu Lys
    130                 135                 140

aga gag ttg ttc gaa ttt ctg ctg aat gca cag aaa gaa aag gga aga      480
Arg Glu Leu Phe Glu Phe Leu Leu Asn Ala Gln Lys Glu Lys Gly Arg
145                 150                 155                 160

tct aag aat gcc aaa agt gac att ggt gcg tca gag caa ttg aaa caa      528
Ser Lys Asn Ala Lys Ser Asp Ile Gly Ala Ser Glu Gln Leu Lys Gln
                165                 170                 175

gct cag gac gat tat caa gaa gaa gca act ctt ttt cta ttc cgg tta      576
Ala Gln Asp Asp Tyr Gln Glu Glu Ala Thr Leu Phe Leu Phe Arg Leu
            180                 185                 190

aag tca ttg aag caa gga cag ttc aga agt ctt ttc aca caa gct gct      624
Lys Ser Leu Lys Gln Gly Gln Phe Arg Ser Leu Phe Thr Gln Ala Ala
        195                 200                 205

cgg cac cat gct gct cag cta aat tta ttc aga aag ggg ctc aaa tct      672
Arg His His Ala Ala Gln Leu Asn Leu Phe Arg Lys Gly Leu Lys Ser
    210                 215                 220

ctt gag gct gta gag cca cat gtt agg ctt gct gct gag cag caa cac      720
Leu Glu Ala Val Glu Pro His Val Arg Leu Ala Ala Glu Gln Gln His
225                 230                 235                 240

att gac cat cag ttc agt gca ctt gac gag gag gat tac tct gtg gat      768
Ile Asp His Gln Phe Ser Ala Leu Asp Glu Glu Asp Tyr Ser Val Asp
                245                 250                 255

gaa gaa aat gag gat gat tac aat gac agt cat gaa gat tta tcc ttt      816
Glu Glu Asn Glu Asp Asp Tyr Asn Asp Ser His Glu Asp Leu Ser Phe
            260                 265                 270

gat tat gga gaa aat aag gaa ggc aca gaa gct ggt cat gct tcc agg      864
Asp Tyr Gly Glu Asn Lys Glu Gly Thr Glu Ala Gly His Ala Ser Arg
        275                 280                 285

agc cct aca gag gaa ctt ctt gac aga agc aaa gca gag tat tcc tct      912
```

```
Ser Pro Thr Glu Glu Leu Leu Asp Arg Ser Lys Ala Glu Tyr Ser Ser
    290                 295                 300

ttt cct ggt gaa agg cag aga tct gga agt cag tca gcc cca ctt ttt       960
Phe Pro Gly Glu Arg Gln Arg Ser Gly Ser Gln Ser Ala Pro Leu Phe
305                 310                 315                 320

cct gaa aaa aaa ctt gag gca gca gag aga ata aaa gag ttg cgg cgt      1008
Pro Glu Lys Lys Leu Glu Ala Ala Glu Arg Ile Lys Glu Leu Arg Arg
                325                 330                 335

tct gct acg agg aag cta tat act tat gtt ttg cct act cca aat gat      1056
Ser Ala Thr Arg Lys Leu Tyr Thr Tyr Val Leu Pro Thr Pro Asn Asp
            340                 345                 350

gtt aga gac acc tct cag aca gtt aca gca aat cct act agt gga tct      1104
Val Arg Asp Thr Ser Gln Thr Val Thr Ala Asn Pro Thr Ser Gly Ser
        355                 360                 365

cct ctt gga aac aaa ggt gca ttc tat tca tct cca ctc cag cca agt      1152
Pro Leu Gly Asn Lys Gly Ala Phe Tyr Ser Ser Pro Leu Gln Pro Ser
    370                 375                 380

aca aat gtg gga gat ttg aga gac aat aag cta cca agt cct acc aga      1200
Thr Asn Val Gly Asp Leu Arg Asp Asn Lys Leu Pro Ser Pro Thr Arg
385                 390                 395                 400

tta tct aat gca cat tca gtt ttg aaa gag agc aat acc aat act aca      1248
Leu Ser Asn Ala His Ser Val Leu Lys Glu Ser Asn Thr Asn Thr Thr
                405                 410                 415

gac acc agg aca atg ctt gtg ctc cct ttg ggt gat ctc tct tta cct      1296
Asp Thr Arg Thr Met Leu Val Leu Pro Leu Gly Asp Leu Ser Leu Pro
            420                 425                 430

ggt tat cat gac tcg aag gct tct gac aac aaa aag gtg aag aga gga      1344
Gly Tyr His Asp Ser Lys Ala Ser Asp Asn Lys Lys Val Lys Arg Gly
        435                 440                 445

tcc ttt tct ggt cct att gtt ccc agg tca agg tca aca gag aat att      1392
Ser Phe Ser Gly Pro Ile Val Pro Arg Ser Arg Ser Thr Glu Asn Ile
    450                 455                 460

gat gtt gtt tct gta cca cct agg cac agc tct tca cat cag ccg tca      1440
Asp Val Val Ser Val Pro Pro Arg His Ser Ser Ser His Gln Pro Ser
465                 470                 475                 480

ata cat gtc aga gtt tct ccc aat acc tcg cca cca ctg ttg tcc tca      1488
Ile His Val Arg Val Ser Pro Asn Thr Ser Pro Pro Leu Leu Ser Ser
                485                 490                 495

cct aaa atc aag gag ctt cat gag ctt cct cgg cca cct gct aat gca      1536
Pro Lys Ile Lys Glu Leu His Glu Leu Pro Arg Pro Pro Ala Asn Ala
            500                 505                 510

tca aaa cat aca aca ttt ccc agt tta gtc gcc cac tct gca ccg ttg      1584
Ser Lys His Thr Thr Phe Pro Ser Leu Val Ala His Ser Ala Pro Leu
        515                 520                 525

gtg cca aat tct gct cct ttg gca ccc aga ggc cag gat cat ttt agg      1632
Val Pro Asn Ser Ala Pro Leu Ala Pro Arg Gly Gln Asp His Phe Arg
    530                 535                 540

ccg agg caa aca cca cca agt gct ccc caa act gca tca cct tta ccg      1680
Pro Arg Gln Thr Pro Pro Ser Ala Pro Gln Thr Ala Ser Pro Leu Pro
545                 550                 555                 560
```

```
aca cca cct ggg cct ata tcc cgc agt ttt tcg ata cct tct agg ggc      1728
Thr Pro Pro Gly Pro Ile Ser Arg Ser Phe Ser Ile Pro Ser Arg Gly
                565                 570                 575

atg aga aca tca ggt att tca gat ggt aaa gag aca gaa gat ctt cag      1776
Met Arg Thr Ser Gly Ile Ser Asp Gly Lys Glu Thr Glu Asp Leu Gln
            580                 585                 590

gat aaa ggg cct gcc aga atg agc ctt tct agt ctt cct tca gca caa      1824
Asp Lys Gly Pro Ala Arg Met Ser Leu Ser Ser Leu Pro Ser Ala Gln
        595                 600                 605

aca tcg ttg gag gat cat cgg cca ttg tca gga gct acc gag tca gtt      1872
Thr Ser Leu Glu Asp His Arg Pro Leu Ser Gly Ala Thr Glu Ser Val
    610                 615                 620

agc aaa aca tag                                                      1884
Ser Lys Thr
625


<210>  24
<211>  627
<212>  PRT
<213>  Oryza sativa

<400>  24

Met Lys Ser Ser Leu Arg Lys Leu Arg Gly Phe Ala Leu Gln Arg His
1               5                   10                  15


Glu Gln Arg Val Asp Arg Arg Gly Gly His Ser Pro Ala Ala Ala Ala
            20                  25                  30


Ala Ala Asn Glu Leu Leu Ala Ala Ser Gln Asp Met Ala Asp Met Arg
        35                  40                  45


Ser Cys Tyr Asp Asn Leu Leu Ser Val Ala Ala Ala Ile Ala Asn Ser
    50                  55                  60


Ala Tyr Glu Phe Ser Glu Ala Leu Gln Glu Met Gly Thr Cys Leu Leu
65                  70                  75                  80


Lys Arg Val Thr Pro Asn Lys Asp Gly Ile Asn Asp Lys Val Leu Leu
                85                  90                  95


Leu Leu Gly Lys Ala Gln Phe Glu Leu Arg Lys Leu Val Asp Ser Tyr
            100                 105                 110


Arg Val His Val Leu Asn Thr Ile Thr Thr Pro Ser Gln Ser Leu Leu
        115                 120                 125


Asn Glu Leu Gln Thr Val Glu Glu Met Lys His Gln Cys Asp Glu Lys
    130                 135                 140


Arg Glu Leu Phe Glu Phe Leu Leu Asn Ala Gln Lys Glu Lys Gly Arg
```

145 150 155 160

Ser Lys Asn Ala Lys Ser Asp Ile Gly Ala Ser Glu Gln Leu Lys Gln
165 170 175

Ala Gln Asp Asp Tyr Gln Glu Glu Ala Thr Leu Phe Leu Phe Arg Leu
180 185 190

Lys Ser Leu Lys Gln Gly Gln Phe Arg Ser Leu Phe Thr Gln Ala Ala
195 200 205

Arg His His Ala Ala Gln Leu Asn Leu Phe Arg Lys Gly Leu Lys Ser
210 215 220

Leu Glu Ala Val Glu Pro His Val Arg Leu Ala Ala Glu Gln Gln His
225 230 235 240

Ile Asp His Gln Phe Ser Ala Leu Asp Glu Glu Asp Tyr Ser Val Asp
245 250 255

Glu Glu Asn Glu Asp Asp Tyr Asn Asp Ser His Glu Asp Leu Ser Phe
260 265 270

Asp Tyr Gly Glu Asn Lys Glu Gly Thr Glu Ala Gly His Ala Ser Arg
275 280 285

Ser Pro Thr Glu Glu Leu Leu Asp Arg Ser Lys Ala Glu Tyr Ser Ser
290 295 300

Phe Pro Gly Glu Arg Gln Arg Ser Gly Ser Gln Ser Ala Pro Leu Phe
305 310 315 320

Pro Glu Lys Lys Leu Glu Ala Ala Glu Arg Ile Lys Glu Leu Arg Arg
325 330 335

Ser Ala Thr Arg Lys Leu Tyr Thr Tyr Val Leu Pro Thr Pro Asn Asp
340 345 350

Val Arg Asp Thr Ser Gln Thr Val Thr Ala Asn Pro Thr Ser Gly Ser
355 360 365

Pro Leu Gly Asn Lys Gly Ala Phe Tyr Ser Ser Pro Leu Gln Pro Ser
370 375 380

Thr Asn Val Gly Asp Leu Arg Asp Asn Lys Leu Pro Ser Pro Thr Arg
385 390 395 400

Leu Ser Asn Ala His Ser Val Leu Lys Glu Ser Asn Thr Asn Thr Thr
405 410 415

```
Asp Thr Arg Thr Met Leu Val Leu Pro Leu Gly Asp Leu Ser Leu Pro
            420                 425                 430

Gly Tyr His Asp Ser Lys Ala Ser Asp Asn Lys Lys Val Lys Arg Gly
        435                 440                 445

Ser Phe Ser Gly Pro Ile Val Pro Arg Ser Arg Ser Thr Glu Asn Ile
    450                 455                 460

Asp Val Val Ser Val Pro Pro Arg His Ser Ser Ser His Gln Pro Ser
465                 470                 475                 480

Ile His Val Arg Val Ser Pro Asn Thr Ser Pro Pro Leu Leu Ser Ser
                485                 490                 495

Pro Lys Ile Lys Glu Leu His Glu Leu Pro Arg Pro Pro Ala Asn Ala
            500                 505                 510

Ser Lys His Thr Thr Phe Pro Ser Leu Val Ala His Ser Ala Pro Leu
        515                 520                 525

Val Pro Asn Ser Ala Pro Leu Ala Pro Arg Gly Gln Asp His Phe Arg
    530                 535                 540

Pro Arg Gln Thr Pro Pro Ser Ala Pro Gln Thr Ala Ser Pro Leu Pro
545                 550                 555                 560

Thr Pro Pro Gly Pro Ile Ser Arg Ser Phe Ser Ile Pro Ser Arg Gly
                565                 570                 575

Met Arg Thr Ser Gly Ile Ser Asp Gly Lys Glu Thr Glu Asp Leu Gln
            580                 585                 590

Asp Lys Gly Pro Ala Arg Met Ser Leu Ser Ser Leu Pro Ser Ala Gln
        595                 600                 605

Thr Ser Leu Glu Asp His Arg Pro Leu Ser Gly Ala Thr Glu Ser Val
    610                 615                 620

Ser Lys Thr
625
```

<210> 25
<211> 1875
<212> DNA
<213> Oryza sativa

<220>
<221> CDS
<222> (1)..(1875)

<400> 25

```
atg aaa tcg cca ttg cgg aag ttc agg gga ttc ggt ctc cac agc cac      48
Met Lys Ser Pro Leu Arg Lys Phe Arg Gly Phe Gly Leu His Ser His
1               5                   10                  15

agg gag agg aag gac cac cgc ccg ccg ccg gcc aag ctc gac gag ctc      96
Arg Glu Arg Lys Asp His Arg Pro Pro Pro Ala Lys Leu Asp Glu Leu
            20                  25                  30

gcc gac gcc gcc cag gaa atg gag gaa atg agg aat tgt tat gac agc      144
Ala Asp Ala Ala Gln Glu Met Glu Glu Met Arg Asn Cys Tyr Asp Ser
        35                  40                  45

ttg ctt tca gct gca gct gca aca atg aac agt gta tat gag ttt gca      192
Leu Leu Ser Ala Ala Ala Ala Thr Met Asn Ser Val Tyr Glu Phe Ala
    50                  55                  60

gaa gct atg gag gaa atg gga act tgc tta ctt gag aaa act gca ttg      240
Glu Ala Met Glu Glu Met Gly Thr Cys Leu Leu Glu Lys Thr Ala Leu
65                  70                  75                  80

aat tat gat gat gat gac agt ggc aga gtt ctg atg atg cta gga aag      288
Asn Tyr Asp Asp Asp Asp Ser Gly Arg Val Leu Met Met Leu Gly Lys
                85                  90                  95

gcc caa ttc gaa ctg cag aag ttt gtt gat aac tat cgt aca aat atc      336
Ala Gln Phe Glu Leu Gln Lys Phe Val Asp Asn Tyr Arg Thr Asn Ile
            100                 105                 110

ata aat acc atc aca aac cca tca gag tca ctt ctc aaa gag cta caa      384
Ile Asn Thr Ile Thr Asn Pro Ser Glu Ser Leu Leu Lys Glu Leu Gln
        115                 120                 125

gtt gta gag gaa atg aag gaa ctc tgt gat cat aaa aga cag gaa tat      432
Val Val Glu Glu Met Lys Glu Leu Cys Asp His Lys Arg Gln Glu Tyr
    130                 135                 140

gaa gcc atg cga gca gcc tat aga gag aaa gga cgg tca agg cat tcc      480
Glu Ala Met Arg Ala Ala Tyr Arg Glu Lys Gly Arg Ser Arg His Ser
145                 150                 155                 160

aaa acc gaa aca tta tcc tcg gaa cag ctg caa gct tat ttt ctt gac      528
Lys Thr Glu Thr Leu Ser Ser Glu Gln Leu Gln Ala Tyr Phe Leu Asp
                165                 170                 175

tat caa gag gat gca gca ttg ttt ata ttc cga ttg aaa tca ttg aag      576
Tyr Gln Glu Asp Ala Ala Leu Phe Ile Phe Arg Leu Lys Ser Leu Lys
            180                 185                 190

caa ggt caa ttc cgt agt att tta aca cag gct gct cgc cac cat tct      624
Gln Gly Gln Phe Arg Ser Ile Leu Thr Gln Ala Ala Arg His His Ser
        195                 200                 205

gct cag ttg agt ttt ttc agg cga ggg ttg aag tat cta gag gct ctg      672
Ala Gln Leu Ser Phe Phe Arg Arg Gly Leu Lys Tyr Leu Glu Ala Leu
    210                 215                 220

gaa cct cat gta aaa gca gtt gct gag aaa cag cac att gaa tac cct      720
Glu Pro His Val Lys Ala Val Ala Glu Lys Gln His Ile Glu Tyr Pro
225                 230                 235                 240

tta aat gga cta gat gac gac aca gat aat gat gag tac agc tct tac      768
Leu Asn Gly Leu Asp Asp Asp Thr Asp Asn Asp Glu Tyr Ser Ser Tyr
                245                 250                 255
```

```
cag ggc aat caa agt gat gac agt gag tta agt ttt gac tat gag ata      816
Gln Gly Asn Gln Ser Asp Asp Ser Glu Leu Ser Phe Asp Tyr Glu Ile
            260                 265                 270

aat gac agg gat aag gac ttc ccc gct tcc aga agt tca atg gat ttg      864
Asn Asp Arg Asp Lys Asp Phe Pro Ala Ser Arg Ser Ser Met Asp Leu
        275                 280                 285

gat cag tct aat cag gca tgt tcc cca gaa cct ctg aag gaa cac aag      912
Asp Gln Ser Asn Gln Ala Cys Ser Pro Glu Pro Leu Lys Glu His Lys
    290                 295                 300

cag gaa tat acc gaa caa ata cag gca gat ttt gca gct cct cga gtg      960
Gln Glu Tyr Thr Glu Gln Ile Gln Ala Asp Phe Ala Ala Pro Arg Val
305                 310                 315                 320

aag ctt gag att ggt acc caa tca gcg cca att tct gct gac aat gtg     1008
Lys Leu Glu Ile Gly Thr Gln Ser Ala Pro Ile Ser Ala Asp Asn Val
                325                 330                 335

ttt gat cca tct aca agg ttt cgg aaa atg aat acg tca aac aga aca     1056
Phe Asp Pro Ser Thr Arg Phe Arg Lys Met Asn Thr Ser Asn Arg Thr
            340                 345                 350

aat tat tcg tac aaa ctc ccg aca cca gat gat gac aag aac tcc act     1104
Asn Tyr Ser Tyr Lys Leu Pro Thr Pro Asp Asp Asp Lys Asn Ser Thr
        355                 360                 365

tca gca cac acc aat aga tct cct cat tca gat caa cca gaa agt aaa     1152
Ser Ala His Thr Asn Arg Ser Pro His Ser Asp Gln Pro Glu Ser Lys
    370                 375                 380

tct cac gta gca gaa aat tta tgg cat tcc tct cca ctg gtc aaa ggt     1200
Ser His Val Ala Glu Asn Leu Trp His Ser Ser Pro Leu Val Lys Gly
385                 390                 395                 400

ttt aaa cca aac tcc atg ttt agt gga cct gtt aag atg cca tca agt     1248
Phe Lys Pro Asn Ser Met Phe Ser Gly Pro Val Lys Met Pro Ser Ser
                405                 410                 415

act gaa ggg ata tca gcg cca ctt gtt tat ccc tac gct act tca gat     1296
Thr Glu Gly Ile Ser Ala Pro Leu Val Tyr Pro Tyr Ala Thr Ser Asp
            420                 425                 430

ttt aag aaa atg aag agg gaa gca ttt tcc ggt cca att cca agc aaa     1344
Phe Lys Lys Met Lys Arg Glu Ala Phe Ser Gly Pro Ile Pro Ser Lys
        435                 440                 445

gca gga ttg aac aag ccc ttg ttt tct gct act gat ctc aga gca cca     1392
Ala Gly Leu Asn Lys Pro Leu Phe Ser Ala Thr Asp Leu Arg Ala Pro
    450                 455                 460

atg aac tat cct cgt gca atg tca aca aaa tca tat ggc cca ggc tgg     1440
Met Asn Tyr Pro Arg Ala Met Ser Thr Lys Ser Tyr Gly Pro Gly Trp
465                 470                 475                 480

cag tca tcc gtg gct cca aaa ttt acc cct agg atc act tca ctc cca     1488
Gln Ser Ser Val Ala Pro Lys Phe Thr Pro Arg Ile Thr Ser Leu Pro
                485                 490                 495

act aca tca ccg aga ata agt gag cta cat gaa ctg cct agg cct cca     1536
Thr Thr Ser Pro Arg Ile Ser Glu Leu His Glu Leu Pro Arg Pro Pro
            500                 505                 510

gca aat gta ggt gct gcc cgt cct ggt ttg gtt gga tat tct ggc cct     1584
Ala Asn Val Gly Ala Ala Arg Pro Gly Leu Val Gly Tyr Ser Gly Pro
```

```
        515                 520                 525

ctg gta tca agg cgg cag gtg cct aat gta ccg acc cgc gcc tca cca      1632
Leu Val Ser Arg Arg Gln Val Pro Asn Val Pro Thr Arg Ala Ser Pro
    530                 535                 540

cca tca caa aca gca tca cct ctt cca cgg cca cct gct gcc atg acc      1680
Pro Ser Gln Thr Ala Ser Pro Leu Pro Arg Pro Pro Ala Ala Met Thr
545                 550                 555                 560

cgc agt tat tct ata cct tca aat agc caa aga aca ccc att ctt acc      1728
Arg Ser Tyr Ser Ile Pro Ser Asn Ser Gln Arg Thr Pro Ile Leu Thr
                565                 570                 575

gtg aat aag ttg ttg gaa gct agg cat agc aga gag agt agt gaa gtt      1776
Val Asn Lys Leu Leu Glu Ala Arg His Ser Arg Glu Ser Ser Glu Val
            580                 585                 590

tcc tct ccc cca tta aca cct ata tct ttg gct gat gtt tcc cgc aga      1824
Ser Ser Pro Pro Leu Thr Pro Ile Ser Leu Ala Asp Val Ser Arg Arg
        595                 600                 605

tca aca gca gaa aca gct ctt gaa aaa aca agg atg atg gaa acc ttg      1872
Ser Thr Ala Glu Thr Ala Leu Glu Lys Thr Arg Met Met Glu Thr Leu
    610                 615                 620

tga                                                                  1875


<210>  26
<211>  624
<212>  PRT
<213>  Oryza sativa

<400>  26

Met Lys Ser Pro Leu Arg Lys Phe Arg Gly Phe Gly Leu His Ser His
1               5                   10                  15

Arg Glu Arg Lys Asp His Arg Pro Pro Pro Ala Lys Leu Asp Glu Leu
            20                  25                  30

Ala Asp Ala Ala Gln Glu Met Glu Glu Met Arg Asn Cys Tyr Asp Ser
        35                  40                  45

Leu Leu Ser Ala Ala Ala Ala Thr Met Asn Ser Val Tyr Glu Phe Ala
    50                  55                  60

Glu Ala Met Glu Glu Met Gly Thr Cys Leu Leu Glu Lys Thr Ala Leu
65                  70                  75                  80

Asn Tyr Asp Asp Asp Asp Ser Gly Arg Val Leu Met Met Leu Gly Lys
                85                  90                  95

Ala Gln Phe Glu Leu Gln Lys Phe Val Asp Asn Tyr Arg Thr Asn Ile
            100                 105                 110

Ile Asn Thr Ile Thr Asn Pro Ser Glu Ser Leu Leu Lys Glu Leu Gln
        115                 120                 125
```

```
Val Val Glu Glu Met Lys Glu Leu Cys Asp His Lys Arg Gln Glu Tyr
    130                 135                 140

Glu Ala Met Arg Ala Ala Tyr Arg Glu Lys Gly Arg Ser Arg His Ser
145                 150                 155                 160

Lys Thr Glu Thr Leu Ser Ser Glu Gln Leu Gln Ala Tyr Phe Leu Asp
                165                 170                 175

Tyr Gln Glu Asp Ala Ala Leu Phe Ile Phe Arg Leu Lys Ser Leu Lys
            180                 185                 190

Gln Gly Gln Phe Arg Ser Ile Leu Thr Gln Ala Ala Arg His His Ser
        195                 200                 205

Ala Gln Leu Ser Phe Phe Arg Arg Gly Leu Lys Tyr Leu Glu Ala Leu
    210                 215                 220

Glu Pro His Val Lys Ala Val Ala Glu Lys Gln His Ile Glu Tyr Pro
225                 230                 235                 240

Leu Asn Gly Leu Asp Asp Asp Thr Asp Asn Asp Glu Tyr Ser Ser Tyr
                245                 250                 255

Gln Gly Asn Gln Ser Asp Asp Ser Glu Leu Ser Phe Asp Tyr Glu Ile
            260                 265                 270

Asn Asp Arg Asp Lys Asp Phe Pro Ala Ser Arg Ser Ser Met Asp Leu
        275                 280                 285

Asp Gln Ser Asn Gln Ala Cys Ser Pro Glu Pro Leu Lys Glu His Lys
    290                 295                 300

Gln Glu Tyr Thr Glu Gln Ile Gln Ala Asp Phe Ala Ala Pro Arg Val
305                 310                 315                 320

Lys Leu Glu Ile Gly Thr Gln Ser Ala Pro Ile Ser Ala Asp Asn Val
                325                 330                 335

Phe Asp Pro Ser Thr Arg Phe Arg Lys Met Asn Thr Ser Asn Arg Thr
            340                 345                 350

Asn Tyr Ser Tyr Lys Leu Pro Thr Pro Asp Asp Asp Lys Asn Ser Thr
        355                 360                 365

Ser Ala His Thr Asn Arg Ser Pro His Ser Asp Gln Pro Glu Ser Lys
    370                 375                 380
```

```
Ser His Val Ala Glu Asn Leu Trp His Ser Ser Pro Leu Val Lys Gly
385                 390                 395                 400

Phe Lys Pro Asn Ser Met Phe Ser Gly Pro Val Lys Met Pro Ser Ser
                405                 410                 415

Thr Glu Gly Ile Ser Ala Pro Leu Val Tyr Pro Tyr Ala Thr Ser Asp
            420                 425                 430

Phe Lys Lys Met Lys Arg Glu Ala Phe Ser Gly Pro Ile Pro Ser Lys
        435                 440                 445

Ala Gly Leu Asn Lys Pro Leu Phe Ser Ala Thr Asp Leu Arg Ala Pro
    450                 455                 460

Met Asn Tyr Pro Arg Ala Met Ser Thr Lys Ser Tyr Gly Pro Gly Trp
465                 470                 475                 480

Gln Ser Ser Val Ala Pro Lys Phe Thr Pro Arg Ile Thr Ser Leu Pro
                485                 490                 495

Thr Thr Ser Pro Arg Ile Ser Glu Leu His Glu Leu Pro Arg Pro Pro
            500                 505                 510

Ala Asn Val Gly Ala Ala Arg Pro Gly Leu Val Gly Tyr Ser Gly Pro
        515                 520                 525

Leu Val Ser Arg Arg Gln Val Pro Asn Val Pro Thr Arg Ala Ser Pro
    530                 535                 540

Pro Ser Gln Thr Ala Ser Pro Leu Pro Arg Pro Pro Ala Ala Met Thr
545                 550                 555                 560

Arg Ser Tyr Ser Ile Pro Ser Asn Ser Gln Arg Thr Pro Ile Leu Thr
                565                 570                 575

Val Asn Lys Leu Leu Glu Ala Arg His Ser Arg Glu Ser Ser Glu Val
            580                 585                 590

Ser Ser Pro Pro Leu Thr Pro Ile Ser Leu Ala Asp Val Ser Arg Arg
        595                 600                 605

Ser Thr Ala Glu Thr Ala Leu Glu Lys Thr Arg Met Met Glu Thr Leu
    610                 615                 620

<210>  27
<211>  1872
<212>  DNA
<213>  Oryza sativa
```

<220>
<221> CDS
<222> (1)..(1872)

<400> 27
```
atg aag tcg ccg ttg ctg agg ctg aag ggc ttc ggc cac cac cag cag         48
Met Lys Ser Pro Leu Leu Arg Leu Lys Gly Phe Gly His His Gln Gln
1               5                   10                  15

cac agg gag agg aag tcg cgg cag ccg cag ccg cag ccg acg ccg gcc         96
His Arg Glu Arg Lys Ser Arg Gln Pro Gln Pro Gln Pro Thr Pro Ala
            20                  25                  30

aag ctc gac gag ctc gcc gac gcc gcc cag gat gtg gaa gaa atg agg        144
Lys Leu Asp Glu Leu Ala Asp Ala Ala Gln Asp Val Glu Glu Met Arg
        35                  40                  45

aac tgt tat gat ggc ttc att tca gca gca gct gct aca acg aat ggc        192
Asn Cys Tyr Asp Gly Phe Ile Ser Ala Ala Ala Ala Thr Thr Asn Gly
    50                  55                  60

gta tat gag ttt gct gag gct tta gag gaa ctg gga agt tgc tta ctt        240
Val Tyr Glu Phe Ala Glu Ala Leu Glu Glu Leu Gly Ser Cys Leu Leu
65                  70                  75                  80

gca aaa cct gtg cta aat gac gac gac gac gat agt ggc aga gtg ttg        288
Ala Lys Pro Val Leu Asn Asp Asp Asp Asp Asp Ser Gly Arg Val Leu
                85                  90                  95

atg atg ctc ggt aag gcc caa tat gaa cta cag aaa tct gcg gat aga        336
Met Met Leu Gly Lys Ala Gln Tyr Glu Leu Gln Lys Ser Ala Asp Arg
            100                 105                 110

tac cga aca aat atc atc cac aca atc aca acc cca tct gaa tct ctt        384
Tyr Arg Thr Asn Ile Ile His Thr Ile Thr Thr Pro Ser Glu Ser Leu
        115                 120                 125

ctt aag gag ctg caa act tta gag gaa atg aaa caa caa tgc gac atg        432
Leu Lys Glu Leu Gln Thr Leu Glu Glu Met Lys Gln Gln Cys Asp Met
    130                 135                 140

aaa agg gac gca tat gaa acc atg agg gca tcc tat agt gac aaa gga        480
Lys Arg Asp Ala Tyr Glu Thr Met Arg Ala Ser Tyr Ser Asp Lys Gly
145                 150                 155                 160

ggg tca agg cat tcc aaa acc gaa tca ttc tcc aca gaa caa ctg gac        528
Gly Ser Arg His Ser Lys Thr Glu Ser Phe Ser Thr Glu Gln Leu Asp
                165                 170                 175

gct tct ttt ctt gaa tac caa gag gat tca gca ctt ttc aca ttt cgc        576
Ala Ser Phe Leu Glu Tyr Gln Glu Asp Ser Ala Leu Phe Thr Phe Arg
            180                 185                 190

ttg aaa tca tta aag caa ggg caa ttc cag agc ctg tta aca cag gct        624
Leu Lys Ser Leu Lys Gln Gly Gln Phe Gln Ser Leu Leu Thr Gln Ala
        195                 200                 205

gct cgc cat cat gct gct cag tta agt ttt ttc agg aaa gga ctc aaa        672
Ala Arg His His Ala Ala Gln Leu Ser Phe Phe Arg Lys Gly Leu Lys
    210                 215                 220

tgt ctc gaa gca ctt gaa cct cgt gtc aaa gca ata tct gag aaa cat        720
Cys Leu Glu Ala Leu Glu Pro Arg Val Lys Ala Ile Ser Glu Lys His
225                 230                 235                 240

cac att gac tat aac ttc agc ggt cta gag gat gat ggt tct gac aat        768
```

```
His Ile Asp Tyr Asn Phe Ser Gly Leu Glu Asp Asp Gly Ser Asp Asn
                245                 250                 255

gat ggc tac agt act tac gat tct tgt agt gat gac gga gaa ctg agt      816
Asp Gly Tyr Ser Thr Tyr Asp Ser Cys Ser Asp Asp Gly Glu Leu Ser
            260                 265                 270

ttc gac tat gaa ata aac gat aga gac caa gat ttt ctt act tcg aga      864
Phe Asp Tyr Glu Ile Asn Asp Arg Asp Gln Asp Phe Leu Thr Ser Arg
        275                 280                 285

ggt tca atg gat ttt gat aaa agt gat cag act act tca cca aag cca      912
Gly Ser Met Asp Phe Asp Lys Ser Asp Gln Thr Thr Ser Pro Lys Pro
    290                 295                 300

atc aaa gaa aac aag cag gaa gag gca aaa caa gca gag gcc gaa ata      960
Ile Lys Glu Asn Lys Gln Glu Glu Ala Lys Gln Ala Glu Ala Glu Ile
305                 310                 315                 320

gtg ttc ccg caa ttg aag ccc gag ttc gct aca cat tca gct cca ctt     1008
Val Phe Pro Gln Leu Lys Pro Glu Phe Ala Thr His Ser Ala Pro Leu
                325                 330                 335

ttt gct ggc aat ttg ctt gat gaa act gac agg ctt cgg caa atg agg     1056
Phe Ala Gly Asn Leu Leu Asp Glu Thr Asp Arg Leu Arg Gln Met Arg
            340                 345                 350

cca tcc tca acc aaa cac tcg tac agg ctc cct acc cca gtt ggt gct     1104
Pro Ser Ser Thr Lys His Ser Tyr Arg Leu Pro Thr Pro Val Gly Ala
        355                 360                 365

gac aac ccc gta cca tca ggt tca cac agg ctg cat cat tcc gcg caa     1152
Asp Asn Pro Val Pro Ser Gly Ser His Arg Leu His His Ser Ala Gln
    370                 375                 380

ttc ttc gaa aca aag ccc cat gcc ccg acg aat ctg tgg cac tcg tct     1200
Phe Phe Glu Thr Lys Pro His Ala Pro Thr Asn Leu Trp His Ser Ser
385                 390                 395                 400

cct ctg aca aaa gac tac aat ggc gcc atg cac aac gcc gct acc aag     1248
Pro Leu Thr Lys Asp Tyr Asn Gly Ala Met His Asn Ala Ala Thr Lys
                405                 410                 415

ccg tcg tca tca tca agc acc gat gat ctg aag aag ctg aag agg gag     1296
Pro Ser Ser Ser Ser Ser Thr Asp Asp Leu Lys Lys Leu Lys Arg Glu
            420                 425                 430

tca tgg tca ggt ccg atc ccg atc aag gcc ggg tca ggc ggc aag ccc     1344
Ser Trp Ser Gly Pro Ile Pro Ile Lys Ala Gly Ser Gly Gly Lys Pro
        435                 440                 445

ttc tct cag gct gac cac agg cca tcg ccc acc atg gct tac cct ggt     1392
Phe Ser Gln Ala Asp His Arg Pro Ser Pro Thr Met Ala Tyr Pro Gly
    450                 455                 460

gca atg cct gcg gcc aag ccg cac gtc cgg cac gcg tcg tcg tcg tct     1440
Ala Met Pro Ala Ala Lys Pro His Val Arg His Ala Ser Ser Ser Ser
465                 470                 475                 480

gtg tca ccc aag gtt tcc cca aag atg tca ccg gtg cca cca gca tcg     1488
Val Ser Pro Lys Val Ser Pro Lys Met Ser Pro Val Pro Pro Ala Ser
                485                 490                 495

tcg ctg aag atc agc gag ctc cac ctg ctg ccg ctg cct cca gcc aat     1536
Ser Leu Lys Ile Ser Glu Leu His Leu Leu Pro Leu Pro Pro Ala Asn
            500                 505                 510
```

```
gtg gat cct gtc cgg cct tct ggt ctg gtc ggc tac tcc ggc cct ctg      1584
Val Asp Pro Val Arg Pro Ser Gly Leu Val Gly Tyr Ser Gly Pro Leu
        515                 520                 525

gtg tcg aag cgc gcg ccg acg ccg gcc cgt gcc tcc ccg aag gcg tca      1632
Val Ser Lys Arg Ala Pro Thr Pro Ala Arg Ala Ser Pro Lys Ala Ser
    530                 535                 540

aga acg gcg tcg ccg ctg cct agg ccg cct gcg gcc ttg gca agg agc      1680
Arg Thr Ala Ser Pro Leu Pro Arg Pro Pro Ala Ala Leu Ala Arg Ser
545                 550                 555                 560

tac tcc ata cct tcc aac agc cag agg acg ccc atc atc act gtg aat      1728
Tyr Ser Ile Pro Ser Asn Ser Gln Arg Thr Pro Ile Ile Thr Val Asn
                565                 570                 575

aag ctc ttg gag gct aag cat agc agg gag ggc agt gat gct tct tcc      1776
Lys Leu Leu Glu Ala Lys His Ser Arg Glu Gly Ser Asp Ala Ser Ser
            580                 585                 590

cca cca ctg act cca ttg tca ttg tct gat ttg tgc cac cag gag aag      1824
Pro Pro Leu Thr Pro Leu Ser Leu Ser Asp Leu Cys His Gln Glu Lys
        595                 600                 605

gca ggg aag gca gct gca ggc aac aca agg aga aag gaa acc ttg tga      1872
Ala Gly Lys Ala Ala Ala Gly Asn Thr Arg Arg Lys Glu Thr Leu
    610                 615                 620


<210>  28
<211>  623
<212>  PRT
<213>  Oryza sativa

<400>  28

Met Lys Ser Pro Leu Leu Arg Leu Lys Gly Phe Gly His His Gln Gln
1               5                   10                  15


His Arg Glu Arg Lys Ser Arg Gln Pro Gln Pro Gln Pro Thr Pro Ala
            20                  25                  30


Lys Leu Asp Glu Leu Ala Asp Ala Ala Gln Asp Val Glu Glu Met Arg
        35                  40                  45


Asn Cys Tyr Asp Gly Phe Ile Ser Ala Ala Ala Ala Thr Thr Asn Gly
    50                  55                  60


Val Tyr Glu Phe Ala Glu Ala Leu Glu Glu Leu Gly Ser Cys Leu Leu
65                  70                  75                  80


Ala Lys Pro Val Leu Asn Asp Asp Asp Asp Asp Ser Gly Arg Val Leu
                85                  90                  95


Met Met Leu Gly Lys Ala Gln Tyr Glu Leu Gln Lys Ser Ala Asp Arg
            100                 105                 110


Tyr Arg Thr Asn Ile Ile His Thr Ile Thr Thr Pro Ser Glu Ser Leu
```

115 120 125

Leu Lys Glu Leu Gln Thr Leu Glu Glu Met Lys Gln Gln Cys Asp Met
130 135 140

Lys Arg Asp Ala Tyr Glu Thr Met Arg Ala Ser Tyr Ser Asp Lys Gly
145 150 155 160

Gly Ser Arg His Ser Lys Thr Glu Ser Phe Ser Thr Glu Gln Leu Asp
165 170 175

Ala Ser Phe Leu Glu Tyr Gln Glu Asp Ser Ala Leu Phe Thr Phe Arg
180 185 190

Leu Lys Ser Leu Lys Gln Gly Gln Phe Gln Ser Leu Leu Thr Gln Ala
195 200 205

Ala Arg His His Ala Ala Gln Leu Ser Phe Phe Arg Lys Gly Leu Lys
210 215 220

Cys Leu Glu Ala Leu Glu Pro Arg Val Lys Ala Ile Ser Glu Lys His
225 230 235 240

His Ile Asp Tyr Asn Phe Ser Gly Leu Glu Asp Asp Gly Ser Asp Asn
245 250 255

Asp Gly Tyr Ser Thr Tyr Asp Ser Cys Ser Asp Asp Gly Glu Leu Ser
260 265 270

Phe Asp Tyr Glu Ile Asn Asp Arg Asp Gln Asp Phe Leu Thr Ser Arg
275 280 285

Gly Ser Met Asp Phe Asp Lys Ser Asp Gln Thr Thr Ser Pro Lys Pro
290 295 300

Ile Lys Glu Asn Lys Gln Glu Glu Ala Lys Gln Ala Glu Ala Glu Ile
305 310 315 320

Val Phe Pro Gln Leu Lys Pro Glu Phe Ala Thr His Ser Ala Pro Leu
325 330 335

Phe Ala Gly Asn Leu Leu Asp Glu Thr Asp Arg Leu Arg Gln Met Arg
340 345 350

Pro Ser Ser Thr Lys His Ser Tyr Arg Leu Pro Thr Pro Val Gly Ala
355 360 365

Asp Asn Pro Val Pro Ser Gly Ser His Arg Leu His His Ser Ala Gln
370 375 380

```
Phe Phe Glu Thr Lys Pro His Ala Pro Thr Asn Leu Trp His Ser Ser
385                 390                 395                 400

Pro Leu Thr Lys Asp Tyr Asn Gly Ala Met His Asn Ala Ala Thr Lys
                405                 410                 415

Pro Ser Ser Ser Ser Ser Thr Asp Asp Leu Lys Lys Leu Lys Arg Glu
            420                 425                 430

Ser Trp Ser Gly Pro Ile Pro Ile Lys Ala Gly Ser Gly Gly Lys Pro
        435                 440                 445

Phe Ser Gln Ala Asp His Arg Pro Ser Pro Thr Met Ala Tyr Pro Gly
    450                 455                 460

Ala Met Pro Ala Ala Lys Pro His Val Arg His Ala Ser Ser Ser Ser
465                 470                 475                 480

Val Ser Pro Lys Val Ser Pro Lys Met Ser Pro Val Pro Pro Ala Ser
                485                 490                 495

Ser Leu Lys Ile Ser Glu Leu His Leu Leu Pro Leu Pro Pro Ala Asn
            500                 505                 510

Val Asp Pro Val Arg Pro Ser Gly Leu Val Gly Tyr Ser Gly Pro Leu
        515                 520                 525

Val Ser Lys Arg Ala Pro Thr Pro Ala Arg Ala Ser Pro Lys Ala Ser
    530                 535                 540

Arg Thr Ala Ser Pro Leu Pro Arg Pro Pro Ala Ala Leu Ala Arg Ser
545                 550                 555                 560

Tyr Ser Ile Pro Ser Asn Ser Gln Arg Thr Pro Ile Ile Thr Val Asn
                565                 570                 575

Lys Leu Leu Glu Ala Lys His Ser Arg Glu Gly Ser Asp Ala Ser Ser
            580                 585                 590

Pro Pro Leu Thr Pro Leu Ser Leu Ser Asp Leu Cys His Gln Glu Lys
        595                 600                 605

Ala Gly Lys Ala Ala Ala Gly Asn Thr Arg Arg Lys Glu Thr Leu
    610                 615                 620
```

<210> 29
<211> 1878
<212> DNA
<213> P.trichocarpa

```
<220>
<221>  CDS
<222>  (1)..(1878)

<400>  29
atg aag aat aaa ctg aga gga ttt gga ctg aaa aga agc gaa aca aag       48
Met Lys Asn Lys Leu Arg Gly Phe Gly Leu Lys Arg Ser Glu Thr Lys
1               5                   10                  15

gaa aaa att gat tta tta cct cct gct cag tta gat gag ctc gct caa       96
Glu Lys Ile Asp Leu Leu Pro Pro Ala Gln Leu Asp Glu Leu Ala Gln
            20                  25                  30

gct gct cgg gac atg caa gac atg aga aat tgc tat gat agt tta ctt      144
Ala Ala Arg Asp Met Gln Asp Met Arg Asn Cys Tyr Asp Ser Leu Leu
        35                  40                  45

ttc gca gct gct gca aca gca aac agt gca tat gaa ttc tca gag tca      192
Phe Ala Ala Ala Ala Thr Ala Asn Ser Ala Tyr Glu Phe Ser Glu Ser
    50                  55                  60

ttg cgg gaa atg ggt tct tgt ttg tta gag aaa aca gca tta cat gat      240
Leu Arg Glu Met Gly Ser Cys Leu Leu Glu Lys Thr Ala Leu His Asp
65                  70                  75                  80

gat gaa gaa agt ggc aag gtt ttg cta atg ttg ggg aat gtg cag ttt      288
Asp Glu Glu Ser Gly Lys Val Leu Leu Met Leu Gly Asn Val Gln Phe
                85                  90                  95

gaa ctt caa aaa ctt gtt gat agt tat cgg tct cat ata ttc ctg aca      336
Glu Leu Gln Lys Leu Val Asp Ser Tyr Arg Ser His Ile Phe Leu Thr
            100                 105                 110

att aca aat cca tct gaa tcc ctg ctc aat gaa ctt cgg aca gtt gag      384
Ile Thr Asn Pro Ser Glu Ser Leu Leu Asn Glu Leu Arg Thr Val Glu
        115                 120                 125

gat atg aag cga caa tgt gat gaa aaa aga aat gtc tat gaa tac atg      432
Asp Met Lys Arg Gln Cys Asp Glu Lys Arg Asn Val Tyr Glu Tyr Met
    130                 135                 140

gtg gca caa caa aag gac aag gga aga tca aaa ggt ggg aaa gat gaa      480
Val Ala Gln Gln Lys Asp Lys Gly Arg Ser Lys Gly Gly Lys Asp Glu
145                 150                 155                 160

agt act act ttg cag caa ttg cga tca gct cgt gaa gaa tat gac gag      528
Ser Thr Thr Leu Gln Gln Leu Arg Ser Ala Arg Glu Glu Tyr Asp Glu
                165                 170                 175

gag gca acc cta tgt gtt ttt cgg ttg aaa tct ctg aaa cag gga cag      576
Glu Ala Thr Leu Cys Val Phe Arg Leu Lys Ser Leu Lys Gln Gly Gln
            180                 185                 190

tct cga agc ctt cta aca cag gta gca cgt cat cat gct gct cag ttg      624
Ser Arg Ser Leu Leu Thr Gln Val Ala Arg His His Ala Ala Gln Leu
        195                 200                 205

aat ttc ttt cag aag gga ctt aaa tca ctt gag aca gtt gaa ccc cac      672
Asn Phe Phe Gln Lys Gly Leu Lys Ser Leu Glu Thr Val Glu Pro His
    210                 215                 220

gta aga ctg att aca gaa cat caa cac att gat tac cat ttc agt gga      720
Val Arg Leu Ile Thr Glu His Gln His Ile Asp Tyr His Phe Ser Gly
225                 230                 235                 240
```

```
ctt gaa gat gat ggt aga gaa gat ggt gag gat tat ggt gag gat gtc      768
Leu Glu Asp Asp Gly Arg Glu Asp Gly Glu Asp Tyr Gly Glu Asp Val
                245                 250                 255

gat gat aca tat gaa ggc agg gaa ttg agt ttt gac tac aga gca aat      816
Asp Asp Thr Tyr Glu Gly Arg Glu Leu Ser Phe Asp Tyr Arg Ala Asn
            260                 265                 270

aat cag ggg cat gct gtt tct gca gca agg aat tct atg gag gtt gat      864
Asn Gln Gly His Ala Val Ser Ala Ala Arg Asn Ser Met Glu Val Asp
        275                 280                 285

gaa gag gat ctt tca ttc ccc caa gca cca gca gca gaa aat gta gag      912
Glu Glu Asp Leu Ser Phe Pro Gln Ala Pro Ala Ala Glu Asn Val Glu
    290                 295                 300

tta aac cca gac aaa acc cct ggt ggt ttc cat ttc cca atc aga gaa      960
Leu Asn Pro Asp Lys Thr Pro Gly Gly Phe His Phe Pro Ile Arg Glu
305                 310                 315                 320

cca aga gga ggc agc cat tca gca cca att ttt cca gaa aga aaa cct     1008
Pro Arg Gly Gly Ser His Ser Ala Pro Ile Phe Pro Glu Arg Lys Pro
                325                 330                 335

gat cca gtt gaa aga ata aga caa ata cag aaa tca tcg agg aag tcc     1056
Asp Pro Val Glu Arg Ile Arg Gln Ile Gln Lys Ser Ser Arg Lys Ser
            340                 345                 350

aac aca tat gtg ctg ccc aca cct gtt gat gca aag ggt gtg atc tct     1104
Asn Thr Tyr Val Leu Pro Thr Pro Val Asp Ala Lys Gly Val Ile Ser
        355                 360                 365

tca aga gca agc ggt tca gtt ccc aac aca agg caa ata gac atc agt     1152
Ser Arg Ala Ser Gly Ser Val Pro Asn Thr Arg Gln Ile Asp Ile Ser
    370                 375                 380

ggg cgc act cac tat ttg tct cat tct tcc cca ttg gaa cag aag aag     1200
Gly Arg Thr His Tyr Leu Ser His Ser Ser Pro Leu Glu Gln Lys Lys
385                 390                 395                 400

aat gag aaa gat tct ggc gat ggt cac ctg cca gag ttc acc cct tca     1248
Asn Glu Lys Asp Ser Gly Asp Gly His Leu Pro Glu Phe Thr Pro Ser
                405                 410                 415

aaa gaa cgt tca gga cat aaa gag agc aac aat cct aat gcc tcc acc     1296
Lys Glu Arg Ser Gly His Lys Glu Ser Asn Asn Pro Asn Ala Ser Thr
            420                 425                 430

caa tta ccc cgt cct tta gtc gga gga atc tca ttt cca cag ctt gat     1344
Gln Leu Pro Arg Pro Leu Val Gly Gly Ile Ser Phe Pro Gln Leu Asp
        435                 440                 445

gtt tac aat gca tct gat aac aaa aaa att aaa cgg caa tcc ttc tct     1392
Val Tyr Asn Ala Ser Asp Asn Lys Lys Ile Lys Arg Gln Ser Phe Ser
    450                 455                 460

ggt ccg ata acc agt aag cca tgg tca atg aaa ttg ggc tta tca tca     1440
Gly Pro Ile Thr Ser Lys Pro Trp Ser Met Lys Leu Gly Leu Ser Ser
465                 470                 475                 480

tcc agt ggt ccc att tct gct act gaa ctt tca caa gaa cct tct aca     1488
Ser Ser Gly Pro Ile Ser Ala Thr Glu Leu Ser Gln Glu Pro Ser Thr
                485                 490                 495

tcc ccc aaa gta tcc cct agt aca tca cct cct ctt gtt tct tca ccc     1536
Ser Pro Lys Val Ser Pro Ser Thr Ser Pro Pro Leu Val Ser Ser Pro
```

```
            500                 505                 510

agg ata agc gag ctt cat gaa ctt cca agg cca cca ggt aat tta gca      1584
Arg Ile Ser Glu Leu His Glu Leu Pro Arg Pro Pro Gly Asn Leu Ala
        515                 520                 525

gcc aaa gct gca aaa tct ctg ggg ccg att gga cac tct gct cca ttg      1632
Ala Lys Ala Ala Lys Ser Leu Gly Pro Ile Gly His Ser Ala Pro Leu
    530                 535                 540

gtc aga aat cct gag ctt tct gga aca aac aag att tct tca ggg gca      1680
Val Arg Asn Pro Glu Leu Ser Gly Thr Asn Lys Ile Ser Ser Gly Ala
545                 550                 555                 560

gca aat ttg gca tca cct ctt ccc acg cca ccg ttg atg gtc cca aca      1728
Ala Asn Leu Ala Ser Pro Leu Pro Thr Pro Pro Leu Met Val Pro Thr
                565                 570                 575

agt ttc tca ata ccc tca att agt cca aga aca atg tct gta cat gtt      1776
Ser Phe Ser Ile Pro Ser Ile Ser Pro Arg Thr Met Ser Val His Val
            580                 585                 590

tct aag ctt ttg gtt tct tct caa cta ctg gat aaa cct ggg gaa cta      1824
Ser Lys Leu Leu Val Ser Ser Gln Leu Leu Asp Lys Pro Gly Glu Leu
        595                 600                 605

agg gtt gct ctt atg ctg tct gat tcc ccc ccc ccc ccc att ttt att      1872
Arg Val Ala Leu Met Leu Ser Asp Ser Pro Pro Pro Pro Ile Phe Ile
    610                 615                 620

gag tga                                                              1878
Glu
625


<210>  30
<211>  625
<212>  PRT
<213>  P.trichocarpa

<400>  30

Met Lys Asn Lys Leu Arg Gly Phe Gly Leu Lys Arg Ser Glu Thr Lys
1               5                   10                  15


Glu Lys Ile Asp Leu Leu Pro Pro Ala Gln Leu Asp Glu Leu Ala Gln
            20                  25                  30


Ala Ala Arg Asp Met Gln Asp Met Arg Asn Cys Tyr Asp Ser Leu Leu
        35                  40                  45


Phe Ala Ala Ala Ala Thr Ala Asn Ser Ala Tyr Glu Phe Ser Glu Ser
    50                  55                  60


Leu Arg Glu Met Gly Ser Cys Leu Leu Glu Lys Thr Ala Leu His Asp
65                  70                  75                  80


Asp Glu Glu Ser Gly Lys Val Leu Leu Met Leu Gly Asn Val Gln Phe
                85                  90                  95
```

```
Glu Leu Gln Lys Leu Val Asp Ser Tyr Arg Ser His Ile Phe Leu Thr
            100                 105                 110

Ile Thr Asn Pro Ser Glu Ser Leu Leu Asn Glu Leu Arg Thr Val Glu
        115                 120                 125

Asp Met Lys Arg Gln Cys Asp Glu Lys Arg Asn Val Tyr Glu Tyr Met
    130                 135                 140

Val Ala Gln Gln Lys Asp Lys Gly Arg Ser Lys Gly Gly Lys Asp Glu
145                 150                 155                 160

Ser Thr Thr Leu Gln Gln Leu Arg Ser Ala Arg Glu Glu Tyr Asp Glu
                165                 170                 175

Glu Ala Thr Leu Cys Val Phe Arg Leu Lys Ser Leu Lys Gln Gly Gln
            180                 185                 190

Ser Arg Ser Leu Leu Thr Gln Val Ala Arg His His Ala Ala Gln Leu
        195                 200                 205

Asn Phe Phe Gln Lys Gly Leu Lys Ser Leu Glu Thr Val Glu Pro His
    210                 215                 220

Val Arg Leu Ile Thr Glu His Gln His Ile Asp Tyr His Phe Ser Gly
225                 230                 235                 240

Leu Glu Asp Asp Gly Arg Glu Asp Gly Glu Asp Tyr Gly Glu Asp Val
                245                 250                 255

Asp Asp Thr Tyr Glu Gly Arg Glu Leu Ser Phe Asp Tyr Arg Ala Asn
            260                 265                 270

Asn Gln Gly His Ala Val Ser Ala Ala Arg Asn Ser Met Glu Val Asp
        275                 280                 285

Glu Glu Asp Leu Ser Phe Pro Gln Ala Pro Ala Ala Glu Asn Val Glu
    290                 295                 300

Leu Asn Pro Asp Lys Thr Pro Gly Gly Phe His Phe Pro Ile Arg Glu
305                 310                 315                 320

Pro Arg Gly Gly Ser His Ser Ala Pro Ile Phe Pro Glu Arg Lys Pro
                325                 330                 335

Asp Pro Val Glu Arg Ile Arg Gln Ile Gln Lys Ser Ser Arg Lys Ser
            340                 345                 350

Asn Thr Tyr Val Leu Pro Thr Pro Val Asp Ala Lys Gly Val Ile Ser
        355                 360                 365
```

```
Ser Arg Ala Ser Gly Ser Val Pro Asn Thr Arg Gln Ile Asp Ile Ser
    370                 375                 380

Gly Arg Thr His Tyr Leu Ser His Ser Ser Pro Leu Glu Gln Lys Lys
385                 390                 395                 400

Asn Glu Lys Asp Ser Gly Asp Gly His Leu Pro Glu Phe Thr Pro Ser
                405                 410                 415

Lys Glu Arg Ser Gly His Lys Glu Ser Asn Asn Pro Asn Ala Ser Thr
            420                 425                 430

Gln Leu Pro Arg Pro Leu Val Gly Gly Ile Ser Phe Pro Gln Leu Asp
        435                 440                 445

Val Tyr Asn Ala Ser Asp Asn Lys Lys Ile Lys Arg Gln Ser Phe Ser
    450                 455                 460

Gly Pro Ile Thr Ser Lys Pro Trp Ser Met Lys Leu Gly Leu Ser Ser
465                 470                 475                 480

Ser Ser Gly Pro Ile Ser Ala Thr Glu Leu Ser Gln Glu Pro Ser Thr
                485                 490                 495

Ser Pro Lys Val Ser Pro Ser Thr Ser Pro Pro Leu Val Ser Ser Pro
            500                 505                 510

Arg Ile Ser Glu Leu His Glu Leu Pro Arg Pro Pro Gly Asn Leu Ala
        515                 520                 525

Ala Lys Ala Ala Lys Ser Leu Gly Pro Ile Gly His Ser Ala Pro Leu
    530                 535                 540

Val Arg Asn Pro Glu Leu Ser Gly Thr Asn Lys Ile Ser Ser Gly Ala
545                 550                 555                 560

Ala Asn Leu Ala Ser Pro Leu Pro Thr Pro Pro Leu Met Val Pro Thr
                565                 570                 575

Ser Phe Ser Ile Pro Ser Ile Ser Pro Arg Thr Met Ser Val His Val
            580                 585                 590

Ser Lys Leu Leu Val Ser Ser Gln Leu Leu Asp Lys Pro Gly Glu Leu
        595                 600                 605

Arg Val Ala Leu Met Leu Ser Asp Ser Pro Pro Pro Pro Ile Phe Ile
    610                 615                 620
```

```
Glu
625


<210>  31
<211>  615
<212>  DNA
<213>  P.trichocarpa


<220>
<221>  CDS
<222>  (1)..(615)

<400>  31
atg gac ttc ttt ttt tca agg ctt gag cca ccg gct ccc gct gaa tct        48
Met Asp Phe Phe Phe Ser Arg Leu Glu Pro Pro Ala Pro Ala Glu Ser
1               5                   10                  15

aag aaa ata aaa aga tat gcc tta tct ggt cct att aca tcc cag cca        96
Lys Lys Ile Lys Arg Tyr Ala Leu Ser Gly Pro Ile Thr Ser Gln Pro
            20                  25                  30

tgg tct aca aag gca gtc tcc gca gaa aat cca caa ttg ttc tct gga       144
Trp Ser Thr Lys Ala Val Ser Ala Glu Asn Pro Gln Leu Phe Ser Gly
        35                  40                  45

ccc ctt ttg cga aat caa aca gcc caa cta ttt tca tca cct ccg aaa       192
Pro Leu Leu Arg Asn Gln Thr Ala Gln Leu Phe Ser Ser Pro Pro Lys
    50                  55                  60

gtg tct cca aga ata tca cca aaa gta tct cct agt tct tcc cct cca       240
Val Ser Pro Arg Ile Ser Pro Lys Val Ser Pro Ser Ser Ser Pro Pro
65                  70                  75                  80

ttt gtg tcc tca ccc aaa atc agc gag cta cat gag ctt ccc aga ccc       288
Phe Val Ser Ser Pro Lys Ile Ser Glu Leu His Glu Leu Pro Arg Pro
                85                  90                  95

cca gtc act tca acc tcc aag tct cca ggg gct gta ggt ttg gtt ggt       336
Pro Val Thr Ser Thr Ser Lys Ser Pro Gly Ala Val Gly Leu Val Gly
            100                 105                 110

cat tca gcg cca ctg ttg ccc aaa ggc cac atg ctt cct ggt aca agc       384
His Ser Ala Pro Leu Leu Pro Lys Gly His Met Leu Pro Gly Thr Ser
        115                 120                 125

aaa acg tca gca tca cat gta gaa tct caa ctg cct aca cct tct caa       432
Lys Thr Ser Ala Ser His Val Glu Ser Gln Leu Pro Thr Pro Ser Gln
    130                 135                 140

gtt gtc cct cgc agt ttt tca ata ccc tct agc aga cat aga gta atg       480
Val Val Pro Arg Ser Phe Ser Ile Pro Ser Ser Arg His Arg Val Met
145                 150                 155                 160

gtc gct cag aat tca ggg att gtt gag aat gtt gcc tca cct cct cta       528
Val Ala Gln Asn Ser Gly Ile Val Glu Asn Val Ala Ser Pro Pro Leu
                165                 170                 175

acc cca ata tct tta tct aac acc cag cca tca tcc act ggt tcc ccg       576
Thr Pro Ile Ser Leu Ser Asn Thr Gln Pro Ser Ser Thr Gly Ser Pro
            180                 185                 190

atc gtc aat cag aca gtt caa atc aga ggt aat tca taa                   615
Ile Val Asn Gln Thr Val Gln Ile Arg Gly Asn Ser
        195                 200
```

<210> 32
<211> 204
<212> PRT
<213> P.trichocarpa

<400> 32

```
Met Asp Phe Phe Phe Ser Arg Leu Glu Pro Pro Ala Pro Ala Glu Ser
1               5                   10                  15

Lys Lys Ile Lys Arg Tyr Ala Leu Ser Gly Pro Ile Thr Ser Gln Pro
            20                  25                  30

Trp Ser Thr Lys Ala Val Ser Ala Glu Asn Pro Gln Leu Phe Ser Gly
        35                  40                  45

Pro Leu Leu Arg Asn Gln Thr Ala Gln Leu Phe Ser Ser Pro Pro Lys
    50                  55                  60

Val Ser Pro Arg Ile Ser Pro Lys Val Ser Pro Ser Ser Ser Pro Pro
65                  70                  75                  80

Phe Val Ser Ser Pro Lys Ile Ser Glu Leu His Glu Leu Pro Arg Pro
                85                  90                  95

Pro Val Thr Ser Thr Ser Lys Ser Pro Gly Ala Val Gly Leu Val Gly
            100                 105                 110

His Ser Ala Pro Leu Leu Pro Lys Gly His Met Leu Pro Gly Thr Ser
        115                 120                 125

Lys Thr Ser Ala Ser His Val Glu Ser Gln Leu Pro Thr Pro Ser Gln
    130                 135                 140

Val Val Pro Arg Ser Phe Ser Ile Pro Ser Ser Arg His Arg Val Met
145                 150                 155                 160

Val Ala Gln Asn Ser Gly Ile Val Glu Asn Val Ala Ser Pro Pro Leu
                165                 170                 175

Thr Pro Ile Ser Leu Ser Asn Thr Gln Pro Ser Ser Thr Gly Ser Pro
            180                 185                 190

Ile Val Asn Gln Thr Val Gln Ile Arg Gly Asn Ser
        195                 200
```

<210> 33
<211> 612
<212> DNA
<213> P.trichocarpa

<220>
<221> CDS
<222> (1)..(612)

<400> 33

```
atg gac acc tgt ttt cac ggc ttg aac cat tgg ctg ctt ttg att cta       48
Met Asp Thr Cys Phe His Gly Leu Asn His Trp Leu Leu Leu Ile Leu
1               5                   10                  15

aga aaa cca gaa gat atg cct ttt ctg gtc cta tta caa gca agc cat       96
Arg Lys Pro Glu Asp Met Pro Phe Leu Val Leu Leu Gln Ala Ser His
            20                  25                  30

tgt cta cca agc tgg tct cag cag aac atc ccc aat tgt tct ctg gac      144
Cys Leu Pro Ser Trp Ser Gln Gln Asn Ile Pro Asn Cys Ser Leu Asp
        35                  40                  45

ccc ttc tgc gaa atc cag ctg act caa tta tta tca cct cca aaa gtg      192
Pro Phe Cys Glu Ile Gln Leu Thr Gln Leu Leu Ser Pro Pro Lys Val
    50                  55                  60

tct cca ata ata tca cca aaa gta tct cct agt gct tcc cct act ttt      240
Ser Pro Ile Ile Ser Pro Lys Val Ser Pro Ser Ala Ser Pro Thr Phe
65                  70                  75                  80

gtg tcc ccg ccc aaa atc agt gag tta cat gag ctt cct aga ccc cca      288
Val Ser Pro Pro Lys Ile Ser Glu Leu His Glu Leu Pro Arg Pro Pro
                85                  90                  95

ctc agt tca acc tcc aag tct cca agg gct gaa ggt ttg gtt ggt cat      336
Leu Ser Ser Thr Ser Lys Ser Pro Arg Ala Glu Gly Leu Val Gly His
            100                 105                 110

tca acc cca ttg ttg ccc aaa ggt cgc atg cat cct gga aca aga aaa      384
Ser Thr Pro Leu Leu Pro Lys Gly Arg Met His Pro Gly Thr Arg Lys
        115                 120                 125

aca cca gcg tca aat gta gca tct caa ctg ccc aca cct tct caa gtt      432
Thr Pro Ala Ser Asn Val Ala Ser Gln Leu Pro Thr Pro Ser Gln Val
    130                 135                 140

gtc act cgc agt ttt tca ata ccc tct aga agt cgt aga ata atg gtc      480
Val Thr Arg Ser Phe Ser Ile Pro Ser Arg Ser Arg Arg Ile Met Val
145                 150                 155                 160

gct cag agt tca ggg att gct gag gat gtt gcc tca cct cct cta acc      528
Ala Gln Ser Ser Gly Ile Ala Glu Asp Val Ala Ser Pro Pro Leu Thr
                165                 170                 175

ccg ata tct tta tgt aac aac tac cca tca tcc acc ggt tcc cat act      576
Pro Ile Ser Leu Cys Asn Asn Tyr Pro Ser Ser Thr Gly Ser His Thr
            180                 185                 190

gtc aat cag acg gtt caa atc aga ggt gcc gat tga                      612
Val Asn Gln Thr Val Gln Ile Arg Gly Ala Asp
        195                 200
```

<210> 34
<211> 203
<212> PRT
<213> P.trichocarpa

<400> 34

Met Asp Thr Cys Phe His Gly Leu Asn His Trp Leu Leu Leu Ile Leu
1 5 10 15

Arg Lys Pro Glu Asp Met Pro Phe Leu Val Leu Leu Gln Ala Ser His
20 25 30

Cys Leu Pro Ser Trp Ser Gln Gln Asn Ile Pro Asn Cys Ser Leu Asp
35 40 45

Pro Phe Cys Glu Ile Gln Leu Thr Gln Leu Leu Ser Pro Pro Lys Val
50 55 60

Ser Pro Ile Ile Ser Pro Lys Val Ser Pro Ser Ala Ser Pro Thr Phe
65 70 75 80

Val Ser Pro Pro Lys Ile Ser Glu Leu His Glu Leu Pro Arg Pro Pro
85 90 95

Leu Ser Ser Thr Ser Lys Ser Pro Arg Ala Glu Gly Leu Val Gly His
100 105 110

Ser Thr Pro Leu Leu Pro Lys Gly Arg Met His Pro Gly Thr Arg Lys
115 120 125

Thr Pro Ala Ser Asn Val Ala Ser Gln Leu Pro Thr Pro Ser Gln Val
130 135 140

Val Thr Arg Ser Phe Ser Ile Pro Ser Arg Ser Arg Arg Ile Met Val
145 150 155 160

Ala Gln Ser Ser Gly Ile Ala Glu Asp Val Ala Ser Pro Pro Leu Thr
165 170 175

Pro Ile Ser Leu Cys Asn Asn Tyr Pro Ser Ser Thr Gly Ser His Thr
180 185 190

Val Asn Gln Thr Val Gln Ile Arg Gly Ala Asp
195 200

<210> 35
<211> 540
<212> DNA
<213> P.trichocarpa

<220>
<221> CDS
<222> (1)..(540)

<400> 35
atg aag ttg gcc tta tca tcc agt ggt ccc att tct gct act gaa ctt 48
Met Lys Leu Ala Leu Ser Ser Ser Gly Pro Ile Ser Ala Thr Glu Leu
1 5 10 15

```
tca caa caa gtt tct gga gtg ctt gct cat gtt gca aat cct cag cct        96
Ser Gln Gln Val Ser Gly Val Leu Ala His Val Ala Asn Pro Gln Pro
            20                  25                  30

tct aca tcc ccc aaa gta tcc cct agt aca tca cct cct ctt gtt tct       144
Ser Thr Ser Pro Lys Val Ser Pro Ser Thr Ser Pro Pro Leu Val Ser
        35                  40                  45

tca ccc agg ata agc gag ctt cat gaa ctt cca agg cca cca ggt aat       192
Ser Pro Arg Ile Ser Glu Leu His Glu Leu Pro Arg Pro Pro Gly Asn
    50                  55                  60

tta gca gcc aaa gct gca aaa tct ctg ggg ctg att gga cac tct gct       240
Leu Ala Ala Lys Ala Ala Lys Ser Leu Gly Leu Ile Gly His Ser Ala
65                  70                  75                  80

cca ttg gtc aga aat cct gag ctt tct gga aca aac aag att tct tca       288
Pro Leu Val Arg Asn Pro Glu Leu Ser Gly Thr Asn Lys Ile Ser Ser
                85                  90                  95

ggg gca gta aat ttg gca tca cct ctt ccc atg cca ccg ttg atg gtc       336
Gly Ala Val Asn Leu Ala Ser Pro Leu Pro Met Pro Pro Leu Met Val
            100                 105                 110

cca aca agt ttc tca ata ccc tca att agt cca aga aca atg act gta       384
Pro Thr Ser Phe Ser Ile Pro Ser Ile Ser Pro Arg Thr Met Thr Val
        115                 120                 125

cat gtt tct aag ctt ttg gat tct tct caa cta ctg gaa aaa cct ggg       432
His Val Ser Lys Leu Leu Asp Ser Ser Gln Leu Leu Glu Lys Pro Gly
    130                 135                 140

gaa gtt gat tct cct cca ctg aca ccg att tcc ctc aca aat atg agg       480
Glu Val Asp Ser Pro Pro Leu Thr Pro Ile Ser Leu Thr Asn Met Arg
145                 150                 155                 160

caa gca cct gcc att tca gaa tcg ata ccg cag tct ggt caa att aga       528
Gln Ala Pro Ala Ile Ser Glu Ser Ile Pro Gln Ser Gly Gln Ile Arg
                165                 170                 175

gga ggg agc tga                                                       540
Gly Gly Ser


<210>  36
<211>  179
<212>  PRT
<213>  P.trichocarpa

<400>  36

Met Lys Leu Ala Leu Ser Ser Ser Gly Pro Ile Ser Ala Thr Glu Leu
1               5                   10                  15

Ser Gln Gln Val Ser Gly Val Leu Ala His Val Ala Asn Pro Gln Pro
            20                  25                  30

Ser Thr Ser Pro Lys Val Ser Pro Ser Thr Ser Pro Pro Leu Val Ser
        35                  40                  45

Ser Pro Arg Ile Ser Glu Leu His Glu Leu Pro Arg Pro Pro Gly Asn
```

50 55 60

Leu Ala Ala Lys Ala Ala Lys Ser Leu Gly Leu Ile Gly His Ser Ala
65 70 75 80

Pro Leu Val Arg Asn Pro Glu Leu Ser Gly Thr Asn Lys Ile Ser Ser
85 90 95

Gly Ala Val Asn Leu Ala Ser Pro Leu Pro Met Pro Pro Leu Met Val
100 105 110

Pro Thr Ser Phe Ser Ile Pro Ser Ile Ser Pro Arg Thr Met Thr Val
115 120 125

His Val Ser Lys Leu Leu Asp Ser Ser Gln Leu Leu Glu Lys Pro Gly
130 135 140

Glu Val Asp Ser Pro Pro Leu Thr Pro Ile Ser Leu Thr Asn Met Arg
145 150 155 160

Gln Ala Pro Ala Ile Ser Glu Ser Ile Pro Gln Ser Gly Gln Ile Arg
165 170 175

Gly Gly Ser

<210> 37
<211> 399
<212> DNA
<213> T.aestivum

<220>
<221> CDS
<222> (1)..(399)
<223> 382 = unsure nucleotide

<220>
<221> misc_feature
<222> (292)..(292)
<223> N = unkown nucleotide

<220>
<221> misc_feature
<222> (293)..(293)
<223> N = unkown nucleotide

<220>
<221> misc_feature
<222> (294)..(294)
<223> N = unkown nucleotide

<220>
<221> misc_feature
<222> (382)..(382)
<223> N = unkown nucleotide

```
<400>  37
atg aaa tca ttg aag caa ggt caa ttc ttg agt att tta aca cag gct        48
Met Lys Ser Leu Lys Gln Gly Gln Phe Leu Ser Ile Leu Thr Gln Ala
1               5                   10                  15

gct cgc cat cat gct gct cag ttg agt ttt ttc agg aaa ggg ttg aag        96
Ala Arg His His Ala Ala Gln Leu Ser Phe Phe Arg Lys Gly Leu Lys
            20                  25                  30

tat cta gag gct cta gaa cct cat gta aaa gca gtt gct gag aag cag       144
Tyr Leu Glu Ala Leu Glu Pro His Val Lys Ala Val Ala Glu Lys Gln
        35                  40                  45

cac att gat tat cac ttc agt gga cta gat gat gac act gat aat gat       192
His Ile Asp Tyr His Phe Ser Gly Leu Asp Asp Asp Thr Asp Asn Asp
    50                  55                  60

gat tac agc tct tac cag gac aat cat agt gag ggc agt gag ttg agt       240
Asp Tyr Ser Ser Tyr Gln Asp Asn His Ser Glu Gly Ser Glu Leu Ser
65                  70                  75                  80

ttt gac tac ggg ata aac ttt cct gct tcc aga agt tca atg gat ttg       288
Phe Asp Tyr Gly Ile Asn Phe Pro Ala Ser Arg Ser Ser Met Asp Leu
                85                  90                  95

gat nnn tct aat atg gcg agt ccc acg aaa cct ctg aag gaa cat gag       336
Asp Xaa Ser Asn Met Ala Ser Pro Thr Lys Pro Leu Lys Glu His Glu
            100                 105                 110

caa gaa cat ggc aaa caa ata gag act gat ttg ccg gtc ctc caa ntg       384
Gln Glu His Gly Lys Gln Ile Glu Thr Asp Leu Pro Val Leu Gln Xaa
        115                 120                 125

aaa cct gaa ttt ggt                                                   399
Lys Pro Glu Phe Gly
    130


<210>  38
<211>  133
<212>  PRT
<213>  T.aestivum

<220>
<221>  misc_feature
<222>  (98)..(98)
<223>  The 'Xaa' at location 98 stands for Lys, Asn, Arg, Ser, Thr, Ile,
       Met, Glu, Asp, Gly, Ala, Val, Gln, His, Pro, Leu, Tyr, Trp, Cys,
       or Phe.

<220>
<221>  misc_feature
<222>  (128)..(128)
<223>  The 'Xaa' at location 128 stands for Met, Val, or Leu.

<400>  38

Met Lys Ser Leu Lys Gln Gly Gln Phe Leu Ser Ile Leu Thr Gln Ala
1               5                   10                  15


Ala Arg His His Ala Ala Gln Leu Ser Phe Phe Arg Lys Gly Leu Lys
            20                  25                  30


Tyr Leu Glu Ala Leu Glu Pro His Val Lys Ala Val Ala Glu Lys Gln
```

```
        35                  40                  45

His Ile Asp Tyr His Phe Ser Gly Leu Asp Asp Asp Thr Asp Asn Asp
    50                  55                  60

Asp Tyr Ser Ser Tyr Gln Asp Asn His Ser Glu Gly Ser Glu Leu Ser
65                  70                  75                  80

Phe Asp Tyr Gly Ile Asn Phe Pro Ala Ser Arg Ser Ser Met Asp Leu
                85                  90                  95

Asp Xaa Ser Asn Met Ala Ser Pro Thr Lys Pro Leu Lys Glu His Glu
            100                 105                 110

Gln Glu His Gly Lys Gln Ile Glu Thr Asp Leu Pro Val Leu Gln Xaa
        115                 120                 125

Lys Pro Glu Phe Gly
    130


<210>  39
<211>  840
<212>  DNA
<213>  T.aestivum


<220>
<221>  CDS
<222>  (1)..(840)

<400>  39
atg ttc tcg agc cca cta cag tca agt aga aca aat tat tcc tac aaa      48
Met Phe Ser Ser Pro Leu Gln Ser Ser Arg Thr Asn Tyr Ser Tyr Lys
1               5                   10                  15

ctc ccg aca cca gct gat gat aaa atc tgc act tca gca ggc acc aac      96
Leu Pro Thr Pro Ala Asp Asp Lys Ile Cys Thr Ser Ala Gly Thr Asn
            20                  25                  30

aga tca cct cat tca gat aaa cca gag agt tca cat gtg gca gca aat     144
Arg Ser Pro His Ser Asp Lys Pro Glu Ser Ser His Val Ala Ala Asn
        35                  40                  45

ttg tgg cat tcc tct ccg ctc gtg aaa gat tat aag ccg agc tcc ttg     192
Leu Trp His Ser Ser Pro Leu Val Lys Asp Tyr Lys Pro Ser Ser Leu
    50                  55                  60

tat agc ggg cct gtt aag atg cca tca agt act gag agg aga tca tca     240
Tyr Ser Gly Pro Val Lys Met Pro Ser Ser Thr Glu Arg Arg Ser Ser
65                  70                  75                  80

cca tta gtt tat tcc tac tcc act tca gat tcc aag aaa atg aag agg     288
Pro Leu Val Tyr Ser Tyr Ser Thr Ser Asp Ser Lys Lys Met Lys Arg
                85                  90                  95

gaa tct ttt tct ggc cca att cct agt aaa gca ggg tta agc aaa ccc     336
Glu Ser Phe Ser Gly Pro Ile Pro Ser Lys Ala Gly Leu Ser Lys Pro
            100                 105                 110
```

```
ttg ttt tca gct gct ggt cac aga gca tca gta aat tat cct cct cgt      384
Leu Phe Ser Ala Ala Gly His Arg Ala Ser Val Asn Tyr Pro Pro Arg
        115                 120                 125

gtt atg tca aca aaa tca cac gga cca ggt tct ctt cca tct gtg gct      432
Val Met Ser Thr Lys Ser His Gly Pro Gly Ser Leu Pro Ser Val Ala
    130                 135                 140

cca aaa gtc cct agg ata act tca ctg cca aca aca tcc ccc aga att      480
Pro Lys Val Pro Arg Ile Thr Ser Leu Pro Thr Thr Ser Pro Arg Ile
145                 150                 155                 160

agt gag ctt cat gag ctg cct agg cct cct gca cct tta gac act ctc      528
Ser Glu Leu His Glu Leu Pro Arg Pro Pro Ala Pro Leu Asp Thr Leu
                165                 170                 175

cgg cct ggt ttg gtt gga tat tct ggt cct ttg gta tca agg cgg caa      576
Arg Pro Gly Leu Val Gly Tyr Ser Gly Pro Leu Val Ser Arg Arg Gln
            180                 185                 190

att cct acc cgt gcc tcc cca ccg tca aat aca gca tca ccg ctt cca      624
Ile Pro Thr Arg Ala Ser Pro Pro Ser Asn Thr Ala Ser Pro Leu Pro
        195                 200                 205

cga cca cct gct gcc atg act cgc agc tat tct ata cct tca aac agc      672
Arg Pro Pro Ala Ala Met Thr Arg Ser Tyr Ser Ile Pro Ser Asn Ser
    210                 215                 220

caa aga aca cct att att act gtg aat aag ttg ttg gag tct agg cat      720
Gln Arg Thr Pro Ile Ile Thr Val Asn Lys Leu Leu Glu Ser Arg His
225                 230                 235                 240

agc aga gag agc agc gaa gtt tcc tcg ccc cca cta aca cct ata tct      768
Ser Arg Glu Ser Ser Glu Val Ser Ser Pro Pro Leu Thr Pro Ile Ser
                245                 250                 255

ttg gca gat gtt tcc cgc aaa gaa ata gca gaa aca act ata gac acc      816
Leu Ala Asp Val Ser Arg Lys Glu Ile Ala Glu Thr Thr Ile Asp Thr
            260                 265                 270

aga agg atg aag gaa acc tcg tga                                      840
Arg Arg Met Lys Glu Thr Ser
        275


<210>  40
<211>  279
<212>  PRT
<213>  T.aestivum

<400>  40

Met Phe Ser Ser Pro Leu Gln Ser Ser Arg Thr Asn Tyr Ser Tyr Lys
1               5                   10                  15


Leu Pro Thr Pro Ala Asp Asp Lys Ile Cys Thr Ser Ala Gly Thr Asn
            20                  25                  30


Arg Ser Pro His Ser Asp Lys Pro Glu Ser Ser His Val Ala Ala Asn
        35                  40                  45


Leu Trp His Ser Ser Pro Leu Val Lys Asp Tyr Lys Pro Ser Ser Leu
    50                  55                  60
```

```
Tyr Ser Gly Pro Val Lys Met Pro Ser Ser Thr Glu Arg Arg Ser Ser
65                  70                  75                  80

Pro Leu Val Tyr Ser Tyr Ser Thr Ser Asp Ser Lys Lys Met Lys Arg
                85                  90                  95

Glu Ser Phe Ser Gly Pro Ile Pro Ser Lys Ala Gly Leu Ser Lys Pro
            100                 105                 110

Leu Phe Ser Ala Ala Gly His Arg Ala Ser Val Asn Tyr Pro Pro Arg
        115                 120                 125

Val Met Ser Thr Lys Ser His Gly Pro Gly Ser Leu Pro Ser Val Ala
    130                 135                 140

Pro Lys Val Pro Arg Ile Thr Ser Leu Pro Thr Thr Ser Pro Arg Ile
145                 150                 155                 160

Ser Glu Leu His Glu Leu Pro Arg Pro Pro Ala Pro Leu Asp Thr Leu
                165                 170                 175

Arg Pro Gly Leu Val Gly Tyr Ser Gly Pro Leu Val Ser Arg Arg Gln
            180                 185                 190

Ile Pro Thr Arg Ala Ser Pro Pro Ser Asn Thr Ala Ser Pro Leu Pro
        195                 200                 205

Arg Pro Pro Ala Ala Met Thr Arg Ser Tyr Ser Ile Pro Ser Asn Ser
    210                 215                 220

Gln Arg Thr Pro Ile Ile Thr Val Asn Lys Leu Leu Glu Ser Arg His
225                 230                 235                 240

Ser Arg Glu Ser Ser Glu Val Ser Ser Pro Pro Leu Thr Pro Ile Ser
                245                 250                 255

Leu Ala Asp Val Ser Arg Lys Glu Ile Ala Glu Thr Thr Ile Asp Thr
            260                 265                 270

Arg Arg Met Lys Glu Thr Ser
        275

<210>  41
<211>  453
<212>  DNA
<213>  T.aestivum

<220>
<221>  CDS
```

<222> (1)..(453)

<400> 41

```
atg ata tcc aag tcg tgt gtt cat gct agg cag ccg tca cca gtt tca        48
Met Ile Ser Lys Ser Cys Val His Ala Arg Gln Pro Ser Pro Val Ser
1               5                   10                  15

ccc aag atg ttc cca cct tca ata gaa tcc ccc aaa atc agt gag ctt        96
Pro Lys Met Phe Pro Pro Ser Ile Glu Ser Pro Lys Ile Ser Glu Leu
            20                  25                  30

cat gag ctg cct agg cct cca gcc aat gtt gag ccc ctc cgg cct tgt       144
His Glu Leu Pro Arg Pro Pro Ala Asn Val Glu Pro Leu Arg Pro Cys
        35                  40                  45

ggt cta gtt ggc tac tcc ggt cct ttg gta tca aag cgc caa gct cct       192
Gly Leu Val Gly Tyr Ser Gly Pro Leu Val Ser Lys Arg Gln Ala Pro
    50                  55                  60

aca gca cct gtc cgt gcc tcg cca aca gcg tcg cag aca gcc tcg ccg       240
Thr Ala Pro Val Arg Ala Ser Pro Thr Ala Ser Gln Thr Ala Ser Pro
65                  70                  75                  80

ctt ccg cgg cca cct gct tcc ttg gct cgc agt ttc tcc ata ccc tcg       288
Leu Pro Arg Pro Pro Ala Ser Leu Ala Arg Ser Phe Ser Ile Pro Ser
                85                  90                  95

aac agc cag aga aca ccc ctc att aca gtc aat aag ttg ctt gag gcc       336
Asn Ser Gln Arg Thr Pro Leu Ile Thr Val Asn Lys Leu Leu Glu Ala
            100                 105                 110

aga agt agc aga gag agc agt gaa att tcc tcc cca cca ctc act ccg       384
Arg Ser Ser Arg Glu Ser Ser Glu Ile Ser Ser Pro Pro Leu Thr Pro
        115                 120                 125

ttg ttt tct tct acc agc caa cac aaa aaa caa tta aag gca gca ctc       432
Leu Phe Ser Ser Thr Ser Gln His Lys Lys Gln Leu Lys Ala Ala Leu
    130                 135                 140

gga gaa aag ggt atg tca tag                                           453
Gly Glu Lys Gly Met Ser
145                 150
```

<210> 42
<211> 150
<212> PRT
<213> T.aestivum

<400> 42

```
Met Ile Ser Lys Ser Cys Val His Ala Arg Gln Pro Ser Pro Val Ser
1               5                   10                  15

Pro Lys Met Phe Pro Pro Ser Ile Glu Ser Pro Lys Ile Ser Glu Leu
            20                  25                  30

His Glu Leu Pro Arg Pro Pro Ala Asn Val Glu Pro Leu Arg Pro Cys
        35                  40                  45

Gly Leu Val Gly Tyr Ser Gly Pro Leu Val Ser Lys Arg Gln Ala Pro
    50                  55                  60
```

```
Thr Ala Pro Val Arg Ala Ser Pro Thr Ala Ser Gln Thr Ala Ser Pro
65                  70                  75                  80

Leu Pro Arg Pro Pro Ala Ser Leu Ala Arg Ser Phe Ser Ile Pro Ser
                85                  90                  95

Asn Ser Gln Arg Thr Pro Leu Ile Thr Val Asn Lys Leu Leu Glu Ala
            100                 105                 110

Arg Ser Ser Arg Glu Ser Ser Glu Ile Ser Ser Pro Pro Leu Thr Pro
        115                 120                 125

Leu Phe Ser Ser Thr Ser Gln His Lys Lys Gln Leu Lys Ala Ala Leu
    130                 135                 140

Gly Glu Lys Gly Met Ser
145                 150
```

<210> 43
<211> 1299
<212> DNA
<213> Zea mays

<220>
<221> CDS
<222> (1)..(1299)

<400> 43

```
atg aag tcg tcg ctg cgg aag ctg cgg ggc ttc gcg ctg cag cgc cag       48
Met Lys Ser Ser Leu Arg Lys Leu Arg Gly Phe Ala Leu Gln Arg Gln
1               5                   10                  15

gag cag cgg gtg gac cgg gac cgc ggc cgg ggc cac gcc acc gcc gcc       96
Glu Gln Arg Val Asp Arg Asp Arg Gly Arg Gly His Ala Thr Ala Ala
            20                  25                  30

gcc acc gcg gcg gac gag ctc ctc gcg gct gcc cag gat atg gca gat      144
Ala Thr Ala Ala Asp Glu Leu Leu Ala Ala Ala Gln Asp Met Ala Asp
        35                  40                  45

atg aga agc tgt tac gat aac ttg ctt tct gtt gct gca gca att gca      192
Met Arg Ser Cys Tyr Asp Asn Leu Leu Ser Val Ala Ala Ala Ile Ala
    50                  55                  60

aat agt gca tat gag ttc tct gaa gca ctt caa gaa atg gga act tgt      240
Asn Ser Ala Tyr Glu Phe Ser Glu Ala Leu Gln Glu Met Gly Thr Cys
65                  70                  75                  80

ttg ctt aaa aga gtt acg cca aat aag gat gga ata aat gat aaa gtt      288
Leu Leu Lys Arg Val Thr Pro Asn Lys Asp Gly Ile Asn Asp Lys Val
                85                  90                  95

ttg ctg ttg ctt ggg aaa tca caa ttt gaa ctt cgg aaa ctt tta gat      336
Leu Leu Leu Leu Gly Lys Ser Gln Phe Glu Leu Arg Lys Leu Leu Asp
            100                 105                 110

agt tat cgt gtg cat gtt ctt aac acc atc acc act cca tcg ctg tcg      384
Ser Tyr Arg Val His Val Leu Asn Thr Ile Thr Thr Pro Ser Leu Ser
```

```
        115                 120                 125

ctt ctt aat gaa ctt caa act gtg gag gaa atg aag cac caa tgc gat        432
Leu Leu Asn Glu Leu Gln Thr Val Glu Glu Met Lys His Gln Cys Asp
    130                 135                 140

gaa aaa aaa gaa ttg tat gaa ttc atg gta aac acg cag aaa gaa aag        480
Glu Lys Lys Glu Leu Tyr Glu Phe Met Val Asn Thr Gln Lys Glu Lys
145                 150                 155                 160

gga agg tct aag aat gct aaa ggt gat aat gga gca tca gag caa ttg        528
Gly Arg Ser Lys Asn Ala Lys Gly Asp Asn Gly Ala Ser Glu Gln Leu
                165                 170                 175

aaa caa gct cag gaa gat tat caa gag gaa gca act ctt ttt cta ttc        576
Lys Gln Ala Gln Glu Asp Tyr Gln Glu Glu Ala Thr Leu Phe Leu Phe
            180                 185                 190

cgg ttg aag tca ttg aag caa gga cag ttc cga agt ctt ttt aca caa        624
Arg Leu Lys Ser Leu Lys Gln Gly Gln Phe Arg Ser Leu Phe Thr Gln
        195                 200                 205

gct gct cgg cat cat gct gca cag cta aat ttg ttc cga aag gga gtc        672
Ala Ala Arg His His Ala Ala Gln Leu Asn Leu Phe Arg Lys Gly Val
    210                 215                 220

aag tcc ctt gag gct gta gag cca cat gtt agg ctc gct gct gag cag        720
Lys Ser Leu Glu Ala Val Glu Pro His Val Arg Leu Ala Ala Glu Gln
225                 230                 235                 240

caa cac att gat cat cag ttc agc gca ctt gag gaa gag gat tac ctt        768
Gln His Ile Asp His Gln Phe Ser Ala Leu Glu Glu Glu Asp Tyr Leu
                245                 250                 255

gtt gag gat gaa aat gat gac gat tac aat ggc agt cat gat gga gag        816
Val Glu Asp Glu Asn Asp Asp Asp Tyr Asn Gly Ser His Asp Gly Glu
            260                 265                 270

tta tct ttt gat tat gga gaa aat aag gaa gtt gag gaa tct ggt aat        864
Leu Ser Phe Asp Tyr Gly Glu Asn Lys Glu Val Glu Glu Ser Gly Asn
        275                 280                 285

gct tct agg aat cat aca gag ggt ttt ttt aac aga agc aaa gaa gag        912
Ala Ser Arg Asn His Thr Glu Gly Phe Phe Asn Arg Ser Lys Glu Glu
    290                 295                 300

tat tct tct gtt cca cat gaa aga caa aga atc gta agt cag tca gca        960
Tyr Ser Ser Val Pro His Glu Arg Gln Arg Ile Val Ser Gln Ser Ala
305                 310                 315                 320

cca ctt ttt cct gag aaa aag ctc aat aca gaa gag aga ata aaa gat       1008
Pro Leu Phe Pro Glu Lys Lys Leu Asn Thr Glu Glu Arg Ile Lys Asp
                325                 330                 335

ttg cgg cgt tct gca aca agg aag ttg aat act tat gtt ttg cct act       1056
Leu Arg Arg Ser Ala Thr Arg Lys Leu Asn Thr Tyr Val Leu Pro Thr
            340                 345                 350

cca aat gac gtt gga gct act tct cag aga gca tca gga aat cct act       1104
Pro Asn Asp Val Gly Ala Thr Ser Gln Arg Ala Ser Gly Asn Pro Thr
        355                 360                 365

tct gaa cct ctc gag agt aaa ggt gcc ttc cat tca tct cca ctc cac       1152
Ser Glu Pro Leu Glu Ser Lys Gly Ala Phe His Ser Ser Pro Leu His
    370                 375                 380
```

```
tca tct gca gac ata aga gat ttg gga gac agt aat cta cct agt cct      1200
Ser Ser Ala Asp Ile Arg Asp Leu Gly Asp Ser Asn Leu Pro Ser Pro
385                 390                 395                 400

aca aga ttg tct aac gta cag tct gtg ctg aaa gat agc aat acc aat      1248
Thr Arg Leu Ser Asn Val Gln Ser Val Leu Lys Asp Ser Asn Thr Asn
                405                 410                 415

aaa aca gaa aca agg aaa gta ctc cca gtg ggt gat ctg ggc ttt acc      1296
Lys Thr Glu Thr Arg Lys Val Leu Pro Val Gly Asp Leu Gly Phe Thr
            420                 425                 430

taa                                                                  1299


<210>  44
<211>  432
<212>  PRT
<213>  Zea mays

<400>  44

Met Lys Ser Ser Leu Arg Lys Leu Arg Gly Phe Ala Leu Gln Arg Gln
1               5                   10                  15


Glu Gln Arg Val Asp Arg Asp Arg Gly Arg Gly His Ala Thr Ala Ala
            20                  25                  30


Ala Thr Ala Ala Asp Glu Leu Leu Ala Ala Ala Gln Asp Met Ala Asp
        35                  40                  45


Met Arg Ser Cys Tyr Asp Asn Leu Leu Ser Val Ala Ala Ala Ile Ala
    50                  55                  60


Asn Ser Ala Tyr Glu Phe Ser Glu Ala Leu Gln Glu Met Gly Thr Cys
65                  70                  75                  80


Leu Leu Lys Arg Val Thr Pro Asn Lys Asp Gly Ile Asn Asp Lys Val
                85                  90                  95


Leu Leu Leu Leu Gly Lys Ser Gln Phe Glu Leu Arg Lys Leu Leu Asp
            100                 105                 110


Ser Tyr Arg Val His Val Leu Asn Thr Ile Thr Thr Pro Ser Leu Ser
        115                 120                 125


Leu Leu Asn Glu Leu Gln Thr Val Glu Glu Met Lys His Gln Cys Asp
    130                 135                 140


Glu Lys Lys Glu Leu Tyr Glu Phe Met Val Asn Thr Gln Lys Glu Lys
145                 150                 155                 160


Gly Arg Ser Lys Asn Ala Lys Gly Asp Asn Gly Ala Ser Glu Gln Leu
                165                 170                 175
```

Lys Gln Ala Gln Glu Asp Tyr Gln Glu Glu Ala Thr Leu Phe Leu Phe
180 185 190

Arg Leu Lys Ser Leu Lys Gln Gly Gln Phe Arg Ser Leu Phe Thr Gln
195 200 205

Ala Ala Arg His His Ala Ala Gln Leu Asn Leu Phe Arg Lys Gly Val
210 215 220

Lys Ser Leu Glu Ala Val Glu Pro His Val Arg Leu Ala Ala Glu Gln
225 230 235 240

Gln His Ile Asp His Gln Phe Ser Ala Leu Glu Glu Glu Asp Tyr Leu
245 250 255

Val Glu Asp Glu Asn Asp Asp Asp Tyr Asn Gly Ser His Asp Gly Glu
260 265 270

Leu Ser Phe Asp Tyr Gly Glu Asn Lys Glu Val Glu Glu Ser Gly Asn
275 280 285

Ala Ser Arg Asn His Thr Glu Gly Phe Phe Asn Arg Ser Lys Glu Glu
290 295 300

Tyr Ser Ser Val Pro His Glu Arg Gln Arg Ile Val Ser Gln Ser Ala
305 310 315 320

Pro Leu Phe Pro Glu Lys Lys Leu Asn Thr Glu Glu Arg Ile Lys Asp
325 330 335

Leu Arg Arg Ser Ala Thr Arg Lys Leu Asn Thr Tyr Val Leu Pro Thr
340 345 350

Pro Asn Asp Val Gly Ala Thr Ser Gln Arg Ala Ser Gly Asn Pro Thr
355 360 365

Ser Glu Pro Leu Glu Ser Lys Gly Ala Phe His Ser Ser Pro Leu His
370 375 380

Ser Ser Ala Asp Ile Arg Asp Leu Gly Asp Ser Asn Leu Pro Ser Pro
385 390 395 400

Thr Arg Leu Ser Asn Val Gln Ser Val Leu Lys Asp Ser Asn Thr Asn
405 410 415

Lys Thr Glu Thr Arg Lys Val Leu Pro Val Gly Asp Leu Gly Phe Thr
420 425 430

<210> 45
<211> 633

```
<212>  DNA
<213>  Zea mays

<220>
<221>  CDS
<222>  (1)..(633)

<400>  45
atg ccg aat tcc atg cac agt gga cct gtt aag atg cca tca agt aac       48
Met Pro Asn Ser Met His Ser Gly Pro Val Lys Met Pro Ser Ser Asn
1               5                   10                  15

gaa ggg gta tca cta gct tat tcc tac tcc acg aca gat ttt aag aaa       96
Glu Gly Val Ser Leu Ala Tyr Ser Tyr Ser Thr Thr Asp Phe Lys Lys
            20                  25                  30

gtg aag agg gaa gct ttt tct ggc cca atc cca agc aag gta gga ttg      144
Val Lys Arg Glu Ala Phe Ser Gly Pro Ile Pro Ser Lys Val Gly Leu
        35                  40                  45

agc acc ccc ctg att tct gcg act gat cgc aaa ccg tca ggg aag cgg      192
Ser Thr Pro Leu Ile Ser Ala Thr Asp Arg Lys Pro Ser Gly Lys Arg
    50                  55                  60

cct tcc tat gtg ttg cca aca agg cct cct ggc cca ggt tgg cag tca      240
Pro Ser Tyr Val Leu Pro Thr Arg Pro Pro Gly Pro Gly Trp Gln Ser
65                  70                  75                  80

tct gtg cct cca aaa gct acg ccc agg gtg acc tcg ctc cca aca acg      288
Ser Val Pro Pro Lys Ala Thr Pro Arg Val Thr Ser Leu Pro Thr Thr
                85                  90                  95

act ccc agg ata agc gag ctg cat gag ctg ccc cgg cct cca gca aat      336
Thr Pro Arg Ile Ser Glu Leu His Glu Leu Pro Arg Pro Pro Ala Asn
            100                 105                 110

gct ggt ttg gtt gga tat tct ggc cct ctg gtt tca agg cgg ccg atg      384
Ala Gly Leu Val Gly Tyr Ser Gly Pro Leu Val Ser Arg Arg Pro Met
        115                 120                 125

cct act gct gta tcc acc acc cgt gtc tca ccc cca tca cat acg gcg      432
Pro Thr Ala Val Ser Thr Thr Arg Val Ser Pro Pro Ser His Thr Ala
    130                 135                 140

tcg cca ctc cca cga cca cct gct gcc atg act cgt agc tac tcc ata      480
Ser Pro Leu Pro Arg Pro Pro Ala Ala Met Thr Arg Ser Tyr Ser Ile
145                 150                 155                 160

cct tca aac agt caa cgg act cct att att acc gtg aac aaa ttg ttg      528
Pro Ser Asn Ser Gln Arg Thr Pro Ile Ile Thr Val Asn Lys Leu Leu
                165                 170                 175

gag gct agg cat agc agg gag agt agc gaa gtt tcc tcc ccc cca gta      576
Glu Ala Arg His Ser Arg Glu Ser Ser Glu Val Ser Ser Pro Pro Val
            180                 185                 190

aca cct ata tca ctg gca gac gtt tcc cgc aga cca aca aca gaa gca      624
Thr Pro Ile Ser Leu Ala Asp Val Ser Arg Arg Pro Thr Thr Glu Ala
        195                 200                 205

gct gtt tga                                                          633
Ala Val
    210
```

<210> 46
<211> 210
<212> PRT
<213> Zea mays

<400> 46

```
Met Pro Asn Ser Met His Ser Gly Pro Val Lys Met Pro Ser Ser Asn
1               5                   10                  15

Glu Gly Val Ser Leu Ala Tyr Ser Tyr Ser Thr Thr Asp Phe Lys Lys
            20                  25                  30

Val Lys Arg Glu Ala Phe Ser Gly Pro Ile Pro Ser Lys Val Gly Leu
        35                  40                  45

Ser Thr Pro Leu Ile Ser Ala Thr Asp Arg Lys Pro Ser Gly Lys Arg
    50                  55                  60

Pro Ser Tyr Val Leu Pro Thr Arg Pro Pro Gly Pro Gly Trp Gln Ser
65                  70                  75                  80

Ser Val Pro Pro Lys Ala Thr Pro Arg Val Thr Ser Leu Pro Thr Thr
                85                  90                  95

Thr Pro Arg Ile Ser Glu Leu His Glu Leu Pro Arg Pro Pro Ala Asn
            100                 105                 110

Ala Gly Leu Val Gly Tyr Ser Gly Pro Leu Val Ser Arg Arg Pro Met
        115                 120                 125

Pro Thr Ala Val Ser Thr Thr Arg Val Ser Pro Pro Ser His Thr Ala
    130                 135                 140

Ser Pro Leu Pro Arg Pro Pro Ala Ala Met Thr Arg Ser Tyr Ser Ile
145                 150                 155                 160

Pro Ser Asn Ser Gln Arg Thr Pro Ile Ile Thr Val Asn Lys Leu Leu
                165                 170                 175

Glu Ala Arg His Ser Arg Glu Ser Ser Glu Val Ser Ser Pro Pro Val
            180                 185                 190

Thr Pro Ile Ser Leu Ala Asp Val Ser Arg Arg Pro Thr Thr Glu Ala
        195                 200                 205

Ala Val
    210
```

<210> 47
<211> 1848
<212> DNA

```
<213>  Zea mays

<220>
<221>  CDS
<222>  (1)..(1848)

<400>  47
atg aaa tcg cca ttg cgc agg ttc cgg ggc ttc gcc ctt cat cac cac        48
Met Lys Ser Pro Leu Arg Arg Phe Arg Gly Phe Ala Leu His His His
1               5                   10                  15

cac cac cac agg gag agg aag gac cac cgc ccg ccg tcc gcc aag ctc        96
His His His Arg Glu Arg Lys Asp His Arg Pro Pro Ser Ala Lys Leu
            20                  25                  30

gac gag ctc gtc tac gcc gcc cag gaa atg gaa gat atg agg aac tgt       144
Asp Glu Leu Val Tyr Ala Ala Gln Glu Met Glu Asp Met Arg Asn Cys
        35                  40                  45

tac gat agc ttg ctt tca gct gca gcc gca acg aca aat agt gta tat       192
Tyr Asp Ser Leu Leu Ser Ala Ala Ala Ala Thr Thr Asn Ser Val Tyr
    50                  55                  60

gag ttt gcg gaa gcc atg ggt gaa atg gga act tgc ttg ctt gaa aaa       240
Glu Phe Ala Glu Ala Met Gly Glu Met Gly Thr Cys Leu Leu Glu Lys
65                  70                  75                  80

gct gca ttg aat tat gat gat gat gaa agt ggt aaa gtg ctg atg aag       288
Ala Ala Leu Asn Tyr Asp Asp Asp Glu Ser Gly Lys Val Leu Met Lys
                85                  90                  95

cta ggg aag gcc caa ttt gaa cta caa aag ttt gtg gat gac tat cgt       336
Leu Gly Lys Ala Gln Phe Glu Leu Gln Lys Phe Val Asp Asp Tyr Arg
            100                 105                 110

aca aat att ata aat aca atc aca aat cca tca gag tcc ctt ctg aaa       384
Thr Asn Ile Ile Asn Thr Ile Thr Asn Pro Ser Glu Ser Leu Leu Lys
        115                 120                 125

gag cta caa gtc gta gag gaa atg aaa gac caa tgt gat caa aaa aga       432
Glu Leu Gln Val Val Glu Glu Met Lys Asp Gln Cys Asp Gln Lys Arg
    130                 135                 140

gtg gaa tat gaa gcc atg cga gca gcc tat ggg gag aaa gga ggg cga       480
Val Glu Tyr Glu Ala Met Arg Ala Ala Tyr Gly Glu Lys Gly Gly Arg
145                 150                 155                 160

ttg agg cac cta aaa aat gaa tca ttc tca ttg gga caa ctg caa act       528
Leu Arg His Leu Lys Asn Glu Ser Phe Ser Leu Gly Gln Leu Gln Thr
                165                 170                 175

tca ttt ctt gag tac caa gag gag gcg gca ttg ttt att ttt cgc ttg       576
Ser Phe Leu Glu Tyr Gln Glu Glu Ala Ala Leu Phe Ile Phe Arg Leu
            180                 185                 190

aaa tca cta aag caa ggt caa ttt tta agt att ttg aca cag gct gct       624
Lys Ser Leu Lys Gln Gly Gln Phe Leu Ser Ile Leu Thr Gln Ala Ala
        195                 200                 205

cgt cat cat gct tct cag ttg agt ttt ttc agg aaa ggg ctg aag cat       672
Arg His His Ala Ser Gln Leu Ser Phe Phe Arg Lys Gly Leu Lys His
    210                 215                 220

cta gag gct ttg gag cct tgt gtc aaa gca gtt gct gag aag cag cac       720
Leu Glu Ala Leu Glu Pro Cys Val Lys Ala Val Ala Glu Lys Gln His
```

```
225                 230                 235                 240

att gac tac cac ttc agt gga ctg gat aat aac agt gat att gat gat       768
Ile Asp Tyr His Phe Ser Gly Leu Asp Asn Asn Ser Asp Ile Asp Asp
                245                 250                 255

tac agc tct tac cag gat aat cat agt gat ggc agt gag tta agt ttt       816
Tyr Ser Ser Tyr Gln Asp Asn His Ser Asp Gly Ser Glu Leu Ser Phe
            260                 265                 270

aac tat gag ata aat gac agg gac aag gac ctc ccc act ttc aga agt       864
Asn Tyr Glu Ile Asn Asp Arg Asp Lys Asp Leu Pro Thr Phe Arg Ser
        275                 280                 285

cca atg gat atg gat caa gct cat cca gtg agt tcc cca aga cct ctg       912
Pro Met Asp Met Asp Gln Ala His Pro Val Ser Ser Pro Arg Pro Leu
    290                 295                 300

aag gaa cag gag cag gaa aat gct gaa gaa ata aag gcg act tta gga       960
Lys Glu Gln Glu Gln Glu Asn Ala Glu Glu Ile Lys Ala Thr Leu Gly
305                 310                 315                 320

gtt cct cat atg aag cct gag att ggt acc caa tca gca cca att ttt      1008
Val Pro His Met Lys Pro Glu Ile Gly Thr Gln Ser Ala Pro Ile Phe
                325                 330                 335

gct gag aat gtt cct gat cca tct atg aga ttc cgg aaa atg aac ttg      1056
Ala Glu Asn Val Pro Asp Pro Ser Met Arg Phe Arg Lys Met Asn Leu
            340                 345                 350

tta acc aga act gtt cac tcc tac aaa ctc cca acg cca gct gat gac      1104
Leu Thr Arg Thr Val His Ser Tyr Lys Leu Pro Thr Pro Ala Asp Asp
        355                 360                 365

aag aac cct gct tca gta att gcc aac aaa tcg cct cat tca gat caa      1152
Lys Asn Pro Ala Ser Val Ile Ala Asn Lys Ser Pro His Ser Asp Gln
    370                 375                 380

ccc gag agt aaa tct cac gtg gca gta aat ttg tgg cat tcc tct cca      1200
Pro Glu Ser Lys Ser His Val Ala Val Asn Leu Trp His Ser Ser Pro
385                 390                 395                 400

ctg gct aaa gat ttt atg ccg aat tcc atg cac agt gga cct gtt aag      1248
Leu Ala Lys Asp Phe Met Pro Asn Ser Met His Ser Gly Pro Val Lys
                405                 410                 415

atg cca tca agt aac gaa ggg gta tca cta gct tat tcc tac tcc acg      1296
Met Pro Ser Ser Asn Glu Gly Val Ser Leu Ala Tyr Ser Tyr Ser Thr
            420                 425                 430

aca gat ttt aag aaa gtg aag agg gaa gct ttt tct ggc cca atc cca      1344
Thr Asp Phe Lys Lys Val Lys Arg Glu Ala Phe Ser Gly Pro Ile Pro
        435                 440                 445

agc aag gta gga ttg agc acc ccc ctg att tct gcg act gat cgc aaa      1392
Ser Lys Val Gly Leu Ser Thr Pro Leu Ile Ser Ala Thr Asp Arg Lys
    450                 455                 460

ccg tca ggg aag cgg cct tcc tat gtg ttg cca aca agg cct cct ggc      1440
Pro Ser Gly Lys Arg Pro Ser Tyr Val Leu Pro Thr Arg Pro Pro Gly
465                 470                 475                 480

cca ggt tgg cag tca tct gtg cct cca aaa gct acg ccc agg gtg acc      1488
Pro Gly Trp Gln Ser Ser Val Pro Pro Lys Ala Thr Pro Arg Val Thr
                485                 490                 495
```

```
tcg ctc cca aca acg act ccc agg ata agc gag ctg cat gag ctg ccc      1536
Ser Leu Pro Thr Thr Thr Pro Arg Ile Ser Glu Leu His Glu Leu Pro
            500                 505                 510

cgg cct cca gca aat gct ggt ttg gtt gga tat tct ggc cct ctg gtt      1584
Arg Pro Pro Ala Asn Ala Gly Leu Val Gly Tyr Ser Gly Pro Leu Val
        515                 520                 525

tca agg cgg ccg atg cct act gct gta tcc acc acc cgt gtc tca ccc      1632
Ser Arg Arg Pro Met Pro Thr Ala Val Ser Thr Thr Arg Val Ser Pro
    530                 535                 540

cca tca cat acg gcg tcg cca ctc cca cga cca cct gct gcc atg act      1680
Pro Ser His Thr Ala Ser Pro Leu Pro Arg Pro Pro Ala Ala Met Thr
545                 550                 555                 560

cgt agc tac tcc ata cct tca aac agt caa cgg act cct att att acc      1728
Arg Ser Tyr Ser Ile Pro Ser Asn Ser Gln Arg Thr Pro Ile Ile Thr
                565                 570                 575

gtg aac aaa ttg ttg gag gct agg cat agc agg gag agt agc gaa gtt      1776
Val Asn Lys Leu Leu Glu Ala Arg His Ser Arg Glu Ser Ser Glu Val
            580                 585                 590

tcc tcc ccc cca gta aca cct ata tca ctg gca gac gtt tcc cgc aga      1824
Ser Ser Pro Pro Val Thr Pro Ile Ser Leu Ala Asp Val Ser Arg Arg
        595                 600                 605

cca aca aca gaa gca gct gtt tga                                      1848
Pro Thr Thr Glu Ala Ala Val
    610                 615


<210>  48
<211>  615
<212>  PRT
<213>  Zea mays

<400>  48

Met Lys Ser Pro Leu Arg Arg Phe Arg Gly Phe Ala Leu His His His
1               5                   10                  15


His His His Arg Glu Arg Lys Asp His Arg Pro Pro Ser Ala Lys Leu
            20                  25                  30


Asp Glu Leu Val Tyr Ala Ala Gln Glu Met Glu Asp Met Arg Asn Cys
        35                  40                  45


Tyr Asp Ser Leu Leu Ser Ala Ala Ala Ala Thr Thr Asn Ser Val Tyr
    50                  55                  60


Glu Phe Ala Glu Ala Met Gly Glu Met Gly Thr Cys Leu Leu Glu Lys
65                  70                  75                  80


Ala Ala Leu Asn Tyr Asp Asp Asp Glu Ser Gly Lys Val Leu Met Lys
                85                  90                  95


Leu Gly Lys Ala Gln Phe Glu Leu Gln Lys Phe Val Asp Asp Tyr Arg
            100                 105                 110
```

```
Thr Asn Ile Ile Asn Thr Ile Thr Asn Pro Ser Glu Ser Leu Leu Lys
        115                 120                 125

Glu Leu Gln Val Val Glu Glu Met Lys Asp Gln Cys Asp Gln Lys Arg
    130                 135                 140

Val Glu Tyr Glu Ala Met Arg Ala Ala Tyr Gly Glu Lys Gly Gly Arg
145                 150                 155                 160

Leu Arg His Leu Lys Asn Glu Ser Phe Ser Leu Gly Gln Leu Gln Thr
                165                 170                 175

Ser Phe Leu Glu Tyr Gln Glu Glu Ala Ala Leu Phe Ile Phe Arg Leu
            180                 185                 190

Lys Ser Leu Lys Gln Gly Gln Phe Leu Ser Ile Leu Thr Gln Ala Ala
        195                 200                 205

Arg His His Ala Ser Gln Leu Ser Phe Phe Arg Lys Gly Leu Lys His
    210                 215                 220

Leu Glu Ala Leu Glu Pro Cys Val Lys Ala Val Ala Glu Lys Gln His
225                 230                 235                 240

Ile Asp Tyr His Phe Ser Gly Leu Asp Asn Asn Ser Asp Ile Asp Asp
                245                 250                 255

Tyr Ser Ser Tyr Gln Asp Asn His Ser Asp Gly Ser Glu Leu Ser Phe
            260                 265                 270

Asn Tyr Glu Ile Asn Asp Arg Asp Lys Asp Leu Pro Thr Phe Arg Ser
        275                 280                 285

Pro Met Asp Met Asp Gln Ala His Pro Val Ser Ser Pro Arg Pro Leu
    290                 295                 300

Lys Glu Gln Glu Gln Glu Asn Ala Glu Glu Ile Lys Ala Thr Leu Gly
305                 310                 315                 320

Val Pro His Met Lys Pro Glu Ile Gly Thr Gln Ser Ala Pro Ile Phe
                325                 330                 335

Ala Glu Asn Val Pro Asp Pro Ser Met Arg Phe Arg Lys Met Asn Leu
            340                 345                 350

Leu Thr Arg Thr Val His Ser Tyr Lys Leu Pro Thr Pro Ala Asp Asp
        355                 360                 365
```

```
Lys Asn Pro Ala Ser Val Ile Ala Asn Lys Ser Pro His Ser Asp Gln
    370                 375                 380

Pro Glu Ser Lys Ser His Val Ala Val Asn Leu Trp His Ser Ser Pro
385                 390                 395                 400

Leu Ala Lys Asp Phe Met Pro Asn Ser Met His Ser Gly Pro Val Lys
                405                 410                 415

Met Pro Ser Ser Asn Glu Gly Val Ser Leu Ala Tyr Ser Tyr Ser Thr
            420                 425                 430

Thr Asp Phe Lys Lys Val Lys Arg Glu Ala Phe Ser Gly Pro Ile Pro
        435                 440                 445

Ser Lys Val Gly Leu Ser Thr Pro Leu Ile Ser Ala Thr Asp Arg Lys
    450                 455                 460

Pro Ser Gly Lys Arg Pro Ser Tyr Val Leu Pro Thr Arg Pro Pro Gly
465                 470                 475                 480

Pro Gly Trp Gln Ser Ser Val Pro Pro Lys Ala Thr Pro Arg Val Thr
                485                 490                 495

Ser Leu Pro Thr Thr Thr Pro Arg Ile Ser Glu Leu His Glu Leu Pro
            500                 505                 510

Arg Pro Pro Ala Asn Ala Gly Leu Val Gly Tyr Ser Gly Pro Leu Val
        515                 520                 525

Ser Arg Arg Pro Met Pro Thr Ala Val Ser Thr Thr Arg Val Ser Pro
    530                 535                 540

Pro Ser His Thr Ala Ser Pro Leu Pro Arg Pro Pro Ala Ala Met Thr
545                 550                 555                 560

Arg Ser Tyr Ser Ile Pro Ser Asn Ser Gln Arg Thr Pro Ile Ile Thr
                565                 570                 575

Val Asn Lys Leu Leu Glu Ala Arg His Ser Arg Glu Ser Ser Glu Val
            580                 585                 590

Ser Ser Pro Pro Val Thr Pro Ile Ser Leu Ala Asp Val Ser Arg Arg
        595                 600                 605

Pro Thr Thr Glu Ala Ala Val
    610                 615

<210>  49
<211>  2982
```

```
<212>  DNA
<213>  P. patens

<220>
<221>  CDS
<222>  (1)..(2982)

<400>  49
atg tcg gtg gag ttt gaa gag gac gga gta gac agg gcg ttg ggg tgc      48
Met Ser Val Glu Phe Glu Glu Asp Gly Val Asp Arg Ala Leu Gly Cys
1               5                   10                  15

tcc ctt gtt cat ctt ggc ctg cgt tgg tgg atg tgt aat ggc act gcg      96
Ser Leu Val His Leu Gly Leu Arg Trp Trp Met Cys Asn Gly Thr Ala
            20                  25                  30

ttg gag aat gca ggg ttt acc gca gta cat gtt tcg cag cgg att gga     144
Leu Glu Asn Ala Gly Phe Thr Ala Val His Val Ser Gln Arg Ile Gly
        35                  40                  45

cgg ggt ggt caa tct tcc gga agt gca atg ttg ggg cgc gat tgg gga     192
Arg Gly Gly Gln Ser Ser Gly Ser Ala Met Leu Gly Arg Asp Trp Gly
    50                  55                  60

ggg atg ttg aat ggt ggt tcg gtc gtt ctg ctg gat ctt aga agt act     240
Gly Met Leu Asn Gly Gly Ser Val Val Leu Leu Asp Leu Arg Ser Thr
65                  70                  75                  80

tcc cga cta gtt act ggc tta gcc aac ctc gac gcc atc gtt act tgc     288
Ser Arg Leu Val Thr Gly Leu Ala Asn Leu Asp Ala Ile Val Thr Cys
                85                  90                  95

ggt ttc tgt gat cca cat caa agg tgg att ctc gct cgt ata gtt caa     336
Gly Phe Cys Asp Pro His Gln Arg Trp Ile Leu Ala Arg Ile Val Gln
            100                 105                 110

aga aca gcg ttt atc att gca ggc tta gag tct tgc ttt ctg cca ggt     384
Arg Thr Ala Phe Ile Ile Ala Gly Leu Glu Ser Cys Phe Leu Pro Gly
        115                 120                 125

ttg aca tca cag tgc ctt ggg gcg acg tca aaa gaa agc ttc tct tca     432
Leu Thr Ser Gln Cys Leu Gly Ala Thr Ser Lys Glu Ser Phe Ser Ser
    130                 135                 140

cgt ctg cat cac tct tcc tgg ttt tcc ata ttc ttc tct gat gga acg     480
Arg Leu His His Ser Ser Trp Phe Ser Ile Phe Phe Ser Asp Gly Thr
145                 150                 155                 160

agc tgt cga gct gcc aca tta ttt tta aca act ctt atc atc gta aca     528
Ser Cys Arg Ala Ala Thr Leu Phe Leu Thr Thr Leu Ile Ile Val Thr
                165                 170                 175

gaa ttt ttt tta ccg tca gca att cct gaa aga gcc ttg tgg agg aat     576
Glu Phe Phe Leu Pro Ser Ala Ile Pro Glu Arg Ala Leu Trp Arg Asn
            180                 185                 190

aga gag aaa atg aag tcg ttc aag aag ctg cgg gac tta gcg cgg cag     624
Arg Glu Lys Met Lys Ser Phe Lys Lys Leu Arg Asp Leu Ala Arg Gln
        195                 200                 205

gga ggc aaa gct aaa gac aag cac aat tat gga aac gat aat aac cgt     672
Gly Gly Lys Ala Lys Asp Lys His Asn Tyr Gly Asn Asp Asn Asn Arg
    210                 215                 220

cga tat gag gcg cat gga gtg ggc tac gtg gag ccg act gag gat gcg     720
```

Arg Tyr Glu Ala His Gly Val Gly Tyr Val Glu Pro Thr Glu Asp Ala
225                 230                 235                 240

gaa ctc gtt acc aat gga atg aag gat att aga agc ata cag aag aaa 768
Glu Leu Val Thr Asn Gly Met Lys Asp Ile Arg Ser Ile Gln Lys Lys
                245                 250                 255

tat gag tcg ctt gtc agc ttg tca acc gaa gtt tcc cat cga gct tac 816
Tyr Glu Ser Leu Val Ser Leu Ser Thr Glu Val Ser His Arg Ala Tyr
            260                 265                 270

gac tta tcc aca gct gta tca gat atg gcc tca tac ttt gtg gct cct 864
Asp Leu Ser Thr Ala Val Ser Asp Met Ala Ser Tyr Phe Val Ala Pro
        275                 280                 285

ggt gtc ttg gac gat caa gat att gtg tgt gta ttg tac att ttc ttt 912
Gly Val Leu Asp Asp Gln Asp Ile Val Cys Val Leu Tyr Ile Phe Phe
    290                 295                 300

ggt aat tgc cac gac aaa tat tgg ttt gct ata gtt gat tta gtt ttt 960
Gly Asn Cys His Asp Lys Tyr Trp Phe Ala Ile Val Asp Leu Val Phe
305                 310                 315                 320

aag gaa acc aaa aag cag tac gat gag aga aga cag tcc tta tat cat 1008
Lys Glu Thr Lys Lys Gln Tyr Asp Glu Arg Arg Gln Ser Leu Tyr His
                325                 330                 335

cat cgg cta cgc ata gca aaa gga aga tct aaa att gga aag acc gat 1056
His Arg Leu Arg Ile Ala Lys Gly Arg Ser Lys Ile Gly Lys Thr Asp
            340                 345                 350

gct cag gag gaa gag cag cta gaa aat gtt aga gaa caa ttt gaa gaa 1104
Ala Gln Glu Glu Glu Gln Leu Glu Asn Val Arg Glu Gln Phe Glu Glu
        355                 360                 365

gtc agc cag ttt tta gga gat cgg cta ctc tcg tta cgc caa ggt cgg 1152
Val Ser Gln Phe Leu Gly Asp Arg Leu Leu Ser Leu Arg Gln Gly Arg
    370                 375                 380

cct cga agc ctt ata act caa act gcc cgc cat cat gct gcc cag atg 1200
Pro Arg Ser Leu Ile Thr Gln Thr Ala Arg His His Ala Ala Gln Met
385                 390                 395                 400

caa ttg ttc agc aaa gta ctt aca tca ctt cat ggc att gag cct cat 1248
Gln Leu Phe Ser Lys Val Leu Thr Ser Leu His Gly Ile Glu Pro His
                405                 410                 415

atg aag caa gtc act aaa gag ttg aac att gac cga cat cta agt tcc 1296
Met Lys Gln Val Thr Lys Glu Leu Asn Ile Asp Arg His Leu Ser Ser
            420                 425                 430

gct gac gga gat gta ttt aat gat gag att gag gat gat gaa gac gtg 1344
Ala Asp Gly Asp Val Phe Asn Asp Glu Ile Glu Asp Asp Glu Asp Val
        435                 440                 445

tca gac att gag cat cat ctc gat gat gct ggg tcc tat ttt gac gac 1392
Ser Asp Ile Glu His His Leu Asp Asp Ala Gly Ser Tyr Phe Asp Asp
    450                 455                 460

gcc gac gaa gag aag gaa cat cac cat gta aga gaa gat gat ttg ggg 1440
Ala Asp Glu Glu Lys Glu His His His Val Arg Glu Asp Asp Leu Gly
465                 470                 475                 480

tcg gaa agc tct gaa att gaa gct aat tca cga ttg aac atg tcc cgg 1488
Ser Glu Ser Ser Glu Ile Glu Ala Asn Ser Arg Leu Asn Met Ser Arg
                485                 490                 495

```
gaa tgg agc tct cac atg gca tca gga agt gat agg cct gga aat tcg      1536
Glu Trp Ser Ser His Met Ala Ser Gly Ser Asp Arg Pro Gly Asn Ser
            500                 505                 510

gag aac gag ggg gat tcc ccg ttg cct cga tgg ata atc aaa ggg agt      1584
Glu Asn Glu Gly Asp Ser Pro Leu Pro Arg Trp Ile Ile Lys Gly Ser
        515                 520                 525

aag tca gca ccc tca tca cca ctt act gcc tct caa ggt gtg gat gat      1632
Lys Ser Ala Pro Ser Ser Pro Leu Thr Ala Ser Gln Gly Val Asp Asp
    530                 535                 540

gtt gac agg gct cct ccc aca tat gtc tct gct gtt cta ccc ccc acc      1680
Val Asp Arg Ala Pro Pro Thr Tyr Val Ser Ala Val Leu Pro Pro Thr
545                 550                 555                 560

aga tcg cca cca cat gtt tct gat aga ggt cac tcg ggg gtg ttg tat      1728
Arg Ser Pro Pro His Val Ser Asp Arg Gly His Ser Gly Val Leu Tyr
                565                 570                 575

agt gac acg cgt cct tat gaa cac aaa ccc tca acc tct gaa cag act      1776
Ser Asp Thr Arg Pro Tyr Glu His Lys Pro Ser Thr Ser Glu Gln Thr
            580                 585                 590

tct cct cgt cat cac aac ggt aca ccc ggg caa acg cca cgg ttt cag      1824
Ser Pro Arg His His Asn Gly Thr Pro Gly Gln Thr Pro Arg Phe Gln
        595                 600                 605

ggg cga gca aca aag atg ggt gtt cca atg tcg atg ttt ccg cca ttc      1872
Gly Arg Ala Thr Lys Met Gly Val Pro Met Ser Met Phe Pro Pro Phe
    610                 615                 620

gat cct tca cag acc act tgg cct atg cct tta cct cca cct agt gac      1920
Asp Pro Ser Gln Thr Thr Trp Pro Met Pro Leu Pro Pro Pro Ser Asp
625                 630                 635                 640

ccg cct ccc ata ttg aaa ttc cca gct cca act gcc gga ggc ttt att      1968
Pro Pro Pro Ile Leu Lys Phe Pro Ala Pro Thr Ala Gly Gly Phe Ile
                645                 650                 655

gcg gca aat tct ggt gct cct ggt aat aag cac aag aga tac agt tat      2016
Ala Ala Asn Ser Gly Ala Pro Gly Asn Lys His Lys Arg Tyr Ser Tyr
            660                 665                 670

tca ggt cct ctt act tct tcc tca aaa ccg agg gat aga gca tat tct      2064
Ser Gly Pro Leu Thr Ser Ser Ser Lys Pro Arg Asp Arg Ala Tyr Ser
        675                 680                 685

tca gat gtt cca agt agt ggg cca ctg cgg cca ttt cct tct tca aca      2112
Ser Asp Val Pro Ser Ser Gly Pro Leu Arg Pro Phe Pro Ser Ser Thr
    690                 695                 700

act tgg tct gat cag tct agg tca cct aaa ctt tct cca tcc att tcc      2160
Thr Trp Ser Asp Gln Ser Arg Ser Pro Lys Leu Ser Pro Ser Ile Ser
705                 710                 715                 720

cct tct aaa gta tct cca cct caa att agt gag tta cac aaa ctc cct      2208
Pro Ser Lys Val Ser Pro Pro Gln Ile Ser Glu Leu His Lys Leu Pro
                725                 730                 735

cca cct ccc ctt ggt tct agc tcg tcc cct ata gca gca tcg tca agt      2256
Pro Pro Pro Leu Gly Ser Ser Ser Ser Pro Ile Ala Ala Ser Ser Ser
            740                 745                 750

ttg atc gcg cac tca gcg cct ttg cat aga cat gct gat agg cat gcc      2304
```

```
Leu Ile Ala His Ser Ala Pro Leu His Arg His Ala Asp Arg His Ala
        755                 760                 765

tct ccc ctt cct cct cct cct ctt ggt aat gca tct cag aac ctt tcc      2352
Ser Pro Leu Pro Pro Pro Pro Leu Gly Asn Ala Ser Gln Asn Leu Ser
    770                 775                 780

gtc gga gga gcc ggt aag ttg caa aat ata cag cga tac aag ggg ttg      2400
Val Gly Gly Ala Gly Lys Leu Gln Asn Ile Gln Arg Tyr Lys Gly Leu
785                 790                 795                 800

gca atc tct gag gat gaa gag aga gct att cca tct gta cag ccc gta      2448
Ala Ile Ser Glu Asp Glu Glu Arg Ala Ile Pro Ser Val Gln Pro Val
                805                 810                 815

cga act tcg cac agt ggg cat agt gga ctg cta act acc cct agc aat      2496
Arg Thr Ser His Ser Gly His Ser Gly Leu Leu Thr Thr Pro Ser Asn
            820                 825                 830

ccg cag att ggt cac aag agt ccc tcg cta caa agg agt gta tcc ggc      2544
Pro Gln Ile Gly His Lys Ser Pro Ser Leu Gln Arg Ser Val Ser Gly
        835                 840                 845

tgt ggg gtc tcc gcg gct cct aga cat gct aca ctc cac ttg aca gct      2592
Cys Gly Val Ser Ala Ala Pro Arg His Ala Thr Leu His Leu Thr Ala
    850                 855                 860

cct ggt aga gtg caa tct gga tca ggt gac aaa aag atg tgg aga ctc      2640
Pro Gly Arg Val Gln Ser Gly Ser Gly Asp Lys Lys Met Trp Arg Leu
865                 870                 875                 880

aac act acg att cac tct tct cca agt ttg tct tta aaa tat tcc gtg      2688
Asn Thr Thr Ile His Ser Ser Pro Ser Leu Ser Leu Lys Tyr Ser Val
                885                 890                 895

gag tca cct cca tct atc gat tct ttt gaa gga aag gaa aga cct cgg      2736
Glu Ser Pro Pro Ser Ile Asp Ser Phe Glu Gly Lys Glu Arg Pro Arg
            900                 905                 910

ttc gcc tcc agc gac ttt gaa aca gct cgt cga tct ttg gac atg gca      2784
Phe Ala Ser Ser Asp Phe Glu Thr Ala Arg Arg Ser Leu Asp Met Ala
        915                 920                 925

tta aca agc ggc acc agc aac aac act aaa cag tcg aag tgg tcg aaa      2832
Leu Thr Ser Gly Thr Ser Asn Asn Thr Lys Gln Ser Lys Trp Ser Lys
    930                 935                 940

tgg aga tcc ttt tca gcc cat gat gga gag gac ttg tcg atg atg tcc      2880
Trp Arg Ser Phe Ser Ala His Asp Gly Glu Asp Leu Ser Met Met Ser
945                 950                 955                 960

cag ttg gga acc aaa cat cat cat cga gat agc ttg caa gcg cat ggt      2928
Gln Leu Gly Thr Lys His His His Arg Asp Ser Leu Gln Ala His Gly
                965                 970                 975

tac cac gct agc ccc agg ctt ggt acc aca acc tat ggc gtt cga cat      2976
Tyr His Ala Ser Pro Arg Leu Gly Thr Thr Thr Tyr Gly Val Arg His
            980                 985                 990

gtt tga                                                              2982
Val
```

<210>  50
<211>  993

<212> PRT
<213> P. patens

<400> 50

Met Ser Val Glu Phe Glu Glu Asp Gly Val Asp Arg Ala Leu Gly Cys
1 5 10 15

Ser Leu Val His Leu Gly Leu Arg Trp Trp Met Cys Asn Gly Thr Ala
20 25 30

Leu Glu Asn Ala Gly Phe Thr Ala Val His Val Ser Gln Arg Ile Gly
35 40 45

Arg Gly Gly Gln Ser Ser Gly Ser Ala Met Leu Gly Arg Asp Trp Gly
50 55 60

Gly Met Leu Asn Gly Gly Ser Val Val Leu Leu Asp Leu Arg Ser Thr
65 70 75 80

Ser Arg Leu Val Thr Gly Leu Ala Asn Leu Asp Ala Ile Val Thr Cys
85 90 95

Gly Phe Cys Asp Pro His Gln Arg Trp Ile Leu Ala Arg Ile Val Gln
100 105 110

Arg Thr Ala Phe Ile Ile Ala Gly Leu Glu Ser Cys Phe Leu Pro Gly
115 120 125

Leu Thr Ser Gln Cys Leu Gly Ala Thr Ser Lys Glu Ser Phe Ser Ser
130 135 140

Arg Leu His His Ser Ser Trp Phe Ser Ile Phe Phe Ser Asp Gly Thr
145 150 155 160

Ser Cys Arg Ala Ala Thr Leu Phe Leu Thr Thr Leu Ile Ile Val Thr
165 170 175

Glu Phe Phe Leu Pro Ser Ala Ile Pro Glu Arg Ala Leu Trp Arg Asn
180 185 190

Arg Glu Lys Met Lys Ser Phe Lys Lys Leu Arg Asp Leu Ala Arg Gln
195 200 205

Gly Gly Lys Ala Lys Asp Lys His Asn Tyr Gly Asn Asp Asn Asn Arg
210 215 220

Arg Tyr Glu Ala His Gly Val Gly Tyr Val Glu Pro Thr Glu Asp Ala
225 230 235 240

Glu Leu Val Thr Asn Gly Met Lys Asp Ile Arg Ser Ile Gln Lys Lys

245 250 255

Tyr Glu Ser Leu Val Ser Leu Ser Thr Glu Val Ser His Arg Ala Tyr
260 265 270

Asp Leu Ser Thr Ala Val Ser Asp Met Ala Ser Tyr Phe Val Ala Pro
275 280 285

Gly Val Leu Asp Asp Gln Asp Ile Val Cys Val Leu Tyr Ile Phe Phe
290 295 300

Gly Asn Cys His Asp Lys Tyr Trp Phe Ala Ile Val Asp Leu Val Phe
305 310 315 320

Lys Glu Thr Lys Lys Gln Tyr Asp Glu Arg Arg Gln Ser Leu Tyr His
325 330 335

His Arg Leu Arg Ile Ala Lys Gly Arg Ser Lys Ile Gly Lys Thr Asp
340 345 350

Ala Gln Glu Glu Glu Gln Leu Glu Asn Val Arg Glu Gln Phe Glu Glu
355 360 365

Val Ser Gln Phe Leu Gly Asp Arg Leu Leu Ser Leu Arg Gln Gly Arg
370 375 380

Pro Arg Ser Leu Ile Thr Gln Thr Ala Arg His His Ala Ala Gln Met
385 390 395 400

Gln Leu Phe Ser Lys Val Leu Thr Ser Leu His Gly Ile Glu Pro His
405 410 415

Met Lys Gln Val Thr Lys Glu Leu Asn Ile Asp Arg His Leu Ser Ser
420 425 430

Ala Asp Gly Asp Val Phe Asn Asp Glu Ile Glu Asp Asp Glu Asp Val
435 440 445

Ser Asp Ile Glu His His Leu Asp Asp Ala Gly Ser Tyr Phe Asp Asp
450 455 460

Ala Asp Glu Glu Lys Glu His His His Val Arg Glu Asp Asp Leu Gly
465 470 475 480

Ser Glu Ser Ser Glu Ile Glu Ala Asn Ser Arg Leu Asn Met Ser Arg
485 490 495

Glu Trp Ser Ser His Met Ala Ser Gly Ser Asp Arg Pro Gly Asn Ser
500 505 510

```
Glu Asn Glu Gly Asp Ser Pro Leu Pro Arg Trp Ile Ile Lys Gly Ser
        515                 520                 525

Lys Ser Ala Pro Ser Ser Pro Leu Thr Ala Ser Gln Gly Val Asp Asp
    530                 535                 540

Val Asp Arg Ala Pro Pro Thr Tyr Val Ser Ala Val Leu Pro Pro Thr
545                 550                 555                 560

Arg Ser Pro Pro His Val Ser Asp Arg Gly His Ser Gly Val Leu Tyr
                565                 570                 575

Ser Asp Thr Arg Pro Tyr Glu His Lys Pro Ser Thr Ser Glu Gln Thr
            580                 585                 590

Ser Pro Arg His His Asn Gly Thr Pro Gly Gln Thr Pro Arg Phe Gln
        595                 600                 605

Gly Arg Ala Thr Lys Met Gly Val Pro Met Ser Met Phe Pro Pro Phe
    610                 615                 620

Asp Pro Ser Gln Thr Thr Trp Pro Met Pro Leu Pro Pro Pro Ser Asp
625                 630                 635                 640

Pro Pro Pro Ile Leu Lys Phe Pro Ala Pro Thr Ala Gly Gly Phe Ile
                645                 650                 655

Ala Ala Asn Ser Gly Ala Pro Gly Asn Lys His Lys Arg Tyr Ser Tyr
            660                 665                 670

Ser Gly Pro Leu Thr Ser Ser Ser Lys Pro Arg Asp Arg Ala Tyr Ser
        675                 680                 685

Ser Asp Val Pro Ser Ser Gly Pro Leu Arg Pro Phe Pro Ser Ser Thr
    690                 695                 700

Thr Trp Ser Asp Gln Ser Arg Ser Pro Lys Leu Ser Pro Ser Ile Ser
705                 710                 715                 720

Pro Ser Lys Val Ser Pro Pro Gln Ile Ser Glu Leu His Lys Leu Pro
                725                 730                 735

Pro Pro Pro Leu Gly Ser Ser Ser Ser Pro Ile Ala Ala Ser Ser Ser
            740                 745                 750

Leu Ile Ala His Ser Ala Pro Leu His Arg His Ala Asp Arg His Ala
        755                 760                 765

Ser Pro Leu Pro Pro Pro Pro Leu Gly Asn Ala Ser Gln Asn Leu Ser
```

```
    770                 775                 780

Val Gly Gly Ala Gly Lys Leu Gln Asn Ile Gln Arg Tyr Lys Gly Leu
785                 790                 795                 800

Ala Ile Ser Glu Asp Glu Glu Arg Ala Ile Pro Ser Val Gln Pro Val
                805                 810                 815

Arg Thr Ser His Ser Gly His Ser Gly Leu Leu Thr Thr Pro Ser Asn
            820                 825                 830

Pro Gln Ile Gly His Lys Ser Pro Ser Leu Gln Arg Ser Val Ser Gly
        835                 840                 845

Cys Gly Val Ser Ala Ala Pro Arg His Ala Thr Leu His Leu Thr Ala
    850                 855                 860

Pro Gly Arg Val Gln Ser Gly Ser Gly Asp Lys Lys Met Trp Arg Leu
865                 870                 875                 880

Asn Thr Thr Ile His Ser Ser Pro Ser Leu Ser Leu Lys Tyr Ser Val
                885                 890                 895

Glu Ser Pro Pro Ser Ile Asp Ser Phe Glu Gly Lys Glu Arg Pro Arg
            900                 905                 910

Phe Ala Ser Ser Asp Phe Glu Thr Ala Arg Arg Ser Leu Asp Met Ala
        915                 920                 925

Leu Thr Ser Gly Thr Ser Asn Asn Thr Lys Gln Ser Lys Trp Ser Lys
    930                 935                 940

Trp Arg Ser Phe Ser Ala His Asp Gly Glu Asp Leu Ser Met Met Ser
945                 950                 955                 960

Gln Leu Gly Thr Lys His His His Arg Asp Ser Leu Gln Ala His Gly
                965                 970                 975

Tyr His Ala Ser Pro Arg Leu Gly Thr Thr Thr Tyr Gly Val Arg His
            980                 985                 990

Val


<210>  51
<211>  1827
<212>  DNA
<213>  P.trichocarpa

<220>
```

<221> CDS
<222> (1)..(1827)

<400> 51

```
atg aag tcc aaa ctg aga gga ttt aga ctc aga aga agc gaa ccg aag      48
Met Lys Ser Lys Leu Arg Gly Phe Arg Leu Arg Arg Ser Glu Pro Lys
1               5                   10                  15

gac aaa att gac ttc tta cct cct gct cag tta gat gag ctc gct caa      96
Asp Lys Ile Asp Phe Leu Pro Pro Ala Gln Leu Asp Glu Leu Ala Gln
            20                  25                  30

gct gct cag aat tct cag agt cat tgc ggg aaa tgg gtt cat gtc tgt     144
Ala Ala Gln Asn Ser Gln Ser His Cys Gly Lys Trp Val His Val Cys
        35                  40                  45

cgg aga aaa cag cat tac atg atg atg aag gaa gtg cgg tct cat ata     192
Arg Arg Lys Gln His Tyr Met Met Met Lys Glu Val Arg Ser His Ile
    50                  55                  60

ttc ctg acg att aca aat cca tca gaa tcc ttg ctc aat gaa ctt cgg     240
Phe Leu Thr Ile Thr Asn Pro Ser Glu Ser Leu Leu Asn Glu Leu Arg
65                  70                  75                  80

aca gtt gag gat atg aag cga caa tgt gat gaa aaa aga aat gtc tat     288
Thr Val Glu Asp Met Lys Arg Gln Cys Asp Glu Lys Arg Asn Val Tyr
                85                  90                  95

gaa tac atg gtg gca caa cag aag gac aaa gga agg tca aaa ggt ggg     336
Glu Tyr Met Val Ala Gln Gln Lys Asp Lys Gly Arg Ser Lys Gly Gly
            100                 105                 110

aag gat gaa agt att act ttg cag caa ttg caa aca gct cgt gaa gaa     384
Lys Asp Glu Ser Ile Thr Leu Gln Gln Leu Gln Thr Ala Arg Glu Glu
        115                 120                 125

tat gac gaa gag gca acc ttg tgt gtt ttt cgg ttg aaa tct ctg aaa     432
Tyr Asp Glu Glu Ala Thr Leu Cys Val Phe Arg Leu Lys Ser Leu Lys
    130                 135                 140

cag gga cag tct cga agt ctt cta aca cag gca gct cgt cat cac gct     480
Gln Gly Gln Ser Arg Ser Leu Leu Thr Gln Ala Ala Arg His His Ala
145                 150                 155                 160

gct cag aag gga ctt aaa tca ctt gag aca gtt gag cca cat gtt aga     528
Ala Gln Lys Gly Leu Lys Ser Leu Glu Thr Val Glu Pro His Val Arg
                165                 170                 175

ctg gtt aca gaa cat caa cac att gac tac cat ttc agt ggg ctt gaa     576
Leu Val Thr Glu His Gln His Ile Asp Tyr His Phe Ser Gly Leu Glu
            180                 185                 190

agt gat ggt aga gaa gac ggc gag gat gat ggc gag gat ggc ggt gat     624
Ser Asp Gly Arg Glu Asp Gly Glu Asp Asp Gly Glu Asp Gly Gly Asp
        195                 200                 205

aca aat gaa cgc agg gaa ctg agt ttt gat tac aga gaa aat aat cag     672
Thr Asn Glu Arg Arg Glu Leu Ser Phe Asp Tyr Arg Glu Asn Asn Gln
    210                 215                 220

ggg aat gct gtt gtt tct gca gca agg ggt tct atg gag gtg gat gaa     720
Gly Asn Ala Val Val Ser Ala Ala Arg Gly Ser Met Glu Val Asp Glu
225                 230                 235                 240

gag gac ctt tca ttc caa gtt cca gca gca gaa aat gta gag tta aac     768
Glu Asp Leu Ser Phe Gln Val Pro Ala Ala Glu Asn Val Glu Leu Asn
```

```
                245                 250                 255

cca gac aaa aac cat ggt ggt ttc cag ttc cca agc aga gaa cca aga       816
Pro Asp Lys Asn His Gly Gly Phe Gln Phe Pro Ser Arg Glu Pro Arg
            260                 265                 270

gga ggc agc cat tca gca cca ata gtt cca gaa aga aaa cct gat cca       864
Gly Gly Ser His Ser Ala Pro Ile Val Pro Glu Arg Lys Pro Asp Pro
        275                 280                 285

gtt gaa aga ata aga caa atg cag caa gca tca agg aag tcc aac aca       912
Val Glu Arg Ile Arg Gln Met Gln Gln Ala Ser Arg Lys Ser Asn Thr
    290                 295                 300

tat gtg ctg ccc aca cct att gat gca aag ggt gca atc tct tca aga       960
Tyr Val Leu Pro Thr Pro Ile Asp Ala Lys Gly Ala Ile Ser Ser Arg
305                 310                 315                 320

aca agt tgt tca gtt ccc aac aca agg caa aca gac atc agt gga cgc      1008
Thr Ser Cys Ser Val Pro Asn Thr Arg Gln Thr Asp Ile Ser Gly Arg
                325                 330                 335

gct cac aat ttg tgg cat tct tcc cca ttg gaa cag aag aag aat gag      1056
Ala His Asn Leu Trp His Ser Ser Pro Leu Glu Gln Lys Lys Asn Glu
            340                 345                 350

aaa gat tct ggt gat ggt cac ctg tca gat ttc act gct tta aaa gca      1104
Lys Asp Ser Gly Asp Gly His Leu Ser Asp Phe Thr Ala Leu Lys Ala
        355                 360                 365

cgc tca gga cat aaa gag agc aac aat cct aat gcc tcc acc caa tta      1152
Arg Ser Gly His Lys Glu Ser Asn Asn Pro Asn Ala Ser Thr Gln Leu
    370                 375                 380

ccc cca cct tta gtt ggt gga atc tca tat ccg cag ctt gat gta cac      1200
Pro Pro Pro Leu Val Gly Gly Ile Ser Tyr Pro Gln Leu Asp Val His
385                 390                 395                 400

aat gca tcc gat tac aaa aaa aat aaa tgg caa tcc ttc tct ggt cca      1248
Asn Ala Ser Asp Tyr Lys Lys Asn Lys Trp Gln Ser Phe Ser Gly Pro
                405                 410                 415

ata acc agt aag cca tgg tca atg aag cct tta tca tcc agt ggt ccc      1296
Ile Thr Ser Lys Pro Trp Ser Met Lys Pro Leu Ser Ser Ser Gly Pro
            420                 425                 430

att tcc tca act gaa ctt tca caa caa gtt tct gga atg cta tct cgt      1344
Ile Ser Ser Thr Glu Leu Ser Gln Gln Val Ser Gly Met Leu Ser Arg
        435                 440                 445

ggg gca aat cct caa cct tct tca tcc ccc aaa gta tcc cca agt aca      1392
Gly Ala Asn Pro Gln Pro Ser Ser Ser Pro Lys Val Ser Pro Ser Thr
    450                 455                 460

tca cct cct ctt gtt tct tca ccc aag ata agt gag ctt cat gaa ctt      1440
Ser Pro Pro Leu Val Ser Ser Pro Lys Ile Ser Glu Leu His Glu Leu
465                 470                 475                 480

cca agg cca cca ggt aat tta gct gcc aaa gct gca aaa cct tca gtg      1488
Pro Arg Pro Pro Gly Asn Leu Ala Ala Lys Ala Ala Lys Pro Ser Val
                485                 490                 495

ctg att gga cac tct gct cca ttg tcg aga aat ccc gag ctt gct gga      1536
Leu Ile Gly His Ser Ala Pro Leu Ser Arg Asn Pro Glu Leu Ala Gly
            500                 505                 510
```

```
aca agc aag att tct aca ggg gca gca aat ttg gca tca cct ctt cct      1584
Thr Ser Lys Ile Ser Thr Gly Ala Ala Asn Leu Ala Ser Pro Leu Pro
        515                 520                 525

ccc cca ccc ttg ata gtc cca cgt agt ttc tca ata ccc tcg agt agt      1632
Pro Pro Pro Leu Ile Val Pro Arg Ser Phe Ser Ile Pro Ser Ser Ser
    530                 535                 540

cag aga gcc atg aca gtg cac gtt tct aag ctt ttg gat tct tct caa      1680
Gln Arg Ala Met Thr Val His Val Ser Lys Leu Leu Asp Ser Ser Gln
545                 550                 555                 560

gta tca tat aaa cct ggg gaa gtc gat tct cct cca tta aca ccc atg      1728
Val Ser Tyr Lys Pro Gly Glu Val Asp Ser Pro Pro Leu Thr Pro Met
                565                 570                 575

tcc ctc gca aat atg agg cca gca cct gcc att tct gaa cca gta ccg      1776
Ser Leu Ala Asn Met Arg Pro Ala Pro Ala Ile Ser Glu Pro Val Pro
            580                 585                 590

cat tct ggt caa att aga ggt aac aag aca tca tgt tca tta att ttc      1824
His Ser Gly Gln Ile Arg Gly Asn Lys Thr Ser Cys Ser Leu Ile Phe
        595                 600                 605

tga                                                                  1827


<210>  52
<211>  608
<212>  PRT
<213>  P.trichocarpa

<400>  52

Met Lys Ser Lys Leu Arg Gly Phe Arg Leu Arg Arg Ser Glu Pro Lys
1               5                   10                  15


Asp Lys Ile Asp Phe Leu Pro Pro Ala Gln Leu Asp Glu Leu Ala Gln
            20                  25                  30


Ala Ala Gln Asn Ser Gln Ser His Cys Gly Lys Trp Val His Val Cys
        35                  40                  45


Arg Arg Lys Gln His Tyr Met Met Met Lys Glu Val Arg Ser His Ile
    50                  55                  60


Phe Leu Thr Ile Thr Asn Pro Ser Glu Ser Leu Leu Asn Glu Leu Arg
65                  70                  75                  80


Thr Val Glu Asp Met Lys Arg Gln Cys Asp Glu Lys Arg Asn Val Tyr
                85                  90                  95


Glu Tyr Met Val Ala Gln Gln Lys Asp Lys Gly Arg Ser Lys Gly Gly
            100                 105                 110


Lys Asp Glu Ser Ile Thr Leu Gln Gln Leu Gln Thr Ala Arg Glu Glu
        115                 120                 125
```

```
Tyr Asp Glu Glu Ala Thr Leu Cys Val Phe Arg Leu Lys Ser Leu Lys
    130                 135                 140

Gln Gly Gln Ser Arg Ser Leu Leu Thr Gln Ala Ala Arg His His Ala
145                 150                 155                 160

Ala Gln Lys Gly Leu Lys Ser Leu Glu Thr Val Glu Pro His Val Arg
                165                 170                 175

Leu Val Thr Glu His Gln His Ile Asp Tyr His Phe Ser Gly Leu Glu
            180                 185                 190

Ser Asp Gly Arg Glu Asp Gly Glu Asp Asp Gly Glu Asp Gly Gly Asp
        195                 200                 205

Thr Asn Glu Arg Arg Glu Leu Ser Phe Asp Tyr Arg Glu Asn Asn Gln
    210                 215                 220

Gly Asn Ala Val Val Ser Ala Ala Arg Gly Ser Met Glu Val Asp Glu
225                 230                 235                 240

Glu Asp Leu Ser Phe Gln Val Pro Ala Ala Glu Asn Val Glu Leu Asn
                245                 250                 255

Pro Asp Lys Asn His Gly Gly Phe Gln Phe Pro Ser Arg Glu Pro Arg
            260                 265                 270

Gly Gly Ser His Ser Ala Pro Ile Val Pro Glu Arg Lys Pro Asp Pro
        275                 280                 285

Val Glu Arg Ile Arg Gln Met Gln Gln Ala Ser Arg Lys Ser Asn Thr
    290                 295                 300

Tyr Val Leu Pro Thr Pro Ile Asp Ala Lys Gly Ala Ile Ser Ser Arg
305                 310                 315                 320

Thr Ser Cys Ser Val Pro Asn Thr Arg Gln Thr Asp Ile Ser Gly Arg
                325                 330                 335

Ala His Asn Leu Trp His Ser Ser Pro Leu Glu Gln Lys Lys Asn Glu
            340                 345                 350

Lys Asp Ser Gly Asp Gly His Leu Ser Asp Phe Thr Ala Leu Lys Ala
        355                 360                 365

Arg Ser Gly His Lys Glu Ser Asn Asn Pro Asn Ala Ser Thr Gln Leu
    370                 375                 380

Pro Pro Pro Leu Val Gly Gly Ile Ser Tyr Pro Gln Leu Asp Val His
385                 390                 395                 400
```

```
Asn Ala Ser Asp Tyr Lys Lys Asn Lys Trp Gln Ser Phe Ser Gly Pro
                405                 410                 415

Ile Thr Ser Lys Pro Trp Ser Met Lys Pro Leu Ser Ser Ser Gly Pro
            420                 425                 430

Ile Ser Ser Thr Glu Leu Ser Gln Gln Val Ser Gly Met Leu Ser Arg
        435                 440                 445

Gly Ala Asn Pro Gln Pro Ser Ser Ser Pro Lys Val Ser Pro Ser Thr
    450                 455                 460

Ser Pro Pro Leu Val Ser Ser Pro Lys Ile Ser Glu Leu His Glu Leu
465                 470                 475                 480

Pro Arg Pro Pro Gly Asn Leu Ala Ala Lys Ala Ala Lys Pro Ser Val
                485                 490                 495

Leu Ile Gly His Ser Ala Pro Leu Ser Arg Asn Pro Glu Leu Ala Gly
            500                 505                 510

Thr Ser Lys Ile Ser Thr Gly Ala Ala Asn Leu Ala Ser Pro Leu Pro
        515                 520                 525

Pro Pro Pro Leu Ile Val Pro Arg Ser Phe Ser Ile Pro Ser Ser Ser
    530                 535                 540

Gln Arg Ala Met Thr Val His Val Ser Lys Leu Leu Asp Ser Ser Gln
545                 550                 555                 560

Val Ser Tyr Lys Pro Gly Glu Val Asp Ser Pro Pro Leu Thr Pro Met
                565                 570                 575

Ser Leu Ala Asn Met Arg Pro Ala Pro Ala Ile Ser Glu Pro Val Pro
            580                 585                 590

His Ser Gly Gln Ile Arg Gly Asn Lys Thr Ser Cys Ser Leu Ile Phe
        595                 600                 605

<210>  53
<211>  1725
<212>  DNA
<213>  P.trichocarpa

<220>
<221>  CDS
<222>  (1)..(1725)

<400>  53
atg ggt gct tgc ctt ctt gcg aaa act gca ttg aat gat gat gaa gaa         48
```

```
Met Gly Ala Cys Leu Leu Ala Lys Thr Ala Leu Asn Asp Asp Glu Glu
1               5                   10                  15

agt ggc agg gtc ttg ctg atg ctg gga aaa gtg caa ttt gaa ctc cag      96
Ser Gly Arg Val Leu Leu Met Leu Gly Lys Val Gln Phe Glu Leu Gln
            20                  25                  30

aaa ctt gtt gac tgc tat cgt tct cac ata aat cag aca atc ata agc      144
Lys Leu Val Asp Cys Tyr Arg Ser His Ile Asn Gln Thr Ile Ile Ser
        35                  40                  45

ccg tca gag tct ctt ctg aat gaa ctt caa ata gtt gag gag atg aag      192
Pro Ser Glu Ser Leu Leu Asn Glu Leu Gln Ile Val Glu Glu Met Lys
    50                  55                  60

cag cag tgt gat gag aaa aga gat gta tat gct cac atg gcg aga cag      240
Gln Gln Cys Asp Glu Lys Arg Asp Val Tyr Ala His Met Ala Arg Gln
65                  70                  75                  80

aga gaa aga gtg agt gga aga aat gga aag gga gag tgt ttt tct atg      288
Arg Glu Arg Val Ser Gly Arg Asn Gly Lys Gly Glu Cys Phe Ser Met
                85                  90                  95

cag caa ata caa gca gct cat gat gaa tat gat gag gag gcc act ttg      336
Gln Gln Ile Gln Ala Ala His Asp Glu Tyr Asp Glu Glu Ala Thr Leu
            100                 105                 110

ttt gtt ttt cgt ctt aaa tcc ctg aag gaa gga caa tct cgc agt ctt      384
Phe Val Phe Arg Leu Lys Ser Leu Lys Glu Gly Gln Ser Arg Ser Leu
        115                 120                 125

ctt aca cag gcg gct cgg cac cat gct gct cag ctc tgc ttc ttt aag      432
Leu Thr Gln Ala Ala Arg His His Ala Ala Gln Leu Cys Phe Phe Lys
    130                 135                 140

aag gct ctt caa tct ctt gaa gct gtt gag ccg cat gtc aaa ttg gtc      480
Lys Ala Leu Gln Ser Leu Glu Ala Val Glu Pro His Val Lys Leu Val
145                 150                 155                 160

tca gaa cag cag cat ata gat tac cac ttt agt ggc ctt gat gat gat      528
Ser Glu Gln Gln His Ile Asp Tyr His Phe Ser Gly Leu Asp Asp Asp
                165                 170                 175

ggg agg gat tat gat gat gat gaa gat tat gat gat gcc att gat gat      576
Gly Arg Asp Tyr Asp Asp Asp Glu Asp Tyr Asp Asp Ala Ile Asp Asp
            180                 185                 190

gga gaa ttg agc ttt gac tat gga caa aat gac cag gag caa gaa gtt      624
Gly Glu Leu Ser Phe Asp Tyr Gly Gln Asn Asp Gln Glu Gln Glu Val
        195                 200                 205

tcc aaa tca ata aag tca atg gag ttg gat tta gag gac att aca ttt      672
Ser Lys Ser Ile Lys Ser Met Glu Leu Asp Leu Glu Asp Ile Thr Phe
    210                 215                 220

ccc caa gtt gtg aca ttg gaa atg gca aag gaa aat cca gat aca agt      720
Pro Gln Val Val Thr Leu Glu Met Ala Lys Glu Asn Pro Asp Thr Ser
225                 230                 235                 240

tac agg aca tcg ttt cct att aag gga gaa ctt agt gca ggc acc caa      768
Tyr Arg Thr Ser Phe Pro Ile Lys Gly Glu Leu Ser Ala Gly Thr Gln
                245                 250                 255

tca gct cca ctt ttt gct caa gtg aaa tct aat cca gct gga aaa aca      816
Ser Ala Pro Leu Phe Ala Gln Val Lys Ser Asn Pro Ala Gly Lys Thr
            260                 265                 270
```

```
aaa caa ctg atg cca tca tcc acc cga aag ttc aac act tat gtg ttg      864
Lys Gln Leu Met Pro Ser Ser Thr Arg Lys Phe Asn Thr Tyr Val Leu
        275                 280                 285

cct act cca gca gac cca aag agt tca aat tct aca gga cct ggt agc      912
Pro Thr Pro Ala Asp Pro Lys Ser Ser Asn Ser Thr Gly Pro Gly Ser
    290                 295                 300

ccg gtt tct caa aca tta aag aca agt ttg agt gga cgt cct ctg aat      960
Pro Val Ser Gln Thr Leu Lys Thr Ser Leu Ser Gly Arg Pro Leu Asn
305                 310                 315                 320

ttg tgg cac tca tcc cct att ggc cat aaa aaa aat gat aag tta cta     1008
Leu Trp His Ser Ser Pro Ile Gly His Lys Lys Asn Asp Lys Leu Leu
                325                 330                 335

gga gtt gaa atg tct agc aag ccc cct gct ata aat tcg cag tca gta     1056
Gly Val Glu Met Ser Ser Lys Pro Pro Ala Ile Asn Ser Gln Ser Val
            340                 345                 350

ctc aag gag agc aac aac aac acg gca tcc acc cga tta cct cct cct     1104
Leu Lys Glu Ser Asn Asn Asn Thr Ala Ser Thr Arg Leu Pro Pro Pro
        355                 360                 365

cta gct gat gga ctc ttt ttt tca agg ctt gag cca cca gct ggc gct     1152
Leu Ala Asp Gly Leu Phe Phe Ser Arg Leu Glu Pro Pro Ala Gly Ala
    370                 375                 380

gaa tct aag aaa atc aaa aga tat gcc ttt tct ggt cct att tca tcc     1200
Glu Ser Lys Lys Ile Lys Arg Tyr Ala Phe Ser Gly Pro Ile Ser Ser
385                 390                 395                 400

cag cca tgg tct acc aag gca gtc tcc gca gaa cat cca caa ttg ttc     1248
Gln Pro Trp Ser Thr Lys Ala Val Ser Ala Glu His Pro Gln Leu Phe
                405                 410                 415

tct gga ccc ctt ttg cga aat cca acg gcc caa tta ttt tca tca cct     1296
Ser Gly Pro Leu Leu Arg Asn Pro Thr Ala Gln Leu Phe Ser Ser Pro
            420                 425                 430

ccg aaa gtg tct cca aga att tca cca aaa gta tct cct agt tct tcc     1344
Pro Lys Val Ser Pro Arg Ile Ser Pro Lys Val Ser Pro Ser Ser Ser
        435                 440                 445

cct cca ttt gtg tcc tca ccc aaa atc agc gag cta cat gag ctt ccc     1392
Pro Pro Phe Val Ser Ser Pro Lys Ile Ser Glu Leu His Glu Leu Pro
    450                 455                 460

aga ccc cca gtc agt tca acc tcc aag tct ccg ggg gct gga ggt ttg     1440
Arg Pro Pro Val Ser Ser Thr Ser Lys Ser Pro Gly Ala Gly Gly Leu
465                 470                 475                 480

gtt ggt cat tca gct cca ctg ttg ccc aaa ggc cac atg ctt cct ggt     1488
Val Gly His Ser Ala Pro Leu Leu Pro Lys Gly His Met Leu Pro Gly
                485                 490                 495

aca agc aaa acg tca gca tca aat gta gaa tct caa ctg cct aca cct     1536
Thr Ser Lys Thr Ser Ala Ser Asn Val Glu Ser Gln Leu Pro Thr Pro
            500                 505                 510

tct caa gtt gtc cct cgc agt ttt tca ata ccc tct agc aga cat aga     1584
Ser Gln Val Val Pro Arg Ser Phe Ser Ile Pro Ser Ser Arg His Arg
        515                 520                 525

gta atg gtc gct cag aat tca ggg att gtt gag aat gtt gcc tca cct     1632
```

```
Val Met Val Ala Gln Asn Ser Gly Ile Val Glu Asn Val Ala Ser Pro
    530                 535                 540

cct cta acc cca ata tct tta tct aac acc cag cca tca tcc act ggt      1680
Pro Leu Thr Pro Ile Ser Leu Ser Asn Thr Gln Pro Ser Ser Thr Gly
545                 550                 555                 560

tcc cgg atc gtc aat cag aca gtt caa atc aga ggt aat tca taa          1725
Ser Arg Ile Val Asn Gln Thr Val Gln Ile Arg Gly Asn Ser
                565                 570


<210>  54
<211>  574
<212>  PRT
<213>  P.trichocarpa

<400>  54

Met Gly Ala Cys Leu Leu Ala Lys Thr Ala Leu Asn Asp Asp Glu Glu
1               5                   10                  15


Ser Gly Arg Val Leu Leu Met Leu Gly Lys Val Gln Phe Glu Leu Gln
            20                  25                  30


Lys Leu Val Asp Cys Tyr Arg Ser His Ile Asn Gln Thr Ile Ile Ser
        35                  40                  45


Pro Ser Glu Ser Leu Leu Asn Glu Leu Gln Ile Val Glu Glu Met Lys
    50                  55                  60


Gln Gln Cys Asp Glu Lys Arg Asp Val Tyr Ala His Met Ala Arg Gln
65                  70                  75                  80


Arg Glu Arg Val Ser Gly Arg Asn Gly Lys Gly Glu Cys Phe Ser Met
                85                  90                  95


Gln Gln Ile Gln Ala Ala His Asp Glu Tyr Asp Glu Glu Ala Thr Leu
            100                 105                 110


Phe Val Phe Arg Leu Lys Ser Leu Lys Glu Gly Gln Ser Arg Ser Leu
        115                 120                 125


Leu Thr Gln Ala Ala Arg His His Ala Ala Gln Leu Cys Phe Phe Lys
    130                 135                 140


Lys Ala Leu Gln Ser Leu Glu Ala Val Glu Pro His Val Lys Leu Val
145                 150                 155                 160


Ser Glu Gln Gln His Ile Asp Tyr His Phe Ser Gly Leu Asp Asp Asp
                165                 170                 175


Gly Arg Asp Tyr Asp Asp Asp Glu Asp Tyr Asp Asp Ala Ile Asp Asp
            180                 185                 190
```

```
Gly Glu Leu Ser Phe Asp Tyr Gly Gln Asn Asp Gln Glu Gln Glu Val
        195                 200                 205

Ser Lys Ser Ile Lys Ser Met Glu Leu Asp Leu Glu Asp Ile Thr Phe
    210                 215                 220

Pro Gln Val Val Thr Leu Glu Met Ala Lys Glu Asn Pro Asp Thr Ser
225                 230                 235                 240

Tyr Arg Thr Ser Phe Pro Ile Lys Gly Glu Leu Ser Ala Gly Thr Gln
                245                 250                 255

Ser Ala Pro Leu Phe Ala Gln Val Lys Ser Asn Pro Ala Gly Lys Thr
            260                 265                 270

Lys Gln Leu Met Pro Ser Ser Thr Arg Lys Phe Asn Thr Tyr Val Leu
        275                 280                 285

Pro Thr Pro Ala Asp Pro Lys Ser Ser Asn Ser Thr Gly Pro Gly Ser
    290                 295                 300

Pro Val Ser Gln Thr Leu Lys Thr Ser Leu Ser Gly Arg Pro Leu Asn
305                 310                 315                 320

Leu Trp His Ser Ser Pro Ile Gly His Lys Lys Asn Asp Lys Leu Leu
                325                 330                 335

Gly Val Glu Met Ser Ser Lys Pro Pro Ala Ile Asn Ser Gln Ser Val
            340                 345                 350

Leu Lys Glu Ser Asn Asn Asn Thr Ala Ser Thr Arg Leu Pro Pro Pro
        355                 360                 365

Leu Ala Asp Gly Leu Phe Phe Ser Arg Leu Glu Pro Pro Ala Gly Ala
    370                 375                 380

Glu Ser Lys Lys Ile Lys Arg Tyr Ala Phe Ser Gly Pro Ile Ser Ser
385                 390                 395                 400

Gln Pro Trp Ser Thr Lys Ala Val Ser Ala Glu His Pro Gln Leu Phe
                405                 410                 415

Ser Gly Pro Leu Leu Arg Asn Pro Thr Ala Gln Leu Phe Ser Ser Pro
            420                 425                 430

Pro Lys Val Ser Pro Arg Ile Ser Pro Lys Val Ser Pro Ser Ser Ser
        435                 440                 445

Pro Pro Phe Val Ser Ser Pro Lys Ile Ser Glu Leu His Glu Leu Pro
```

```
    450                 455                 460

Arg Pro Pro Val Ser Ser Thr Ser Lys Ser Pro Gly Ala Gly Gly Leu
465                 470                 475                 480

Val Gly His Ser Ala Pro Leu Leu Pro Lys Gly His Met Leu Pro Gly
                485                 490                 495

Thr Ser Lys Thr Ser Ala Ser Asn Val Glu Ser Gln Leu Pro Thr Pro
            500                 505                 510

Ser Gln Val Val Pro Arg Ser Phe Ser Ile Pro Ser Ser Arg His Arg
        515                 520                 525

Val Met Val Ala Gln Asn Ser Gly Ile Val Glu Asn Val Ala Ser Pro
    530                 535                 540

Pro Leu Thr Pro Ile Ser Leu Ser Asn Thr Gln Pro Ser Ser Thr Gly
545                 550                 555                 560

Ser Arg Ile Val Asn Gln Thr Val Gln Ile Arg Gly Asn Ser
                565                 570


<210>  55
<211>  552
<212>  DNA
<213>  P.trichocarpa


<220>
<221>  CDS
<222>  (1)..(552)

<400>  55
atg aag tcc aaa ctg aga gga ttt aga ctc aga aga agc gaa ccg aag       48
Met Lys Ser Lys Leu Arg Gly Phe Arg Leu Arg Arg Ser Glu Pro Lys
1               5                   10                  15

gac aaa att gac ttc tta cct cct gct cag tta gat gag ctc gct caa       96
Asp Lys Ile Asp Phe Leu Pro Pro Ala Gln Leu Asp Glu Leu Ala Gln
            20                  25                  30

gct gct cag gat atg aaa gac atg aag aat tgc tat gat agt tta ctt      144
Ala Ala Gln Asp Met Lys Asp Met Lys Asn Cys Tyr Asp Ser Leu Leu
        35                  40                  45

tct gca gct gca gca aca gca aac agt gca tat gaa ttc tca gag tca      192
Ser Ala Ala Ala Ala Thr Ala Asn Ser Ala Tyr Glu Phe Ser Glu Ser
    50                  55                  60

ttg cgg gaa atg ggt tca tgt ctg tcg gag aaa aca gca tta cat gat      240
Leu Arg Glu Met Gly Ser Cys Leu Ser Glu Lys Thr Ala Leu His Asp
65                  70                  75                  80

gat gaa gga agt ggt agg gtt ttg cta atg ttg ggg aaa gtg cag ttt      288
Asp Glu Gly Ser Gly Arg Val Leu Leu Met Leu Gly Lys Val Gln Phe
                85                  90                  95
```

```
gaa ctt caa aaa ctt ctt gat agt tat cgg tct cat ata ttc ctg acg       336
Glu Leu Gln Lys Leu Leu Asp Ser Tyr Arg Ser His Ile Phe Leu Thr
            100                 105                 110

att aca aat cca tca gaa tcc ttg ctc aat gaa ctt cgg aca gtt gag       384
Ile Thr Asn Pro Ser Glu Ser Leu Leu Asn Glu Leu Arg Thr Val Glu
        115                 120                 125

gat atg aag cga caa tgt gat gaa aaa aga aat gtc tat gaa tac atg       432
Asp Met Lys Arg Gln Cys Asp Glu Lys Arg Asn Val Tyr Glu Tyr Met
    130                 135                 140

gtg gca caa cag aag gac aaa gga agg tca aaa ggt ggg aag gat gaa       480
Val Ala Gln Gln Lys Asp Lys Gly Arg Ser Lys Gly Gly Lys Asp Glu
145                 150                 155                 160

agt att act ttg cag caa ttg caa aca gct cgt gaa gaa tat gac gaa       528
Ser Ile Thr Leu Gln Gln Leu Gln Thr Ala Arg Glu Glu Tyr Asp Glu
                165                 170                 175

gag gca gcc att cag cac caa tag                                       552
Glu Ala Ala Ile Gln His Gln
            180
```

<210> 56
<211> 183
<212> PRT
<213> P.trichocarpa

<400> 56

```
Met Lys Ser Lys Leu Arg Gly Phe Arg Leu Arg Arg Ser Glu Pro Lys
1               5                   10                  15

Asp Lys Ile Asp Phe Leu Pro Pro Ala Gln Leu Asp Glu Leu Ala Gln
            20                  25                  30

Ala Ala Gln Asp Met Lys Asp Met Lys Asn Cys Tyr Asp Ser Leu Leu
        35                  40                  45

Ser Ala Ala Ala Ala Thr Ala Asn Ser Ala Tyr Glu Phe Ser Glu Ser
    50                  55                  60

Leu Arg Glu Met Gly Ser Cys Leu Ser Glu Lys Thr Ala Leu His Asp
65                  70                  75                  80

Asp Glu Gly Ser Gly Arg Val Leu Leu Met Leu Gly Lys Val Gln Phe
                85                  90                  95

Glu Leu Gln Lys Leu Leu Asp Ser Tyr Arg Ser His Ile Phe Leu Thr
            100                 105                 110

Ile Thr Asn Pro Ser Glu Ser Leu Leu Asn Glu Leu Arg Thr Val Glu
        115                 120                 125

Asp Met Lys Arg Gln Cys Asp Glu Lys Arg Asn Val Tyr Glu Tyr Met
    130                 135                 140
```

```
Val Ala Gln Gln Lys Asp Lys Gly Arg Ser Lys Gly Gly Lys Asp Glu
145                 150                 155                 160

Ser Ile Thr Leu Gln Gln Leu Gln Thr Ala Arg Glu Glu Tyr Asp Glu
                165                 170                 175

Glu Ala Ala Ile Gln His Gln
            180

<210>  57
<211>  531
<212>  DNA
<213>  B. napus

<220>
<221>  CDS
<222>  (1)..(531)

<400>  57
atg aaa gct tcg atg ggg atg ctg agg aga tta aca tcg cac aag gtc       48
Met Lys Ala Ser Met Gly Met Leu Arg Arg Leu Thr Ser His Lys Val
1               5                   10                  15

gac gcg aag gag aaa gga gag ctc gtc gct aca gcg cag att gaa gaa       96
Asp Ala Lys Glu Lys Gly Glu Leu Val Ala Thr Ala Gln Ile Glu Glu
            20                  25                  30

ctc gat cga gcc ggg aag gat atg caa gac atg aga gaa tgc tat gat      144
Leu Asp Arg Ala Gly Lys Asp Met Gln Asp Met Arg Glu Cys Tyr Asp
        35                  40                  45

agg cta ctc gct gca gct gct gcc acc gca aat agt gct tat gag ttc      192
Arg Leu Leu Ala Ala Ala Ala Ala Thr Ala Asn Ser Ala Tyr Glu Phe
    50                  55                  60

tct gag tcg ttg gga gag atg ggt tct tgt ttg gag caa atc gcg cct      240
Ser Glu Ser Leu Gly Glu Met Gly Ser Cys Leu Glu Gln Ile Ala Pro
65                  70                  75                  80

cat aac gac gaa gaa agc agt aga atc ttg ttt atg tgc ggc aaa gtt      288
His Asn Asp Glu Glu Ser Ser Arg Ile Leu Phe Met Cys Gly Lys Val
                85                  90                  95

cag ttt gag att caa aaa ctt ctt gac act tat cga agt cat ata ttc      336
Gln Phe Glu Ile Gln Lys Leu Leu Asp Thr Tyr Arg Ser His Ile Phe
            100                 105                 110

aag acc att acg tcc cca tca gaa gcg ctt ctt aag gac ctc aga act      384
Lys Thr Ile Thr Ser Pro Ser Glu Ala Leu Leu Lys Asp Leu Arg Thr
        115                 120                 125

gtt gag gat atg aag caa caa tgc gat gag aag aga aac gtg ttt gag      432
Val Glu Asp Met Lys Gln Gln Cys Asp Glu Lys Arg Asn Val Phe Glu
    130                 135                 140

atg tcg ctt gtg aaa gat aaa gga agg tct aaa ggc agt aaa gga gag      480
Met Ser Leu Val Lys Asp Lys Gly Arg Ser Lys Gly Ser Lys Gly Glu
145                 150                 155                 160

aga cat att cct cat gga ctt cga cga cct gca tac aat gag ttt cca      528
```

Arg His Ile Pro His Gly Leu Arg Arg Pro Ala Tyr Asn Glu Phe Pro
165 170 175

tga 531

<210> 58
<211> 176
<212> PRT
<213> B. napus

<400> 58

Met Lys Ala Ser Met Gly Met Leu Arg Arg Leu Thr Ser His Lys Val
1 5 10 15

Asp Ala Lys Glu Lys Gly Glu Leu Val Ala Thr Ala Gln Ile Glu Glu
20 25 30

Leu Asp Arg Ala Gly Lys Asp Met Gln Asp Met Arg Glu Cys Tyr Asp
35 40 45

Arg Leu Leu Ala Ala Ala Ala Ala Thr Ala Asn Ser Ala Tyr Glu Phe
50 55 60

Ser Glu Ser Leu Gly Glu Met Gly Ser Cys Leu Glu Gln Ile Ala Pro
65 70 75 80

His Asn Asp Glu Glu Ser Ser Arg Ile Leu Phe Met Cys Gly Lys Val
85 90 95

Gln Phe Glu Ile Gln Lys Leu Leu Asp Thr Tyr Arg Ser His Ile Phe
100 105 110

Lys Thr Ile Thr Ser Pro Ser Glu Ala Leu Leu Lys Asp Leu Arg Thr
115 120 125

Val Glu Asp Met Lys Gln Gln Cys Asp Glu Lys Arg Asn Val Phe Glu
130 135 140

Met Ser Leu Val Lys Asp Lys Gly Arg Ser Lys Gly Ser Lys Gly Glu
145 150 155 160

Arg His Ile Pro His Gly Leu Arg Arg Pro Ala Tyr Asn Glu Phe Pro
165 170 175

<210> 59
<211> 56
<212> DNA
<213> Artificial

<220>
<223> Primer

<400> 59

ggggacaagt ttgtacaaaa aagcaggctt aaacaatgaa atcatttagg aaattg 56

<210> 60
<211> 50
<212> DNA
<213> Artificial

<220>
<223> Primer

<400> 60
ggggaccact ttgtacaaga aagctgggta agaatagaat caaactgcac 50

<210> 61
<211> 645
<212> PRT
<213> Populus trichocarpa

<400> 61

```
Met Lys Ser Phe Arg Lys Leu Arg Gly Phe Ala Ser Leu His Lys Gln
1               5                   10                  15

Val Val His Lys Asn Pro Arg Asp Leu Pro Ser Phe Ser Gln Ser His
            20                  25                  30

Glu Leu Ala Lys Ala Ser Gln Asp Met Lys Asp Met Lys Asp Cys Tyr
        35                  40                  45

Asp Ser Leu Leu Ser Ala Ala Ala Gly Thr Ala Asn Cys Ala Phe Glu
    50                  55                  60

Phe Ser Glu Ser Leu Arg Glu Met Gly Ala Cys Leu Leu Ala Lys Thr
65                  70                  75                  80

Ala Leu Asn Asp Asp Glu Glu Ser Gly Arg Val Leu Leu Met Leu Gly
                85                  90                  95

Lys Val Gln Phe Glu Leu Gln Lys Leu Val Asp Cys Tyr Arg Ser His
            100                 105                 110

Ile Asn Gln Thr Ile Ile Ser Pro Ser Glu Ser Leu Leu Asn Glu Leu
        115                 120                 125

Gln Ile Val Glu Glu Met Lys Gln Gln Cys Asp Glu Lys Arg Asp Val
    130                 135                 140

Tyr Ala His Met Ala Arg Gln Arg Glu Arg Val Ser Gly Arg Asn Gly
145                 150                 155                 160

Lys Gly Glu Cys Phe Ser Met Gln Gln Ile Gln Ala Ala His Asp Glu
                165                 170                 175
```

Tyr Asp Glu Glu Ala Thr Leu Phe Val Phe Arg Leu Lys Ser Leu Lys
180 185 190

Glu Gly Gln Ser Arg Ser Leu Leu Thr Gln Ala Ala Arg His His Ala
195 200 205

Ala Gln Leu Cys Phe Phe Lys Lys Ala Leu Gln Ser Leu Glu Ala Val
210 215 220

Glu Pro His Val Lys Leu Val Ser Glu Gln Gln His Ile Asp Tyr His
225 230 235 240

Phe Ser Gly Leu Asp Asp Asp Gly Arg Asp Tyr Asp Asp Asp Glu Asp
245 250 255

Tyr Asp Asp Ala Ile Asp Asp Gly Glu Leu Ser Phe Asp Tyr Gly Gln
260 265 270

Asn Asp Gln Glu Gln Glu Val Ser Lys Ser Ile Lys Ser Met Glu Leu
275 280 285

Asp Leu Glu Asp Ile Thr Phe Pro Gln Val Val Thr Leu Glu Met Ala
290 295 300

Lys Glu Asn Pro Asp Arg Ser Tyr Arg Thr Ser Phe Pro Ile Lys Gly
305 310 315 320

Glu Leu Ser Ala Gly Thr Gln Ser Ala Pro Leu Phe Ala Gln Val Lys
325 330 335

Ser Asn Pro Ala Gly Lys Thr Lys Gln Leu Met Pro Ser Ser Thr Arg
340 345 350

Lys Phe Asn Thr Tyr Val Leu Pro Thr Pro Ala Asp Pro Lys Ser Ser
355 360 365

Asn Ser Thr Gly Pro Gly Ser Pro Val Ser Gln Thr Leu Lys Thr Ser
370 375 380

Leu Ser Gly Arg Pro Leu Asn Leu Trp His Ser Ser Pro Ile Gly His
385 390 395 400

Lys Lys Asn Asp Lys Leu Leu Gly Val Glu Met Ser Ser Lys Pro Pro
405 410 415

Ala Ile Asn Ser Arg Ser Val Leu Lys Glu Ser Asn Asn Asn Thr Ala
420 425 430

Ser Thr Arg Leu Pro Pro Pro Leu Ala Asp Gly Leu Phe Phe Ser Arg
435 440 445

Leu Glu Pro Pro Ala Gly Ala Glu Ser Lys Lys Ile Lys Arg Tyr Ala
    450                 455                 460

Phe Ser Gly Pro Ile Thr Ser Gln Pro Trp Ser Thr Lys Ala Val Ser
465                 470                 475                 480

Ala Glu His Pro Gln Leu Phe Ser Gly Pro Leu Leu Arg Asn Pro Thr
                485                 490                 495

Ala Gln Leu Phe Ser Ser Pro Pro Lys Val Ser Pro Arg Ile Ser Pro
            500                 505                 510

Lys Val Ser Pro Ser Ser Ser Pro Pro Phe Val Ser Ser Pro Lys Ile
        515                 520                 525

Ser Glu Leu His Glu Leu Pro Arg Pro Pro Val Ser Ser Thr Ser Lys
    530                 535                 540

Ser Pro Gly Ala Gly Gly Leu Val Gly His Ser Ala Pro Leu Leu Pro
545                 550                 555                 560

Lys Gly His Met Leu Pro Gly Thr Ser Lys Thr Ser Ala Ser Asn Val
                565                 570                 575

Glu Ser Gln Leu Pro Thr Pro Ser Gln Val Val Pro Arg Ser Phe Ser
            580                 585                 590

Ile Pro Ser Ser Arg His Arg Val Met Val Ala Gln Asn Pro Gly Ile
        595                 600                 605

Val Glu Asn Val Ala Ser Pro Pro Leu Thr Pro Ile Ser Leu Ser Asn
    610                 615                 620

Thr Gln Pro Ser Ser Thr Gly Ser Arg Ile Val Asn Gln Thr Val Gln
625                 630                 635                 640

Ile Arg Gly Ala Val
                645

<210> 62
<211> 645
<212> PRT
<213> Populus trichocarpa

<400> 62

Met Lys Ser Phe Arg Lys Leu Arg Gly Phe Ala Ser Leu His Lys Gln
1               5                   10                  15

Val Val His Lys Asn Pro Arg Asp Leu Pro Ser Phe Ser Gln Ser His

20 25 30

Glu Leu Ala Lys Ala Ser Gln Asp Met Lys Asp Met Lys Asp Cys Tyr
35 40 45

Asp Ser Leu Leu Ser Ala Ala Ala Gly Thr Ala Asn Cys Ala Phe Glu
50 55 60

Phe Ser Glu Ser Leu Arg Glu Met Gly Ala Cys Leu Leu Ala Lys Thr
65 70 75 80

Ala Leu Asn Asp Asp Glu Glu Ser Gly Arg Val Leu Leu Met Leu Gly
85 90 95

Lys Val Gln Phe Glu Leu Gln Lys Leu Val Asp Cys Tyr Arg Ser His
100 105 110

Ile Asn Gln Thr Ile Ile Ser Pro Ser Glu Ser Leu Leu Asn Glu Leu
115 120 125

Gln Ile Val Glu Glu Met Lys Gln Gln Cys Asp Glu Lys Arg Asp Val
130 135 140

Tyr Ala His Met Ala Arg Gln Arg Glu Arg Val Ser Gly Arg Asn Gly
145 150 155 160

Lys Gly Glu Cys Phe Ser Met Gln Gln Ile Gln Ala Ala His Asp Glu
165 170 175

Tyr Asp Glu Glu Ala Thr Leu Phe Val Phe Arg Leu Lys Ser Leu Lys
180 185 190

Glu Gly Gln Ser Arg Ser Leu Leu Thr Gln Ala Ala Arg His His Ala
195 200 205

Ala Gln Leu Cys Phe Phe Lys Lys Ala Leu Gln Ser Leu Glu Ala Val
210 215 220

Glu Pro His Val Lys Leu Val Ser Glu Gln Gln His Ile Asp Tyr His
225 230 235 240

Phe Ser Gly Leu Asp Asp Asp Gly Arg Asp Tyr Asp Asp Asp Glu Asp
245 250 255

Tyr Asp Asp Ala Ile Asp Asp Gly Glu Leu Ser Phe Asp Tyr Gly Gln
260 265 270

Asn Asp Gln Glu Gln Glu Val Ser Lys Ser Ile Lys Ser Met Glu Leu
275 280 285

```
Asp Leu Glu Asp Ile Thr Phe Pro Gln Val Val Thr Leu Glu Met Ala
    290                 295                 300

Lys Glu Asn Pro Asp Thr Ser Tyr Arg Thr Ser Phe Pro Ile Lys Gly
305                 310                 315                 320

Glu Leu Ser Ala Gly Thr Gln Ser Ala Pro Leu Phe Ala Gln Val Lys
                325                 330                 335

Ser Asn Pro Ala Gly Lys Thr Lys Gln Leu Met Pro Ser Ser Thr Arg
            340                 345                 350

Lys Phe Asn Thr Tyr Val Leu Pro Thr Pro Ala Asp Pro Lys Ser Ser
        355                 360                 365

Asn Ser Thr Gly Pro Gly Ser Pro Val Ser Gln Thr Leu Lys Thr Ser
    370                 375                 380

Leu Ser Gly Arg Pro Leu Asn Leu Trp His Ser Ser Pro Ile Gly His
385                 390                 395                 400

Lys Lys Asn Asp Lys Leu Leu Gly Val Glu Met Ser Ser Lys Pro Pro
                405                 410                 415

Ala Ile Asn Ser Gln Ser Val Leu Lys Glu Ser Asn Asn Asn Thr Ala
            420                 425                 430

Ser Thr Arg Leu Pro Pro Pro Leu Ala Asp Gly Leu Phe Phe Ser Arg
        435                 440                 445

Leu Glu Pro Pro Ala Gly Ala Glu Ser Lys Lys Ile Lys Arg Tyr Ala
    450                 455                 460

Phe Ser Gly Pro Ile Ser Ser Gln Pro Trp Ser Thr Lys Ala Val Ser
465                 470                 475                 480

Ala Glu His Pro Gln Leu Phe Ser Gly Pro Leu Leu Arg Asn Pro Thr
                485                 490                 495

Ala Gln Leu Phe Ser Ser Pro Pro Lys Val Ser Pro Arg Ile Ser Pro
            500                 505                 510

Lys Val Ser Pro Ser Ser Ser Pro Pro Phe Val Ser Ser Pro Lys Ile
        515                 520                 525

Ser Glu Leu His Glu Leu Pro Arg Pro Pro Val Ser Ser Thr Ser Lys
    530                 535                 540

Ser Pro Gly Ala Gly Gly Leu Val Gly His Ser Ala Pro Leu Leu Pro
```

545 550 555 560

Lys Gly His Met Leu Pro Gly Thr Ser Lys Thr Ser Ala Ser Asn Val
565 570 575

Glu Ser Gln Leu Pro Thr Pro Ser Gln Val Val Pro Arg Ser Phe Ser
580 585 590

Ile Pro Ser Ser Arg His Arg Val Met Val Ala Gln Asn Ser Gly Ile
595 600 605

Val Glu Asn Val Ala Ser Pro Pro Leu Thr Pro Ile Ser Leu Ser Asn
610 615 620

Thr Gln Pro Ser Ser Thr Gly Ser Arg Ile Val Asn Gln Thr Val Gln
625 630 635 640

Ile Arg Gly Ala Val
645

<210> 63
<211> 623
<212> PRT
<213> Arabidopsis thaliana

<400> 63

Met Lys Thr Ser Leu Arg Arg Leu Arg Gly Val Leu His Lys His Glu
1 5 10 15

Ser Lys Asp Arg Arg Asp Leu Arg Ala Leu Val Gln Lys Asp Glu Leu
20 25 30

Ala Gln Ala Ser Gln Asp Val Glu Asp Met Arg Asp Cys Tyr Asp Ser
35 40 45

Leu Leu Asn Ala Ala Ala Ala Thr Ala Asn Ser Ala Tyr Glu Phe Ser
50 55 60

Glu Ser Leu Arg Glu Leu Gly Ala Cys Leu Leu Glu Lys Thr Ala Leu
65 70 75 80

Asn Asp Asp Glu Glu Ser Gly Arg Val Leu Ile Met Leu Gly Lys Leu
85 90 95

Gln Phe Glu Leu Gln Lys Leu Val Asp Lys Tyr Arg Ser His Ile Phe
100 105 110

Gln Thr Ile Thr Ile Pro Ser Glu Ser Leu Leu Asn Glu Leu Arg Ile
115 120 125

```
Val Glu Glu Met Gln Arg Leu Cys Asp Glu Lys Arg Asn Val Tyr Glu
    130                 135                 140

Gly Met Leu Thr Arg Gln Arg Glu Lys Gly Arg Ser Lys Gly Gly Lys
145                 150                 155                 160

Gly Glu Thr Phe Ser Pro Gln Gln Leu Gln Glu Ala His Asp Asp Tyr
                165                 170                 175

Glu Asn Glu Thr Thr Leu Phe Val Phe Arg Leu Lys Ser Leu Lys Gln
            180                 185                 190

Gly Gln Thr Arg Ser Leu Leu Thr Gln Ala Ala Arg His His Ala Ala
        195                 200                 205

Gln Leu Cys Phe Phe Lys Lys Ala Leu Ser Ser Leu Glu Glu Val Asp
    210                 215                 220

Pro His Val Gln Met Val Thr Glu Ser Gln His Ile Asp Tyr His Phe
225                 230                 235                 240

Ser Gly Leu Glu Asp Asp Asp Gly Asp Asp Glu Ile Glu Asn Asn Glu
                245                 250                 255

Asn Asp Gly Ser Glu Val His Asp Asp Gly Glu Leu Ser Phe Glu Tyr
            260                 265                 270

Arg Val Asn Asp Lys Asp Gln Asp Ala Asp Ser Ser Ala Gly Gly Ser
        275                 280                 285

Ser Glu Leu Gly Asn Ser Asp Ile Thr Phe Pro Gln Ile Gly Gly Pro
    290                 295                 300

Tyr Thr Ala Gln Glu Asn Glu Glu Gly Asn Tyr Arg Lys Ser His Ser
305                 310                 315                 320

Phe Arg Arg Asp Val Arg Ala Val Ser Gln Ser Ala Pro Leu Phe Pro
                325                 330                 335

Glu Asn Arg Thr Thr Pro Pro Ser Glu Lys Leu Leu Arg Met Arg Ser
            340                 345                 350

Thr Leu Thr Arg Lys Phe Asn Thr Tyr Ala Leu Pro Thr Pro Val Glu
        355                 360                 365

Thr Thr Arg Ser Pro Ser Ser Thr Thr Ser Pro Gly His Lys Asn Val
    370                 375                 380

Gly Ser Ser Asn Pro Thr Lys Ala Ile Thr Lys Gln Ile Trp Tyr Ser
385                 390                 395                 400
```

Ser Pro Leu Glu Thr Arg Gly Pro Ala Lys Val Ser Ser Arg Ser Met
405 410 415

Val Ala Leu Lys Glu Gln Val Leu Arg Glu Ser Asn Lys Asn Thr Ser
420 425 430

Arg Leu Pro Pro Pro Leu Ala Asp Gly Leu Leu Phe Ser Arg Leu Gly
435 440 445

Thr Leu Lys Arg Arg Ser Phe Ser Gly Pro Leu Thr Ser Lys Pro Leu
450 455 460

Pro Asn Lys Pro Leu Ser Thr Thr Ser His Leu Tyr Ser Gly Pro Ile
465 470 475 480

Pro Arg Asn Pro Val Ser Lys Leu Pro Lys Val Ser Ser Ser Pro Thr
485 490 495

Ala Ser Pro Thr Phe Val Ser Thr Pro Lys Ile Ser Glu Leu His Glu
500 505 510

Leu Pro Arg Pro Pro Pro Arg Ser Ser Thr Lys Ser Ser Arg Glu Leu
515 520 525

Gly Tyr Ser Ala Pro Leu Val Ser Arg Ser Gln Leu Leu Ser Lys Pro
530 535 540

Leu Ile Thr Asn Ser Ala Ser Pro Leu Pro Ile Pro Pro Ala Ile Thr
545 550 555 560

Arg Ser Phe Ser Ile Pro Thr Ser Asn Leu Arg Ala Ser Asp Leu Asp
565 570 575

Met Ser Lys Thr Ser Leu Gly Thr Lys Lys Leu Gly Thr Pro Ser Pro
580 585 590

Pro Leu Thr Pro Met Ser Leu Ile His Pro Pro Pro Gln Ala Leu Pro
595 600 605

Glu Arg Ala Asp His Leu Met Met Ser Lys Gln Glu Arg Arg Ile
610 615 620

<210> 64
<211> 608
<212> PRT
<213> A.thaliana

<400> 64

Met Lys Ala Ser Ile Glu Lys Leu Arg Arg Leu Thr Ser His Ser His

```
1               5                   10                  15

Lys Val Asp Val Lys Glu Lys Gly Asp Val Met Ala Thr Thr Gln Ile
            20                  25                  30

Asp Glu Leu Asp Arg Ala Gly Lys Asp Met Gln Asp Met Arg Glu Cys
        35                  40                  45

Tyr Asp Arg Leu Leu Ala Ala Ala Ala Ala Thr Ala Asn Ser Ala Tyr
    50                  55                  60

Glu Phe Ser Glu Ser Leu Gly Glu Met Gly Ser Cys Leu Glu Gln Ile
65                  70                  75                  80

Ala Pro His Asn Asp Glu Glu Ser Ser Arg Ile Leu Phe Met Leu Gly
                85                  90                  95

Lys Val Gln Ser Glu Leu Gln Arg Leu Leu Asp Thr Tyr Arg Ser His
            100                 105                 110

Ile Phe Glu Thr Ile Thr Ser Pro Ser Glu Ala Leu Leu Lys Asp Leu
        115                 120                 125

Arg Tyr Val Glu Asp Met Lys Gln Gln Cys Asp Gly Lys Arg Asn Val
    130                 135                 140

Tyr Glu Met Ser Leu Val Lys Glu Lys Gly Arg Pro Lys Ser Ser Lys
145                 150                 155                 160

Gly Glu Arg His Ile Pro Pro Glu Ser Arg Pro Ala Tyr Ser Glu Phe
                165                 170                 175

His Asp Glu Ala Thr Met Cys Ile Phe Arg Leu Lys Ser Leu Lys Glu
            180                 185                 190

Gly Gln Ala Arg Ser Leu Leu Ile Gln Ala Val Arg His His Thr Ala
        195                 200                 205

Gln Met Arg Leu Phe His Thr Gly Leu Lys Ser Leu Glu Ala Val Glu
    210                 215                 220

Arg His Val Lys Val Ala Val Glu Lys Gln His Ile Asp Cys Asp Leu
225                 230                 235                 240

Ser Val His Gly Asn Glu Met Glu Ala Ser Glu Asp Asp Asp Asp Asp
                245                 250                 255

Gly Arg Tyr Met Asn Arg Glu Gly Glu Leu Ser Phe Asp Tyr Arg Thr
            260                 265                 270
```

```
Asn Glu Gln Lys Val Glu Ala Ser Ser Leu Ser Thr Pro Trp Ala Thr
        275                 280                 285

Lys Met Asp Asp Thr Asp Leu Ser Phe Pro Arg Pro Ser Thr Thr Arg
    290                 295                 300

Pro Ala Ala Val Asn Ala Asp His Arg Glu Glu Tyr Pro Val Ser Thr
305                 310                 315                 320

Arg Asp Lys Tyr Leu Ser Ser His Ser Ala Pro Leu Phe Pro Glu Lys
                325                 330                 335

Lys Pro Asp Val Ser Glu Arg Leu Arg Gln Ala Asn Pro Ser Phe Asn
            340                 345                 350

Ala Tyr Val Leu Pro Thr Pro Asn Asp Ser Arg Tyr Ser Lys Pro Val
        355                 360                 365

Ser Gln Ala Leu Asn Pro Arg Pro Thr Asn His Ser Ala Gly Asn Ile
    370                 375                 380

Trp His Ser Ser Pro Leu Glu Pro Ile Lys Ser Gly Lys Asp Gly Lys
385                 390                 395                 400

Asp Ala Glu Ser Asn Ser Phe Tyr Gly Arg Leu Pro Arg Pro Ser Thr
                405                 410                 415

Thr Asp Thr His His His Gln Gln Gln Ala Ala Gly Arg His Ala Phe
            420                 425                 430

Ser Gly Pro Leu Arg Pro Ser Ser Thr Lys Pro Ile Thr Met Ala Asp
        435                 440                 445

Ser Tyr Ser Gly Ala Phe Cys Pro Leu Pro Thr Pro Pro Val Leu Gln
    450                 455                 460

Ser His Pro His Ser Ser Ser Ser Pro Arg Val Ser Pro Thr Ala Ser
465                 470                 475                 480

Pro Pro Pro Ala Ser Ser Pro Arg Leu Asn Glu Leu His Glu Leu Pro
                485                 490                 495

Arg Pro Pro Gly His Phe Ala Pro Pro Pro Arg Arg Ala Lys Ser Pro
            500                 505                 510

Gly Leu Val Gly His Ser Ala Pro Leu Thr Ala Trp Asn Gln Glu Arg
        515                 520                 525

Ser Thr Val Thr Val Ala Val Pro Ser Ala Thr Asn Ile Val Ala Ser
```

530 535 540

Pro Leu Pro Val Pro Pro Leu Val Val Pro Arg Ser Tyr Ser Ile Pro
545 550 555 560

Ser Arg Asn Gln Arg Val Val Ser Gln Arg Leu Val Glu Arg Arg Asp
565 570 575

Asp Ile Val Ala Ser Pro Pro Leu Thr Pro Met Ser Leu Ser Arg Pro
580 585 590

Leu Pro Gln Ala Thr Gly Val Ala Gln Thr Ser Gln Ile Arg Gly Tyr
595 600 605

<210> 65
<211> 586
<212> PRT
<213> Arabidopsis thaliana

<400> 65

Met Met Lys Ala Ser Phe Gly Arg Leu Arg Arg Phe Ala Leu Pro Lys
1 5 10 15

Ala Asp Ala Ile Asp Ile Gly Glu Leu Phe Pro Thr Ala Gln Ile Glu
20 25 30

Gly Leu Ala Arg Ala Ala Lys Asp Met Gln Asp Met Arg Glu Gly Tyr
35 40 45

Asp Arg Leu Leu Glu Val Ala Ala Ala Met Ala Asn Ser Ala Tyr Glu
50 55 60

Phe Ser Glu Ser Leu Gly Glu Met Gly Ser Cys Leu Glu Gln Ile Ala
65 70 75 80

Pro His Asn Asp Gln Glu Ser Gly Gly Ile Leu Leu Met Leu Gly Lys
85 90 95

Val Gln Phe Glu Leu Lys Lys Leu Val Asp Thr Tyr Arg Ser Gln Ile
100 105 110

Phe Lys Thr Ile Thr Arg Pro Ser Glu Ser Leu Leu Ser Asp Leu Arg
115 120 125

Thr Val Glu Asp Met Lys Gln Gln Cys Glu Glu Lys Arg Asp Val Val
130 135 140

Lys His Met Leu Met Glu His Val Lys Asp Lys Val Gln Val Lys Gly
145 150 155 160

Thr Lys Gly Glu Arg Leu Ile Arg Arg Gln Leu Glu Thr Ala Arg Asp
165 170 175

Glu Leu Gln Asp Glu Ala Thr Leu Cys Ile Phe Arg Leu Lys Ser Leu
180 185 190

Lys Glu Gly Gln Ala Arg Ser Leu Leu Thr Gln Ala Ala Arg His His
195 200 205

Thr Ala Gln Met His Met Phe Phe Ala Gly Leu Lys Ser Leu Glu Ala
210 215 220

Val Glu Gln His Val Arg Ile Ala Ala Asp Arg Gln His Ile Asp Cys
225 230 235 240

Val Leu Ser Asp Pro Gly Asn Glu Met Asp Cys Ser Glu Asp Asn Asp
245 250 255

Asp Asp Asp Arg Leu Val Asn Arg Asp Gly Glu Leu Ser Phe Asp Tyr
260 265 270

Ile Thr Ser Glu Gln Arg Val Glu Val Ile Ser Thr Pro His Gly Ser
275 280 285

Met Lys Met Asp Asp Thr Asp Leu Ser Phe Gln Arg Pro Ser Pro Ala
290 295 300

Gly Ser Ala Thr Val Asn Ala Asp Pro Arg Glu Glu His Ser Val Ser
305 310 315 320

Asn Arg Asp Arg Arg Thr Ser Ser His Ser Ala Pro Leu Phe Pro Asp
325 330 335

Lys Lys Ala Asp Leu Ala Asp Arg Ser Met Arg Gln Met Thr Pro Ser
340 345 350

Ala Asn Ala Tyr Ile Leu Pro Thr Pro Val Asp Ser Lys Ser Ser Pro
355 360 365

Ile Phe Thr Lys Pro Val Thr Gln Thr Asn His Ser Ala Asn Leu Trp
370 375 380

His Ser Ser Pro Leu Glu Pro Ile Lys Thr Ala His Lys Asp Ala Glu
385 390 395 400

Ser Asn Leu Tyr Ser Arg Leu Pro Arg Pro Ser Glu His Ala Phe Ser
405 410 415

Gly Pro Leu Lys Pro Ser Ser Thr Arg Leu Pro Val Pro Val Ala Val
420 425 430

Gln Ala Gln Ser Ser Ser Pro Arg Ile Ser Pro Thr Ala Ser Pro Pro
        435                 440                 445

Leu Ala Ser Ser Pro Arg Ile Asn Glu Leu His Glu Leu Pro Arg Pro
    450                 455                 460

Pro Gly Gln Phe Ala Pro Pro Arg Arg Ser Lys Ser Pro Gly Leu Val
465                 470                 475                 480

Gly His Ser Ala Pro Leu Thr Ala Trp Asn Gln Glu Arg Ser Asn Val
                485                 490                 495

Val Val Ser Thr Asn Ile Val Ala Ser Pro Leu Pro Val Pro Pro Leu
            500                 505                 510

Val Val Pro Arg Ser Tyr Ser Ile Pro Ser Arg Asn Gln Arg Ala Met
        515                 520                 525

Ala Gln Gln Pro Leu Pro Glu Arg Asn Gln Asn Arg Val Ala Ser Pro
    530                 535                 540

Pro Pro Leu Pro Leu Thr Pro Ala Ser Leu Met Asn Leu Arg Ser Leu
545                 550                 555                 560

Ser Arg Ser His Val Gly Glu Val Ala Gln Ser Gly Leu Ile Arg Gly
                565                 570                 575

Asn Gly Asn Lys Thr Asn Leu Leu Tyr Leu
            580                 585

<210> 66
<211> 305
<212> PRT
<213> Glycine max

<400> 66

Met Leu Pro Pro Ser Gln Ser Phe Ser Leu Trp Pro Ser Leu Thr Ser
1               5                   10                  15

Glu Leu Gly Leu Pro Ser His Leu Phe Leu Glu Asn Asp Ile Ile Pro
            20                  25                  30

Tyr Arg Thr Asn Gly Trp Ile Glu Gln Glu Pro Leu Gln Glu Asp Leu
        35                  40                  45

Leu Ile Leu Leu Ile Leu Met Thr Cys Cys Leu Thr Thr Phe Val Ser
    50                  55                  60

Phe Thr Val Gly Leu Gly Lys Leu Asn Ala Ser Glu Lys Leu Arg Gln

65 70 75 80

Met Arg Pro Ser Leu Ser Arg Lys Phe Ser Ser Tyr Val Leu Ala Thr
85 90 95

Pro Val Asp Ala Lys Ser Ser Thr Ser Ser Gly Ser Asn Asn Pro Lys
100 105 110

Pro Ser Asn Met Gln Ala Asn Leu Arg Glu Pro Thr Lys Asn Leu Trp
115 120 125

His Ser Ser Pro Leu Glu Gln Lys Lys His Asp Lys Asp Met Gly Asp
130 135 140

Glu Phe Ser Gly Ser Ile Ile Arg Ser Ala Gln Ser Leu Leu Lys Glu
145 150 155 160

Ser Asn Cys Leu Leu Gln His Ala Phe Ser Gly Pro Leu Thr Ser Asn
165 170 175

Leu Gly Pro Thr Asn Pro Val Asn Cys Ser Leu Ser Ser Ser Pro Lys
180 185 190

Val Ser Pro Ser Ala Ser Leu Thr Leu Met Thr Ser Pro Gln Ile Ser
195 200 205

Glu Leu His Glu Leu Pro Arg Pro Pro Thr Ser Phe Pro Ser Asn Ser
210 215 220

Arg Leu Leu Gly Leu Val Gly His Ser Gly Pro Leu Val Ser Arg Val
225 230 235 240

Ala Ala Leu His Val Pro Arg Thr Leu Glu Phe Ser His Arg Ser Ser
245 250 255

Ile Phe Glu Asn Ile Ala Ser Pro Pro Arg Met Pro Ile Glu Leu Ser
260 265 270

Ser Ile Ile Arg Trp Leu Gly Leu Thr Arg Thr Ala Ile Gly Ile Gly
275 280 285

His Leu Ser Leu Arg Lys Phe Tyr Leu Gln Ser Phe Gly Phe Ile Arg
290 295 300

Lys
305

<210> 67
<211> 628
<212> PRT

<213> M.truncatula

<400> 67

```
Met Lys Ser Ser Leu Lys Lys Leu Arg Gly Leu Ala Leu His Asn His
1               5                   10                  15

His His His His His His His His Lys His Asp Thr Ile Asn Asn Lys
            20                  25                  30

Thr Ile Leu Pro Leu Lys Gln Leu Asp Glu Leu Glu Lys Ala Thr Arg
        35                  40                  45

Glu Met Gln Asp Met Arg Asp Cys Tyr Asp Thr Leu Leu Ser Ala Ala
    50                  55                  60

Ala Ala Thr Ala Ser Ser Ala Tyr Glu Phe Ala Glu Ser Leu Arg Asp
65                  70                  75                  80

Met Gly Ser Cys Leu Leu Glu Lys Thr Ala Leu Asn Asp His Glu Glu
                85                  90                  95

Glu Thr Gly Lys Val Leu Leu Met Leu Gly Lys Ile Gln Phe Lys Leu
            100                 105                 110

Gln Lys Leu Ile Asp Asn Tyr Arg Ser His Ile Ile Gln Thr Ile Thr
        115                 120                 125

Val Pro Ser Glu Ser Leu Leu Asn Glu Leu Arg Ile Val Glu Glu Met
    130                 135                 140

Lys Arg Gln Cys Asp Glu Lys Arg Asp Val Tyr Glu Tyr Met Ile Ala
145                 150                 155                 160

Arg Tyr Arg Glu Arg Gly Arg Ser Lys Gly Gly Lys Gly Glu Thr Phe
                165                 170                 175

Thr Leu Gln Gln Leu Gln Ala Ala Arg Asp Glu Tyr Asp Glu Glu Ala
            180                 185                 190

Thr Leu Phe Val Phe Arg Leu Lys Ser Leu Lys Gln Gly Gln Ser Arg
        195                 200                 205

Ser Leu Leu Thr Gln Ala Ala Arg His His Ala Ser Gln Ser Cys Phe
    210                 215                 220

Phe Lys Lys Ala Ala Lys Ser Leu Glu Thr Val Glu Pro His Val Lys
225                 230                 235                 240

Ser Val Thr Glu Gln Gln His Ile Asp Tyr His Phe Ser Gly Leu Glu
                245                 250                 255
```

```
Glu Glu Asp Gly Asp Glu Gly Asp Tyr Val Asp Glu Asp Asp Glu Gly
            260                 265                 270

Tyr Asp Glu Asn Asp Asp Gly Glu Leu Ser Phe Asp Tyr Gly Pro Asn
        275                 280                 285

Glu Gln Glu Arg Asp Val Ser Thr Ser Arg Asn Ser Met Glu Leu Asp
    290                 295                 300

Gln Val Glu His Thr Leu Pro Arg Gly Ser Pro Ala Gly Gly Ala Lys
305                 310                 315                 320

Glu Asn Leu Asp Lys Leu Gln Arg Asn Leu Phe Ser Phe Lys Val Arg
                325                 330                 335

Ala Gly Ser Gln Ser Ala Pro Leu Phe Ala Asp Asn Lys Pro Asp Ser
            340                 345                 350

Ser Glu Lys Leu Arg Gln Met Arg Pro Ser Leu Ser Arg Lys Phe Ser
        355                 360                 365

Ser Tyr Val Leu Pro Thr Pro Val Asp Ala Lys Ser Pro Ile Ser Phe
    370                 375                 380

Phe Pro Asp Lys Pro Lys Pro Ser Thr Met Gln Thr Asn Leu Asn Glu
385                 390                 395                 400

Pro Thr Lys Asn Leu Trp His Ser Ser Pro Leu Asp Gln Lys Lys His
                405                 410                 415

Glu Lys Asp Ile Arg Asp Glu His Ser Asp Pro Thr Ile Arg Asn Thr
            420                 425                 430

Gln Ser Ala Leu Arg Glu Ser Asn Asn Asn Ala Ser Phe Thr Arg Leu
        435                 440                 445

Pro Leu Pro Leu Val Asp Gly Pro Ala Ser Leu Asn His Asp Asn Val
    450                 455                 460

Ser Ala Tyr Ser Lys Lys Ile Lys Arg His Ala Phe Ser Gly Pro Leu
465                 470                 475                 480

Thr Ser Asn Pro Trp Pro Thr Arg Pro Val Ser Met Glu Asn Ile Gln
                485                 490                 495

Leu Phe Ser Gly Pro Leu Leu Pro Thr Arg Ile Pro Gln Pro Pro Ser
            500                 505                 510
```

Ser Ser Pro Lys Val Ser Pro Ser Ala Ser Pro Thr Ile Leu Ser Ser
515 520 525

Pro Lys Ile Ser Glu Leu His Glu Leu Pro Arg Pro Pro Ala Asn Ser
530 535 540

Pro Pro Asn Ser Arg Leu Leu Gly Leu Met Gly His Ser Gly Pro Leu
545 550 555 560

Val Ser Arg Gly Gln Asn Val Ser Ala Ala Asn Asn Leu Val Val Ser
565 570 575

Ser Val Ala Ser Pro Leu Pro Met Pro Pro Gln Ala Met Ser Arg Ser
580 585 590

Phe Ser Ile Pro Ser Ser Gly Ser Ala Arg Val Ala Ala Leu Met Gly
595 600 605

Gln Gly Asp Glu Asn Pro Leu Ile His His Leu Tyr Pro Arg Lys Leu
610 615 620

Leu Leu Leu Arg
625

<210> 68
<211> 544
<212> PRT
<213> M.truncatula

<400> 68

Met Lys Ser Phe Asn Lys Leu Lys Arg Ile Ala Leu His Lys Thr Val
1 5 10 15

Gly Lys Glu Lys Lys Glu Phe Leu Pro Ser Val Lys Val Asp Glu Leu
20 25 30

Ala Leu Ala Ala Lys Tyr Met Gln Glu Met Arg Asp Cys Tyr Asp Ser
35 40 45

Leu Leu Ala Ala Ala Ala Ala Thr Glu Asn Ser Ala Tyr Glu Phe Ser
50 55 60

Glu Ser Leu Gln Glu Met Gly Thr Cys Leu Leu Glu Lys Thr Ala Leu
65 70 75 80

Asn Asp Asp Glu Glu Ser Gly Lys Val Leu Gly Met Leu Gly Asn Val
85 90 95

Gln Leu Asp Leu Gln Lys Leu Val Asp Gly Tyr Arg Ser His Val Ala
100 105 110

```
Leu Thr Ile Thr Arg Pro Ser Glu Ser Leu Leu Asn Glu Leu Arg Thr
            115                 120                 125

Val Glu Asp Met Lys Arg Gln Cys Asp Glu Lys Arg Glu Val Tyr Glu
    130                 135                 140

Tyr Met Ile Ala Gln Gln Lys Glu Lys Gly Lys Ser Lys Ser Gly Lys
145                 150                 155                 160

Gly Glu Asn Ile Thr Ser Gln His Leu Lys Ala Ala His Asp Glu Tyr
                165                 170                 175

Glu Glu Glu Ala Thr Leu Cys Ala Phe Arg Leu Lys Ser Leu Lys Gln
            180                 185                 190

Gly Gln Ser Arg Ser Leu Leu Thr Gln Ala Ala Arg His His Ala Ala
        195                 200                 205

Gln Leu Asn Phe Phe Arg Lys Gly Leu Lys Ser Leu Glu Ala Val Glu
    210                 215                 220

Pro His Val Arg Met Val Ala Glu Leu Gln His Ile Asp Tyr Gln Phe
225                 230                 235                 240

Ser Gly Leu Glu Asp Asp Asp Gly Glu Gly Ser Phe Glu Tyr Asp Gly
                245                 250                 255

Asn Glu Tyr Glu Tyr Glu Ala Thr Glu Gly Gly Glu Leu Ser Phe Asn
            260                 265                 270

Tyr Arg Ser Asn Lys Asp Val Pro Thr Ser Pro Asn Ser Ala Glu Val
        275                 280                 285

Glu Glu Ser Asp Arg Ser Asn Ile Arg Ala Ser Thr Thr Glu Thr Ala
    290                 295                 300

Glu Thr Ser Leu Asp Lys Ser His Val Asp Phe Lys Val Ser Asn Arg
305                 310                 315                 320

Asp Pro Pro Arg Val Ser Ser Tyr Ser Ala Pro Ile Phe Ala Glu Lys
                325                 330                 335

Lys Phe Asp Pro Ala Glu Lys Val Arg Gln Leu Leu Ser Ser Ser Ala
            340                 345                 350

Ala Lys Pro Asn Ala Tyr Val Leu Pro Leu Pro Val Asn Ile Lys Glu
        355                 360                 365

Thr Lys Thr Ala Pro Arg Leu Ser Ala Ser Ala Ser Ser His Asp Leu
```

370 375 380

Trp His Ser Ser Pro Leu Asp Glu Lys Lys Asn Gly Lys Asp Phe Ala
385 390 395 400

Asp Gly Lys Leu Ser Glu Pro Ala Ile Pro Arg Val Ser Ile Leu Lys
405 410 415

Glu Ser Asn Ser Asp Thr Ser Ser Ala Gln Leu Pro Arg Pro Ser Thr
420 425 430

Glu Gly Gln Ser Leu Pro Gln Val Asp Ile Phe Asn Ala Ser Asp His
435 440 445

Lys Lys Ile Lys Arg His Ala Phe Ser Gly Pro Leu Thr Asn Lys Pro
450 455 460

Leu Ser Val Lys Pro Val Ser Gly Gly Phe Ser Arg Leu Pro Met Pro
465 470 475 480

Gln Pro Ser Ser Pro Lys Ala Ser Pro Gly Ala Ser Pro Pro Leu Val
485 490 495

Ser Ser Pro Arg Ile Ser Glu Leu His Glu Leu Pro Arg Pro Pro Gly
500 505 510

Asn Gln Thr Ser Lys Ala Thr Lys Ser Ser Arg Phe Ser Ile Ser Thr
515 520 525

Ser Asn Ser Thr His Tyr Arg Leu Ser Glu Phe Phe His Thr Leu Lys
530 535 540

<210> 69
<211> 627
<212> PRT
<213> Oryza sativa

<400> 69

Met Lys Ser Ser Leu Arg Lys Leu Arg Gly Phe Ala Leu Gln Arg His
1 5 10 15

Glu Gln Arg Val Asp Arg Arg Gly Gly His Ser Pro Ala Ala Ala Ala
20 25 30

Ala Ala Asn Glu Leu Leu Ala Ala Ser Gln Asp Met Ala Asp Met Arg
35 40 45

Ser Cys Tyr Asp Asn Leu Leu Ser Val Ala Ala Ala Ile Ala Asn Ser
50 55 60

```
Ala Tyr Glu Phe Ser Glu Ala Leu Gln Glu Met Gly Thr Cys Leu Leu
65                  70                  75                  80

Lys Arg Val Thr Pro Asn Lys Asp Gly Ile Asn Asp Lys Val Leu Leu
                85                  90                  95

Leu Leu Gly Lys Ala Gln Phe Glu Leu Arg Lys Leu Val Asp Ser Tyr
            100                 105                 110

Arg Val His Val Leu Asn Thr Ile Thr Thr Pro Ser Gln Ser Leu Leu
        115                 120                 125

Asn Glu Leu Gln Thr Val Glu Glu Met Lys His Gln Cys Asp Glu Lys
    130                 135                 140

Arg Glu Leu Phe Glu Phe Leu Leu Asn Ala Gln Lys Glu Lys Gly Arg
145                 150                 155                 160

Ser Lys Asn Ala Lys Ser Asp Ile Gly Ala Ser Glu Gln Leu Lys Gln
                165                 170                 175

Ala Gln Asp Asp Tyr Gln Glu Glu Ala Thr Leu Phe Leu Phe Arg Leu
            180                 185                 190

Lys Ser Leu Lys Gln Gly Gln Phe Arg Ser Leu Phe Thr Gln Ala Ala
        195                 200                 205

Arg His His Ala Ala Gln Leu Asn Leu Phe Arg Lys Gly Leu Lys Ser
    210                 215                 220

Leu Glu Ala Val Glu Pro His Val Arg Leu Ala Ala Glu Gln Gln His
225                 230                 235                 240

Ile Asp His Gln Phe Ser Ala Leu Asp Glu Glu Asp Tyr Ser Val Asp
                245                 250                 255

Glu Glu Asn Glu Asp Asp Tyr Asn Asp Ser His Glu Asp Leu Ser Phe
            260                 265                 270

Asp Tyr Gly Glu Asn Lys Glu Gly Thr Glu Ala Gly His Ala Ser Arg
        275                 280                 285

Ser Pro Thr Glu Glu Leu Leu Asp Arg Ser Lys Ala Glu Tyr Ser Ser
    290                 295                 300

Phe Pro Gly Glu Arg Gln Arg Ser Gly Ser Gln Ser Ala Pro Leu Phe
305                 310                 315                 320

Pro Glu Lys Lys Leu Glu Ala Ala Glu Arg Ile Lys Glu Leu Arg Arg
                325                 330                 335
```

Ser Ala Thr Arg Lys Leu Tyr Thr Tyr Val Leu Pro Thr Pro Asn Asp
340 345 350

Val Arg Asp Thr Ser Gln Thr Val Thr Ala Asn Pro Thr Ser Gly Ser
355 360 365

Pro Leu Gly Asn Lys Gly Ala Phe Tyr Ser Ser Pro Leu Gln Pro Ser
370 375 380

Thr Asn Val Gly Asp Leu Arg Asp Asn Lys Leu Pro Ser Pro Thr Arg
385 390 395 400

Leu Ser Asn Ala His Ser Val Leu Lys Glu Ser Asn Thr Asn Thr Thr
405 410 415

Asp Thr Arg Thr Met Leu Val Leu Pro Leu Gly Asp Leu Ser Leu Pro
420 425 430

Gly Tyr His Asp Ser Lys Ala Ser Asp Asn Lys Lys Val Lys Arg Gly
435 440 445

Ser Phe Ser Gly Pro Ile Val Pro Arg Ser Arg Ser Thr Glu Asn Ile
450 455 460

Asp Val Val Ser Val Pro Pro Arg His Ser Ser Ser His Gln Pro Ser
465 470 475 480

Ile His Val Arg Val Ser Pro Asn Thr Ser Pro Pro Leu Leu Ser Ser
485 490 495

Pro Lys Ile Lys Glu Leu His Glu Leu Pro Arg Pro Pro Ala Asn Ala
500 505 510

Ser Lys His Thr Thr Phe Pro Ser Leu Val Ala His Ser Ala Pro Leu
515 520 525

Val Pro Asn Ser Ala Pro Leu Ala Pro Arg Gly Gln Asp His Phe Arg
530 535 540

Pro Arg Gln Thr Pro Pro Ser Ala Pro Gln Thr Ala Ser Pro Leu Pro
545 550 555 560

Thr Pro Pro Gly Pro Ile Ser Arg Ser Phe Ser Ile Pro Ser Arg Gly
565 570 575

Met Arg Thr Ser Gly Ile Ser Asp Gly Lys Glu Thr Glu Asp Leu Gln
580 585 590

Asp Lys Gly Pro Ala Arg Met Ser Leu Ser Ser Leu Pro Ser Ala Gln
595 600 605

Thr Ser Leu Glu Asp His Arg Pro Leu Ser Gly Ala Thr Glu Ser Val
610 615 620

Ser Lys Thr
625

<210> 70
<211> 624
<212> PRT
<213> Oryza sativa

<400> 70

Met Lys Ser Pro Leu Arg Lys Phe Arg Gly Phe Gly Leu His Ser His
1 5 10 15

Arg Glu Arg Lys Asp His Arg Pro Pro Pro Ala Lys Leu Asp Glu Leu
20 25 30

Ala Asp Ala Ala Gln Glu Met Glu Glu Met Arg Asn Cys Tyr Asp Ser
35 40 45

Leu Leu Ser Ala Ala Ala Ala Thr Met Asn Ser Val Tyr Glu Phe Ala
50 55 60

Glu Ala Met Glu Glu Met Gly Thr Cys Leu Leu Glu Lys Thr Ala Leu
65 70 75 80

Asn Tyr Asp Asp Asp Asp Ser Gly Arg Val Leu Met Met Leu Gly Lys
85 90 95

Ala Gln Phe Glu Leu Gln Lys Phe Val Asp Asn Tyr Arg Thr Asn Ile
100 105 110

Ile Asn Thr Ile Thr Asn Pro Ser Glu Ser Leu Leu Lys Glu Leu Gln
115 120 125

Val Val Glu Glu Met Lys Glu Leu Cys Asp His Lys Arg Gln Glu Tyr
130 135 140

Glu Ala Met Arg Ala Ala Tyr Arg Glu Lys Gly Arg Ser Arg His Ser
145 150 155 160

Lys Thr Glu Thr Leu Ser Ser Glu Gln Leu Gln Ala Tyr Phe Leu Asp
165 170 175

Tyr Gln Glu Asp Ala Ala Leu Phe Ile Phe Arg Leu Lys Ser Leu Lys
180 185 190

```
Gln Gly Gln Phe Arg Ser Ile Leu Thr Gln Ala Ala Arg His His Ser
        195                 200                 205

Ala Gln Leu Ser Phe Phe Arg Arg Gly Leu Lys Tyr Leu Glu Ala Leu
    210                 215                 220

Glu Pro His Val Lys Ala Val Ala Glu Lys Gln His Ile Glu Tyr Pro
225                 230                 235                 240

Leu Asn Gly Leu Asp Asp Asp Thr Asp Asn Asp Glu Tyr Ser Ser Tyr
                245                 250                 255

Gln Gly Asn Gln Ser Asp Asp Ser Glu Leu Ser Phe Asp Tyr Glu Ile
            260                 265                 270

Asn Asp Arg Asp Lys Asp Phe Pro Ala Ser Arg Ser Ser Met Asp Leu
        275                 280                 285

Asp Gln Ser Asn Gln Ala Cys Ser Pro Glu Pro Leu Lys Glu His Lys
    290                 295                 300

Gln Glu Tyr Thr Glu Gln Ile Gln Ala Asp Phe Ala Ala Pro Arg Val
305                 310                 315                 320

Lys Leu Glu Ile Gly Thr Gln Ser Ala Pro Ile Ser Ala Asp Asn Val
                325                 330                 335

Phe Asp Pro Ser Thr Arg Phe Arg Lys Met Asn Thr Ser Asn Arg Thr
            340                 345                 350

Asn Tyr Ser Tyr Lys Leu Pro Thr Pro Asp Asp Asp Lys Asn Ser Thr
        355                 360                 365

Ser Ala His Thr Asn Arg Ser Pro His Ser Asp Gln Pro Glu Ser Lys
    370                 375                 380

Ser His Val Ala Glu Asn Leu Trp His Ser Ser Pro Leu Val Lys Gly
385                 390                 395                 400

Phe Lys Pro Asn Ser Met Phe Ser Gly Pro Val Lys Met Pro Ser Ser
                405                 410                 415

Thr Glu Gly Ile Ser Ala Pro Leu Val Tyr Pro Tyr Ala Thr Ser Asp
            420                 425                 430

Phe Lys Lys Met Lys Arg Glu Ala Phe Ser Gly Pro Ile Pro Ser Lys
        435                 440                 445

Ala Gly Leu Asn Lys Pro Leu Phe Ser Ala Thr Asp Leu Arg Ala Pro
```

450 455 460

Met Asn Tyr Pro Arg Ala Met Ser Thr Lys Ser Tyr Gly Pro Gly Trp
465 470 475 480

Gln Ser Ser Val Ala Pro Lys Phe Thr Pro Arg Ile Thr Ser Leu Pro
485 490 495

Thr Thr Ser Pro Arg Ile Ser Glu Leu His Glu Leu Pro Arg Pro Pro
500 505 510

Ala Asn Val Gly Ala Ala Arg Pro Gly Leu Val Gly Tyr Ser Gly Pro
515 520 525

Leu Val Ser Arg Arg Gln Val Pro Asn Val Pro Thr Arg Ala Ser Pro
530 535 540

Pro Ser Gln Thr Ala Ser Pro Leu Pro Arg Pro Pro Ala Ala Met Thr
545 550 555 560

Arg Ser Tyr Ser Ile Pro Ser Asn Ser Gln Arg Thr Pro Ile Leu Thr
565 570 575

Val Asn Lys Leu Leu Glu Ala Arg His Ser Arg Glu Ser Ser Glu Val
580 585 590

Ser Ser Pro Pro Leu Thr Pro Ile Ser Leu Ala Asp Val Ser Arg Arg
595 600 605

Ser Thr Ala Glu Thr Ala Leu Glu Lys Thr Arg Met Met Glu Thr Leu
610 615 620

<210> 71
<211> 623
<212> PRT
<213> Oryza sativa

<400> 71

Met Lys Ser Pro Leu Leu Arg Leu Lys Gly Phe Gly His His Gln Gln
1 5 10 15

His Arg Glu Arg Lys Ser Arg Gln Pro Gln Pro Gln Pro Thr Pro Ala
20 25 30

Lys Leu Asp Glu Leu Ala Asp Ala Ala Gln Asp Val Glu Glu Met Arg
35 40 45

Asn Cys Tyr Asp Gly Phe Ile Ser Ala Ala Ala Ala Thr Thr Asn Gly
50 55 60

```
Val Tyr Glu Phe Ala Glu Ala Leu Glu Glu Leu Gly Ser Cys Leu Leu
65                  70                  75                  80

Ala Lys Pro Val Leu Asn Asp Asp Asp Asp Asp Ser Gly Arg Val Leu
                85                  90                  95

Met Met Leu Gly Lys Ala Gln Tyr Glu Leu Gln Lys Ser Ala Asp Arg
            100                 105                 110

Tyr Arg Thr Asn Ile Ile His Thr Ile Thr Thr Pro Ser Glu Ser Leu
        115                 120                 125

Leu Lys Glu Leu Gln Thr Leu Glu Glu Met Lys Gln Gln Cys Asp Met
    130                 135                 140

Lys Arg Asp Ala Tyr Glu Thr Met Arg Ala Ser Tyr Ser Asp Lys Gly
145                 150                 155                 160

Gly Ser Arg His Ser Lys Thr Glu Ser Phe Ser Thr Glu Gln Leu Asp
                165                 170                 175

Ala Ser Phe Leu Glu Tyr Gln Glu Asp Ser Ala Leu Phe Thr Phe Arg
            180                 185                 190

Leu Lys Ser Leu Lys Gln Gly Gln Phe Gln Ser Leu Leu Thr Gln Ala
        195                 200                 205

Ala Arg His His Ala Ala Gln Leu Ser Phe Phe Arg Lys Gly Leu Lys
    210                 215                 220

Cys Leu Glu Ala Leu Glu Pro Arg Val Lys Ala Ile Ser Glu Lys His
225                 230                 235                 240

His Ile Asp Tyr Asn Phe Ser Gly Leu Glu Asp Asp Gly Ser Asp Asn
                245                 250                 255

Asp Gly Tyr Ser Thr Tyr Asp Ser Cys Ser Asp Asp Gly Glu Leu Ser
            260                 265                 270

Phe Asp Tyr Glu Ile Asn Asp Arg Asp Gln Asp Phe Leu Thr Ser Arg
        275                 280                 285

Gly Ser Met Asp Phe Asp Lys Ser Asp Gln Thr Thr Ser Pro Lys Pro
    290                 295                 300

Ile Lys Glu Asn Lys Gln Glu Glu Ala Lys Gln Ala Glu Ala Glu Ile
305                 310                 315                 320

Val Phe Pro Gln Leu Lys Pro Glu Phe Ala Thr His Ser Ala Pro Leu
                325                 330                 335
```

```
Phe Ala Gly Asn Leu Leu Asp Glu Thr Asp Arg Leu Arg Gln Met Arg
            340                 345                 350

Pro Ser Ser Thr Lys His Ser Tyr Arg Leu Pro Thr Pro Val Gly Ala
        355                 360                 365

Asp Asn Pro Val Pro Ser Gly Ser His Arg Leu His His Ser Ala Gln
    370                 375                 380

Phe Phe Glu Thr Lys Pro His Ala Pro Thr Asn Leu Trp His Ser Ser
385                 390                 395                 400

Pro Leu Thr Lys Asp Tyr Asn Gly Ala Met His Asn Ala Ala Thr Lys
                405                 410                 415

Pro Ser Ser Ser Ser Ser Thr Asp Asp Leu Lys Lys Leu Lys Arg Glu
            420                 425                 430

Ser Trp Ser Gly Pro Ile Pro Ile Lys Ala Gly Ser Gly Gly Lys Pro
        435                 440                 445

Phe Ser Gln Ala Asp His Arg Pro Ser Pro Thr Met Ala Tyr Pro Gly
    450                 455                 460

Ala Met Pro Ala Ala Lys Pro His Val Arg His Ala Ser Ser Ser Ser
465                 470                 475                 480

Val Ser Pro Lys Val Ser Pro Lys Met Ser Pro Val Pro Pro Ala Ser
                485                 490                 495

Ser Leu Lys Ile Ser Glu Leu His Leu Leu Pro Leu Pro Pro Ala Asn
            500                 505                 510

Val Asp Pro Val Arg Pro Ser Gly Leu Val Gly Tyr Ser Gly Pro Leu
        515                 520                 525

Val Ser Lys Arg Ala Pro Thr Pro Ala Arg Ala Ser Pro Lys Ala Ser
    530                 535                 540

Arg Thr Ala Ser Pro Leu Pro Arg Pro Pro Ala Ala Leu Ala Arg Ser
545                 550                 555                 560

Tyr Ser Ile Pro Ser Asn Ser Gln Arg Thr Pro Ile Ile Thr Val Asn
                565                 570                 575

Lys Leu Leu Glu Ala Lys His Ser Arg Glu Gly Ser Asp Ala Ser Ser
            580                 585                 590
```

Pro Pro Leu Thr Pro Leu Ser Leu Ser Asp Leu Cys His Gln Glu Lys
595 600 605

Ala Gly Lys Ala Ala Ala Gly Asn Thr Arg Arg Lys Glu Thr Leu
610 615 620

<210> 72
<211> 625
<212> PRT
<213> P.trichocarpa

<400> 72

Met Lys Asn Lys Leu Arg Gly Phe Gly Leu Lys Arg Ser Glu Thr Lys
1 5 10 15

Glu Lys Ile Asp Leu Leu Pro Pro Ala Gln Leu Asp Glu Leu Ala Gln
20 25 30

Ala Ala Arg Asp Met Gln Asp Met Arg Asn Cys Tyr Asp Ser Leu Leu
35 40 45

Phe Ala Ala Ala Ala Thr Ala Asn Ser Ala Tyr Glu Phe Ser Glu Ser
50 55 60

Leu Arg Glu Met Gly Ser Cys Leu Leu Glu Lys Thr Ala Leu His Asp
65 70 75 80

Asp Glu Glu Ser Gly Lys Val Leu Leu Met Leu Gly Asn Val Gln Phe
85 90 95

Glu Leu Gln Lys Leu Val Asp Ser Tyr Arg Ser His Ile Phe Leu Thr
100 105 110

Ile Thr Asn Pro Ser Glu Ser Leu Leu Asn Glu Leu Arg Thr Val Glu
115 120 125

Asp Met Lys Arg Gln Cys Asp Glu Lys Arg Asn Val Tyr Glu Tyr Met
130 135 140

Val Ala Gln Gln Lys Asp Lys Gly Arg Ser Lys Gly Gly Lys Asp Glu
145 150 155 160

Ser Thr Thr Leu Gln Gln Leu Arg Ser Ala Arg Glu Glu Tyr Asp Glu
165 170 175

Glu Ala Thr Leu Cys Val Phe Arg Leu Lys Ser Leu Lys Gln Gly Gln
180 185 190

Ser Arg Ser Leu Leu Thr Gln Val Ala Arg His His Ala Ala Gln Leu
195 200 205

```
Asn Phe Phe Gln Lys Gly Leu Lys Ser Leu Glu Thr Val Glu Pro His
    210                 215                 220

Val Arg Leu Ile Thr Glu His Gln His Ile Asp Tyr His Phe Ser Gly
225                 230                 235                 240

Leu Glu Asp Asp Gly Arg Glu Asp Gly Glu Asp Tyr Gly Glu Asp Val
                245                 250                 255

Asp Asp Thr Tyr Glu Gly Arg Glu Leu Ser Phe Asp Tyr Arg Ala Asn
            260                 265                 270

Asn Gln Gly His Ala Val Ser Ala Ala Arg Asn Ser Met Glu Val Asp
        275                 280                 285

Glu Glu Asp Leu Ser Phe Pro Gln Ala Pro Ala Ala Glu Asn Val Glu
    290                 295                 300

Leu Asn Pro Asp Lys Thr Pro Gly Gly Phe His Phe Pro Ile Arg Glu
305                 310                 315                 320

Pro Arg Gly Gly Ser His Ser Ala Pro Ile Phe Pro Glu Arg Lys Pro
                325                 330                 335

Asp Pro Val Glu Arg Ile Arg Gln Ile Gln Lys Ser Ser Arg Lys Ser
            340                 345                 350

Asn Thr Tyr Val Leu Pro Thr Pro Val Asp Ala Lys Gly Val Ile Ser
        355                 360                 365

Ser Arg Ala Ser Gly Ser Val Pro Asn Thr Arg Gln Ile Asp Ile Ser
    370                 375                 380

Gly Arg Thr His Tyr Leu Ser His Ser Ser Pro Leu Glu Gln Lys Lys
385                 390                 395                 400

Asn Glu Lys Asp Ser Gly Asp Gly His Leu Pro Glu Phe Thr Pro Ser
                405                 410                 415

Lys Glu Arg Ser Gly His Lys Glu Ser Asn Asn Pro Asn Ala Ser Thr
            420                 425                 430

Gln Leu Pro Arg Pro Leu Val Gly Gly Ile Ser Phe Pro Gln Leu Asp
        435                 440                 445

Val Tyr Asn Ala Ser Asp Asn Lys Lys Ile Lys Arg Gln Ser Phe Ser
    450                 455                 460

Gly Pro Ile Thr Ser Lys Pro Trp Ser Met Lys Leu Gly Leu Ser Ser
```

465 470 475 480

Ser Ser Gly Pro Ile Ser Ala Thr Glu Leu Ser Gln Glu Pro Ser Thr
485 490 495

Ser Pro Lys Val Ser Pro Ser Thr Ser Pro Pro Leu Val Ser Ser Pro
500 505 510

Arg Ile Ser Glu Leu His Glu Leu Pro Arg Pro Pro Gly Asn Leu Ala
515 520 525

Ala Lys Ala Ala Lys Ser Leu Gly Pro Ile Gly His Ser Ala Pro Leu
530 535 540

Val Arg Asn Pro Glu Leu Ser Gly Thr Asn Lys Ile Ser Ser Gly Ala
545 550 555 560

Ala Asn Leu Ala Ser Pro Leu Pro Thr Pro Pro Leu Met Val Pro Thr
565 570 575

Ser Phe Ser Ile Pro Ser Ile Ser Pro Arg Thr Met Ser Val His Val
580 585 590

Ser Lys Leu Leu Val Ser Ser Gln Leu Leu Asp Lys Pro Gly Glu Leu
595 600 605

Arg Val Ala Leu Met Leu Ser Asp Ser Pro Pro Pro Pro Ile Phe Ile
610 615 620

Glu
625

<210> 73
<211> 204
<212> PRT
<213> P.trichocarpa

<400> 73

Met Asp Phe Phe Phe Ser Arg Leu Glu Pro Pro Ala Pro Ala Glu Ser
1 5 10 15

Lys Lys Ile Lys Arg Tyr Ala Leu Ser Gly Pro Ile Thr Ser Gln Pro
20 25 30

Trp Ser Thr Lys Ala Val Ser Ala Glu Asn Pro Gln Leu Phe Ser Gly
35 40 45

Pro Leu Leu Arg Asn Gln Thr Ala Gln Leu Phe Ser Ser Pro Pro Lys
50 55 60

Val Ser Pro Arg Ile Ser Pro Lys Val Ser Pro Ser Ser Ser Pro Pro
65 70 75 80

Phe Val Ser Ser Pro Lys Ile Ser Glu Leu His Glu Leu Pro Arg Pro
85 90 95

Pro Val Thr Ser Thr Ser Lys Ser Pro Gly Ala Val Gly Leu Val Gly
100 105 110

His Ser Ala Pro Leu Leu Pro Lys Gly His Met Leu Pro Gly Thr Ser
115 120 125

Lys Thr Ser Ala Ser His Val Glu Ser Gln Leu Pro Thr Pro Ser Gln
130 135 140

Val Val Pro Arg Ser Phe Ser Ile Pro Ser Ser Arg His Arg Val Met
145 150 155 160

Val Ala Gln Asn Ser Gly Ile Val Glu Asn Val Ala Ser Pro Pro Leu
165 170 175

Thr Pro Ile Ser Leu Ser Asn Thr Gln Pro Ser Ser Thr Gly Ser Pro
180 185 190

Ile Val Asn Gln Thr Val Gln Ile Arg Gly Asn Ser
195 200

<210> 74
<211> 203
<212> PRT
<213> P.trichocarpa

<400> 74

Met Asp Thr Cys Phe His Gly Leu Asn His Trp Leu Leu Leu Ile Leu
1 5 10 15

Arg Lys Pro Glu Asp Met Pro Phe Leu Val Leu Leu Gln Ala Ser His
20 25 30

Cys Leu Pro Ser Trp Ser Gln Gln Asn Ile Pro Asn Cys Ser Leu Asp
35 40 45

Pro Phe Cys Glu Ile Gln Leu Thr Gln Leu Leu Ser Pro Pro Lys Val
50 55 60

Ser Pro Ile Ile Ser Pro Lys Val Ser Pro Ser Ala Ser Pro Thr Phe
65 70 75 80

Val Ser Pro Pro Lys Ile Ser Glu Leu His Glu Leu Pro Arg Pro Pro
85 90 95

```
Leu Ser Ser Thr Ser Lys Ser Pro Arg Ala Glu Gly Leu Val Gly His
            100                 105                 110

Ser Thr Pro Leu Leu Pro Lys Gly Arg Met His Pro Gly Thr Arg Lys
        115                 120                 125

Thr Pro Ala Ser Asn Val Ala Ser Gln Leu Pro Thr Pro Ser Gln Val
    130                 135                 140

Val Thr Arg Ser Phe Ser Ile Pro Ser Arg Ser Arg Arg Ile Met Val
145                 150                 155                 160

Ala Gln Ser Ser Gly Ile Ala Glu Asp Val Ala Ser Pro Pro Leu Thr
                165                 170                 175

Pro Ile Ser Leu Cys Asn Asn Tyr Pro Ser Ser Thr Gly Ser His Thr
            180                 185                 190

Val Asn Gln Thr Val Gln Ile Arg Gly Ala Asp
        195                 200


<210>  75
<211>  179
<212>  PRT
<213>  P.trichocarpa

<400>  75

Met Lys Leu Ala Leu Ser Ser Ser Gly Pro Ile Ser Ala Thr Glu Leu
1               5                   10                  15

Ser Gln Gln Val Ser Gly Val Leu Ala His Val Ala Asn Pro Gln Pro
            20                  25                  30

Ser Thr Ser Pro Lys Val Ser Pro Ser Thr Ser Pro Pro Leu Val Ser
        35                  40                  45

Ser Pro Arg Ile Ser Glu Leu His Glu Leu Pro Arg Pro Pro Gly Asn
    50                  55                  60

Leu Ala Ala Lys Ala Ala Lys Ser Leu Gly Leu Ile Gly His Ser Ala
65                  70                  75                  80

Pro Leu Val Arg Asn Pro Glu Leu Ser Gly Thr Asn Lys Ile Ser Ser
                85                  90                  95

Gly Ala Val Asn Leu Ala Ser Pro Leu Pro Met Pro Pro Leu Met Val
            100                 105                 110

Pro Thr Ser Phe Ser Ile Pro Ser Ile Ser Pro Arg Thr Met Thr Val
        115                 120                 125
```

His Val Ser Lys Leu Leu Asp Ser Ser Gln Leu Leu Glu Lys Pro Gly
130 135 140

Glu Val Asp Ser Pro Pro Leu Thr Pro Ile Ser Leu Thr Asn Met Arg
145 150 155 160

Gln Ala Pro Ala Ile Ser Glu Ser Ile Pro Gln Ser Gly Gln Ile Arg
165 170 175

Gly Gly Ser

<210> 76
<211> 132
<212> PRT
<213> T.aestivum

<220>
<221> MISC_FEATURE
<222> (127)..(127)
<223> unkown amino acid

<400> 76

Met Lys Ser Leu Lys Gln Gly Gln Phe Leu Ser Ile Leu Thr Gln Ala
1 5 10 15

Ala Arg His His Ala Ala Gln Leu Ser Phe Phe Arg Lys Gly Leu Lys
20 25 30

Tyr Leu Glu Ala Leu Glu Pro His Val Lys Ala Val Ala Glu Lys Gln
35 40 45

His Ile Asp Tyr His Phe Ser Gly Leu Asp Asp Asp Thr Asp Asn Asp
50 55 60

Asp Tyr Ser Ser Tyr Gln Asp Asn His Ser Glu Gly Ser Glu Leu Ser
65 70 75 80

Phe Asp Tyr Gly Ile Asn Phe Pro Ala Ser Arg Ser Ser Met Asp Leu
85 90 95

Asp Ser Asn Met Ala Ser Pro Thr Lys Pro Leu Lys Glu His Glu Gln
100 105 110

Glu His Gly Lys Gln Ile Glu Thr Asp Leu Pro Val Leu Gln Xaa Lys
115 120 125

Pro Glu Phe Gly
130

<210> 77
<211> 279
<212> PRT
<213> T.aestivum

<400> 77

Met Phe Ser Ser Pro Leu Gln Ser Ser Arg Thr Asn Tyr Ser Tyr Lys
1 5 10 15

Leu Pro Thr Pro Ala Asp Asp Lys Ile Cys Thr Ser Ala Gly Thr Asn
20 25 30

Arg Ser Pro His Ser Asp Lys Pro Glu Ser Ser His Val Ala Ala Asn
35 40 45

Leu Trp His Ser Ser Pro Leu Val Lys Asp Tyr Lys Pro Ser Ser Leu
50 55 60

Tyr Ser Gly Pro Val Lys Met Pro Ser Ser Thr Glu Arg Arg Ser Ser
65 70 75 80

Pro Leu Val Tyr Ser Tyr Ser Thr Ser Asp Ser Lys Lys Met Lys Arg
85 90 95

Glu Ser Phe Ser Gly Pro Ile Pro Ser Lys Ala Gly Leu Ser Lys Pro
100 105 110

Leu Phe Ser Ala Ala Gly His Arg Ala Ser Val Asn Tyr Pro Pro Arg
115 120 125

Val Met Ser Thr Lys Ser His Gly Pro Gly Ser Leu Pro Ser Val Ala
130 135 140

Pro Lys Val Pro Arg Ile Thr Ser Leu Pro Thr Thr Ser Pro Arg Ile
145 150 155 160

Ser Glu Leu His Glu Leu Pro Arg Pro Pro Ala Pro Leu Asp Thr Leu
165 170 175

Arg Pro Gly Leu Val Gly Tyr Ser Gly Pro Leu Val Ser Arg Arg Gln
180 185 190

Ile Pro Thr Arg Ala Ser Pro Pro Ser Asn Thr Ala Ser Pro Leu Pro
195 200 205

Arg Pro Pro Ala Ala Met Thr Arg Ser Tyr Ser Ile Pro Ser Asn Ser
210 215 220

Gln Arg Thr Pro Ile Ile Thr Val Asn Lys Leu Leu Glu Ser Arg His
225 230 235 240

Ser Arg Glu Ser Ser Glu Val Ser Ser Pro Pro Leu Thr Pro Ile Ser
245 250 255

Leu Ala Asp Val Ser Arg Lys Glu Ile Ala Glu Thr Thr Ile Asp Thr
260 265 270

Arg Arg Met Lys Glu Thr Ser
275

<210> 78
<211> 150
<212> PRT
<213> T.aestivum

<400> 78

Met Ile Ser Lys Ser Cys Val His Ala Arg Gln Pro Ser Pro Val Ser
1 5 10 15

Pro Lys Met Phe Pro Pro Ser Ile Glu Ser Pro Lys Ile Ser Glu Leu
20 25 30

His Glu Leu Pro Arg Pro Pro Ala Asn Val Glu Pro Leu Arg Pro Cys
35 40 45

Gly Leu Val Gly Tyr Ser Gly Pro Leu Val Ser Lys Arg Gln Ala Pro
50 55 60

Thr Ala Pro Val Arg Ala Ser Pro Thr Ala Ser Gln Thr Ala Ser Pro
65 70 75 80

Leu Pro Arg Pro Pro Ala Ser Leu Ala Arg Ser Phe Ser Ile Pro Ser
85 90 95

Asn Ser Gln Arg Thr Pro Leu Ile Thr Val Asn Lys Leu Leu Glu Ala
100 105 110

Arg Ser Ser Arg Glu Ser Ser Glu Ile Ser Ser Pro Pro Leu Thr Pro
115 120 125

Leu Phe Ser Ser Thr Ser Gln His Lys Lys Gln Leu Lys Ala Ala Leu
130 135 140

Gly Glu Lys Gly Met Ser
145 150

<210> 79
<211> 432
<212> PRT
<213> Zea mays

<400> 79

```
Met Lys Ser Ser Leu Arg Lys Leu Arg Gly Phe Ala Leu Gln Arg Gln
1               5                   10                  15

Glu Gln Arg Val Asp Arg Asp Arg Gly Arg Gly His Ala Thr Ala Ala
            20                  25                  30

Ala Thr Ala Ala Asp Glu Leu Leu Ala Ala Ala Gln Asp Met Ala Asp
        35                  40                  45

Met Arg Ser Cys Tyr Asp Asn Leu Leu Ser Val Ala Ala Ala Ile Ala
    50                  55                  60

Asn Ser Ala Tyr Glu Phe Ser Glu Ala Leu Gln Glu Met Gly Thr Cys
65                  70                  75                  80

Leu Leu Lys Arg Val Thr Pro Asn Lys Asp Gly Ile Asn Asp Lys Val
                85                  90                  95

Leu Leu Leu Leu Gly Lys Ser Gln Phe Glu Leu Arg Lys Leu Leu Asp
            100                 105                 110

Ser Tyr Arg Val His Val Leu Asn Thr Ile Thr Thr Pro Ser Leu Ser
        115                 120                 125

Leu Leu Asn Glu Leu Gln Thr Val Glu Glu Met Lys His Gln Cys Asp
    130                 135                 140

Glu Lys Lys Glu Leu Tyr Glu Phe Met Val Asn Thr Gln Lys Glu Lys
145                 150                 155                 160

Gly Arg Ser Lys Asn Ala Lys Gly Asp Asn Gly Ala Ser Glu Gln Leu
                165                 170                 175

Lys Gln Ala Gln Glu Asp Tyr Gln Glu Glu Ala Thr Leu Phe Leu Phe
            180                 185                 190

Arg Leu Lys Ser Leu Lys Gln Gly Gln Phe Arg Ser Leu Phe Thr Gln
        195                 200                 205

Ala Ala Arg His His Ala Ala Gln Leu Asn Leu Phe Arg Lys Gly Val
    210                 215                 220

Lys Ser Leu Glu Ala Val Glu Pro His Val Arg Leu Ala Ala Glu Gln
225                 230                 235                 240

Gln His Ile Asp His Gln Phe Ser Ala Leu Glu Glu Glu Asp Tyr Leu
                245                 250                 255
```

```
Val Glu Asp Glu Asn Asp Asp Asp Tyr Asn Gly Ser His Asp Gly Glu
            260                 265                 270

Leu Ser Phe Asp Tyr Gly Glu Asn Lys Glu Val Glu Glu Ser Gly Asn
        275                 280                 285

Ala Ser Arg Asn His Thr Glu Gly Phe Phe Asn Arg Ser Lys Glu Glu
    290                 295                 300

Tyr Ser Ser Val Pro His Glu Arg Gln Arg Ile Val Ser Gln Ser Ala
305                 310                 315                 320

Pro Leu Phe Pro Glu Lys Lys Leu Asn Thr Glu Glu Arg Ile Lys Asp
                325                 330                 335

Leu Arg Arg Ser Ala Thr Arg Lys Leu Asn Thr Tyr Val Leu Pro Thr
            340                 345                 350

Pro Asn Asp Val Gly Ala Thr Ser Gln Arg Ala Ser Gly Asn Pro Thr
        355                 360                 365

Ser Glu Pro Leu Glu Ser Lys Gly Ala Phe His Ser Ser Pro Leu His
    370                 375                 380

Ser Ser Ala Asp Ile Arg Asp Leu Gly Asp Ser Asn Leu Pro Ser Pro
385                 390                 395                 400

Thr Arg Leu Ser Asn Val Gln Ser Val Leu Lys Asp Ser Asn Thr Asn
                405                 410                 415

Lys Thr Glu Thr Arg Lys Val Leu Pro Val Gly Asp Leu Gly Phe Thr
            420                 425                 430


<210>  80
<211>  210
<212>  PRT
<213>  Zea mays

<400>  80

Met Pro Asn Ser Met His Ser Gly Pro Val Lys Met Pro Ser Ser Asn
1               5                   10                  15

Glu Gly Val Ser Leu Ala Tyr Ser Tyr Ser Thr Thr Asp Phe Lys Lys
            20                  25                  30

Val Lys Arg Glu Ala Phe Ser Gly Pro Ile Pro Ser Lys Val Gly Leu
        35                  40                  45

Ser Thr Pro Leu Ile Ser Ala Thr Asp Arg Lys Pro Ser Gly Lys Arg
    50                  55                  60
```

```
Pro Ser Tyr Val Leu Pro Thr Arg Pro Pro Gly Pro Gly Trp Gln Ser
65                  70                  75                  80

Ser Val Pro Pro Lys Ala Thr Pro Arg Val Thr Ser Leu Pro Thr Thr
                85                  90                  95

Thr Pro Arg Ile Ser Glu Leu His Glu Leu Pro Arg Pro Pro Ala Asn
            100                 105                 110

Ala Gly Leu Val Gly Tyr Ser Gly Pro Leu Val Ser Arg Arg Pro Met
        115                 120                 125

Pro Thr Ala Val Ser Thr Thr Arg Val Ser Pro Pro Ser His Thr Ala
    130                 135                 140

Ser Pro Leu Pro Arg Pro Pro Ala Ala Met Thr Arg Ser Tyr Ser Ile
145                 150                 155                 160

Pro Ser Asn Ser Gln Arg Thr Pro Ile Ile Thr Val Asn Lys Leu Leu
                165                 170                 175

Glu Ala Arg His Ser Arg Glu Ser Ser Glu Val Ser Ser Pro Pro Val
            180                 185                 190

Thr Pro Ile Ser Leu Ala Asp Val Ser Arg Arg Pro Thr Thr Glu Ala
        195                 200                 205

Ala Val
    210


<210>  81
<211>  615
<212>  PRT
<213>  Zea mays

<400>  81

Met Lys Ser Pro Leu Arg Arg Phe Arg Gly Phe Ala Leu His His His
1               5                   10                  15

His His His Arg Glu Arg Lys Asp His Arg Pro Pro Ser Ala Lys Leu
            20                  25                  30

Asp Glu Leu Val Tyr Ala Ala Gln Glu Met Glu Asp Met Arg Asn Cys
        35                  40                  45

Tyr Asp Ser Leu Leu Ser Ala Ala Ala Ala Thr Thr Asn Ser Val Tyr
    50                  55                  60

Glu Phe Ala Glu Ala Met Gly Glu Met Gly Thr Cys Leu Leu Glu Lys
65                  70                  75                  80
```

```
Ala Ala Leu Asn Tyr Asp Asp Asp Glu Ser Gly Lys Val Leu Met Lys
                85                  90                  95

Leu Gly Lys Ala Gln Phe Glu Leu Gln Lys Phe Val Asp Asp Tyr Arg
            100                 105                 110

Thr Asn Ile Ile Asn Thr Ile Thr Asn Pro Ser Glu Ser Leu Leu Lys
        115                 120                 125

Glu Leu Gln Val Val Glu Glu Met Lys Asp Gln Cys Asp Gln Lys Arg
    130                 135                 140

Val Glu Tyr Glu Ala Met Arg Ala Ala Tyr Gly Glu Lys Gly Gly Arg
145                 150                 155                 160

Leu Arg His Leu Lys Asn Glu Ser Phe Ser Leu Gly Gln Leu Gln Thr
                165                 170                 175

Ser Phe Leu Glu Tyr Gln Glu Glu Ala Ala Leu Phe Ile Phe Arg Leu
            180                 185                 190

Lys Ser Leu Lys Gln Gly Gln Phe Leu Ser Ile Leu Thr Gln Ala Ala
        195                 200                 205

Arg His His Ala Ser Gln Leu Ser Phe Phe Arg Lys Gly Leu Lys His
    210                 215                 220

Leu Glu Ala Leu Glu Pro Cys Val Lys Ala Val Ala Glu Lys Gln His
225                 230                 235                 240

Ile Asp Tyr His Phe Ser Gly Leu Asp Asn Asn Ser Asp Ile Asp Asp
                245                 250                 255

Tyr Ser Ser Tyr Gln Asp Asn His Ser Asp Gly Ser Glu Leu Ser Phe
            260                 265                 270

Asn Tyr Glu Ile Asn Asp Arg Asp Lys Asp Leu Pro Thr Phe Arg Ser
        275                 280                 285

Pro Met Asp Met Asp Gln Ala His Pro Val Ser Ser Pro Arg Pro Leu
    290                 295                 300

Lys Glu Gln Glu Gln Glu Asn Ala Glu Glu Ile Lys Ala Thr Leu Gly
305                 310                 315                 320

Val Pro His Met Lys Pro Glu Ile Gly Thr Gln Ser Ala Pro Ile Phe
                325                 330                 335
```

```
Ala Glu Asn Val Pro Asp Pro Ser Met Arg Phe Arg Lys Met Asn Leu
            340                 345                 350

Leu Thr Arg Thr Val His Ser Tyr Lys Leu Pro Thr Pro Ala Asp Asp
        355                 360                 365

Lys Asn Pro Ala Ser Val Ile Ala Asn Lys Ser Pro His Ser Asp Gln
    370                 375                 380

Pro Glu Ser Lys Ser His Val Ala Val Asn Leu Trp His Ser Ser Pro
385                 390                 395                 400

Leu Ala Lys Asp Phe Met Pro Asn Ser Met His Ser Gly Pro Val Lys
                405                 410                 415

Met Pro Ser Ser Asn Glu Gly Val Ser Leu Ala Tyr Ser Tyr Ser Thr
            420                 425                 430

Thr Asp Phe Lys Lys Val Lys Arg Glu Ala Phe Ser Gly Pro Ile Pro
        435                 440                 445

Ser Lys Val Gly Leu Ser Thr Pro Leu Ile Ser Ala Thr Asp Arg Lys
    450                 455                 460

Pro Ser Gly Lys Arg Pro Ser Tyr Val Leu Pro Thr Arg Pro Pro Gly
465                 470                 475                 480

Pro Gly Trp Gln Ser Ser Val Pro Pro Lys Ala Thr Pro Arg Val Thr
                485                 490                 495

Ser Leu Pro Thr Thr Thr Pro Arg Ile Ser Glu Leu His Glu Leu Pro
            500                 505                 510

Arg Pro Pro Ala Asn Ala Gly Leu Val Gly Tyr Ser Gly Pro Leu Val
        515                 520                 525

Ser Arg Arg Pro Met Pro Thr Ala Val Ser Thr Thr Arg Val Ser Pro
    530                 535                 540

Pro Ser His Thr Ala Ser Pro Leu Pro Arg Pro Pro Ala Ala Met Thr
545                 550                 555                 560

Arg Ser Tyr Ser Ile Pro Ser Asn Ser Gln Arg Thr Pro Ile Ile Thr
                565                 570                 575

Val Asn Lys Leu Leu Glu Ala Arg His Ser Arg Glu Ser Ser Glu Val
            580                 585                 590

Ser Ser Pro Pro Val Thr Pro Ile Ser Leu Ala Asp Val Ser Arg Arg
        595                 600                 605
```

```
Pro Thr Thr Glu Ala Ala Val
    610                 615


<210>  82
<211>  2005
<212>  PRT
<213>  Glycine max

<400>  82

Met Lys Arg Ser Leu Arg Lys Leu Gly Val Leu Ala Val Glu Lys His
1               5                   10                  15

Glu Arg Asp Arg Arg Asn Ile Met Pro Leu Ser Gln Leu Glu Glu Leu
            20                  25                  30

Ala Gln Ala Thr Gln Glu Met Gln Asp Met Arg Asp Cys His Asp Ser
        35                  40                  45

Leu Leu Ser Ala Ala Ala Val Ala Ala Asn Ser Ala Tyr Glu Phe Ser
    50                  55                  60

Glu Ser Leu Gly Glu Leu Gly Ser Cys Leu Leu Glu Lys Thr Ala Leu
65                  70                  75                  80

His Asp Asp Glu Glu Ser Gly Lys Val Leu Ile Met Leu Gly Lys Met
                85                  90                  95

Gln Phe Gln Leu Gln Lys Leu Ile Asp Asn Tyr Arg Ser His Ile Thr
            100                 105                 110

Gln Thr Ile Thr Ile Pro Ser Asp Ser Leu Leu Asn Glu Leu Arg Ile
        115                 120                 125

Val Glu Asp Met Lys Gln His Cys Asp Glu Lys Arg Glu Val Tyr Glu
    130                 135                 140

Ser Met Leu Thr Arg Tyr Arg Glu Arg Gly Arg Ser Arg Ser Gly Lys
145                 150                 155                 160

Ala Glu Asn Ile Ser Leu Gln His Leu Gln Ile Ala Arg Asp Glu Tyr
                165                 170                 175

Asp Glu Glu Ala Thr Leu Phe Val Phe Arg Leu Lys Ser Leu Lys Gln
            180                 185                 190

Gly Gln Ser Trp Ser Leu Leu Thr Gln Ala Ala Arg His His Ala Ala
        195                 200                 205

Gln Leu Cys Phe Phe Lys Lys Ala Val Lys Ser Leu Glu Thr Val Glu
```

```
    210                 215                 220

Pro His Val Lys Ser Val Thr Glu Gln His His Ile Asp Tyr Gln Phe
225                 230                 235                 240

Ile Gly Ile Glu Gly Glu Asp Glu Asp Glu Asp Glu Asp Glu Asp Asp
                245                 250                 255

Val Asp His Asp Asp Asp Ser His Asp Glu Asn Asp Asp Gly Glu Leu
            260                 265                 270

Ser Phe Asp Tyr Ala Gln Asn Glu Cys Glu Gln Asp Asp Ser Thr Leu
        275                 280                 285

Glu Asn Ser Met Lys Lys Ser Thr Met Asn Glu Thr Ser Ile Asn Asn
    290                 295                 300

Met Glu Ser Tyr Leu Tyr Leu His Pro Ser Glu Asn Pro Ala Thr Ala
305                 310                 315                 320

Leu Val Ser Pro Val Leu Asp Ser Thr Asn Tyr His Ser Trp Ser Arg
                325                 330                 335

Ser Met Val Thr Ala Leu Ser Ala Lys Asn Lys Val Glu Phe Ile Asp
            340                 345                 350

Gly Ser Ala Pro Glu Pro Leu Lys Thr Asp Arg Met His Gly Ala Trp
        355                 360                 365

Cys Arg Cys Asn Asn Met Val Val Ser Trp Ile Val His Ser Val Ala
    370                 375                 380

Thr Ser Ile Arg Gln Ser Ile Leu Trp Met Asp Lys Ala Glu Glu Ile
385                 390                 395                 400

Trp Arg Asp Leu Lys Ser Arg Tyr Ser Gln Gly Asp Leu Leu Arg Ile
                405                 410                 415

Ser Asp Leu Gln Gln Glu Ala Ser Thr Met Lys Gln Gly Thr Leu Thr
            420                 425                 430

Val Thr Glu Tyr Phe Thr Cys Leu Arg Val Ile Trp Asp Glu Ile Glu
        435                 440                 445

Asn Phe Arg Pro Asp Pro Ile Cys Ser Cys Asn Ile Arg Cys Ser Cys
    450                 455                 460

Asn Ala Phe Thr Ile Ile Ala Gln Arg Lys Leu Glu Asp Arg Ala Met
465                 470                 475                 480
```

```
Gln Phe Leu Arg Gly Leu Asn Glu Gln Tyr Ala Asn Ile Arg Ser His
                485                 490                 495

Val Leu Leu Met Asp Pro Ile Pro Thr Ile Ser Lys Ile Phe Ser Tyr
            500                 505                 510

Val Ala Gln Gln Glu Arg Gln Leu Leu Gly Asn Thr Gly Pro Gly Ile
        515                 520                 525

Asn Phe Glu Pro Lys Asp Ile Ser Ile Asn Ala Ala Lys Thr Val Cys
    530                 535                 540

Asp Phe Cys Gly Arg Ile Gly His Val Glu Ser Thr Cys Tyr Lys Lys
545                 550                 555                 560

His Gly Val Pro Ser Asn Tyr Asp Ala Arg Asn Lys Ser Asn Gly Arg
                565                 570                 575

Lys Ala Cys Thr His Cys Gly Lys Ile Gly His Thr Val Asp Val Cys
            580                 585                 590

Tyr Arg Lys His Gly Tyr Pro Pro Gly Tyr Lys Pro Tyr Ser Gly Arg
        595                 600                 605

Thr Thr Val Asn Asn Val Val Ala Val Glu Ser Lys Ala Thr Asp Asp
    610                 615                 620

Gln Ala Gln His His Glu Ser His Glu Phe Val Arg Phe Ser Pro Glu
625                 630                 635                 640

Gln Tyr Lys Ala Leu Leu Ala Leu Ile Gln Glu Pro Ser Ala Gly Asn
                645                 650                 655

Thr Ala Leu Thr Gln Pro Lys Gln Val Ala Ser Ile Ser Ser Cys Thr
            660                 665                 670

Val Asn Asn Pro Thr Asn Pro Gly Met Ser Leu Ser Leu Ser Ala Ser
        675                 680                 685

Leu Thr Ser Trp Ile Leu Asp Ser Gly Ala Thr Asp His Val Thr Cys
    690                 695                 700

Ser Leu His Asn Leu His Ser His Lys Arg Ile Asn Pro Ile Thr Val
705                 710                 715                 720

Lys Leu Pro Asn Gly Gln Tyr Val His Ala Thr His Ser Gly Thr Val
                725                 730                 735

Gln Leu Ser Ser Asn Ile Thr Leu His Asp Val Leu Tyr Ile Pro Ser
```

740 745 750

Phe Thr Phe Asn Leu Ile Ser Ile Ser Lys Leu Val Ser Ser Ile Asn
755 760 765

Cys Glu Leu Ile Phe Ser Ser Thr Ser Cys Val Leu Gln Glu Met Asn
770 775 780

Asn His Met Lys Ile Gly Ile Val Glu Ala Lys His Gly Leu Tyr His
785 790 795 800

Leu Ile Pro Asn Gln Leu Thr Thr Lys Ala Val Asn Ser Thr Ile Thr
805 810 815

His Pro Arg Cys Asn Val Ile Pro Ile Asp Leu Trp His Phe Arg Leu
820 825 830

Gly His Pro Ser Ala Glu Arg Ile Gln Cys Met Lys Thr Tyr Tyr Pro
835 840 845

Leu Leu Arg Asn Asn Lys Asn Phe Val Cys Asn Thr Cys His Tyr Ala
850 855 860

Lys His Lys Lys Met Pro Phe Ser Leu Ser Asn Ser His Ala Ser His
865 870 875 880

Ala Phe Asp Leu Leu His Met Asp Ile Arg Gly Pro Cys Ser Lys Pro
885 890 895

Ser Met His Gly His Lys Tyr Phe Leu Thr Ile Val Asp Asp Cys Ser
900 905 910

Arg Phe Thr Trp Val His Leu Met Lys Ser Lys Ala Glu Thr Arg Gln
915 920 925

Val Ile Met Asn Phe Ile Thr Phe Ile Glu Thr Gln Tyr Asn Gly Lys
930 935 940

Val Lys Ile Ile Arg Ser Asp Asn Gly Ile Glu Phe Phe Met His His
945 950 955 960

Tyr Tyr Ala Ser Lys Gly Ile Ile His Gln Thr Thr Cys Val Glu Thr
965 970 975

Pro Glu Gln Asn Gly Ile Val Glu Arg Lys His Gln His Leu Leu Asn
980 985 990

Ile Thr Arg Ala Leu Leu Phe Gln Ala Ser Leu Pro Pro Ser Phe Trp
995 1000 1005

Cys Tyr Ala Leu Pro His Ala Thr Tyr Leu Ile Asn Cys Ile Pro
1010 1015 1020

Thr Pro Tyr Leu His Asn Ile Ser Pro Tyr Glu Lys Leu His Lys
1025 1030 1035

His Pro Cys Asp Ile Ser Asn Leu Arg Val Phe Gly Gly Leu Cys
1040 1045 1050

Tyr Ile Asn Thr Leu Lys Ala Asn Arg Gln Lys Leu Asp Ala Arg
1055 1060 1065

Ala His Pro Cys Ile Phe Ile Gly Phe Lys Thr His Thr Lys Gly
1070 1075 1080

Tyr Leu Val Tyr Asp Leu His Ser Asn Asp Val Thr Val Ser Arg
1085 1090 1095

Asn Val Thr Phe Tyr Glu Asp His Phe Pro Tyr Tyr Ser Glu Thr
1100 1105 1110

Gln His Ile Asn Ser Glu His Ser Ala Pro Ser Pro Gly Pro Phe
1115 1120 1125

Ser Gly Lys Asn Leu Asp Pro Gln Ile Glu Asn Cys Ser Ser Gln
1130 1135 1140

Pro Thr Ile Ser Val Pro Ser Ser Asn Glu Pro Ser Asn Glu Gln
1145 1150 1155

Pro Leu Pro His Leu Arg Arg Ser Thr Arg Ala Lys Asn Thr Pro
1160 1165 1170

Thr Tyr Leu Gln Asp Tyr His Arg Asp Leu Ala Ser Ser Thr Pro
1175 1180 1185

Asn Thr Ser Ala Ile Val Arg Tyr Pro Leu Ser Ser Val Leu Ser
1190 1195 1200

Tyr Ser Arg Leu Ser Pro Ala His Arg Asn Phe Val Met Ser Ile
1205 1210 1215

Ser Leu Thr Ala Glu Pro Thr Ser Tyr Thr Glu Ala Ser Arg His
1220 1225 1230

Asp Cys Trp Ile Lys Ala Met Lys Val Glu Leu Gln Ala Leu Gln
1235 1240 1245

Ser Asn Asn Thr Trp Arg Leu Thr Pro Leu Pro Pro His Lys Thr

```
        1250                1255                1260

Ala Ile  Gly Cys Arg Trp Ile  Tyr Lys Ile Lys Tyr  Arg Thr Asp
    1265                1270                1275

Gly Ser  Ile Glu Arg His Lys  Ala Arg Leu Val Ala  Lys Gly Tyr
    1280                1285                1290

Thr Gln  Met Glu Gly Leu Asp  Tyr Leu Asp Thr Phe  Ser Pro Val
    1295                1300                1305

Ala Lys  Leu Thr Thr Val Arg  Leu Leu Leu Ala Ile  Ala Ala Leu
    1310                1315                1320

Asn Gln  Trp His Leu Arg Gln  Leu Asp Val Asn Asn  Ala Phe Leu
    1325                1330                1335

His Gly  Glu Leu Asp Glu Glu  Val Tyr Met Gln Ile  Pro Pro Gly
    1340                1345                1350

Leu Ser  Val Asp Asn Pro Gln  Leu Val Cys His Leu  Gln Arg Phe
    1355                1360                1365

Leu Ser  Ser His Gly Phe Gln  Gln Ser Asn Ala Asp  His Ser Leu
    1370                1375                1380

Phe Leu  Arg Phe Thr Gly Val  Ile Thr Thr Ile Leu  Leu Val Tyr
    1385                1390                1395

Val Asp  Asp Ile Ile Leu Thr  Gly Asn Asn Ile Ala  Glu Ile Gln
    1400                1405                1410

Thr Met  Ile Thr Leu Leu Asp  Arg Glu Phe Arg Ile  Lys Asp Leu
    1415                1420                1425

Gly Asp  Leu Lys Phe Phe Leu  Gly Leu Glu Ile Ala  Arg Thr Ser
    1430                1435                1440

Lys Gly  Ile His Leu Cys Gln  Arg Lys Tyr Thr Leu  Asp Ile Leu
    1445                1450                1455

Ser Asp  Ser Gly Met Leu Gly  Cys Lys Pro Asn Ser  Thr Pro Met
    1460                1465                1470

Asp Tyr  Ser Thr Lys Leu Gln  Ala Asp Ser Gly Ser  Leu Leu Ser
    1475                1480                1485

Ala Glu  Ser Ser Ser Ser Tyr  Arg Arg Leu Ile Gly  Lys Leu Ile
    1490                1495                1500
```

```
Tyr Leu  Thr Asn Thr Arg Pro  Asp Ile Thr Tyr Ala  Val Gln Gln
    1505                 1510                 1515

Leu Ser  Gln Tyr Met Ala Thr  Pro Thr Asn Val His  Leu Gln Ala
    1520                 1525                 1530

Ala Phe  Arg Ile Leu Arg Tyr  Leu Lys Gly Thr Pro  Gly Ser Gly
    1535                 1540                 1545

Leu Phe  Phe Ala Ala Thr Gly  Thr Pro Gln Leu Arg  Ala Phe Ser
    1550                 1555                 1560

Asp Ser  Asp Trp Ala Gly Cys  Lys Asp Ser Arg Lys  Ser Thr Pro
    1565                 1570                 1575

Gly Tyr  Leu Val Tyr Leu Gly  Ser Ser Leu Val Ser  Trp Gln Ser
    1580                 1585                 1590

Lys Lys  Gln Ser Thr Val Ser  Arg Ser Ser Ser Glu  Ala Glu Tyr
    1595                 1600                 1605

Arg Ala  Leu Ala Ser Thr Thr  Cys Glu Leu Gln Trp  Leu Thr Phe
    1610                 1615                 1620

Leu Leu  Gln Asp Phe Arg Ala  Thr Phe Ile Gln Pro  Ala Thr Leu
    1625                 1630                 1635

Tyr Cys  Asp Asn Gln Ser Thr  Ile Gln Ile Ala Thr  Asn Pro Val
    1640                 1645                 1650

Phe His  Glu Arg Thr Lys His  Ile Glu Ile Asp Cys  His Ile Val
    1655                 1660                 1665

Arg Gln  Lys Leu Asn Ser Ala  Leu Ile Lys Leu Leu  Pro Ser Asn
    1670                 1675                 1680

Arg Lys  Lys Ser His Phe Val  Val Val Arg Ser Ala  Ser Gln Ser
    1685                 1690                 1695

Ala Pro  Leu Phe Val Asp Asn  Lys Arg Asp Ser Ser  Glu Lys Leu
    1700                 1705                 1710

Arg Gln  Met Arg Pro Thr Leu  Ser Arg Lys Phe Asn  Ser Tyr Val
    1715                 1720                 1725

Leu Pro  Thr Pro Val Asp Ala  Lys Ser Ser Ile Ser  Met Arg Ser
    1730                 1735                 1740

Ser Asn  Gln Val Pro Leu Lys  Ile Lys Thr Asn Leu  Asn Glu Pro
```

```
        1745                1750                1755

Met Lys  Asn Leu Trp His Ser  Ser Pro Leu Glu Gln  Lys Lys Tyr
    1760                1765                1770

Glu Asn  Ile Phe Gly Asp Gly  Gly Leu Ser Gly Pro  Asn Ala Gln
    1775                1780                1785

Ser Val  Leu Lys Glu Ser Asn  Ser Asn Thr Ala Tyr  Ser Arg Leu
    1790                1795                1800

Pro Pro  Pro Leu Ile Asp Gly  Asn Leu Ser Ser Ser  His Asp Tyr
    1805                1810                1815

Ile Thr  Ala Tyr Ser Lys Lys  Ile Lys Arg His Ala  Phe Ser Gly
    1820                1825                1830

Pro Leu  Val Ser Asn Ala Trp  Pro Thr Lys Pro Val  Ser Leu Glu
    1835                1840                1845

Ser Val  Gln Leu Phe Ser Gly  Pro Leu Leu Arg Thr  Ser Ile Pro
    1850                1855                1860

Gln Pro  Pro Ser Ser Ser Pro  Lys Val Ser Pro Ser  Ala Ser Pro
    1865                1870                1875

Pro Leu  Ser Ser Ser Pro Lys  Ile Asn Glu Leu His  Glu Leu Pro
    1880                1885                1890

Arg Pro  Pro Thr Ile Ser Pro  Ser Asp Ser Lys Leu  Leu Gly Leu
    1895                1900                1905

Val Gly  His Ser Gly Pro Leu  Val Ser Arg Gly Pro  Gln Leu Ser
    1910                1915                1920

Ala Ala  Asn Tyr Leu Val Thr  Ser Asn Ala Ala Ser  Pro Leu Pro
    1925                1930                1935

Met Pro  Ala Ser Ala Met Ala  Arg Ser Phe Ser Thr  Ser Tyr Arg
    1940                1945                1950

Gly Ala  Lys Val Ala Ala Ile  His Asp Ser Arg Pro  Leu Glu Asp
    1955                1960                1965

Pro Asn  Lys Ser Thr Ile Ser  Glu Asp Ile Asp Ser  Pro Pro Leu
    1970                1975                1980

Met Gln  Ile Ala Leu Ser Thr  Ser Gln Pro Ser Ser  Asp Gly Ser
    1985                1990                1995
```

```
Asp Thr  Val Ala Gln Ala Val
    2000                2005


<210>  83
<211>  209
<212>  PRT
<213>  M.truncatula

<400>  83

Met Arg Pro Ser Lys Leu His Gln Ile Ala Ala Leu Thr Ser Lys Val
1               5                   10                  15

Asp Lys Asn Ile Lys Lys Asn Gly Lys Phe Gly Pro Ile Arg Val Pro
            20                  25                  30

Arg Gly Val Asn Asn Gln Ile Phe Glu Asp Ser Asn Ser Leu Lys Val
        35                  40                  45

Lys Glu Lys Asp Asn Met Lys His His Cys Asp Glu Lys Arg Glu Val
    50                  55                  60

Tyr Glu Tyr Met Ile Ile Gln Pro Lys Glu Lys Gly Lys Ser Asn Ser
65                  70                  75                  80

Gly Lys Gly Glu His Ile Thr Ser Arg Pro Leu Gln Ala Ala His Asp
                85                  90                  95

Glu Tyr Glu Glu Glu Ala Thr Leu Ala Lys Gln Gly Cys Pro His Ile
            100                 105                 110

Ala Lys Leu Gly Lys Gly Arg Arg Ile Lys Pro Leu Lys His Ser Asn
        115                 120                 125

Phe Asn Glu Arg Asn Cys Arg Asp Lys Phe Arg Gln Asp Pro Cys Ser
    130                 135                 140

Glu Ala Met Ser Pro Leu Asp Val Tyr Ser Asp Ser Val Tyr Asp Val
145                 150                 155                 160

Asp Thr Asn Ser Asp Thr Glu Asp Ile Glu Val Asn Asn Asp Ile Asn
                165                 170                 175

Val Arg Ser Asp Arg Asp Val Gly Ser Met Glu Phe Asn His Gly Asn
            180                 185                 190

Gly Val Cys Ser Glu Gln Gly Lys Arg Glu Phe Gln Trp Leu Gln Met
        195                 200                 205

His
```

<210> 84
<211> 993
<212> PRT
<213> P. patents

<400> 84

```
Met Ser Val Glu Phe Glu Glu Asp Gly Val Asp Arg Ala Leu Gly Cys
1               5                   10                  15

Ser Leu Val His Leu Gly Leu Arg Trp Trp Met Cys Asn Gly Thr Ala
            20                  25                  30

Leu Glu Asn Ala Gly Phe Thr Ala Val His Val Ser Gln Arg Ile Gly
        35                  40                  45

Arg Gly Gly Gln Ser Ser Gly Ser Ala Met Leu Gly Arg Asp Trp Gly
    50                  55                  60

Gly Met Leu Asn Gly Gly Ser Val Val Leu Leu Asp Leu Arg Ser Thr
65                  70                  75                  80

Ser Arg Leu Val Thr Gly Leu Ala Asn Leu Asp Ala Ile Val Thr Cys
                85                  90                  95

Gly Phe Cys Asp Pro His Gln Arg Trp Ile Leu Ala Arg Ile Val Gln
            100                 105                 110

Arg Thr Ala Phe Ile Ile Ala Gly Leu Glu Ser Cys Phe Leu Pro Gly
        115                 120                 125

Leu Thr Ser Gln Cys Leu Gly Ala Thr Ser Lys Glu Ser Phe Ser Ser
    130                 135                 140

Arg Leu His His Ser Ser Trp Phe Ser Ile Phe Phe Ser Asp Gly Thr
145                 150                 155                 160

Ser Cys Arg Ala Ala Thr Leu Phe Leu Thr Thr Leu Ile Ile Val Thr
                165                 170                 175

Glu Phe Phe Leu Pro Ser Ala Ile Pro Glu Arg Ala Leu Trp Arg Asn
            180                 185                 190

Arg Glu Lys Met Lys Ser Phe Lys Lys Leu Arg Asp Leu Ala Arg Gln
        195                 200                 205

Gly Gly Lys Ala Lys Asp Lys His Asn Tyr Gly Asn Asp Asn Asn Arg
    210                 215                 220

Arg Tyr Glu Ala His Gly Val Gly Tyr Val Glu Pro Thr Glu Asp Ala
```

```
225                 230                 235                 240

Glu Leu Val Thr Asn Gly Met Lys Asp Ile Arg Ser Ile Gln Lys Lys
                245                 250                 255

Tyr Glu Ser Leu Val Ser Leu Ser Thr Glu Val Ser His Arg Ala Tyr
            260                 265                 270

Asp Leu Ser Thr Ala Val Ser Asp Met Ala Ser Tyr Phe Val Ala Pro
        275                 280                 285

Gly Val Leu Asp Asp Gln Asp Ile Val Cys Val Leu Tyr Ile Phe Phe
    290                 295                 300

Gly Asn Cys His Asp Lys Tyr Trp Phe Ala Ile Val Asp Leu Val Phe
305                 310                 315                 320

Lys Glu Thr Lys Lys Gln Tyr Asp Glu Arg Arg Gln Ser Leu Tyr His
                325                 330                 335

His Arg Leu Arg Ile Ala Lys Gly Arg Ser Lys Ile Gly Lys Thr Asp
            340                 345                 350

Ala Gln Glu Glu Glu Gln Leu Glu Asn Val Arg Glu Gln Phe Glu Glu
        355                 360                 365

Val Ser Gln Phe Leu Gly Asp Arg Leu Leu Ser Leu Arg Gln Gly Arg
    370                 375                 380

Pro Arg Ser Leu Ile Thr Gln Thr Ala Arg His His Ala Ala Gln Met
385                 390                 395                 400

Gln Leu Phe Ser Lys Val Leu Thr Ser Leu His Gly Ile Glu Pro His
                405                 410                 415

Met Lys Gln Val Thr Lys Glu Leu Asn Ile Asp Arg His Leu Ser Ser
            420                 425                 430

Ala Asp Gly Asp Val Phe Asn Asp Glu Ile Glu Asp Asp Glu Asp Val
        435                 440                 445

Ser Asp Ile Glu His His Leu Asp Asp Ala Gly Ser Tyr Phe Asp Asp
    450                 455                 460

Ala Asp Glu Glu Lys Glu His His His Val Arg Glu Asp Asp Leu Gly
465                 470                 475                 480

Ser Glu Ser Ser Glu Ile Glu Ala Asn Ser Arg Leu Asn Met Ser Arg
                485                 490                 495
```

```
Glu Trp Ser Ser His Met Ala Ser Gly Ser Asp Arg Pro Gly Asn Ser
          500                 505                 510

Glu Asn Glu Gly Asp Ser Pro Leu Pro Arg Trp Ile Ile Lys Gly Ser
      515                 520                 525

Lys Ser Ala Pro Ser Ser Pro Leu Thr Ala Ser Gln Gly Val Asp Asp
    530                 535                 540

Val Asp Arg Ala Pro Pro Thr Tyr Val Ser Ala Val Leu Pro Pro Thr
545                 550                 555                 560

Arg Ser Pro Pro His Val Ser Asp Arg Gly His Ser Gly Val Leu Tyr
                565                 570                 575

Ser Asp Thr Arg Pro Tyr Glu His Lys Pro Ser Thr Ser Glu Gln Thr
            580                 585                 590

Ser Pro Arg His His Asn Gly Thr Pro Gly Gln Thr Pro Arg Phe Gln
        595                 600                 605

Gly Arg Ala Thr Lys Met Gly Val Pro Met Ser Met Phe Pro Pro Phe
    610                 615                 620

Asp Pro Ser Gln Thr Thr Trp Pro Met Pro Leu Pro Pro Pro Ser Asp
625                 630                 635                 640

Pro Pro Pro Ile Leu Lys Phe Pro Ala Pro Thr Ala Gly Gly Phe Ile
                645                 650                 655

Ala Ala Asn Ser Gly Ala Pro Gly Asn Lys His Lys Arg Tyr Ser Tyr
            660                 665                 670

Ser Gly Pro Leu Thr Ser Ser Ser Lys Pro Arg Asp Arg Ala Tyr Ser
        675                 680                 685

Ser Asp Val Pro Ser Ser Gly Pro Leu Arg Pro Phe Pro Ser Ser Thr
    690                 695                 700

Thr Trp Ser Asp Gln Ser Arg Ser Pro Lys Leu Ser Pro Ser Ile Ser
705                 710                 715                 720

Pro Ser Lys Val Ser Pro Pro Gln Ile Ser Glu Leu His Lys Leu Pro
                725                 730                 735

Pro Pro Pro Leu Gly Ser Ser Ser Ser Pro Ile Ala Ala Ser Ser Ser
            740                 745                 750

Leu Ile Ala His Ser Ala Pro Leu His Arg His Ala Asp Arg His Ala
```

755 760 765

Ser Pro Leu Pro Pro Pro Pro Leu Gly Asn Ala Ser Gln Asn Leu Ser
770 775 780

Val Gly Gly Ala Gly Lys Leu Gln Asn Ile Gln Arg Tyr Lys Gly Leu
785 790 795 800

Ala Ile Ser Glu Asp Glu Glu Arg Ala Ile Pro Ser Val Gln Pro Val
805 810 815

Arg Thr Ser His Ser Gly His Ser Gly Leu Leu Thr Thr Pro Ser Asn
820 825 830

Pro Gln Ile Gly His Lys Ser Pro Ser Leu Gln Arg Ser Val Ser Gly
835 840 845

Cys Gly Val Ser Ala Ala Pro Arg His Ala Thr Leu His Leu Thr Ala
850 855 860

Pro Gly Arg Val Gln Ser Gly Ser Gly Asp Lys Lys Met Trp Arg Leu
865 870 875 880

Asn Thr Thr Ile His Ser Ser Pro Ser Leu Ser Leu Lys Tyr Ser Val
885 890 895

Glu Ser Pro Pro Ser Ile Asp Ser Phe Glu Gly Lys Glu Arg Pro Arg
900 905 910

Phe Ala Ser Ser Asp Phe Glu Thr Ala Arg Arg Ser Leu Asp Met Ala
915 920 925

Leu Thr Ser Gly Thr Ser Asn Asn Thr Lys Gln Ser Lys Trp Ser Lys
930 935 940

Trp Arg Ser Phe Ser Ala His Asp Gly Glu Asp Leu Ser Met Met Ser
945 950 955 960

Gln Leu Gly Thr Lys His His His Arg Asp Ser Leu Gln Ala His Gly
965 970 975

Tyr His Ala Ser Pro Arg Leu Gly Thr Thr Thr Tyr Gly Val Arg His
980 985 990

Val

<210> 85
<211> 608
<212> PRT

<213> P.trichocarpa

<400> 85

```
Met Lys Ser Lys Leu Arg Gly Phe Arg Leu Arg Arg Ser Glu Pro Lys
1               5                   10                  15

Asp Lys Ile Asp Phe Leu Pro Pro Ala Gln Leu Asp Glu Leu Ala Gln
            20                  25                  30

Ala Ala Gln Asn Ser Gln Ser His Cys Gly Lys Trp Val His Val Cys
        35                  40                  45

Arg Arg Lys Gln His Tyr Met Met Met Lys Glu Val Arg Ser His Ile
    50                  55                  60

Phe Leu Thr Ile Thr Asn Pro Ser Glu Ser Leu Leu Asn Glu Leu Arg
65                  70                  75                  80

Thr Val Glu Asp Met Lys Arg Gln Cys Asp Glu Lys Arg Asn Val Tyr
                85                  90                  95

Glu Tyr Met Val Ala Gln Gln Lys Asp Lys Gly Arg Ser Lys Gly Gly
            100                 105                 110

Lys Asp Glu Ser Ile Thr Leu Gln Gln Leu Gln Thr Ala Arg Glu Glu
        115                 120                 125

Tyr Asp Glu Glu Ala Thr Leu Cys Val Phe Arg Leu Lys Ser Leu Lys
    130                 135                 140

Gln Gly Gln Ser Arg Ser Leu Leu Thr Gln Ala Ala Arg His His Ala
145                 150                 155                 160

Ala Gln Lys Gly Leu Lys Ser Leu Glu Thr Val Glu Pro His Val Arg
                165                 170                 175

Leu Val Thr Glu His Gln His Ile Asp Tyr His Phe Ser Gly Leu Glu
            180                 185                 190

Ser Asp Gly Arg Glu Asp Gly Glu Asp Asp Gly Glu Asp Gly Gly Asp
        195                 200                 205

Thr Asn Glu Arg Arg Glu Leu Ser Phe Asp Tyr Arg Glu Asn Asn Gln
    210                 215                 220

Gly Asn Ala Val Val Ser Ala Ala Arg Gly Ser Met Glu Val Asp Glu
225                 230                 235                 240

Glu Asp Leu Ser Phe Gln Val Pro Ala Ala Glu Asn Val Glu Leu Asn
                245                 250                 255
```

```
Pro Asp Lys Asn His Gly Gly Phe Gln Phe Pro Ser Arg Glu Pro Arg
            260                 265                 270

Gly Gly Ser His Ser Ala Pro Ile Val Pro Glu Arg Lys Pro Asp Pro
        275                 280                 285

Val Glu Arg Ile Arg Gln Met Gln Gln Ala Ser Arg Lys Ser Asn Thr
    290                 295                 300

Tyr Val Leu Pro Thr Pro Ile Asp Ala Lys Gly Ala Ile Ser Ser Arg
305                 310                 315                 320

Thr Ser Cys Ser Val Pro Asn Thr Arg Gln Thr Asp Ile Ser Gly Arg
                325                 330                 335

Ala His Asn Leu Trp His Ser Ser Pro Leu Glu Gln Lys Lys Asn Glu
            340                 345                 350

Lys Asp Ser Gly Asp Gly His Leu Ser Asp Phe Thr Ala Leu Lys Ala
        355                 360                 365

Arg Ser Gly His Lys Glu Ser Asn Asn Pro Asn Ala Ser Thr Gln Leu
    370                 375                 380

Pro Pro Pro Leu Val Gly Gly Ile Ser Tyr Pro Gln Leu Asp Val His
385                 390                 395                 400

Asn Ala Ser Asp Tyr Lys Lys Asn Lys Trp Gln Ser Phe Ser Gly Pro
                405                 410                 415

Ile Thr Ser Lys Pro Trp Ser Met Lys Pro Leu Ser Ser Ser Gly Pro
            420                 425                 430

Ile Ser Ser Thr Glu Leu Ser Gln Gln Val Ser Gly Met Leu Ser Arg
        435                 440                 445

Gly Ala Asn Pro Gln Pro Ser Ser Ser Pro Lys Val Ser Pro Ser Thr
    450                 455                 460

Ser Pro Pro Leu Val Ser Ser Pro Lys Ile Ser Glu Leu His Glu Leu
465                 470                 475                 480

Pro Arg Pro Pro Gly Asn Leu Ala Ala Lys Ala Ala Lys Pro Ser Val
                485                 490                 495

Leu Ile Gly His Ser Ala Pro Leu Ser Arg Asn Pro Glu Leu Ala Gly
            500                 505                 510
```

Thr Ser Lys Ile Ser Thr Gly Ala Ala Asn Leu Ala Ser Pro Leu Pro
515 520 525

Pro Pro Pro Leu Ile Val Pro Arg Ser Phe Ser Ile Pro Ser Ser Ser
530 535 540

Gln Arg Ala Met Thr Val His Val Ser Lys Leu Leu Asp Ser Ser Gln
545 550 555 560

Val Ser Tyr Lys Pro Gly Glu Val Asp Ser Pro Pro Leu Thr Pro Met
565 570 575

Ser Leu Ala Asn Met Arg Pro Ala Pro Ala Ile Ser Glu Pro Val Pro
580 585 590

His Ser Gly Gln Ile Arg Gly Asn Lys Thr Ser Cys Ser Leu Ile Phe
595 600 605

<210> 86
<211> 574
<212> PRT
<213> P.trichocarpa

<400> 86

Met Gly Ala Cys Leu Leu Ala Lys Thr Ala Leu Asn Asp Asp Glu Glu
1 5 10 15

Ser Gly Arg Val Leu Leu Met Leu Gly Lys Val Gln Phe Glu Leu Gln
20 25 30

Lys Leu Val Asp Cys Tyr Arg Ser His Ile Asn Gln Thr Ile Ile Ser
35 40 45

Pro Ser Glu Ser Leu Leu Asn Glu Leu Gln Ile Val Glu Glu Met Lys
50 55 60

Gln Gln Cys Asp Glu Lys Arg Asp Val Tyr Ala His Met Ala Arg Gln
65 70 75 80

Arg Glu Arg Val Ser Gly Arg Asn Gly Lys Gly Glu Cys Phe Ser Met
85 90 95

Gln Gln Ile Gln Ala Ala His Asp Glu Tyr Asp Glu Glu Ala Thr Leu
100 105 110

Phe Val Phe Arg Leu Lys Ser Leu Lys Glu Gly Gln Ser Arg Ser Leu
115 120 125

Leu Thr Gln Ala Ala Arg His His Ala Ala Gln Leu Cys Phe Phe Lys
130 135 140

Lys Ala Leu Gln Ser Leu Glu Ala Val Glu Pro His Val Lys Leu Val
145 150 155 160

Ser Glu Gln Gln His Ile Asp Tyr His Phe Ser Gly Leu Asp Asp Asp
165 170 175

Gly Arg Asp Tyr Asp Asp Asp Glu Asp Tyr Asp Asp Ala Ile Asp Asp
180 185 190

Gly Glu Leu Ser Phe Asp Tyr Gly Gln Asn Asp Gln Glu Gln Glu Val
195 200 205

Ser Lys Ser Ile Lys Ser Met Glu Leu Asp Leu Glu Asp Ile Thr Phe
210 215 220

Pro Gln Val Val Thr Leu Glu Met Ala Lys Glu Asn Pro Asp Thr Ser
225 230 235 240

Tyr Arg Thr Ser Phe Pro Ile Lys Gly Glu Leu Ser Ala Gly Thr Gln
245 250 255

Ser Ala Pro Leu Phe Ala Gln Val Lys Ser Asn Pro Ala Gly Lys Thr
260 265 270

Lys Gln Leu Met Pro Ser Ser Thr Arg Lys Phe Asn Thr Tyr Val Leu
275 280 285

Pro Thr Pro Ala Asp Pro Lys Ser Ser Asn Ser Thr Gly Pro Gly Ser
290 295 300

Pro Val Ser Gln Thr Leu Lys Thr Ser Leu Ser Gly Arg Pro Leu Asn
305 310 315 320

Leu Trp His Ser Ser Pro Ile Gly His Lys Lys Asn Asp Lys Leu Leu
325 330 335

Gly Val Glu Met Ser Ser Lys Pro Pro Ala Ile Asn Ser Gln Ser Val
340 345 350

Leu Lys Glu Ser Asn Asn Asn Thr Ala Ser Thr Arg Leu Pro Pro Pro
355 360 365

Leu Ala Asp Gly Leu Phe Phe Ser Arg Leu Glu Pro Pro Ala Gly Ala
370 375 380

Glu Ser Lys Lys Ile Lys Arg Tyr Ala Phe Ser Gly Pro Ile Ser Ser
385 390 395 400

Gln Pro Trp Ser Thr Lys Ala Val Ser Ala Glu His Pro Gln Leu Phe

```
                405                 410                 415

Ser Gly Pro Leu Leu Arg Asn Pro Thr Ala Gln Leu Phe Ser Ser Pro
            420                 425                 430

Pro Lys Val Ser Pro Arg Ile Ser Pro Lys Val Ser Pro Ser Ser Ser
        435                 440                 445

Pro Pro Phe Val Ser Ser Pro Lys Ile Ser Glu Leu His Glu Leu Pro
    450                 455                 460

Arg Pro Pro Val Ser Ser Thr Ser Lys Ser Pro Gly Ala Gly Gly Leu
465                 470                 475                 480

Val Gly His Ser Ala Pro Leu Leu Pro Lys Gly His Met Leu Pro Gly
                485                 490                 495

Thr Ser Lys Thr Ser Ala Ser Asn Val Glu Ser Gln Leu Pro Thr Pro
            500                 505                 510

Ser Gln Val Val Pro Arg Ser Phe Ser Ile Pro Ser Ser Arg His Arg
        515                 520                 525

Val Met Val Ala Gln Asn Ser Gly Ile Val Glu Asn Val Ala Ser Pro
    530                 535                 540

Pro Leu Thr Pro Ile Ser Leu Ser Asn Thr Gln Pro Ser Ser Thr Gly
545                 550                 555                 560

Ser Arg Ile Val Asn Gln Thr Val Gln Ile Arg Gly Asn Ser
                565                 570


<210>  87
<211>  183
<212>  PRT
<213>  P.trichocarpa

<400>  87

Met Lys Ser Lys Leu Arg Gly Phe Arg Leu Arg Arg Ser Glu Pro Lys
1               5                   10                  15

Asp Lys Ile Asp Phe Leu Pro Pro Ala Gln Leu Asp Glu Leu Ala Gln
            20                  25                  30

Ala Ala Gln Asp Met Lys Asp Met Lys Asn Cys Tyr Asp Ser Leu Leu
        35                  40                  45

Ser Ala Ala Ala Ala Thr Ala Asn Ser Ala Tyr Glu Phe Ser Glu Ser
    50                  55                  60
```

```
Leu Arg Glu Met Gly Ser Cys Leu Ser Glu Lys Thr Ala Leu His Asp
65                  70                  75                  80

Asp Glu Gly Ser Gly Arg Val Leu Leu Met Leu Gly Lys Val Gln Phe
                85                  90                  95

Glu Leu Gln Lys Leu Leu Asp Ser Tyr Arg Ser His Ile Phe Leu Thr
            100                 105                 110

Ile Thr Asn Pro Ser Glu Ser Leu Leu Asn Glu Leu Arg Thr Val Glu
        115                 120                 125

Asp Met Lys Arg Gln Cys Asp Glu Lys Arg Asn Val Tyr Glu Tyr Met
    130                 135                 140

Val Ala Gln Gln Lys Asp Lys Gly Arg Ser Lys Gly Gly Lys Asp Glu
145                 150                 155                 160

Ser Ile Thr Leu Gln Gln Leu Gln Thr Ala Arg Glu Glu Tyr Asp Glu
                165                 170                 175

Glu Ala Ala Ile Gln His Gln
            180


<210>  88
<211>  176
<212>  PRT
<213>  B. napus

<400>  88

Met Lys Ala Ser Met Gly Met Leu Arg Arg Leu Thr Ser His Lys Val
1               5                   10                  15

Asp Ala Lys Glu Lys Gly Glu Leu Val Ala Thr Ala Gln Ile Glu Glu
            20                  25                  30

Leu Asp Arg Ala Gly Lys Asp Met Gln Asp Met Arg Glu Cys Tyr Asp
        35                  40                  45

Arg Leu Leu Ala Ala Ala Ala Ala Thr Ala Asn Ser Ala Tyr Glu Phe
    50                  55                  60

Ser Glu Ser Leu Gly Glu Met Gly Ser Cys Leu Glu Gln Ile Ala Pro
65                  70                  75                  80

His Asn Asp Glu Glu Ser Ser Arg Ile Leu Phe Met Cys Gly Lys Val
                85                  90                  95

Gln Phe Glu Ile Gln Lys Leu Leu Asp Thr Tyr Arg Ser His Ile Phe
            100                 105                 110
```

```
Lys Thr Ile Thr Ser Pro Ser Glu Ala Leu Leu Lys Asp Leu Arg Thr
        115                 120                 125

Val Glu Asp Met Lys Gln Gln Cys Asp Glu Lys Arg Asn Val Phe Glu
    130                 135                 140

Met Ser Leu Val Lys Asp Lys Gly Arg Ser Lys Gly Ser Lys Gly Glu
145                 150                 155                 160

Arg His Ile Pro His Gly Leu Arg Arg Pro Ala Tyr Asn Glu Phe Pro
                165                 170                 175


<210>  89
<211>  2194
<212>  DNA
<213>  O.sativa


<220>
<221>  promoter
<222>  (1)..(2194)

<400>  89
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt     420

ttgtgcattc gtcatatcgc acatcattaa ggacatgtct tactccatcc caatttttat     480

ttagtaatta aagacaattg acttattttt attatttatc ttttttcgat tagatgcaag     540

gtacttacgc acacactttg tgctcatgtg catgtgtgag tgcacctcct caatacacgt     600

tcaactagca acacatctct aatatcactc gcctatttaa tacatttagg tagcaatatc     660

tgaattcaag cactccacca tcaccagacc acttttaata atatctaaaa tacaaaaaat     720

aattttacag aatagcatga aaagtatgaa acgaactatt taggtttttc acatacaaaa     780

aaaaaaagaa ttttgctcgt gcgcgagcgc caatctccca tattgggcac acaggcaaca     840

acagagtggc tgcccacaga acaacccaca aaaaacgatg atctaacgga ggacagcaag     900

tccgcaacaa ccttttaaca gcaggctttg cggccaggag agaggaggag aggcaaagaa     960

aaccaagcat cctccttctc ccatctataa attcctcccc ccttttcccc tctctatata    1020

ggaggcatcc aagccaagaa gagggagagc accaaggaca cgcgactagc agaagccgag    1080

cgaccgcctt ctcgatccat atcttccggt cgagttcttg gtcgatctct tccctcctcc    1140
```

```
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                               2194
```